(19) Europäisches Patentamt
European Patent Office
Office européen des brevets

(11) EP 4 455 136 A1

(12) **EUROPEAN PATENT APPLICATION**
published in accordance with Art. 153(4) EPC

(43) Date of publication:
**30.10.2024 Bulletin 2024/44**

(21) Application number: **22910086.2**

(22) Date of filing: **21.12.2022**

(51) International Patent Classification (IPC):
***C07D 403/04*** *(2006.01)* ***C07D 205/02*** *(2006.01)*
***A61K 31/397*** *(2006.01)* ***A61P 35/00*** *(2006.01)*

(52) Cooperative Patent Classification (CPC):
**A61K 31/397; A61P 35/00; C07D 205/02; C07D 403/04**

(86) International application number:
**PCT/CN2022/140731**

(87) International publication number:
**WO 2023/116774 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2021 CN 202111569042**
**01.04.2022 CN 202210347097**
**14.12.2022 CN 202211606701**

(71) Applicant: **Scinnohub Pharmaceutical Co., Ltd.**
**Chengdu, Sichuan 610094 (CN)**

(72) Inventors:
- **LI, Jianzong**
  **Chengdu, Sichuan 610094 (CN)**
- **WANG, Zao**
  **Chengdu, Sichuan 610094 (CN)**
- **ZHAO, Shuchun**
  **Chengdu, Sichuan 610094 (CN)**
- **SHAO, Tao**
  **Chengdu, Sichuan 610094 (CN)**
- **ZHOU, Ruijie**
  **Chengdu, Sichuan 610094 (CN)**
- **WANG, Jing**
  **Chengdu, Sichuan 610094 (CN)**
- **HU, Xiao**
  **Chengdu, Sichuan 610094 (CN)**
- **WANG, Yeye**
  **Chengdu, Sichuan 610094 (CN)**
- **ZHANG, Xiaodong**
  **Chengdu, Sichuan 610094 (CN)**
- **TANG, Jun**
  **Chengdu, Sichuan 610094 (CN)**
- **TANG, Yuanqing**
  **Chengdu, Sichuan 610094 (CN)**

(74) Representative: **Cabinet Becker et Associés**
**25, rue Louis le Grand**
**75002 Paris (FR)**

# (54) COMPOUND CONTAINING BIS(AZANYLYLIDENE) SULFONYL STRUCTURE AND USE THEREOF IN MEDICINE

(57) A compound containing a bis(azanylylidene) sulfonyl structure of formula (I) as a PKR agonist and/or USP9X inhibitor, which can be used for treating related diseases mediated and regulated by PKR and USP9X, wherein the related diseases comprise, but are not limited to: sickle-cell anemia, β-thalassemia, hereditary non-spherocytic hemolytic anemia, hemolytic anemia, hereditary spherocytosis, hereditary elliptocytosis, abetalipoproteinemia, paroxysmal nocturnal hemoglobinuria, acquired hemolytic anemia, congenital anemia, anemia of chronic diseases, colorectal cancer, kidney cancer, pancreatic cancer, breast cancer, lung cancer, esophageal cancer, melanoma, lymphoma, glioblastoma, or multiple myeloma. ;,

EP 4 455 136 A1

## Description

## TECHNICAL FIELD

The present invention belongs to the field of pharmaceutical technology, and specifically relates to a compound containing a diazanylidenesulfonyl structure and use thereof as a PKR agonist and/or USP9X inhibitor in the treatment of related diseases.

## BACKGROUND

Hemoglobin (Hb) is an oxygen-binding tetrameric protein in a red blood cell (RBC). Fetal hemoglobin (HbF) is composed of two α-chains and two γ-chains (α2γ2). It is modulated by a globin switching developmental regulatory mechanism 6-12 months after birth and is converted into α2β2 hemoglobin (HbA). A β- or α-chain gene mutation may cause HbA structural abnormalities, reduce the amount of the produced HbA or form an abnormal HbA, leading to a decrease in the oxygen-carrying capacity of RBC. A disease caused by these mutations are called hemoglobinopathy. A common hemoglobinopathy includes β-thalassemia and sickle cell disease (SCD) (Nat Genet50, 478-480 (2018)).

The β-thalassemia is an autosomal-recessive genetic disease. Due to point mutations or deletions of the β-chain gene, the synthesis of the β-chain is partially or completely inhibited, resulting in an imbalance in the ratio of the α-chain to the β-chain. The α-chains accumulate near the cell membrane to form insoluble haemichromes and result in a reduction in the total oxygen-carrying capacity of the RBC and meanwhile an increase in free heme and iron, which increase the level of reactive oxygen species (ROSs) and oxidize cell membrane proteins and lipids, leading to cytolysis and premature apoptosis and inhibiting RBC differentiation and maturation stages. Therefore, RBCs in patients with β-thalassemia consume more ATP, which is a key factor in the shortened lifespan and increased hemolysis of RBCs (Int J Mol Sci 22, 7229 (2021)). The patients with β-thalassemia have different clinical manifestations, ranging from asymptomatic, non-transfusion-dependent β-thalassemia (NTDT) to transfusion-dependent β-thalassemia (TDT), which are mainly related to the degree of β-chain deletion. Patients with TDT produce little HbA and usually develop anemia after HbF is converted to HbA. It is estimated that 5% of the world's population has at least one β-thalassemia gene variation, and there are approximately 300,000 patients with β-thalassemia worldwide, who are distributed along the Mediterranean coast and throughout Southeast Asia (N Engl J Med 371, 1908-1916 (2014)), and mainly distributed in the south of the Yangtze River in China. In 2017, a Meta-analysis result showed that the incidence rate of β-thalassemia in Chinese mainland was 2.21% (Sci Rep 7, 920 (2017)).

SCD is an autosomal dominant inherited hemoglobinopathy. Since glutamic acid at position 6 of the β-peptide chain is replaced by valine, hemoglobin S (HbS) is prone to polymerization to form a sickle-shaped red blood cell when it is deoxygenated. Clinical manifestations include chronic hemolytic anemia, organ and tissue damage caused by chronic ischemia, splenic infarction, susceptibility to infection, and vaso-occlusive crises (VOCs) (Lancet 376, 2018-2031 (2010)). SCD mutated genes are distributed around the world, including African Americans (about 7%-10%), sub-Saharan Africa (30%), Mediterranean countries (especially Greece), Middle East (0.2%-27%) and India (13%), and the like districts (J Neonatal Screen 4, 31 (2018)). To sum up, the global prevalence of SCD is estimated to be approximately 300 million people (Engl J Med 375, 435-442 (2016)). In patients with β-thalassemia and SCD, the imbalance of an α-/non-α-chain ratio leads to ineffective erythropoiesis (IE), triggers the apoptosis of erythroid precursors, and is accompanied by increased apoptosis, leading to anemia, splenomegaly and decreased production of hepcidin, leading to systemic iron overload. IE is the main pathogenesis and pathological feature of β-thalassemia. In the bone marrow of a patient with β-thalassemia, the content of the erythroid precursors is about 6 times higher than that of normal people, and the apoptosis rate is about 4 times higher than that of normal people (Int J Mol Sci 22, 7229 (2021)). Moreover, the life span of circulating red blood cells in the patients with SCD is shortened, and late erythroblast death also occurs in the body. For the aforementioned two hemolytic anemia diseases, hematopoietic stem cell (HSC) transplantation is currently the only curative measure. However, it is limited by the availability of donors, the need for post-transplantation immunosuppression, and the relatively higher cost of treatment. The proportion of patients suitable for this therapy is very small. Supportive treatments include blood transfusion, folic acid supplementation, splenectomy, and the administration of iron chelators to reduce iron overload, etc. To improve IE caused by α-/non-α-chain ratio imbalance, treatment means currently being studied include three aspects of the gene, protein, and small molecule compound treatments.

In terms of gene treatment, it was reported in 2018 that 22 cases of TDT patients used autologous HSCs with LentiGlobin BB305 vectors encoding T87Q variant, with significant clinical benefits, but no curative outcomes (N Engl J Med 378, 1479-1493 (2018)). Moreover, based on CRISPR-Cas9 technology, CTX001 is used for inhibiting BCL11A to induce HbF, which can reduce the number of blood transfusion in patients with severe SCD (N Engl J Med 384, 252-260 (2021)).

The recombinant fusion protein Luspatercept (ACE-536) was approved for the treatment of adult patients with TDT in 2019. Luspatercept can bind to transforming growth factor TGF β superfamily ligands, inhibit a SMAD2/3 pathway,

promote RBC maturation, and reduce the number of blood transfusions for the patients (J Cell Mol Med 24, 6162-6177 (2020)). Crizanlizumab, a monoclonal antibody against P-selectin that has been approved by FDA, is used for reducing the frequency of occurrence of VOCs by reducing the interaction between endothelial cells and circulating blood cells, but it cannot alter HbS aggregation (J Pain Res 14, 849-856 (2021)).

Although there are currently many treatment options mentioned above, these treatment manners, including hematopoietic stem cell transplantation, gene treatment, recombinant fusion protein treatment, and treatment with blood transfusion combined with iron chelators, generally have shortcomings such as the risk of surgical complications, high economic burden, and impact on the patient's quality of life. There is still a huge number of unmet needs. In contrast, the chemical small molecule drug therapy has unique clinical advantages.

Among small molecule compounds, the Janus kinase 2 inhibitor Ruxolitinib can improve IE and reduce splenomegaly in a β-thalassemia mouse model (Blood 131, 263-265 (2018)). Bitopertin (RO-4917838), an orally available potent and highly selective glycine transporter 1 inhibitor, can improve anemia and hemolysis in patients with β-thalassemia and improve RBC survival rate *in vivo* (Br J Haematol 194, 474-477 (2021)). VIT-2763, an orally active small-molecule ferroportin inhibitor, blocks iron efflux by inhibiting the binding of hepcidin to ferroportin (J Clin Invest 130, 491-506 (2020)). Inducing continuous production of HbF can alleviate the clinical symptoms of patients with β-chain abnormalities, such as hydroxyurea (HU), which is clinically used in the treatment of SCD and can stimulate HbF production and reduce HbS aggregation. However, HU will inhibit a bone marrow function. IMR-687, a phosphodiesterase (PDE9) inhibitor, has an obvious therapeutic effect in a SCD preclinical model by inhibiting PDE9, increasing intracellular cGMP levels and stimulating HbF production (Haematologica 105, 623-631 (2020)).

A pyruvate kinase (PK) is a key enzyme in glycolysis, which catalyzes the conversion of phosphoenolpyruvate into enolpyruvate and produces adenosine triphosphate (ATP). A R-type pyruvate kinase (PKR) is expressed in a mature RBC. The functional loss of PKR caused by gene mutation reduces ATP production, leading to hemolytic anemia and the occurrence of hemoglobinopathies such as β-thalassemia and SCD. Therefore, stimulating PKR to increase ATP in red blood cells is expected to play a positive role in the treatment of patients with hemoglobinopathies such as β-thalassemia and SCD who are deficient in PKR.

Currently, research literatures on small molecule drugs targeting PKR have been disclosed one after another. However, compared with other popular targets, there are still relatively few studies on the small molecule drugs, which are mainly as follows:

Mitapivat (AG-348), which is an oral small-molecule allosteric agonist, has shown efficacy and safety in clinical trials in patients with PKR deficiency (N Engl J Med 381, 933-944 (2019)).

FORMA Therapeutics Inc discloses a pyrrolopyrrole compound as a PKR agonist in WO2018175474 and WO2020061255.

Global Blood Therapeutics Inc discloses a pyrrolidinopyrazole compound as a pyruvate kinase activator in WO2021202796.

Agios Pharmaceuticals Inc discloses an azetidine arylsulfonamide derivative compound as a pyruvate kinase modulator in WO2012083246, WO2011002817, WO2012092442, WO2014139325, etc.

The aforementioned compounds related to PKR effects are all different from those in the present invention. Considering the current status of the treatment of hemoglobinopathies, in order to meet urgent clinical needs, providing a richer treatment modality or clinical drug selection that is more cost-effective, reduces the risk of complications, or improves the quality of life of patients is an urgent problem to be addressed.

Ubiquitination is one of manners of post-translation modifications of proteins, is dynamically regulated by ubiquitinases and deubiquitinases (DUBs). It is a basic mechanism for regulating protein turnover and stability in the body. The basic function of DUBs is to specifically remove ubiquitin from ubiquitinated proteins, thereby controlling the stability, interaction, or cellular localization of proteins in cells (Biomark Res 9, 66 (2021)). DUBs are involved in a variety of cell life activities, including DNA methylation, DNA damage repair, survival, differentiation and apoptosis. Dysregulation of ubiquitin balance has been implicated in the pathogenesis of human diseases, especially cancer, infection, neurodegenerative diseases, and immune disorders, etc., and thus DUBs have become attractive drug targets.

The human genome expresses nearly 100 DUBs, which are mainly divided into five families, namely a ubiquitin carboxyl-terminal hydrolase (UCH) family, a ubiquitin-specific protease (USP/UBP) family, an Otubaim (OTU) family, a Josephin domain protein family, and a JAMM family, respectively. USP is the largest subclass of DUBs, and has at least 54 members in human. Most USPs have both tumorigenic and tumorsuppressive effects, especially USP9X, USP10, USP18, USP22 and USP28. Different DUBs play different roles in tumorigenesis, mainly by affecting the stability, enzymatic activity or cellular localization of their substrate proteins. Different roles of USP9X in human cancer depend on

its different substrates (Cancer Med8, 6730-6740 (2019)).

In terms of anti-tumor effects, USP9X inhibits colorectal cancer, kidney cancer and pancreatic cancer by stabilizing FBW7 (a tumor suppressor that targets ubiquitination of oncoproteins such as c-MYC), AMOT (a YAP1 inhibitor), and LATS2 (a kinase that inhibits the Hippo pathway) substrate molecules (Biochim Biophys Acta Rev Cancer 1872, 188312 (2019)). In a USP9X-knockout colorectal cancer mouse model, USP9X has a significant tumorsuppressive effect (J Clin Invest 128, 1326-1337 (2018)).

On the other hand, USP9X is overexpressed in a variety of malignant tumors. In breast cancer, lung cancer, melanoma, lymphoma, and glioblastoma, USP9X expression is upregulated, promoting tumorigenesis and increasing resistance to chemotherapy drugs. For example, USP9X can stabilize CEP131 (as a key protein for centrosomal amplification, interfering with cell division and inducing tumorigenesis), SMURF1 (a key E3 ligase that regulates cell migration), and YAP1 (a downstream transcriptional regulator of the Hippo pathway) in breast cancer cells, and therefore, USP9X may induce malignant transformation, cancer cell survival, distant metastasis, and chemotherapy resistance in breast cancer by regulating these oncogenic signaling pathways (Nat Commun 8, 14866

(2017)). Degrasyn (WP1130) is a selective DUBs inhibitor that inhibits the DUBs activity of USP9X, USP5, USP14 and UCH37, leading to the rapid accumulation of polyubiquitinated proteins in abnormal protein inclusion bodies and tumor cell apoptosis. The compound EOAI3402143 can dose-dependently inhibit the activities of USP9X and USP24, increase tumor cell apoptosis, and inhibit cancer progression in multiple myeloma (Blood 125, 3588-3597 (2015)). Expression of USP9X in pancreatic cancer cells is positively correlated with gemcitabine resistance, and inhibiting USP9X enables pancreatic cancer cells to be sensitive to gemcitabine (Cancer Lett436, 129-138 (2018)). Low expression of USP9X in tumor cells can reduce MCL-1, thereby increasing the sensitivity to imatinib (BiomarkRes 9, 66 (2021)), cisplatin, and doxorubicin (Cancers (Basel). 11, 344 (2019)).

Studies have found that USP9X is an unfavorable prognostic indicator in glioma, lymphoma, multiple myeloma, and esophageal cancer, while its expression status is associated with better prognosis in patients with colorectal and pancreatic cancer (Diagn Pathol 8, 177 (2013), Nature 486, 266-270 (2012)). In view of the above, it is more likely to be believed that USP9X is a potential context-dependent USP that is regulated by a variety of upstream molecules in tumor cells (Biochim Biophys Acta Rev Cancer 1872, 188312 (2019)).

Protein ubiquitination downstream of immune signaling pathways is critical for positive and negative regulation of almost all immune responses, especially T cell activation. A large number of studies have shown that modulation of ubiquitin-dependent pathways can significantly alter T cell activation and enhance anti-tumor responses. The deubiquitinases USP9X and USP12 actively regulate the NF-κB pathway by removing inhibitory ubiquitin chains from BCL10, thereby blocking the assembly of a CBM signaling complex, which plays a key role in transducing signals from antigen receptors in T and B cells to NF-κB. Accordingly, T cells with USP9X deficiency exhibit lower levels of NF-κB activation upon activation of T cell receptors (Am J Physiol Cell Physiol 317, C534-C543 (2019)). The development of a T cell-centered immunotherapy is a current research hotspot. A tumor PD-L1 level is an important determinant of tumor immunity, and studies have shown that cancer cells utilize EGFR signaling to stabilize PD-L1 expression, so as to evade T cell immunity (Nat Commun 12, 2346 (2021)). PD-L1 can be stabilized by OTUB1, USP9X, and USP7. Inhibiting or eliminating these DUBs can re-stimulate an anti-tumor response and increase the sensitivity of cancer cells to T cell killing (Cancer Med 7, 4004-4011 (2018)). An inhibitor targeting USP9X is likely to be an alternative to improve a targeted tumor immunotherapy (/nt J Mol Sci 22,10800 (2021)).

Cancer treatment drugs targeting USP9X are being actively developed. Highly specific USP9X small-molecule inhibitors show great potential in inhibiting tumors, participating in chemotherapy drug resistance, participating in T cell activation, stabilizing PD-L1 expression, and the like.

Typical USP9X small molecule inhibitors include a pyrrolopyrrole or pyrazolopyrrolidine compound as a USP9X inhibitor disclosed by FORMA Therapeutics Inc in WO2020061261. In view of the above, in order to meet unmet clinical needs, it is necessary to design new compounds with potential medical values.

## SUMMARY OF THE INVENTION

In order to solve the aforementioned problems existed in the prior art, the purpose of the present invention is to provide a new compound containing a diazanylidenesulfonyl structure as a PKR agonist and/or USP9X inhibitor for the prevention and treatment of PKR and/or USP9X-mediated related diseases, such as sickle cell anemia, β-thalassemia, colorectal cancer, renal cancer, pancreatic cancer, breast cancer, lung cancer, esophageal cancer, melanoma, lymphoma, glioblastoma, or multiple myeloma, etc.

Detailed description of the present invention:

Firstly, the present invention provides a compound of formula I, or an isomer, pharmaceutically acceptable salt or solvate thereof:

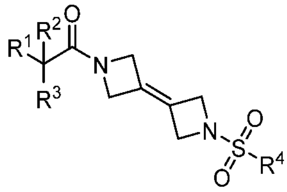
( I )

wherein:

$R^1$, $R^2$ and $R^3$ are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ or $-C(O)OR^g$, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)\ NR^cR^d$, $-NR^cS(O)_2R^e$, $-NR^cS(O)R^e$, $-C(O)R^f$ and $-C(O)OR^g$;

or, $R^1$ and $R^2$, $R^1$ and $R^3$, or $R^2$ and $R^3$ together with atoms to which they are attached optionally bind to form $-(C_3-C_8)$cycloalkyl, 3-14 membered heterocyclyl containing 1-4 heteroatoms independently selected from O, N and S, $-(C_5-C_8)$spirocyclyl, 5-8 membered spiroheterocyclyl containing 1-4 heteroatoms independently selected from O, N and S, 7-14 membered fused ring group containing 0-4 heteroatoms independently selected from O, N and S, or 5-14 membered heteroaryl containing 1-4 heteroatoms independently selected from O, N and S;

$R^4$ is $-NH_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$, $-C(O)OR^g$ or $-NR^c(CR^hR^i)_t-R^a$, wherein each of the alkyl, alkenyl, alkynyl, cycloalkyl, cycloalkenyl, heterocyclyl, aryl, heteroaryl, or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ and $-C(O)OR^g$;

t is 0, 1, 2 or 3;

$R^a$, $R^b$, $R^c$, $R^d$, $R^e$, $R^f$, $R^g$, $R^h$ and $R^i$ at each occurrence are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H or -C(O)OH, wherein each of the alkyl, alkenyl, alkynyl, cycloalkyl, cycloalkenyl, heterocyclyl, aryl, heteroaryl, or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, $-(C_1-C_6)$alkyl, $-(C_3-C_8)$cycloalkyl and 3-14 membered heterocyclyl containing 1-4 heteroatoms independently selected from O, N and S, or wherein each of the alkyl, alkenyl, alkynyl, cycloalkyl, cycloalkenyl, heterocyclyl, aryl, heteroaryl, or fused ring group is optionally substituted by one or more substituents selected from: $-S(O)_2OH$, -S(O)OH, $-NHS(O)_2OH$ or -NHS(O)OH; or, $R^a$, $R^b$, $R^c$, $R^d$, $R^e$, $R^f$, $R^g$, $R^h$ and $R^i$ at each occurrence are independently $-S(O)_2OH$, -S(O)OH, $-NHS(O)_2OH$ or -NHS(O)OH;

or, any two groups on adjacent atoms selected from $R^a$, $R^b$, $R^c$, $R^d$, $R^e$, $R^f$, $R^g$, $R^h$ and $R^i$ together with atoms to which they are attached optionally bind to form $C_6-C_{14}$ aryl, 5-14 membered heteroaryl containing 1-4 heteroatoms independently selected from O, N and S, $(C_3-C_8)$cycloalkyl, or 3-14 membered heterocyclyl containing 1-4 heteroatoms independently selected from O, N and S, which is optionally substituted by one or more $R^a$. In some embodiments, the present invention provides specific isomeric compounds of the aforementioned formula I, having formula I-1 or I-2,

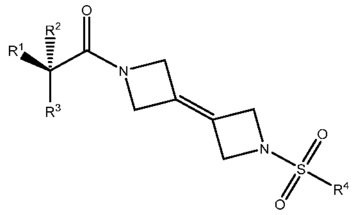 ( I −1), 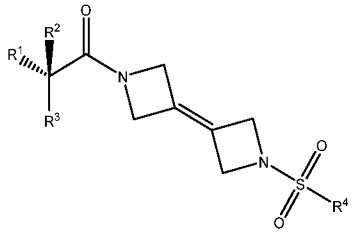 ( I −2).

In some embodiments, in the aforementioned compounds of formula I, I-1 or I-2 provided by the present invention, $R^1$ and $R^2$ can be further independently selected from the following:
In one embodiment,

$R^1$ and $R^2$ are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$ or $-NR^cR^d$, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, $-R^a$, $-OR^b$, $-NO_2$, and $-NR^cR^d$;
or, $R^1$ and $R^2$ together with atoms to which they are attached optionally bind to form $-(C_3-C_8)$cycloalkyl or 3-14 membered heterocyclyl containing 1-4 heteroatoms independently selected from O, N and S;
$R^a$, $R^b$, $R^c$, and $R^d$ at each occurrence are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, $-NO_2$, $-NH_2$, $-(C_1-C_6)$alkyl, $-(C_3-C_8)$cycloalkyl, and 3-14 membered heterocyclyl containing 1-4 heteroatoms independently selected from O, N and S.

In another embodiment,

$R^1$ and $R^2$ are independently -H, halogen, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, 5-8 membered heteroaryl containing 1-4 heteroatoms independently selected from O, N and S, 7-14 membered fused ring group containing 0-4 heteroatoms independently selected from O, N and S, $-OR^b$ or $-NR^cR^d$, wherein each of the alkyl, cycloalkyl, heterocyclyl, aryl, heteroaryl or fused ring group is optionally substituted by one or more substituents selected from: =O, halogen, -CN, $-R^a$, $-OR^b$, $-NO_2$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;
$R^a$, $R^b$, $R^c$, and $R^d$ at each occurrence are independently -H, halogen, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, 5-8 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -OH, $-NO_2$, $-NH_2$, $-(C_1-C_6)$alkyl, $-(C_3-C_6)$cycloalkyl, and 3-6 membered heterocyclyl containing 1-4 heteroatoms independently selected from O, N and S.

In another embodiment,

$R^1$ and $R^2$ are independently -H, -F, -Cl, -Br, -OH, $-NH_2$, -CN, $-NO_2$, $-(C_1-C_6)$ linear alkyl, $-(C_1-C_6)$ branched alkyl, $-(C_3-C_6)$cycloalkyl, 3-6 membered heterocyclyl containing 1-4 heteroatoms independently selected from O, N and S, $C_6-C_8$ aryl, 7-14 membered bicyclic or tricyclic fused ring group containing 0-4 heteroatoms independently selected from O, N and S, $-OR^b$ or $-NR^cR^d$, wherein each of the alkyl, cycloalkyl, heterocyclyl, aryl, heteroaryl or fused ring group is optionally substituted by one or more substituents selected from: -F, -Cl, -Br, -CN, $-R^a$, $-OR^b$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;

$R^a$, $R^b$, $R^c$, and $R^d$ at each occurrence are independently -H, -F, -Cl, -Br, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, or 5-8 membered heteroaryl containing 1-4 heteroatoms independently selected from O, N and S, wherein each of the alkyl, cycloalkyl, heterocyclyl, aryl, heteroaryl or fused ring group is optionally substituted by one or more substituents selected from: -F, -Cl, -Br, -CN, -OH, $-NO_2$, $-NH_2$, and $-(C_1-C_6)$alkyl.

In another embodiment,

$R^1$ and $R^2$ are independently -H, -F, -Cl, -Br, -OH, $-NH_2$, -CN, $-NO_2$, $-(C_1-C_6)$ linear alkyl, $-(C_1-C_6)$ branched alkyl, $-(C_3-C_6)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, $-R^a$, $-OR^b$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;
$R^a$, $R^b$, $R^c$, and $R^d$ at each occurrence are independently -H, -F, -Cl, -Br, -OH, $-NH_2$, -CN, $-NO_2$, $-(C_1-C_6)$alkyl, $-(C_3-C_6)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, -OH, $-NO_2$, $-NH_2$, and $-(C_1-C_6)$alkyl.

In another embodiment,

$R^2$ is -H, -F, -Cl or -Br;
$R^1$ is -H, -F, -Cl, -Br, -OH, $-NH_2$, $-(CH_2)_qCH_3$, $-(CH_2)_qOH$, $-CH(OH)(CH_2)_qCH_3$, $-C(OH)((CH_2)_qCH_3)_2$,

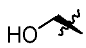 ,

$-(CH_2)_qNH_2$, $-(CH_2)_qNH(CH_2)_qCH_3$, $-(CH_2)_qN((CH_2)_qCH_3)_2$, 3-6 membered heterocycloalkyl containing 1 N atom, or $-(C_1-C_6)$alkyl substituted by 3-6 membered heterocycloalkyl containing 1 N atom;
q at each occurrence is independently 0, 1, 2, 3 or 4;
or, $R^1$ and $R^2$ together with atoms to which they are attached may optionally bind to form a cyclopropane ring, a cyclobutane ring, a cyclopentane ring, a cyclohexane ring, tetrahydrofuran, tetrahydropyran, morpholine, dioxane or 2,3-dihydrobenzofuran ring; or, $R^1$ and $R^2$ together with the atoms to which they are attached may optionally bind to form a cyclopropane ring, a cyclobutane ring, a cyclopentane ring, a cyclohexane ring, tetrahydrofuran, tetrahydropyran, morpholine, dioxane, a 2,3-dihydrobenzofuran ring, or tetrahydro-2H-pyran.

In another embodiment,

$R^2$ is -H, -F, -Cl or -Br;

$R^1$ is -H, -F, -Cl, -Br, -OH, $-NH_2$,

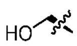, 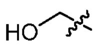, 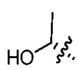, 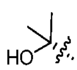, 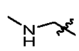, 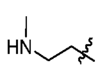, 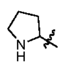

or

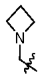;

or, $R^1$ and $R^2$ together with the atoms to which they are attached may optionally bind to form

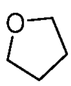;

or, $R^1$ and $R^2$ together with the atoms to which they are attached may optionally

bind to form

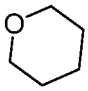.

In another embodiment,

$R^2$ is -H, -F, -Cl or -Br;

$R^1$ is -H, -F, -Cl, -Br, -OH, $-NH_2$, $-CH_3$, $-CH(OH)CH_3$, $-(CH_2)_qCH_3$, $-(CH_2)_qOH$, $-CH(OH)(CH_2)_qCH_3$, $-C(OH)((CH_2)_qCH_3)_2$, $-C(OH)((CH_2)_qCH_3)(CH_3)$, $-C(OH)(CH_3)_2$,

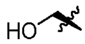, 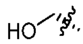,

$-CH_2F$, $-CHF_2$, $-(CH_2)_qCH_2F$, $-(CH_2)_qCHF_2$, $-CH_2Cl$, $-CHCl_2$, $-(CH_2)_qCH_2Cl$, $-(CH_2)_qCHCl_2$, $-(CH_2)_qNH_2$, $-NHCH_3$, $-NH(CH_2)_qCH_3$, $-(CH_2)_qNHCH_3$, $-(CH_2)_qNH(CH_2)_qCH_3$, $-N(CH_3)_2$, $-N((CH_2)_qCH_3)_2$, $-(CH_2)_qN(CH_3)((CH_2)_qCH_3)$, $-(CH_2)_qN(CH_3)_2$, $-(CH_2)_qN((CH_2)_qCH_3)_2$, 3-6 membered heterocycloalkyl containing 1 N atom, or $-(C_1-C_6)$alkyl substituted by 3-6 membered heterocycloalkyl containing 1 N atom;

q at each occurrence is independently 1, 2, 3 or 4;

or, $R^1$ and $R^2$ together with atoms to which they are attached optionally bind to form a cyclopropane ring, a cyclobutane ring, a cyclopentane ring, a cyclohexane ring, oxetane, tetrahydrofuran, tetrahydropyran, morpholine, dioxane, a 2,3-dihydrobenzofuran ring, or tetrahydro-2H-pyran.

In another embodiment,

$R^2$ is -H, -F, -Cl or -Br;

$R^1$ is -H, -F, -Cl, -Br, -OH, $-NH_2$,

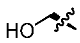, 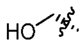, 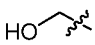, 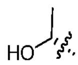, 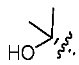, 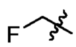, 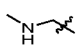,

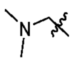, 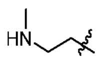, 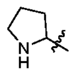 or 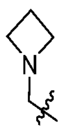;

or, $R^1$ and $R^2$ together with the atoms to which they are attached may optionally bind to form

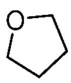 or 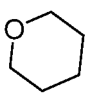;

or, $R^1$ and $R^2$ together with the atoms to which they are attached may optionally bind to form

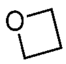.

In some embodiments, in the compounds of formula I, I-1 or I-2 provided by the present invention, on the basis that $R^1$ and $R^2$ are independently made any of the selections and combinations as described above, $R^3$ can be further

independently selected from the following:
In one embodiment,

$R^3$ is -H, halogen, -($C_1$-$C_6$)alkyl, -($C_3$-$C_8$)cycloalkyl, 3-14 membered heterocyclyl containing 1-4 heteroatoms independently selected from O, N and S, $C_6$-$C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -$R^a$, -$OR^b$, -$NO_2$, and -$NR^cR^d$;
$R^a$, $R^b$, $R^c$, and $R^d$ at each occurrence are independently -H, halogen, -OH, -$NH_2$, -CN, -$NO_2$, -($C_1$-$C_6$)alkyl, -($C_2$-$C_6$)alkenyl, -($C_2$-$C_6$)alkynyl, -($C_3$-$C_8$)cycloalkyl, -($C_4$-$C_8$)cycloalkenyl, 3-14 membered heterocyclyl containing 1-4 heteroatoms independently selected from O, N and S, $C_6$-$C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, -$NO_2$, -$NH_2$, -($C_1$-$C_6$)alkyl, -($C_3$-$C_8$)cycloalkyl, and 3-14 membered heterocyclyl containing 1-4 heteroatoms independently selected from O, N and S.

In another embodiment,

$R^3$ is -H, halogen, -($C_1$-$C_6$)alkyl, -($C_3$-$C_6$)cycloalkyl, 3-6 membered heterocyclyl containing 1-4 heteroatoms independently selected from O, N and S, $C_6$-$C_8$ aryl, 5-8 membered heteroaryl containing 1-4 heteroatoms independently selected from O, N and S, or 7-14 membered bicyclic or tricyclic fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -$R^a$, -$OR^b$, -$NO_2$, and -$NR^cR^d$;
$R^a$, $R^b$, $R^c$, and $R^d$ at each occurrence are independently -H, halogen, -OH, -$NH_2$, -CN, -$NO_2$, -($C_1$-$C_6$)alkyl, -($C_3$-$C_8$)cycloalkyl, 3-6 membered heterocyclyl containing 1-4 heteroatoms independently selected from O, N and S, $C_6$-$C_8$ aryl, 5-8 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -OH, -$NO_2$, -$NH_2$, -($C_1$-$C_6$)alkyl, -($C_3$-$C_6$)cycloalkyl, and 3-6 membered heterocyclyl containing 1-4 heteroatoms independently selected from O, N and S.

In another embodiment,

$R^3$ is -H, -F, -Cl, -Br, -($C_1$-$C_6$)alkyl, -($C_3$-$C_6$)cycloalkyl, $C_6$-$C_8$ aryl, 5-8 membered heteroaryl containing 1-4 heteroatoms independently selected from O, N and S, or 7-14 membered bicyclic fused ring group containing 0-4 heteroatoms independently selected from O, N and S, wherein each of the alkyl, cycloalkyl, aryl, heteroaryl or fused ring group is optionally substituted by one or more substituents selected from: -F, -Cl, -Br, -CN, -$R^a$ and -$OR^b$;
$R^a$ and $R^b$ at each occurrence are independently -H, halogen, -OH, -$NH_2$, -CN, -$NO_2$, -($C_1$-$C_6$)alkyl, -($C_3$-$C_8$)cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, -OH, -$NO_2$, -$NH_2$, and -($C_1$-$C_6$)alkyl.

In another embodiment,
$R^3$ is -H, -F, -Cl, -Br, -($C_1$-$C_6$)alkyl, -($C_3$-$C_6$)cycloalkyl, pyridyl, phenyl, benzothiazolyl, benzomorpholinyl, or benzopyrrolidinyl, wherein the pyridyl, phenyl, benzothiazolyl, benzomorpholinyl, or benzopyrrolidinyl is optionally substituted by one or more substituents selected from: -F, -Cl, -Br, -($C_1$-$C_6$)alkyl, -($C_3$-$C_6$)cycloalkyl, -O-($C_1$-$C_6$)alkyl, -O-($C_3$-$C_6$)cycloalkyl, piperazinyl, and piperazinyl substituted by any number of halogen or -($C_1$-$C_6$)alkyl.
In another embodiment,
$R^3$ is -H, -F, -Cl, -Br, -$CH_3$, -$CH_2CH_3$, pyridyl,

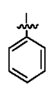, 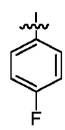, 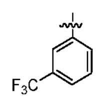, 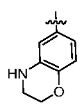, 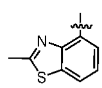, 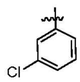,

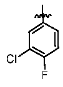, 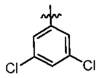, 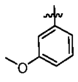, 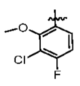, 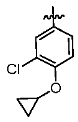, 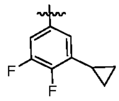, 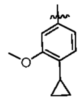, 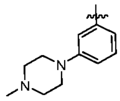, 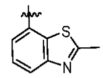, 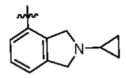, 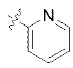,

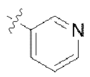, 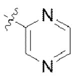, 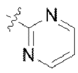, 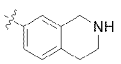, 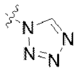, 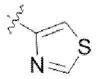, 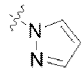, 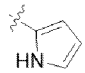, 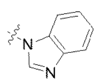, 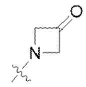, 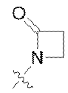,

or

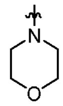.

In another embodiment, $R^3$ is -H, -F, -Cl, -Br, -$(C_1$-$C_6)$alkyl, -$(C_3$-$C_6)$cycloalkyl, pyridyl, phenyl, naphthyl, benzothiazolyl, benzomorpholinyl, benzopyrrolidinyl or

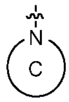,

wherein the pyridyl, phenyl, benzothiazolyl, benzomorpholinyl, benzopyrrolidinyl, or

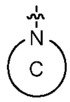

is optionally substituted by one or more substituents selected from: -F, -Cl, -Br, -CN, -$(C_1$-$C_6)$alkyl, -$(C_3$-$C_6)$cycloalkyl, -O-$(C_1$-$C_6)$alkyl, -O-$(C_3$-$C_6)$cycloalkyl, -$(C_1$-$C_6)$haloalkyl, -$(C_3$-$C_6)$halocycloalkyl, -O-$(C_1$-$C_6)$haloalkyl, -O-$(C_3$-$C_6)$halocycloalkyl, pyrazolyl, pyrazolyl substituted by any number of halogen or -$(C_1$-$C_6)$alkyl, piperazinyl, piperazinyl substituted by any number of halogen or -$(C_1$-$C_6)$alkyl; and
the ring

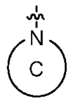

is 3-6 membered saturated or unsaturated heterocyclyl connected through an N atom, and in addition to the N atom, the heterocyclyl optionally contains 1-2 heteroatoms independently selected from O, N and S.

In another embodiment,
$R^3$ is -H, -F, -Cl, -Br, $-CH_3$, $-CH_2CH_3$,

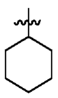, 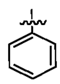, 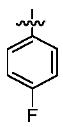, 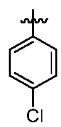, 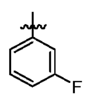, 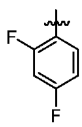, 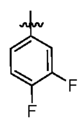,

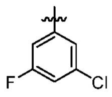, 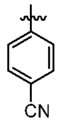, 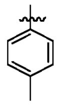, 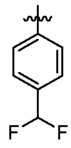, 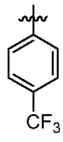, 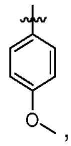, 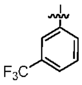, 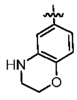, 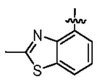, 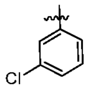, 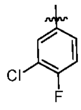, 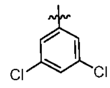,

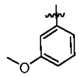, 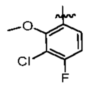, 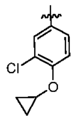, 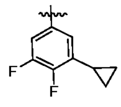, 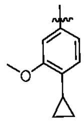, 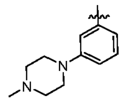, 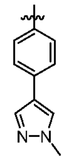, 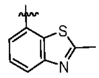, 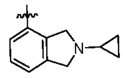, 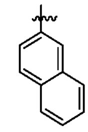, 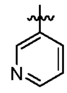,

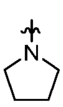, 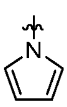, 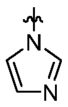, 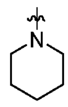, 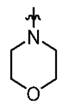, 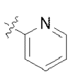, 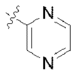, 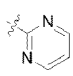, 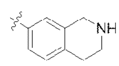, 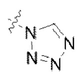, 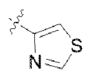, 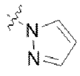,

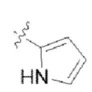, 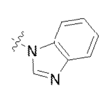, 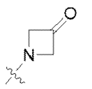 or 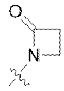.

In some embodiments, in the compounds of formula I, I-1 or I-2 provided by the present invention, on the basis that $R^1$, $R^2$ and $R^3$ are independently made any of the selections and combinations as described above, $R^4$ can be further selected from the following:
In a first kind of embodiments:

$R^4$ is

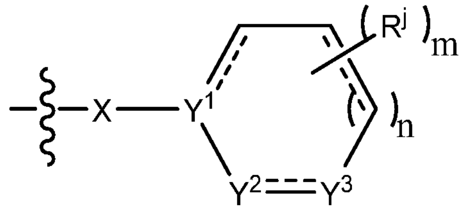;

X is a chemical bond, $-(CR^hR^i)_t-$, $-NR^c(CR^hR^i)_t-$ or -O-; wherein $R^c$, $R^h$, $R^i$ and t are optionally as defined anywhere they are occurred in the present invention;

- - - - - - represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: -H, =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, 3-14 membered heterocyclyl containing 1-4 heteroatoms independently selected from O, N and S, $C_6-C_{14}$ aryl or 5-14 membered heteroaryl containing 1-4 heteroatoms independently selected from O, N and S, or wherein each of the alkyl, alkoxy, alkenyl, alkynyl, cycloalkyl, cycloalkenyl, heterocyclyl, aryl, heteroaryl or fused ring group is optionally substituted by one or more substituents selected from: $-S(O)_2OH$, -S(O)OH, $-NHS(O)_2OH$ or -NHS(O)OH; or, each $R^j$ is independently and optionally $-O-(C_3-C_6)$cycloalkyl, $-S(O)_2OH$, -S(O)OH, $-NHS(O)_2OH$ or -NHS(O)OH;

m is an integer selected from 0-6;

n is 0, 1 or 2.

In some of the first kind of embodiments,

$R^4$ is

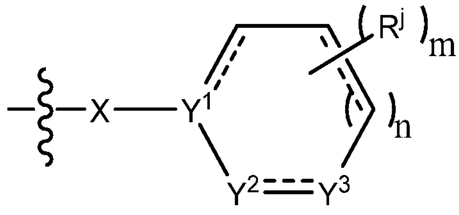

;

X is a chemical bond, $-CH_2-$, $-CH_2CH_2-$, $-NHCH_2-$, $-NHCH_2CH_2-$ or -O-;

------ represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-(C_1-C_6)$alkyl, $-(C_1-C_6)$alkoxy, $-(C_1-C_6)$alkyl or $-(C_1-C_6)$alkoxy substituted by any number of halogen, oxazolyl, thiazolyl and triazolyl;

m is an integer selected from 0-6;

n is 0, 1 or 2.

In some of the first kind of embodiments,

$R^4$ is

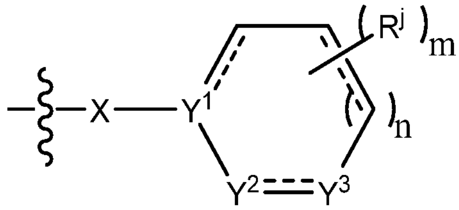

;

X is a chemical bond, $-CH_2-$, $-CH_2CH_2-$, $-NHCH_2-$, $-NHCH_2CH_2-$ or -O-;

------ represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-(C_1-C_6)$alkyl, $-(C_3-C_6)$cycloalkyl, $-(C_1-C_6)$alkoxy, $-O-(C_3-C_6)$cycloalkyl, $-(C_1-C_6)$alkyl substituted by any number of halogen, $-(C_1-C_6)$alkoxy substituted by any number of halogen, $-(C_3-C_6)$cycloalkyl substituted by any number of halogen, $-O-(C_3-C_6)$cycloalkyl substituted by any number of halogen, oxazolyl, thiazolyl and triazolyl;

m is an integer selected from 0-6;

n is 0, 1 or 2.

In a second kind of embodiments:

$R^4$ is

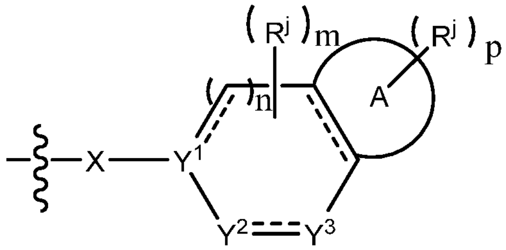

;

X is a chemical bond, $-(CR^hR^i)_t-$, $-NR^c(CR^hR^i)_t-$ or -O-; wherein $R^c$, $R^h$, $R^i$ and t are optionally as defined anywhere they are occurred in the present invention;
------ represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, 3-14 membered heterocyclyl containing 1-4 heteroatoms independently selected from O, N and S, $C_6-C_{14}$ aryl or 5-14 membered heteroaryl containing 1-4 heteroatoms independently selected from O, N and S, or wherein each of the alkyl, alkoxy, alkenyl, alkynyl, cycloalkyl, cycloalkenyl, heterocyclyl, aryl, heteroaryl or fused ring group is optionally substituted by one or more substituents selected from: $-S(O)_2OH$, -S(O)OH, $-NHS(O)_2OH$ or -NHS(O)OH; or, each $R^j$ is independently and optionally =O, $-S(O)_2OH$, -S(O)OH, $-NHS(O)_2OH$ or -NHS(O)OH;
m and p are independently integers selected from 0-6;
n is 0, 1 or 2;
ring A is $(C_3-C_8)$cycloalkyl, $(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S.

In some of the second kind of embodiments,

$R^4$ is

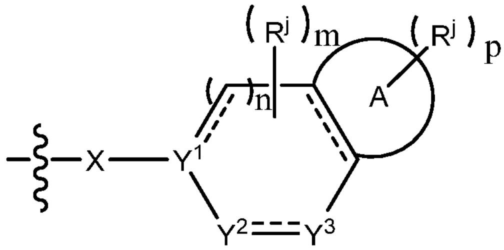

;

X is a chemical bond, $-CH_2-$, $-CH_2CH_2-$, $-NHCH_2-$, $-NHCH_2CH_2-$ or -O-;
------ represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, $-(C_1-C_6)$alkyl, $-(C_1-C_6)$alkoxy, $-(C_1-C_6)$alkyl or $-(C_1-C_6)$alkoxy substituted by any number of halogen, oxazolyl, thiazolyl or triazolyl;
m and p are independently integers selected from 0-6;
n is 0, 1 or 2;
ring A is furan, thiophene, oxazole, thiazole, triazole, piperidine, pyridine, pyran, thiopyran, morpholine, 1,4-dioxane, piperazine, pyrazine, or triazine.

In some of the second kind of embodiments,

$R^4$ is

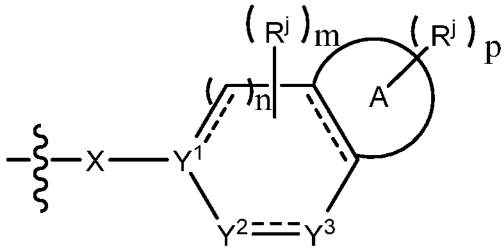

;

X is a chemical bond, $-CH_2-$, $-CH_2CH_2-$, $-NHCH_2-$, $-NHCH_2CH_2-$ or -O-;
------ represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, =O, $-(C_1-C_6)$alkyl, $-(C_1-C_6)$alkoxy, $-(C_1-C_6)$alkyl substituted by any number of halogen, $-(C_1-C_6)$alkoxy substituted by any number of halogen, oxazolyl, thiazolyl, or triazolyl;
m and p are independently integers selected from 0-6;
n is 0, 1 or 2;
ring A is furan, thiophene, oxazole, thiazole, triazole, piperidine, pyridine, pyran, thiopyran, morpholine, 1,4-dioxane, piperazine, pyrazine, triazine, 4,5-dihydro-1H-imidazole, or 1,3-dioxolane.

In some other specific embodiments, $R^4$ can be further selected as

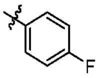, 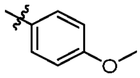,

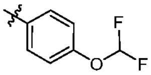, 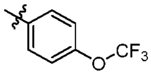, 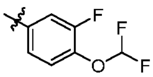, 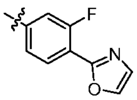, 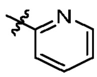, 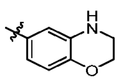, 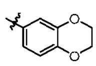, 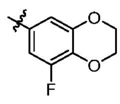,

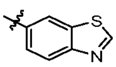, 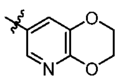, 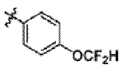, 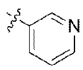, 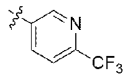, 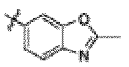, 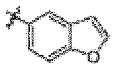, 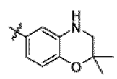,

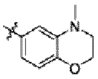, 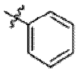, 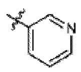 or 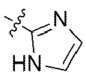.

In some other specific embodiments, $R^4$ can be further selected as

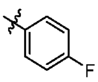, 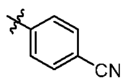,

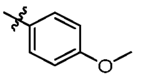, 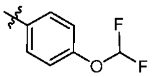, 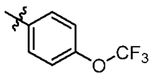, 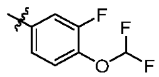, 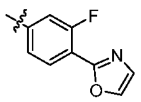, 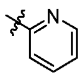, 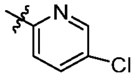,

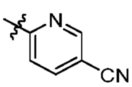, 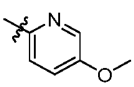, 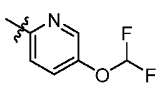, 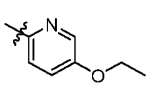, 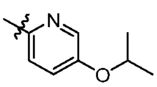, 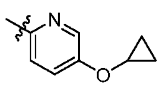, 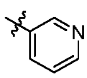,

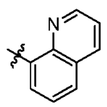, 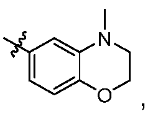, 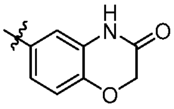, 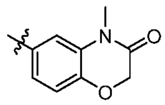, 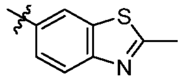, 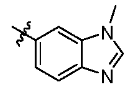, 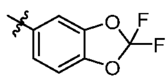,

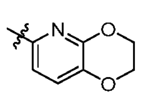, 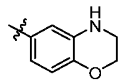, 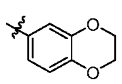, 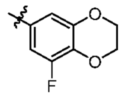, 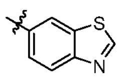, 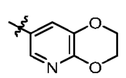, 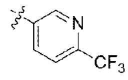, 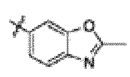,

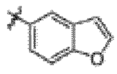, 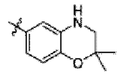, 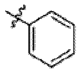 or 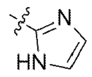.

As the first one of the preferred options, in some embodiments, the present invention provides a compound of formula I, I-1 or I-2, or an isomer, pharmaceutically acceptable salt or solvate thereof:

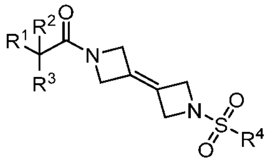 ( I ), 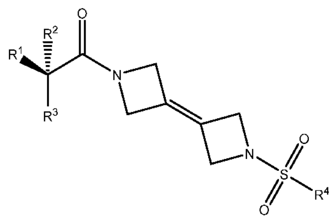 ( I -1), 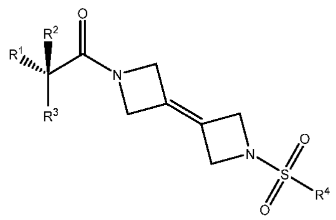 ( I -2)

wherein,

$R^1$ and $R^2$ are independently -H, -F, -Cl, -Br, -OH, $-NH_2$, -CN, $-NO_2$, $-(C_1-C_6)$ linear alkyl, $-(C_1-C_6)$ branched alkyl, $-(C_3-C_6)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, $-R^a$, $-OR^b$, and $-NR^cR^d$;

or, $R^1$ and $R^2$ together with atoms to which they are attached can optionally bind to form $-(C_3-C_8)$cycloalkyl or 3-6 membered heterocyclyl containing 1-4 heteroatoms independently selected from O, N and S;

$R^3$ is -H, -F, -Cl, -Br, $-(C_1-C_6)$alkyl, $-(C_3-C_6)$cycloalkyl, $-(C_6-C_8)$ aryl, or 7-14 membered bicyclic fused ring group containing 0-4 heteroatoms independently selected from O, N and S, wherein each of the alkyl, cycloalkyl, aryl or fused ring group is optionally substituted by one or more substituents selected from: -F, -Cl, -Br, -CN, $-R^a$ and $-OR^b$;

$R^a$, $R^b$, $R^c$, and $R^d$ at each occurrence are independently -H, -F, -Cl, -Br, -OH, $-NH_2$, -CN, $-NO_2$, $-(C_1-C_6)$alkyl, $-(C_3-C_8)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, -OH, $-NO_2$, $-NH_2$, and $-(C_1-C_6)$alkyl;

$R^4$ is

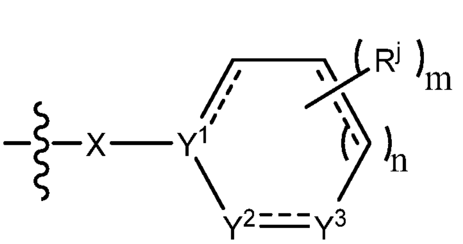 or 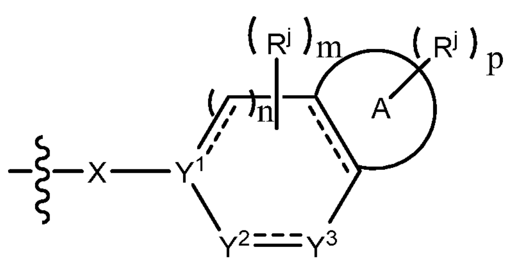 ;

X is a chemical bond, $-CH_2-$, $-CH_2CH_2-$, $-NHCH_2-$, $-NHCH_2CH_2-$ or -O-;

------ represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-(C_1-C_6)$alkyl, $-O-(C_1-C_6)$alkyl, $-(C_1-C_6)$alkyl substituted by

any number of halogen, -O-($C_1$-$C_6$)alkyl substituted by any number of halogen, oxazolyl, thiazolyl or triazolyl; or, each $R^j$ can be further independently selected from =O, -($C_3$-$C_6$)cycloalkyl, -O-($C_3$-$C_6$)cycloalkyl, -($C_3$-$C_6$)cycloalkyl substituted by any number of halogen, and -O-($C_3$-$C_6$)cycloalkyl substituted by any number of halogen;

m and p are independently 0, 1, 2, 3, 4, 5 or 6;

n is 0, 1 or 2;

ring A is furan, thiophene, oxazole, thiazole, triazole, piperidine, pyridine, pyran, thiopyran, morpholine, 1,4-dioxane, piperazine, pyrazine or triazine; or, the ring A is 4,5-dihydro-1H-imidazole or 1,3-dioxolane.

As the second one of the preferred options, in some embodiments, the present invention provides a compound of formula I, I-1 or I-2, or an isomer, pharmaceutically acceptable salt or solvate thereof:

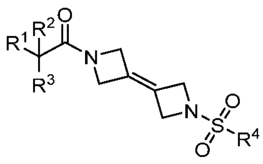 ( I ), 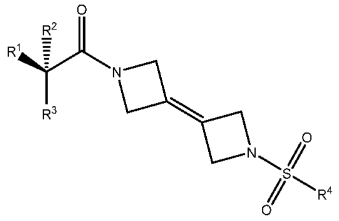 ( I -1), 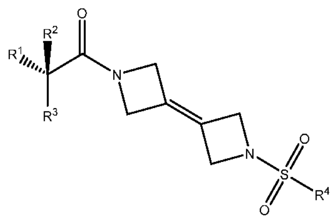 ( I -2)

wherein,

$R^2$ is -H, -F, -Cl or -Br;

$R^1$ is -H, -F, -Cl, -Br, -OH, -$NH_2$, -$(CH_2)_qCH_3$, -$(CH_2)_qOH$, -$CH(OH)(CH_2)_qCH_3$, -$C(OH)((CH_2)_qCH_3)_2$,

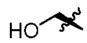 ,

-$(CH_2)_qNH_2$, -$(CH_2)_qNH(CH_2)_qCH_3$, -$(CH_2)_qN((CH_2)_qCH_3)_2$, 3-6 membered heterocycloalkyl containing 1 N atom, or -($C_1$-$C_6$)alkyl substituted by 3-6 membered heterocycloalkyl containing 1 N atom; or, $R^1$ is

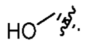 ,

-$CH_2F$, -$CHF_2$, -$(CH_2)_qCH_2F$, -$(CH_2)_qCHF_2$, -$CH_2Cl$, -$CHCl_2$, -$(CH_2)_qCH_2Cl$ or -$(CH_2)_qCHCl_2$;

q at each occurrence is independently 0, 1, 2, 3 or 4;

or, $R^1$ and $R^2$ together with atoms to which they are attached may optionally bind to form a cyclopropane ring, a cyclobutane ring, a cyclopentane ring, a cyclohexane ring, tetrahydrofuran, tetrahydropyran, morpholine, dioxane or 2,3-dihydrobenzofuran ring; or, $R^1$ and $R^2$ together with the atoms to which they are attached may optionally bind to form tetrahydro-2H-pyran or oxetane.

$R^3$ is -H, -F, -Cl, -Br, -($C_1$-$C_6$)alkyl, -($C_3$-$C_6$)cycloalkyl, pyridyl, phenyl, benzothiazolyl, benzomorpholinyl or benzopyrrolidinyl, wherein the pyridyl, phenyl, benzothiazolyl, benzomorpholinyl, or benzopyrrolidinyl is optionally substituted by one or more substituents selected from: -F, -Cl, -Br, -($C_1$-$C_6$)alkyl, -($C_3$-$C_6$)cycloalkyl, -O-($C_1$-$C_6$)alkyl, -O-($C_3$-$C_6$)cycloalkyl, piperazinyl, and piperazinyl substituted by any number of halogen or -($C_1$-$C_6$)alkyl;or, $R^3$ is

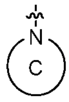 ,

wherein ring

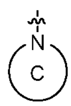

is a 3-6 membered saturated or unsaturated N-containing heterocyclyl connected through an N atom, in addition to

the N atom, the heterocyclyl optionally contains 1-2 heteroatoms independently selected from O, N and S, and

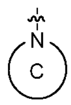

is optionally substituted by one or more substituents selected from: -F, -Cl, -Br, -CN, -($C_1$-$C_6$)alkyl, -($C_3$-$C_6$)cycloalkyl, -O-($C_1$-$C_6$)alkyl, -O-($C_3$-$C_6$)cycloalkyl, -($C_1$-$C_6$)haloalkyl, -($C_3$-$C_6$)halocycloalkyl, -O-($C_1$-$C_6$)haloalkyl, -O-($C_3$-$C_6$)halocycloalkyl, pyrazolyl, pyrazolyl substituted by any number of halogen or -($C_1$-$C_6$)alkyl, piperazinyl, piperazinyl substituted by any number of halogen or -($C_1$-$C_6$)alkyl.

$R^4$ is

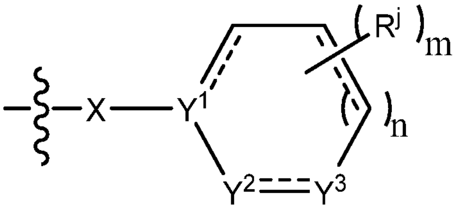 or 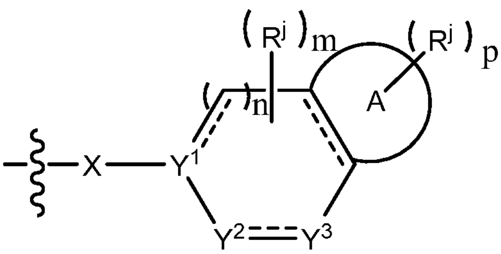 ;

X is a chemical bond, $-CH_2-$, $-CH_2CH_2-$, $-NHCH_2-$, $-NHCH_2CH_2-$ or -O-;

- - - - - - represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, -($C_1$-$C_6$)alkyl, -O-($C_1$-$C_6$)alkyl, -($C_1$-$C_6$)alkyl substituted by any number of halogen, -O-($C_1$-$C_6$)alkyl substituted by any number of halogen, oxazolyl, thiazolyl or triazolyl; alternatively, each $R^j$ can be further independently selected from =O, ($C_3$-$C_6$)cycloalkyl, -O-($C_3$-$C_6$)cycloalkyl, -($C_3$-$C_6$)cycloalkyl substituted by any number of halogen, or -O-($C_3$-$C_6$)cycloalkyl substituted by any number of halogen;

m and p are independently 0, 1, 2, 3, 4, 5 or 6;

n is 0, 1 or 2;

ring A is furan, thiophene, oxazole, thiazole, triazole, piperidine, pyridine, pyran, thiopyran, morpholine, 1,4-dioxane, piperazine, pyrazine or triazine; or, the ring A is 4,5-dihydro-1H-imidazole or 1,3-dioxolane.

As the third one of the preferred options, in some embodiments, the present invention provides a compound of formula I, I-1 or I-2, or an isomer, pharmaceutically acceptable salt or solvate thereof:

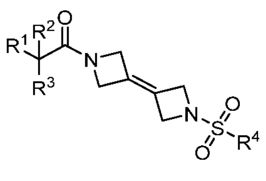 ( I ), 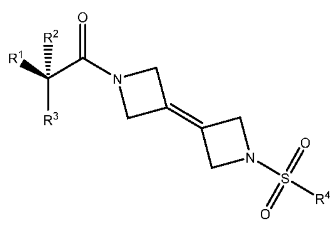 ( I −1), 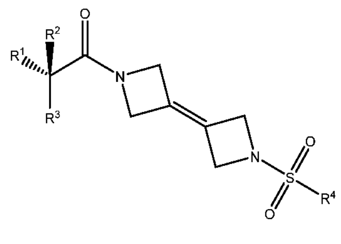 ( I −2)

wherein,

$R^2$ is -H, -F, -Cl or -Br;

$R^1$ is -H, -F, -Cl, -Br, -OH, $-NH_2$,

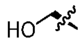, 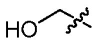, 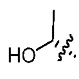, 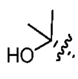, 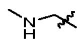, 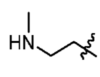, 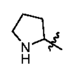 or

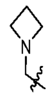;

alternatively, $R^1$ is

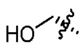, 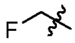 or 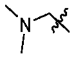;

alternatively, $R^1$ and $R^2$ together with the atoms to which they are attached may optionally bind to form

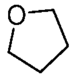;

or, $R^1$ and $R^2$ together with the atoms to which they are attached may optionally bind to form

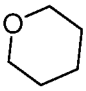 or 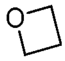;

$R^3$ is -H, -F, -Cl, -Br, $-CH_3$, $-CH_2CH_3$, pyridyl,

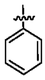, 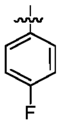, 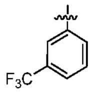, 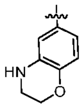, 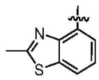, 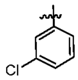,

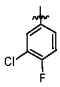, 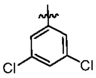, 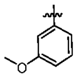, 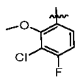, 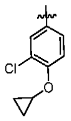, 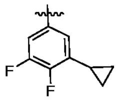, 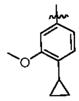, 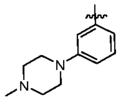, 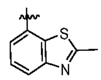, 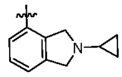, 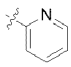,

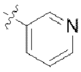, 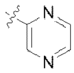, 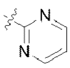, 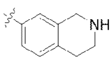, 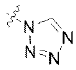, 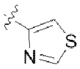, 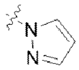, 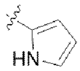, 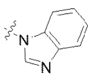, 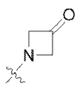, 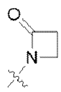 or 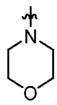;

alternatively $R^3$ is

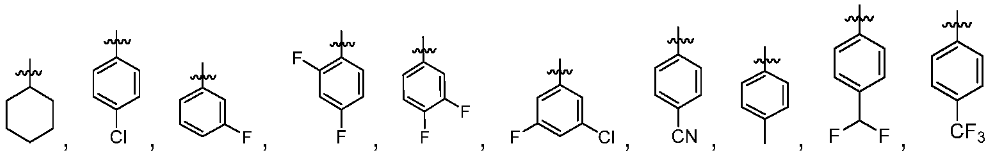, , , , , , , , , ,

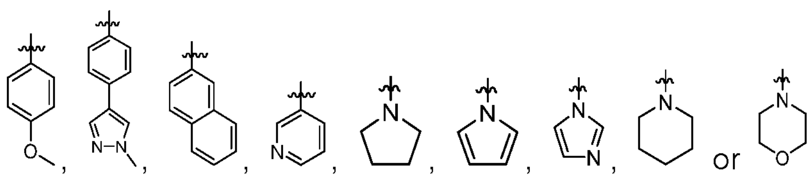, , , , , , , or ;

$R^4$ is

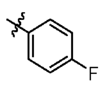, 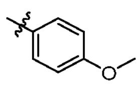, 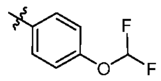, 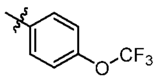, 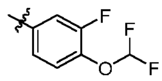, 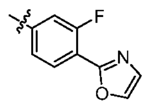, 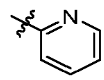,

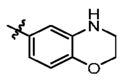, 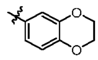, 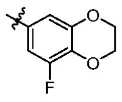, 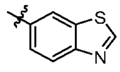, 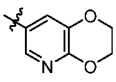, 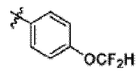, 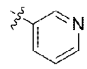, 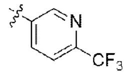, 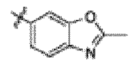,

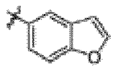, 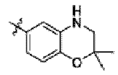, 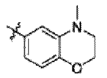, 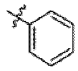, 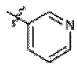 or 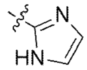; or $R^4$ is 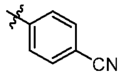, 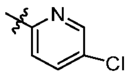,

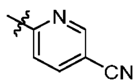, 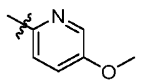, 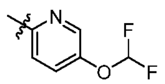, 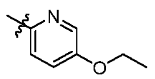, 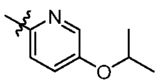, 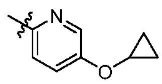, 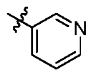,

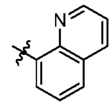, 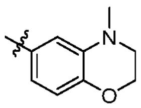, 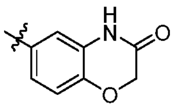, 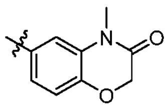, 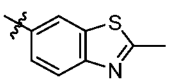, 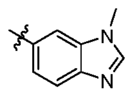, 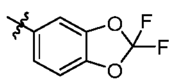

or

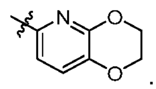.

Preferably, the present invention provides the compounds shown in the following table, or isomers pharmaceutically acceptable salts or solvates thereof:

| | |
|---|---|
| 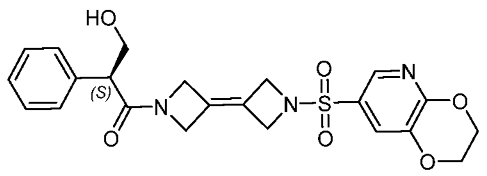 | 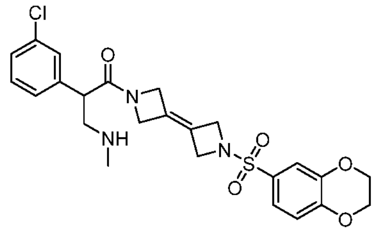 |
| 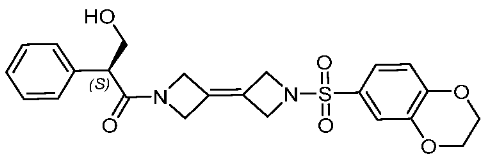 | 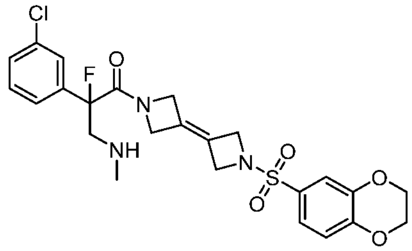 |
| 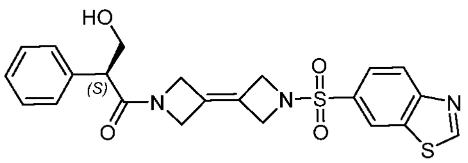 | 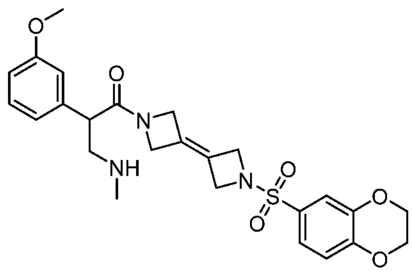 |
| 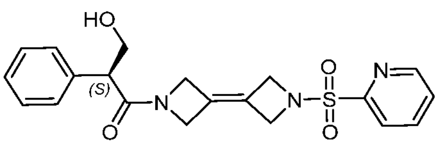 | 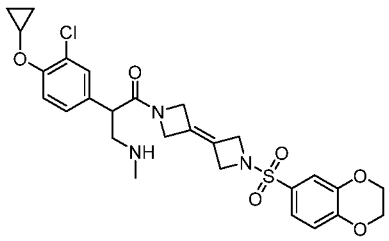 |

| | |
|---|---|
| 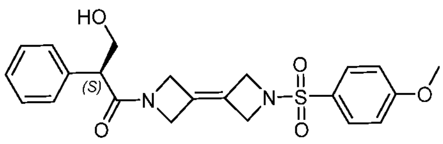 | 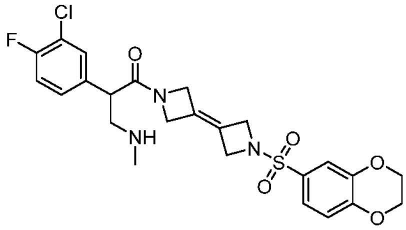 |
| 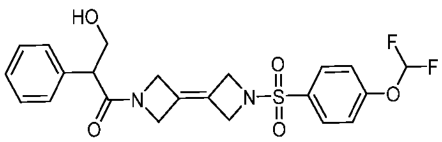 | 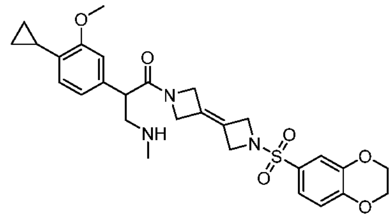 |
| 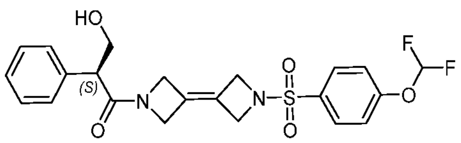 | 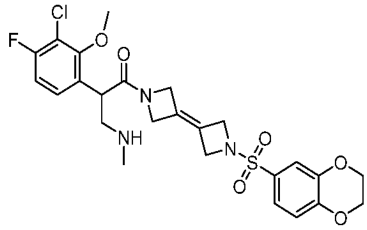 |
| 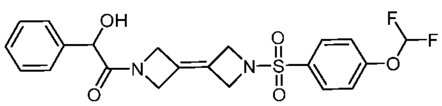 | 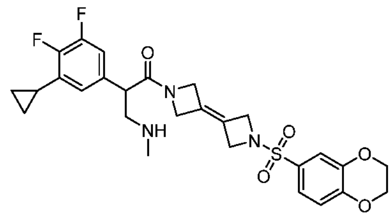 |
| 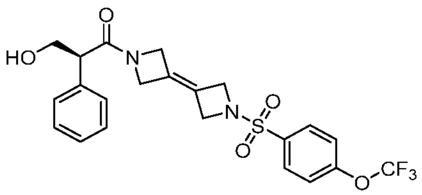 | 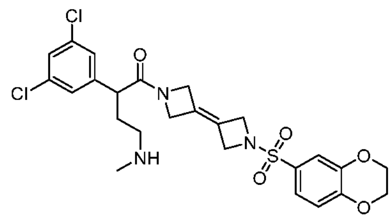 |
| 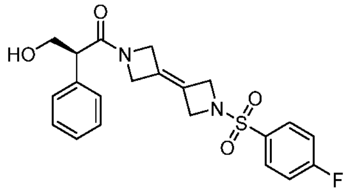 | 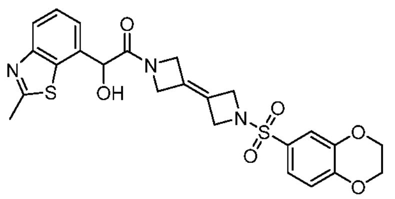 |
| 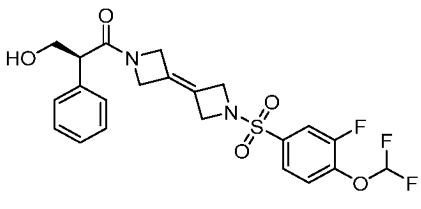 | 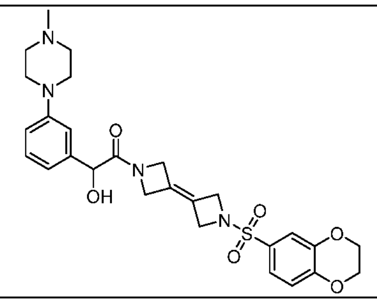 |

| | |
|---|---|
| 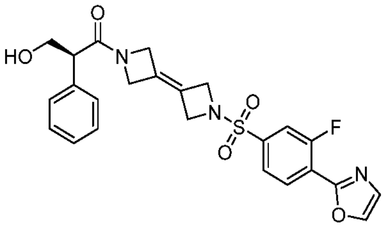 | 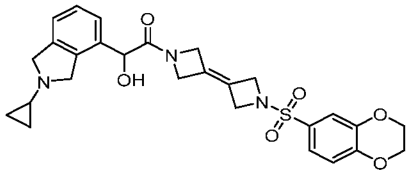 |
| 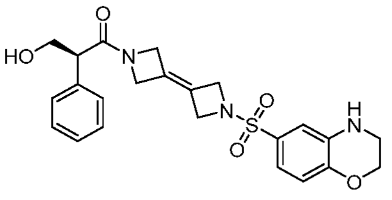 | 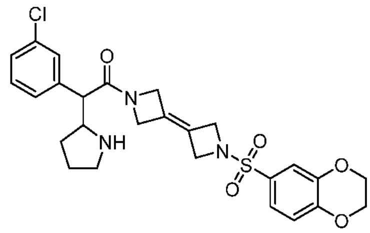 |
| 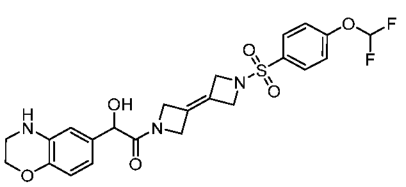 | 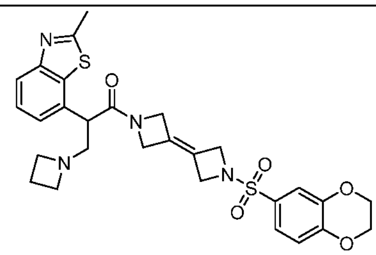 |
| 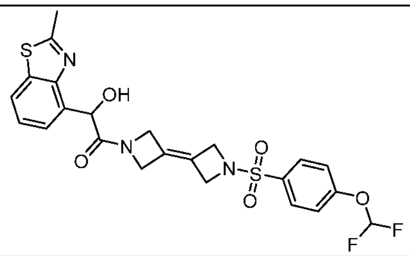 | 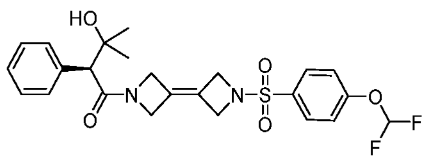 |
| 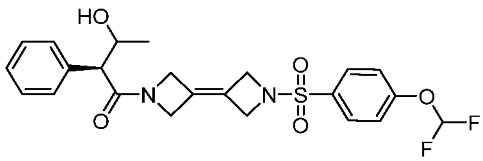 | 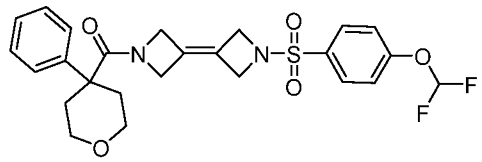 |
| 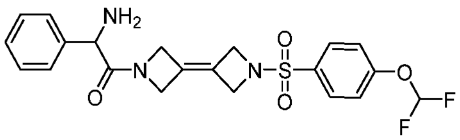 | 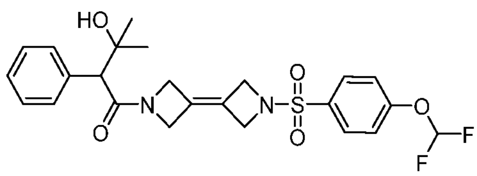 |
| 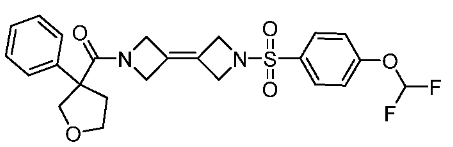 | 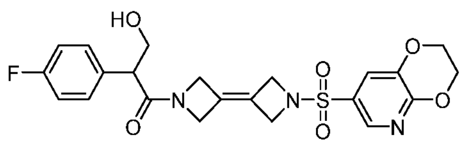 |
| 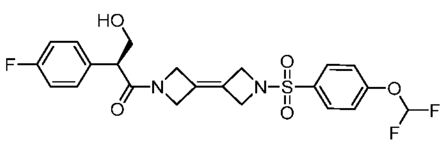 | 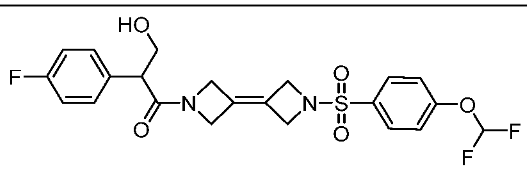 |

| | |
|---|---|
| 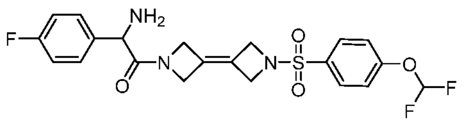 | 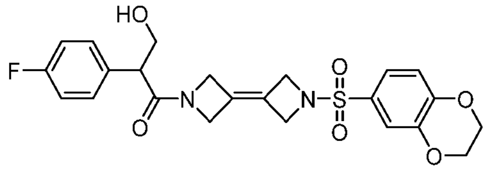 |
| 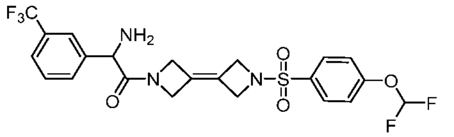 | 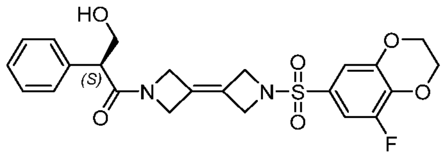 |

Preferably, the present invention further provides the compounds shown in the following table or isomers pharmaceutically acceptable salts or solvates thereof:

| | |
|---|---|
| 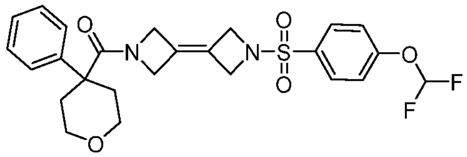 | 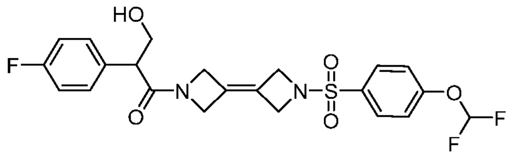 |
| 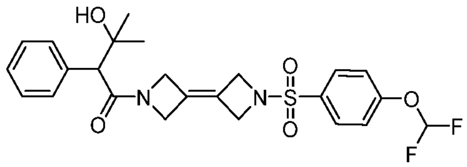 | 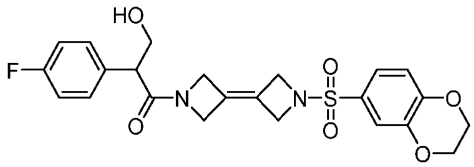 |
| 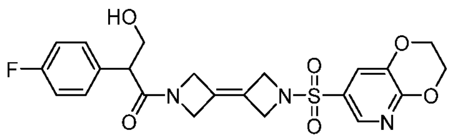 | 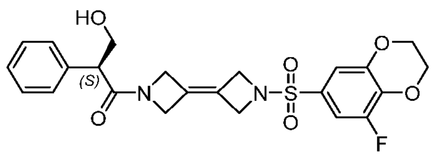 |

Preferably, the present invention further provides the compounds shown in the following table or isomers pharmaceutically acceptable salts or solvates thereof:

| | | |
|---|---|---|
| 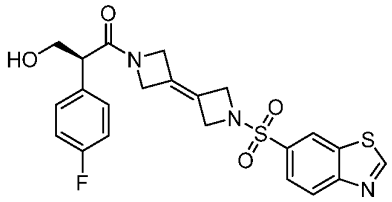 | 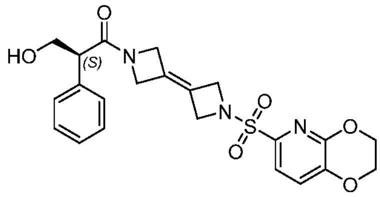 | 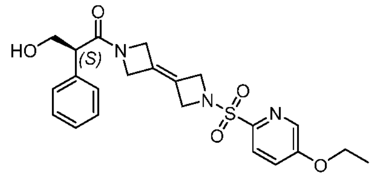 |
| 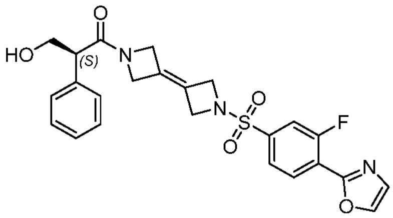 | 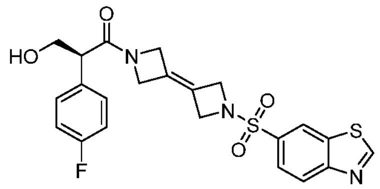 | 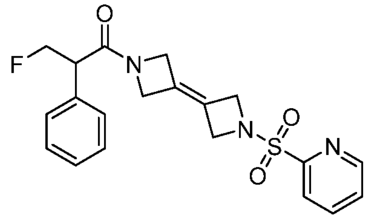 |
| 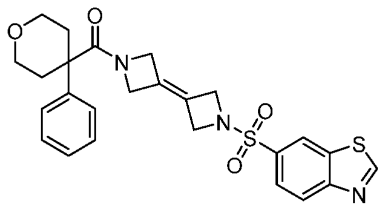 | 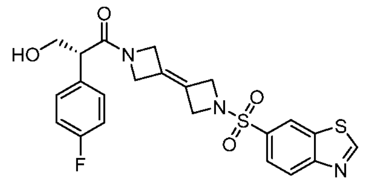 | 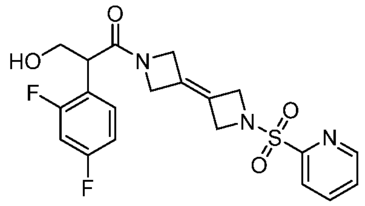 |

| | | |
|---|---|---|
| 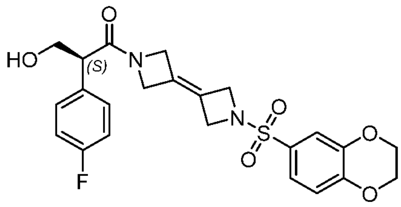 | 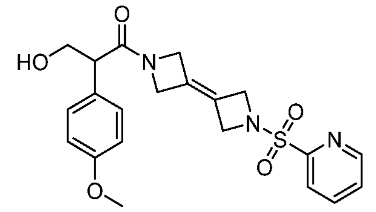 | 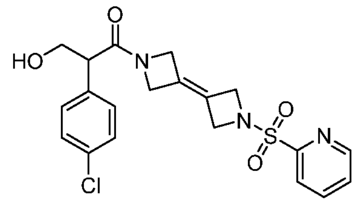 |
| 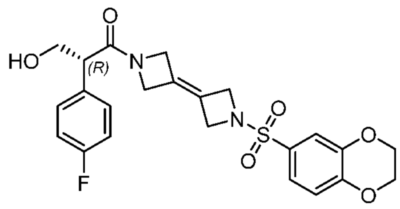 | 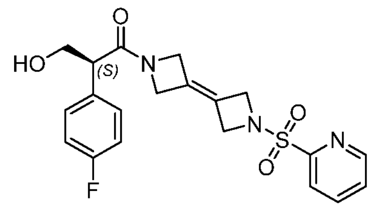 | 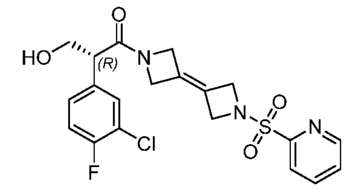 |
| 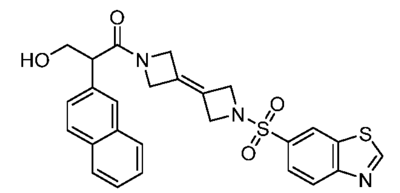 | 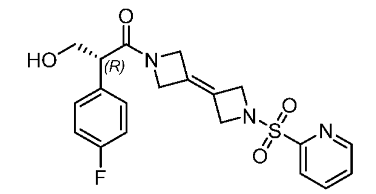 | 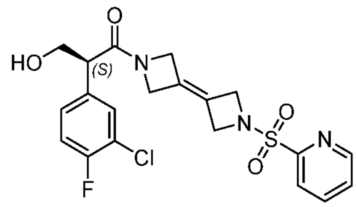 |
| 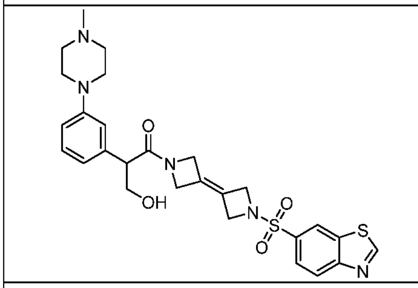 | 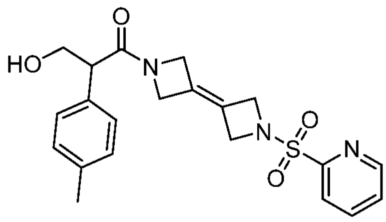 | 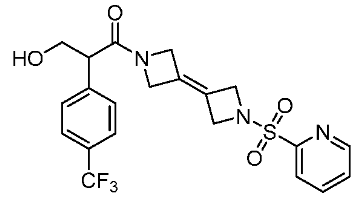 |
| 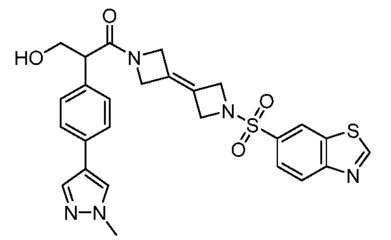 | 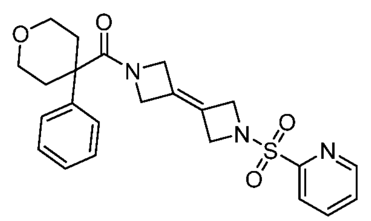 | 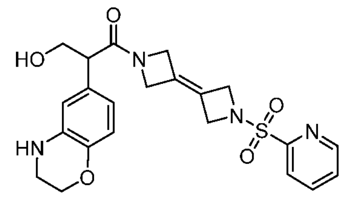 |
| 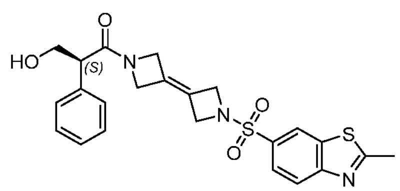 | 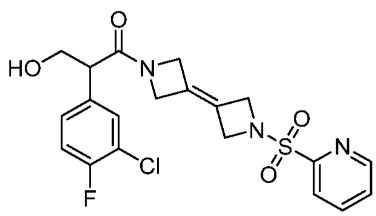 | 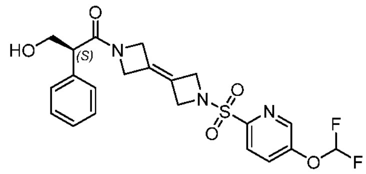 |
| 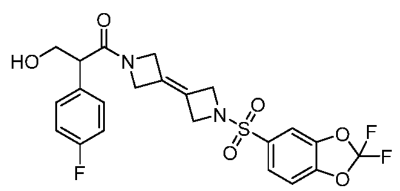 | 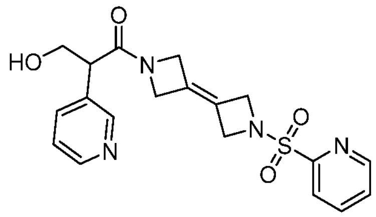 | 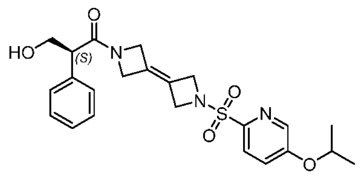 |

| | | |
|---|---|---|
| 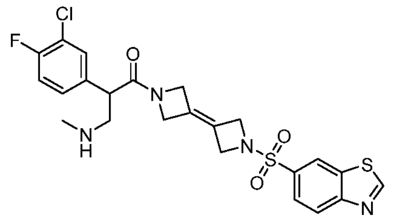 | 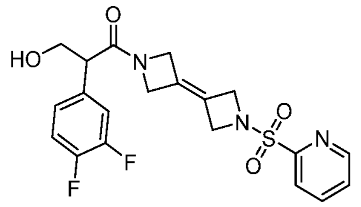 | 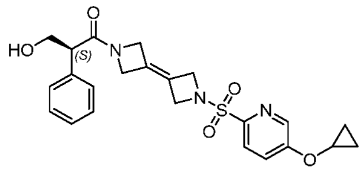 |
| 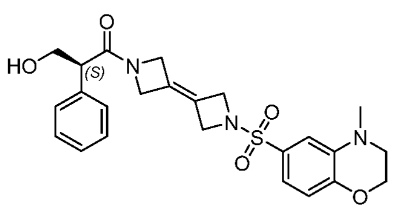 | 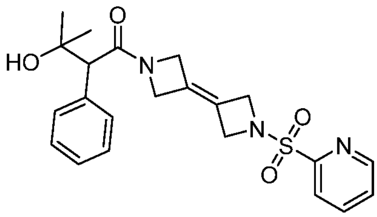 | 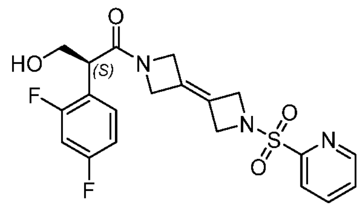 |
| 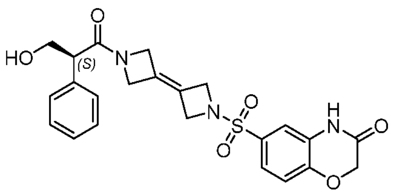 | 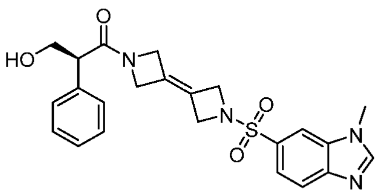 | 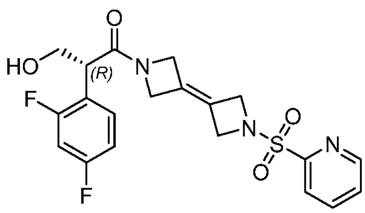 |
| 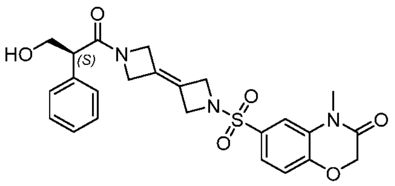 | 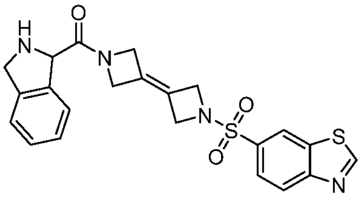 | 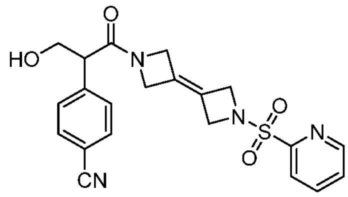 |
| 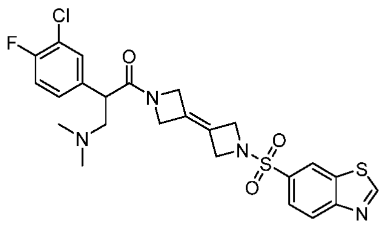 | 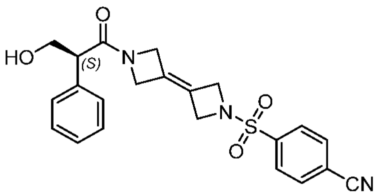 | 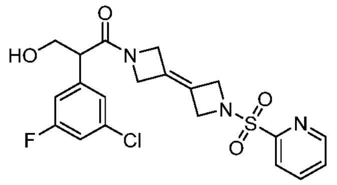 |
| 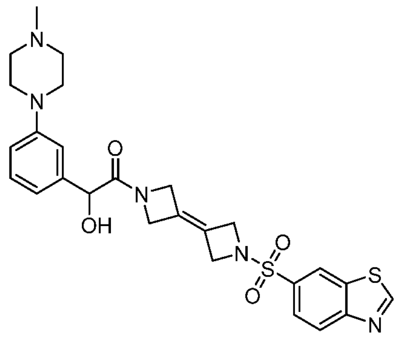 | 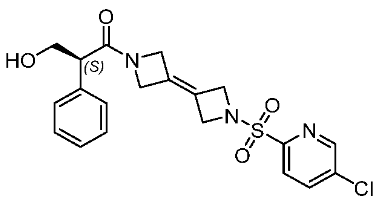 | 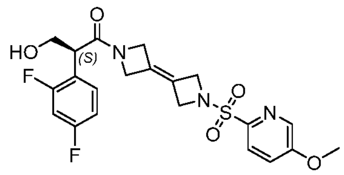 |
| 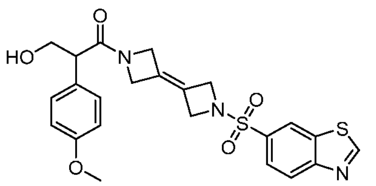 | 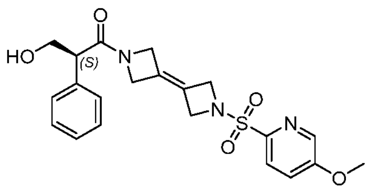 | 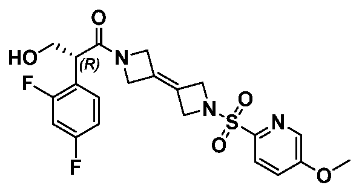 |

| | | |
|---|---|---|
| 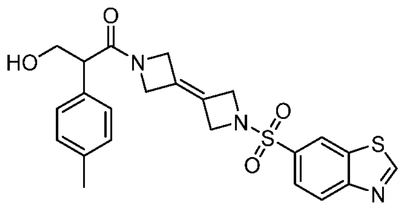 | 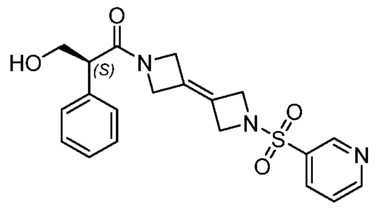 | 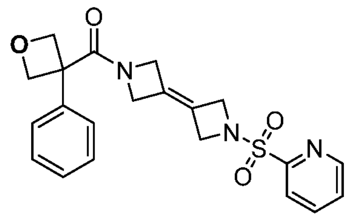 |
| 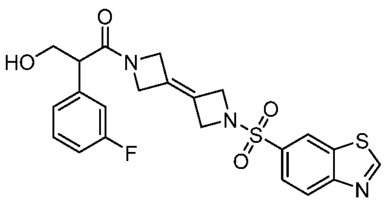 | 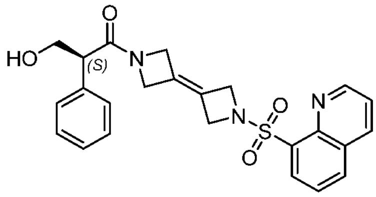 | 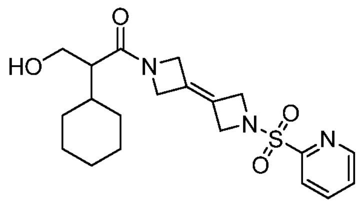 |
| 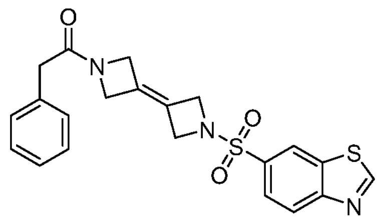 | 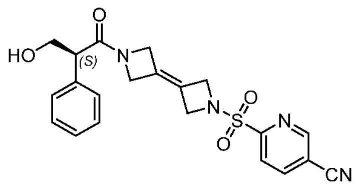 | |
| 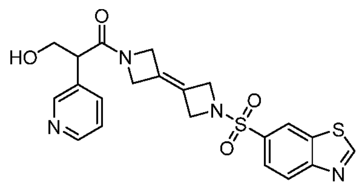 | 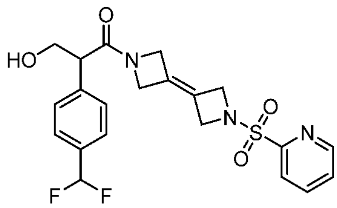 | |
| 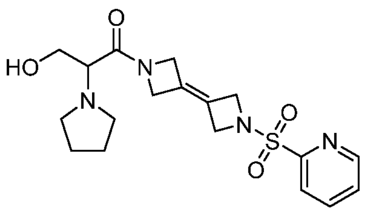 | 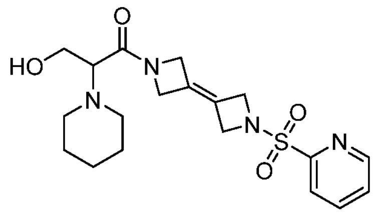 | 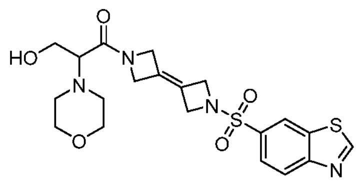 |
| 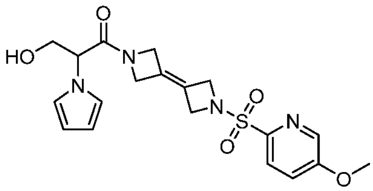 | 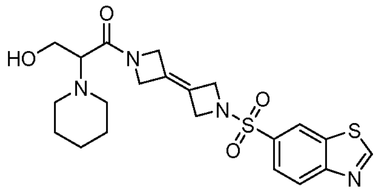 | 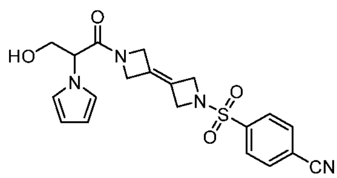 |
| 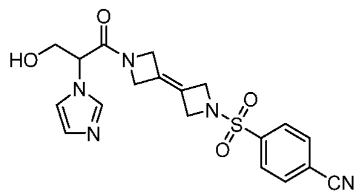 | 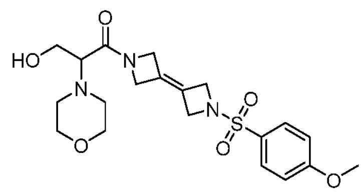 | 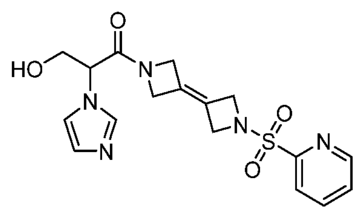 |

On the basis of the above, the present invention further provides a compound of formula II, or an isomer, pharmaceutically acceptable salt or solvate thereof:

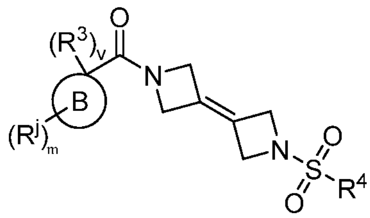

(II)

in the formula II:

$R^3$ and $R^4$ are defined as any corresponding $R^3$ and $R^4$ of the aforementioned compound of formula I, I-1 or I-2;
each $R^j$ is independently -H, halogen, -OH, $-NH_2$, -CN, =O, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$ or -NHS(O)OH, -C(O)H or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: -H, =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$ or -NHS(O)OH, -C(O)H, -C(O)OH, 3-14 membered heterocyclyl containing 1-4 heteroatoms independently selected from O, N and S, $C_6-C_{14}$ aryl, or 5-14 membered heteroaryl containing 1-4 heteroatoms independently selected from O, N and S;
m is independently an integer selected from 0-6;
v is 0 or 1;
ring B is $(C_3-C_8)$cycloalkyl, $(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S.

In some other specific embodiments,

each $R^j$ is independently -H, halogen, -OH, $-NH_2$, -CN, =O, $-(C_1-C_6)$alkyl, $-(C_1-C_6)$alkoxy, $-(C_1-C_6)$alkyl substituted by any number of halogen, $-(C_1-C_6)$alkoxy substituted by any number of halogen, oxazolyl, thiazolyl, and triazolyl;
the ring B is $(C_3-C_8)$cycloalkyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered bicyclic or tricyclic fused ring group containing 0-4 heteroatoms independently selected from O, N and S.

In some other specific embodiments,
the ring B is 3-7 membered oxacycloalkyl or 8-10 membered azabicyclic fused heteroaryl. Alternatively, the ring B is oxacycloheptane, oxacyclohexane, tetrahydrofuran, oxetane, oxacyclopropane, 1H-indole or isoindoline.

In some other specific embodiments, the ring B is

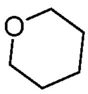 , 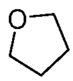 , 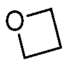 , 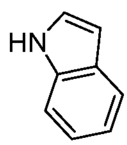 or 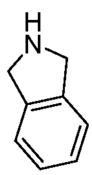 .

Preferably, the present invention provides the compounds shown in the following table, or isomers, pharmaceutically acceptable salts or solvates thereof:

| | |
|---|---|
| 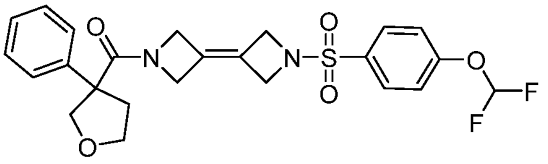 | 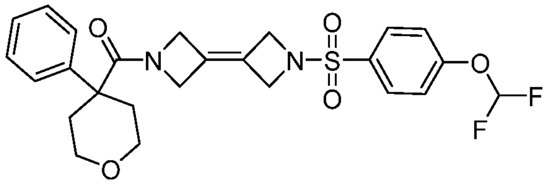 |
| 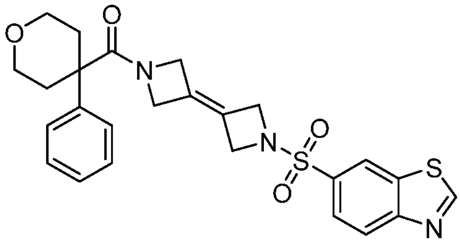 | 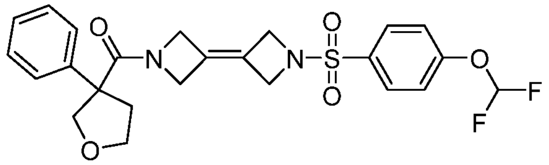 |
| 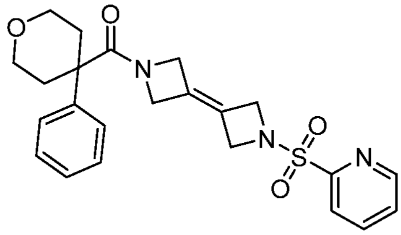 | 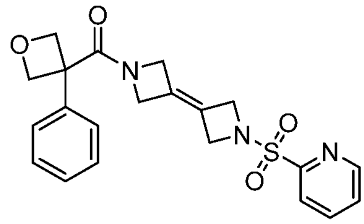 |
| 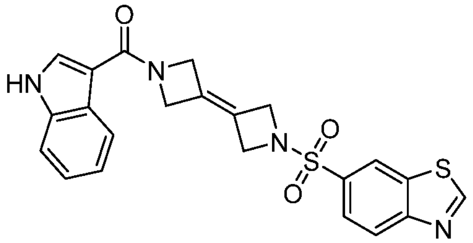 | 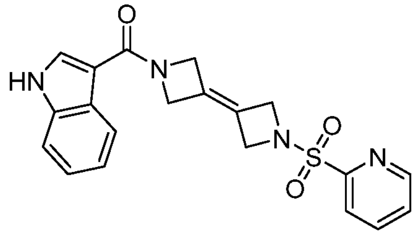 |
| 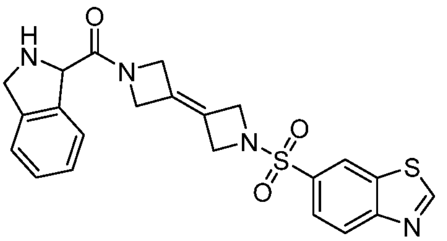 | |

On the basis of the above, the present invention further provides a deuterated compound of the aforementioned formula I or II, or an isomer, pharmaceutically acceptable salt or solvate thereof:

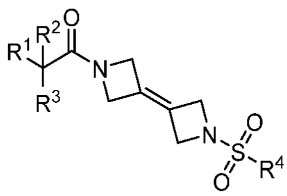 ( I ),

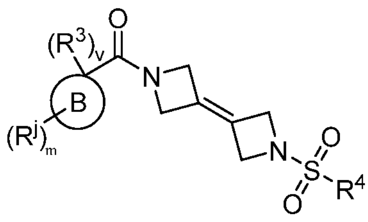

( II ),

wherein, $R^1$, $R^2$, $R^3$, $R^4$, $R^j$, m, v and the ring B are defined as any corresponding $R^1$, $R^2$, $R^3$, $R^4$, $R^j$, m, v and ring B in the aforementioned compound of formula I or II;

any one or more H in the compound of formula I or II are substituted by D.

In some other specific embodiments,
in the compound of formula I or II, any one or more H in the structure

“ 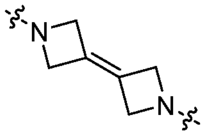 ”

are substituted by D, or any one or more H in $R^4$ are substituted by D. Alternatively, in the compound of formula I or II, all H in the structure

“ 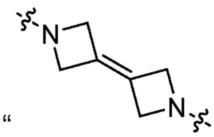 ”

are substituted by D.

In some other specific embodiments,
$R^1$, $R^2$ or $R^3$ is independently and optionally substituted by D when being selected as H. Preferably, the present invention provides the compounds shown in the following table, or isomers, pharmaceutically acceptable salts or solvates thereof:

| | |
|---|---|
| 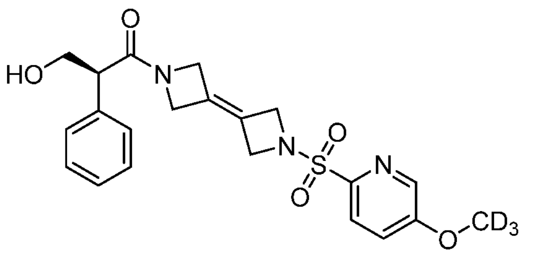 | 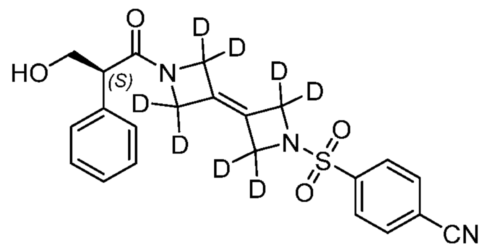 |
| 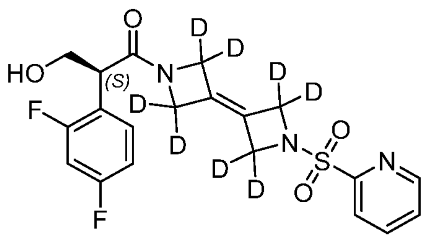 | 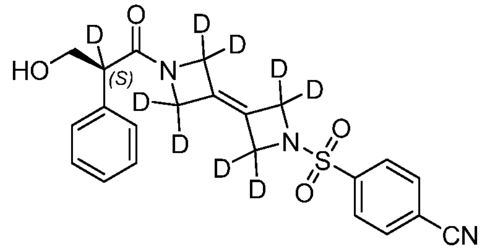 |

On the basis of the above, the present invention further provides a key intermediate of formula III for preparing the aforementioned compound of formula I or II:

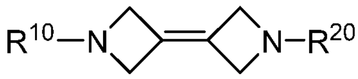 (III),

wherein,

$R^{10}$ is -H or an amino-protecting group, $R^{20}$ is -H, an amino-protecting group or

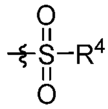 ,

$R^4$ is defined as any corresponding $R^4$ in the aforementioned compound of formula I or II;

or, $R^{10}$ is -H, an amino-protecting group,

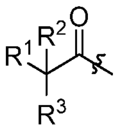 , 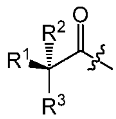 , 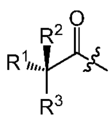 or 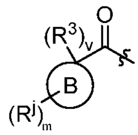 ,

wherein $R^1$, $R^2$ and $R^3$ are defined as any corresponding $R^1$, $R^2$ and $R^3$ in the aforementioned compound of formula I or II, m, v, $R^j$ and ring B are defined as any corresponding m, v, $R^j$ and ring B in the aforementioned compound of formula I or II, and $R^{20}$ is -H or an amino-protecting group.

In some other specific embodiments, each of the aforementioned amino-protecting groups is independently selected from -Cbz, -Boc, -Fmoc, -PMB, -Bn, -Trt, -Tos, -Pht, or -Alloc.

In some other specific embodiments, any one or more H in the compound of formula III are substituted by D.

Preferably, the present invention provides intermediate compounds shown in the following table:

| | |
|---|---|
| 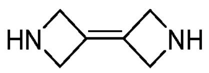 | 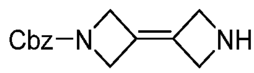 |
| 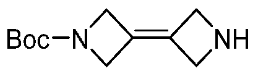 | 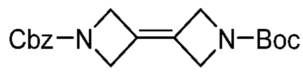 |
| 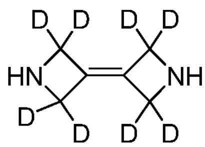 | 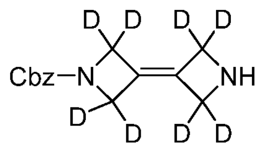 |
| 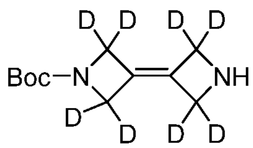 | 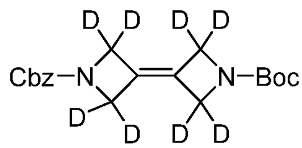 |

In the present invention, when the subsequent technical solution further limits the previous technical solution, it may only further limit some of the technical features. At this time, the undefined technical features can optionally have the definitions in the previous technical solutions or at anywhere they are occurred.

As mentioned above, the present invention provides a class of compounds with the structural features of general formula I. Research has found that the class of compounds can effectively stimulate PKR and/or inhibit USP9X kinase activity, thereby preventing or treating diseases related to PKR and/or USP9X and the like kinases. Specifically, the compounds of the present invention have high PKR agonist and/or USP9X inhibitory activities. Some other experimental

data show that the exposure of the compound of the present invention to rats through oral administration can reach 1700 h*ng/mL or more. Secondly, the present invention further provides a pharmaceutical composition comprising any one of the aforementioned compounds of the present invention. The pharmaceutical composition further comprises a pharmaceutically acceptable excipient. The pharmaceutical composition can be presented in any pharmaceutically acceptable dosage form and route of administration. Acceptable dosage forms include, but are not limited to, tablets, capsules, granules, pills, pastes, powder, tinctures, films, patches, liniment, implants, injections, lozenges, drops, sprays, aerosols, nebulized inhalers, gels, suppositories, ointments, etc. Acceptable routes of administration include, but are not limited to, oral, intravenous, rectal, parenteral, topical, transdermal, ocular, nasal, buccal or pulmonary (inhalation) administration, etc.

Thirdly, the present invention further provides use of the aforementioned compound or pharmaceutical composition of the present invention for preventing or treating a disease. The compound or pharmaceutical composition prevent or treat related diseases by stimulating PKR or inhibiting USP9X.

As a PKR agonist, activating PKR via administering an effective amount of the compound or pharmaceutical composition of the present invention helps to increase the lifespan of red blood cells in the patient's body, and helps to prevent or treat the conditions with the following manifestations: hereditary non-spherocytic hemolytic anemia, hemolytic anemia (e.g., chronic hemolytic anemia caused by deficiency of phosphoglycerate kinase), hereditary spherocytosis, hereditary elliptocytosis, abetalipoproteinemia (or Bassen-Kornzweig syndrome), paroxysmal nocturnal hemoglobinuria, acquired hemolytic anemia (e.g., congenital anemia (e.g., enzymopathy)), or anemia of chronic disease. In some embodiments, the disease or disorder is hereditary non-spherocytic hemolytic anemia. In some embodiments, the disease is hemolytic anemia (e.g., in patients diagnosed with PKD), SCD (e.g., sickle cell anemia), or thalassemia (e.g., β-thalassemia).

As a USP9X inhibitor, inhibiting USP9X via administering an effective amount of the compound or pharmaceutical composition of the present invention helps to prevent or treat USP9X-mediated related diseases, such as cancer or tumors, including but not limited to colorectal cancer, renal cancer, pancreatic cancer, breast cancer, lung cancer, esophageal cancer, melanoma, lymphoma, glioblastoma, or multiple myeloma, etc. Based on the corresponding application potential of the compound or pharmaceutical composition provided by the present invention as a PKR agonist and/or USP9X inhibitor, the present invention further provides pharmaceutical applications corresponding thereto, which can be applied to prepare drugs for treating corresponding diseases.

## DETAILED DESCRIPTION

Unless otherwise stated, the following terms used in the specification and claims have the following meanings.

"Alkyl" refers to an aliphatic hydrocarbon group, and refers to a saturated hydrocarbon group, which may be substituted or unsubstituted. The alkyl moiety may be a linear or branched alkyl. For example, it is $-(C_1-C_6)$alkyl or $-(C_1-C_3)$alkyl. The $-(C_1-C_6)$alkyl refers to an alkyl with 1 to 6 carbon atoms, for example an alkyl with 1 carbon atom, 2 carbon atoms, 3 carbon atoms, 4 carbon atoms, 5 carbon atoms, or 6 carbon atoms. Non-limiting examples of the alkyl include methyl, ethyl, n-propyl, isopropyl, n-butyl, isobutyl, sec-butyl, tert-butyl, n-pentyl, neopentyl, n-hexyl, and the like. The alkyl may be unsubstituted or substituted by one or more substituents, including but not limited to alkyl, alkoxy, cyano, hydroxyl, carbonyl, carboxyl, aryl, heteroaryl, amido, halogen, sulfonyl, sulfinyl, phosphonyl, etc.

"Alkenyl" refers to an unsaturated hydrocarbyl containing a carbon-carbon double bond in its structure, which may be substituted or unsubstituted. The alkenyl moiety may be a linear or branched alkenyl. For example, it is $-(C_2-C_6)$alkenyl or $-(C_2-C_4)$alkenyl. $-(C_2-C_6)$alkenyl refers to an alkenyl with 2 to 6 carbon atoms, for example an alkenyl with 2 carbon atoms, 3 carbon atoms, 4 carbon atoms, 5 carbon atoms, or 6 carbon atoms. Non-limiting examples of the alkenyl include vinyl, n-propenyl, isopropenyl, n-butenyl, isobutenyl, n-hexenyl, and the like. The alkenyl may be unsubstituted or substituted by one or more substituents, including but not limited to alkyl, alkoxy, cyano, hydroxyl, carbonyl, carboxyl, aryl, heteroaryl, amido, halogen, sulfonyl, sulfinyl, phosphonyl, etc.

"Alkynyl" refers to an unsaturated hydrocarbyl containing a carbon-carbon triple bond in its structure, which may be substituted or unsubstituted. The alkynyl moiety may be a linear or branched alkynyl. For example, it is $-(C_2-C_6)$alkynyl or $-(C_2-C_4)$alkynyl. $-(C_2-C_6)$alkynyl refers to an alkynyl with 2 to 6 carbon atoms, for example an alkynyl with 2 carbon atoms, 3 carbon atoms, 4 carbon atoms, 5 carbon atoms, or 6 carbon atoms. Non-limiting examples of the alkynyl include ethynyl, n-propynyl, isopropynyl, n-butynyl, isobutynyl, n-hexynyl, and the like. The alkynyl may be unsubstituted or substituted by one or more substituents, including but not limited to alkyl, alkoxy, cyano, hydroxyl, carbonyl, carboxyl, aryl, heteroaryl, amido, halogen, sulfonyl, sulfinyl, phosphonyl, etc.

"Ring" refers to any covalently closed structure, including, for example, a carbocycle (e.g., aryl, cycloalkyl, or cycloalkenyl), heterocyclyl (e.g., heteroaryl, heterocycloalkyl, or heterocycloalkenyl), an aromatic group (e.g., aryl or heteroaryl), and a non-aromatic group (e.g., cycloalkyl, heterocycloalkyl, cycloalkenyl, heterocycloalkenyl), which may be substituted or unsubstituted. The ring may be optionally substituted and may be monocyclic or polycyclic. Typical polycyclic rings generally include bicyclic rings and tricyclic rings. The ring of the present application usually has 3-20

ring atoms, for example 3, 4, 5, 6, 7, 8, 9, 10, 11, 12, 13, 14, 15, 16, 17, 18, 19 or 20 ring atoms.

The "membered" refers to the number of skeleton atoms constituting a ring. A typical 5-membered ring includes, for example, cyclopentyl, pyrrole, imidazole, thiazole, furan and thiophene, etc.; and a typical 6-membered ring includes, for example, cyclohexyl, cyclohexenyl, pyridine, pyran, pyrazine, thiopyran, pyridazine, pyrimidine, benzene, etc. A ring containing a heteroatom among skeleton atoms is a heterocyclic ring.

"Heteroatom" refers to an atom other than carbon or hydrogen. One or more heteroatoms in the heterocycle of the present application can be independently selected from O, S, N, Si and P, but are not limited thereto.

An "aromatic group" refers to a cyclic group with a conjugated π electron system, including an aryl in which the skeleton atoms constituting the ring are all carbon atoms and a heteroaryl in which the skeleton atoms constituting the ring contain a heteroatom. "Aryl" preferably refers to a monocyclic or fused polycyclic (i.e., rings sharing pairs of adjacent carbon atoms) group of 6 to 14 carbon atoms (6 to 14 membered) having a conjugated π electron system, preferably having 6 to 10 atoms, such as phenyl and naphthyl, which may be substituted or unsubstituted. More preferred is phenyl. "Heteroaryl" preferably refers to a heteroaromatic system containing 1 to 4 (e.g. 1, 2, 3 or 4) heteroatoms, and 5 to 14 (e.g. 5, 6, 7, 8, 9, 10, 11, 12, 13, or 14) ring atoms, wherein the heteroatom is selected from oxygen, sulfur and nitrogen, and the heteroaryl may be substituted or unsubstituted. The heteroaryl preferably is 5 to 10 membered and contains 1 to 3 heteroatoms; more preferably is 5 or 6 membered and contains 1 to 2 heteroatoms; and preferably, for example, imidazolyl, furanyl, thienyl, thiazolyl, pyrazolyl, oxazolyl, pyrrolyl, triazolyl, tetrazolyl, pyridinyl, pyranyl, thiopyranyl, pyrimidinyl, thiadiazole, pyrazinyl, triazinyl, etc., preferably imidazolyl, thiazolyl, pyrazolyl or pyrimidinyl, thiazolyl; and more preferably pyrazolyl such as 1H-pyrazol-4-yl or thiazolyl. The heteroaryl ring can be fused to an aryl, heterocycloalkyl, cycloalkyl ring, or another heteroaryl to form a fused heteroaryl. The fused heteroaryl is preferably an 8-10 membered fused heteroaryl, including but not limited to: indolyl for example 1H-indol-5-yl, 2-oxo-2,3-dihydro-1H-benzo[d]imidazolyl for example 2-oxo-2,3-dihydro-1H-benzo[d]imidazol-5-yl, or 1H-benzo[d]imidazolyl for example 1H-benzo[d]imidazol-6-yl.

A "non-aromatic group" refers to a cyclic group that does not have a conjugated π electron system and is not aromatic. It is also generally called an alicyclic group, i.e., an alicyclic radical, including a cycloalkyl or cycloalkenyl in which skeleton atoms constituting the ring are all carbon atoms, and heterocycloalkyl or heterocycloalkenyl in which skeleton atoms constituting the ring contain heteroatoms.

The "fused" refers to rings that share adjacent pairs of carbon atoms, and typically is bicyclic or tricyclic fused.

A "fused ring group" includes a "fused non-aromatic group" and a "fused aromatic group", wherein the "fused non-aromatic group" includes a "fused cycloalkyl", a "fused heterocycloalkyl", a "fused cycloalkenyl" and a "fused heterocycloalkenyl", and the "fused aromatic group" includes a "fused aryl" and a "fused heteroaryl", which may be all substituted or unsubstituted.

The "cycloalkyl" refers to a saturated cyclic hydrocarbon substituent containing 1-3 rings, including monocycloalkyl, bicycloalkyl and tricycloalkyl, which contains 3-20 carbon atoms that can form a ring, preferably 3-10 carbon atoms (i.e., 3-10 membered cycloalkyl, also known as -($C_3$-$C_{10}$)cycloalkyl), e.g., 3 to 8, 3 to 7, 3 to 6, 5 to 6 carbon atoms. Preferably, the cycloalkyl is selected from a monovalent cycloalkyl derived from the following rings:

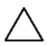 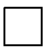 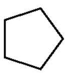 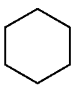 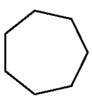 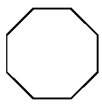 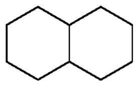 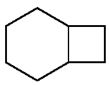 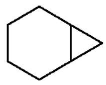 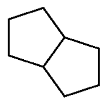 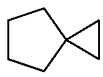 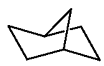 ,

preferably cyclopropyl, cyclobutyl, cyclopentyl, cyclohexyl, cycloheptyl or cyclooctyl. The cycloalkyl may be substituted or unsubstituted.

It should be understood that when a cycloalkyl is linked to two groups according to a structure or context, the cycloalkyl is a divalent group, i.e., having two linking sites. At this time, it may also be named as a cycloalkylene group. Examples of preferred cycloalkylene groups include, but are not limited to, monocyclic structures, such as cyclopropylene, cyclobutylene, cyclopentylene (e.g. cyclopent-1,2-diyl, cyclopent-1, 3-diyl), cyclohexylene (e.g. cyclohex-1,2-diyl, cyclohex-1,3-diyl, cyclohex-1,4-diyl), cycloheptanylene or cycloctanylene, etc.

"Heterocycloalkyl" and "cycloheteroalkyl" are used interchangeably and refer to a saturated and nonaromatic monocyclic ring, fused ring, bridged ring or spiro ring containing one or more (e.g., 1, 2, 3, or 4) heteroatoms. The heteroatom may be N, O, S or $SO_2$

( 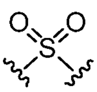 ),

and preferably N, O and/or S. The heterocycloalkyl may be a 3 to 10 membered (e.g., 3, 4, 5, 6, 7, 8, 9, or 10 membered, i.e., including 3, 4, 5, 6, 7, 8, 9 or 10 ring atoms) monocyclic, bicyclic or tricyclic group. A typical heterocycloalkyl includes, but is not limited to, monovalent groups derived from the following rings:

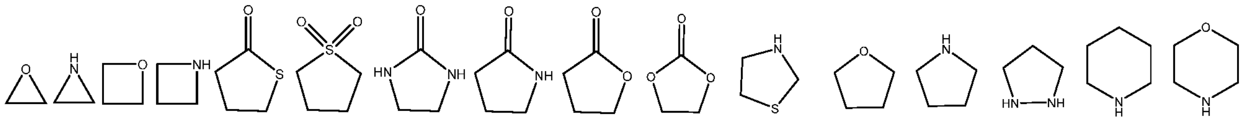

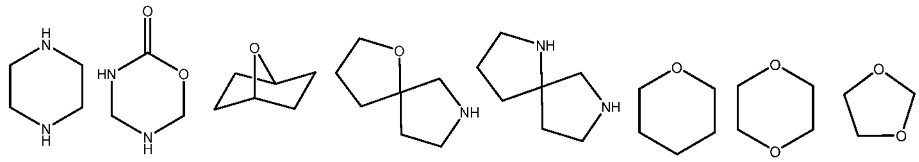
.

These heterocycloalkyl groups can also be represented by commonly understood structural formulae, for example:

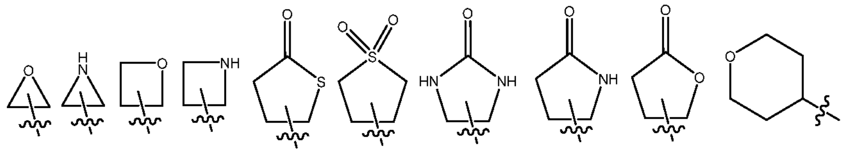
.

It should be understood that when a heterocycloalkyl is linked to two groups according to a structure or context, the heterocycloalkyl is a divalent group, i.e., having two linking sites. At this time, it may also be named as a heterocycloalkylene group. Examples of the heterocycloalkylene group include, but are not limited to, divalent groups formed from the aforementioned groups, for example:

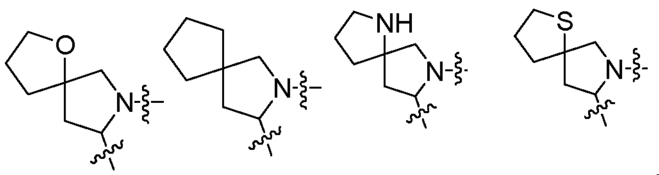
.

"Cycloalkenyl" refers to a cyclic hydrocarbon substituent including 1-3 rings, which contains one or more double bonds, but no ring of which has a completely conjugated $\pi$ electron system and which is not aromatic, including monocycloalkenyl, bicycloalkenyl and tricycloalkenyl, which contain 3-20 carbon atoms that can form a ring, preferably 3-10 carbon atoms (i.e., 3-10 membered cycloalkenyl, also known as -($C_3$-$C_{10}$)cycloalkenyl), for example, 3 to 8, 3 to 7, 3 to 6, or 5 to 6 carbon atoms. Preferably, the cycloalkenyl is selected from a monovalent cycloalkenyl derived from the following rings:

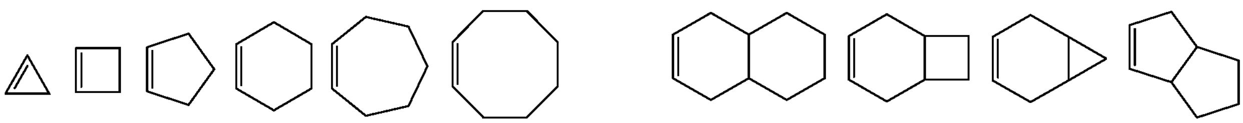

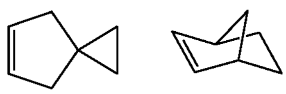
,

preferably cyclopropenyl, cyclopentenyl, and cyclohexenyl. The cycloalkenyl may be substituted or unsubstituted. It should be understood that when a cycloalkenyl is linked to two groups according to a structure or context, the cycloalkenyl is a divalent group, i.e., having two linking sites. At this time, it may also be named as a cycloalkenylene group.

"Heterocycloalkenyl" refers to an unsaturated and nonaromatic monocyclic ring, fused ring, bridged ring or spiro ring containing one or more (e.g., 1, 2, 3, or 4) heteroatoms. The heteroatom may be N, O, S or $SO_2$

( 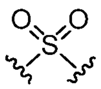 ),

and preferably N, O and/or S. The heterocycloalkenyl can be a 3 to 10 membered (e.g., 3, 4, 5, 6, 7, 8, 9, or 10 membered, i.e., including 3, 4, 5, 6, 7, 8, 9 or 10 ring atoms) monocyclic, bicyclic or tricyclic group. Typical heterocycloalkenyl groups include, but are not limited to, monovalent groups derived from the following rings:

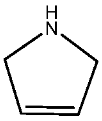 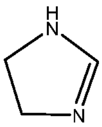 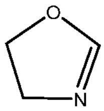 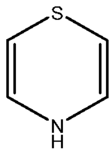 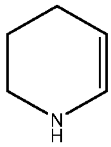 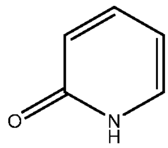 .

These heterocycloalkenyl groups can also be represented by commonly understood structural formulae, for example:

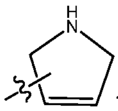 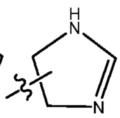 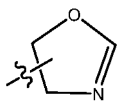 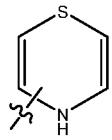 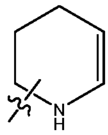 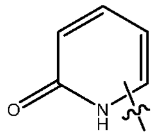 .

It should be understood that when a heterocycloalkenyl is linked to two groups according to a structure or context, the heterocycloalkenyl is a divalent group, i.e., having two linking sites. At this time, it may also be named as a heterocycloalkenylene group.

"Oxo" refers to that a hydrogen on the carbon is replaced by =O.

"Halogen" or "halo" refers to fluorine, chlorine, bromine or iodine.

"Haloalkyl" refers to an alkyl in which at least one hydrogen is replaced by a halogen atom, for example $CF_3$.

The "substituted" means that one or more hydrogen atoms, preferably up to 5 (e.g., 1, 2, 3, 4, or 5), more preferably 1-3 hydrogen atoms in a group can be independently substituted by corresponding number of substituents from each other. It goes without saying that, the substituents are only in their possible chemical positions, and those of skills in the art are able to determine (either experimentally or theoretically) possible or impossible substitutions without undue effort. For example, an amino or hydroxyl group with a free hydrogen may be unstable when binds with a carbon atom with an unsaturated (e.g. olefinic) bond.

"Inhibitor" refers to a substance that reduces enzyme activity.

"Agonist" refers to a substance that increases enzyme activity.

"Optionally" means that the subsequently described event or environment can but not necessarily occur, indicating that the event or environment occurs or doesn't occur.

The term "substituted or unsubstituted" herein means that any group is mono- or poly-substituted by a specified substituent to the extent that such mono- or poly-substitution (including multiple substitutions on the same moiety) is chemically permissible, wherein each substituent may be located at any available position on the group and may be linked through any available atom on the substituent. "Any available position" refers to any position on a group that is chemically accessible by methods known in the art or methods taught herein and does not produce an unduly unstable molecule. When there are two or more substituents on any group, each substituent is defined independently of any other substituent and therefore may be the same or different.

As for "stereoisomerism", the "stereoisomer" described in the present invention refers to that when a compound of the present invention contains one or more asymmetric centers, the compound can be existed in forms of racemates, racemic mixtures, single enantiomer, diastereomeric mixtures and single diastereoisomer. The compound of the present invention may have an asymmetric center, and thus results in the presence of two optical isomers. The scope of the present invention encompasses all possible optical isomers and mixtures thereof. If the compound of the present invention contains an olefin double bond, the scope of the present invention includes *cis* and *trans* isomers, unless otherwise specified. The compound of the present invention may be existed in forms of tautomers (one of functional group isomers) that have different linking sites of hydrogens through shifts of one or more double bonds. For example, a ketone and an enol form thereof are keto-enol tautomers. Each tautomer and mixtures thereof are within the scope of the present invention. Enantiomers, diastereomers, racemates, mesomers, cis-trans isomers, tautomers, geometric isomers, epimers and mixtures of all compounds are all within the scope of the present invention.

The term "compound of the present invention" used herein is intended to encompass compounds of general formulae (I) and (II) as defined herein or any preferred or specific embodiment thereof (including compounds of formulae (I-1) and (I-2) and the like, and example compounds), and stereoisomers, pharmaceutically acceptable salts, tautomers or solvates thereof.

The term "pharmaceutically acceptable" used herein means molecular entities and compositions that are approved by or approvable by respective authorities in various countries, or listed in a generally recognized pharmacopoeia for use in animals and more particularly human, or do not produce adverse, allergic or other adverse responses when being administered in appropriate amount to animals, such as human.

The term "pharmaceutically acceptable salt" used herein means a salt of a compound of the present invention that is pharmaceutically acceptable and possesses the desired pharmacological activity of the parent compound. Specifically, such salts are non-toxic and can be inorganic or organic acid addition salts and alkali addition salts.

The term "individual" used herein includes human or non-human animals. Exemplary human individuals include human individuals (referred to as patients) suffering from a disease (e.g., disease described herein) or normal individuals. In the present invention, "non-human animals" include all vertebrates, e.g., non-mammals (e.g., birds, amphibians, reptiles) and mammals, e.g. non-human primates, livestocks and/or domesticated animals (e.g., sheep, dogs, cats, cows, pigs, etc.).

"Pharmaceutical composition" mentioned in the present invention refers to a composition including one or more compounds of formula (I), formula (I-1), formula (I-2) or formula (II) or stereoisomers, tautomers, pharmaceutically acceptable salts or solvates thereof, and carriers or excipients generally accepted in the art for delivering the biologically active compound to an organism (e.g., human).

The term "pharmaceutical combination" used herein refers to that the compound of the present invention may be combined with other active agents for achieving the purposes of the present invention. The other active agents may be one or more additional compounds of the present invention, or may be a second or additional (e.g., a third) compound that is compatible with the compound of the present invention, i.e., does not adversely affect each other, or has complementary activity. Such active agents are suitably present in combination in an amount effective to achieve the intended purpose. The other active agents may be co-administered with the compound of the present invention in a single pharmaceutical composition, or may be administered separately from the compound of the present invention in different discrete units, and may be administered simultaneously or sequentially when being administered separately. The sequential administrations may be close or distant in time.

It should be understood that when selecting each group in the structure of the compound of the present invention, the groups that have connection, coordination or influence with each other should be selected accordingly on the premise of complying with the rules of chemical valence bonding.

According to the content of the present invention, other modifications, substitutions or changes in various forms can also be made according to the common technical knowledge and means in the art, without departing from the aforementioned basic technical concept of the present invention.

## DETAILED DESCRIPTION

In order to further illustrate the present invention, the active compounds provided by the present invention and preparation methods and use thereof are described in details hereafter in conjunction with examples.

The following abbreviations or terms have the following meanings:

Grubb’s 2 represents a Grubbs second generation catalyst;
DMF represents N,N-dimethylformamide;
DMSO represents dimethyl sulfoxide;
HATU represents 2-(7-azabenzotriazole)-N,N,N’,N’-tetramethyluronium hexafluorophosphate;
DIPEA represents N,N-diisopropylethylamine;
$Pd_2(dba)_3$ represents tris(dibenzylideneacetone)dipalladium;
Xantphos represents 4,5-bis(diphenylphosphino)-9,9-dimethylxanthene;
-Cbz represents a benzyloxycarbonyl protecting group;
-Boc represents a tert-butoxycarbonyl protecting group;
-Fmoc represents a 9-fluorenylmethoxycarbonyl protecting group;
-PMB represents a p-methoxybenzyl protecting group;
-Bn represents a benzyl protecting group;
-Trt represents a trityl protecting group;
-Tos represents a tosyl protecting group;
-Pht represents a phthalyl protecting group;
-Alloc represents an allyloxycarbonyl protecting group;

Tris buffer represents a tris(hydroxymethyl)aminomethane buffer;
-TBS represents tert-butyldimethylsilyl;
-TBDPS represents tert-butyldiphenylsilyl.

This patent further provides a synthesis method of the aforementioned compound. The synthesis method of the present invention is mainly based on the preparation method reported in the chemical literatures or using commercially available chemical reagents as starting materials for related synthesis.

Method:

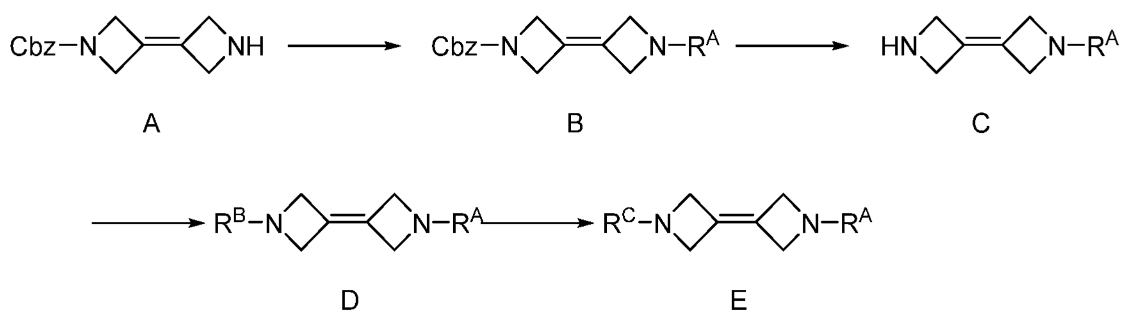

A B C D E

In the aforementioned reaction formula, "$R^A$" refers to

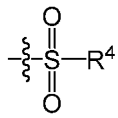
,

wherein $R^4$ is as defined wherever it appears in the present invention; "$R^C$" refers to

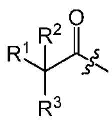 , 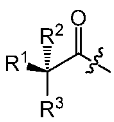 , 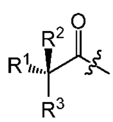 or

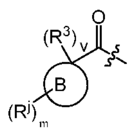
,

wherein $R^1$, $R^2$, $R^3$, $R^j$, m, v and ring B are as defined wherever they appear in the present invention; "$R^B$" refers to $R^C$ protected by a protecting group, $R^B$ becomes $R^C$ after the protecting group is removed. The protecting group includes but is not limited to -TBS or -TBDPS.

The following steps involve a compound Z-$R^A$ and a compound Z-$R^B$, wherein "Z" is a group capable of undergoing a condensation reaction with an amino group to connect $R^A$ or $R^B$ onto

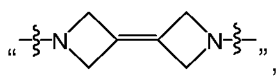
,

and the condensation reaction is preferably a condensation reaction between sulfonyl chloride and an amino group or a condensation reaction between a carboxyl group and an amino group, "Z" can be independently selected implementing groups in different compounds, including but not limited to -Cl, -F, -Br, -OH. The compound Z-$R^A$ is preferably Cl-$R^A$, and the compound Z-$R^B$ is preferably OH-$R^B$.

Step 1:

**Compound A** is dissolved in a tetrahydrofuran solution, then added with triethylamine and a compound Z-$R^A$ to react at 25°C. After the reaction is completed, the product is extracted with ethyl acetate for three times, and the organic phases are combined, dried over anhydrous sodium sulfate, filtered, concentrated and then separated and purified through a chromatography column to obtain **compound B.**

Step 2:

The **compound B** is dissolved in trifluoroacetic acid to react at 60°C. After the reaction is completed, the product is concentrated to dryness and then separated and purified through a chromatography column to obtain **compound C.**

Step 3:

The **compound C** is dissolved in a DMF solution, and then sequentially added with a compound Z-$R^B$, HATU and DIPEA to conduct a condensation reaction at 25°C. After the reaction was completed, the reaction solution was poured into water and extracted with ethyl acetate for three times, and the organic phases are combined, dried over anhydrous sodium sulfate, filtered, concentrated, and then separated and purified through a chromatography column to obtain **compound D.**

Step 4:

The **compound D** is dissolved in a tetrahydrofuran solution, and then added with tetrabutylammonium fluoride to conduct a deprotection reaction to finally obtain a **compound E.**

**Example** 1: preparation of (*S*)-1-(1'-((2,3-dihydro-[1,4]dioxino[2,3-*b*]pyridin-7-yl)sulfonyl)-1',4'-dihydro-2*H*,2' *H*-[3,3'-diazetidinylidene]-1(4*H*)-yl)-3-hydroxy-2-phenylpropan-1-one **(compound 1)** The synthesis steps were as follows:

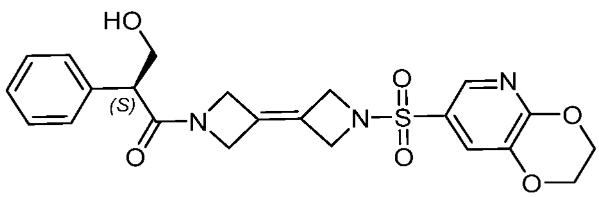

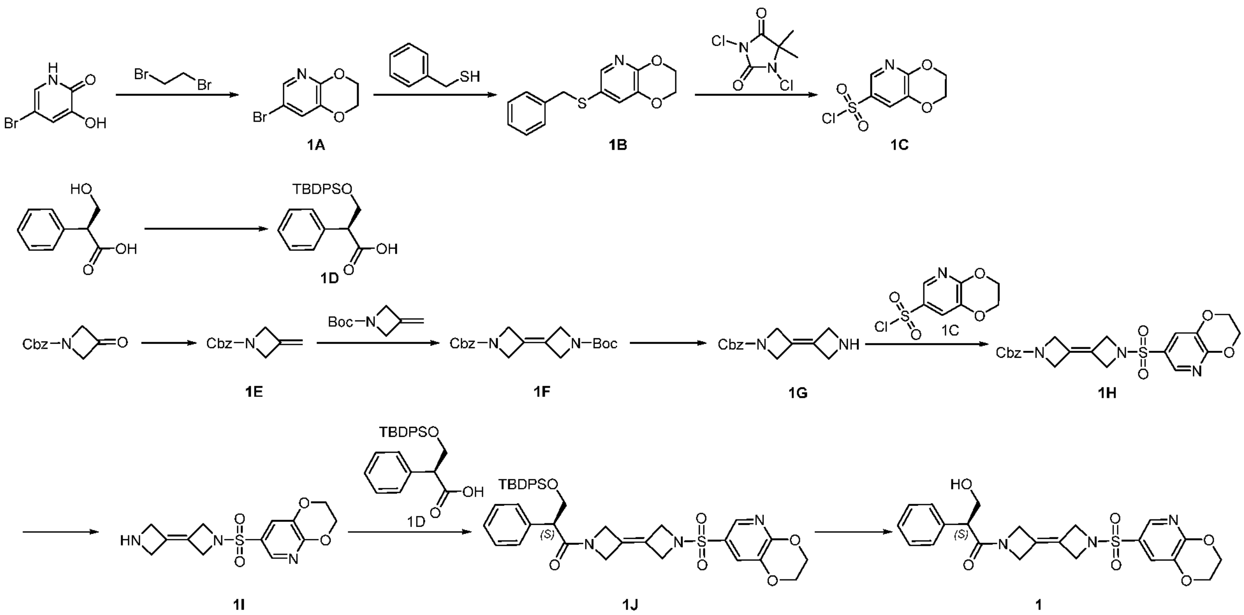

1A 1B 1C 1D 1E 1F 1G 1H 1I 1J 1

Step 1: preparation of 7-bromo-2,3-dihydro-[1,4]dioxino[2,3-b]pyridine **(compound 1A)**

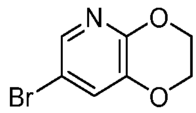

**1A**

5-bromopyridine-2,3-diol (13.0 g, 68.4 mmol) was dissolved in DMF (130 mL), and then added with 1,2-dibromoethane (19.3 g, 102.6 mmol) and potassium carbonate (28.3 g, 205 mmol), and the reaction system was stirred at 100°C overnight, until the reaction was complete. The reaction solution was concentrated to dryness under reduced pressure, and the residue was separated and purified through a silica gel column to obtain **compound 1A.** MS (ESI) m/z 216.0 $(M+H)^+$.

**Step 2:** preparation of 7-(benzylthio)-2,3-dihydro-[1,4]dioxino[2,3-*b*]pyridine **(compound 1B)**

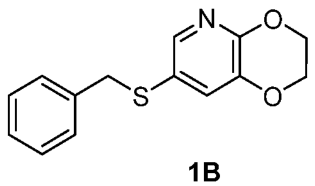

**1B**

Under the protection of argon, 7-bromo-2,3-dihydro-[1 ,4]dioxino[2,3-*b*]pyridine (1 .0 g, 4.6 mmol) was dissolved in 1,4-dioxane solution (20 mL), and then added with benzyl mercaptan (570 mg, 4.6 mmol), $Pd_2(dba)_3$ (240 mg, 0.23 mmol), Xantphos (270 mg, 0.46 mmol) and DIPEA (1,190 mg, 9.3 mmol). The reaction system was stirred at 80°C overnight. After the reaction was complete, the reaction solution was concentrated to dryness under reduced pressure, and the residue was separated and purified through a silica gel column to obtain **compound 1B.**
MS (ESI) m/z 260.1 $(M+H)^+$.

**Step 3:** preparation of 2,3-dihydro-[1,4]dioxino[2,3-*b*]pyridine-7-sulfonyl chloride **(compound 1C)**

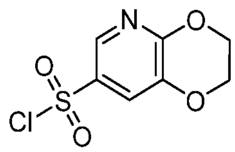

**1C**

7-(benzylthio)-2,3-dihydro-[1,4]dioxino[2,3-*b*]pyridine (800 mg, 3 mmol) and 1,3-dichloro-5,5-dimethylhydantoin (820 mg, 6 mmol) were dissolved in a mixed solution of acetonitrile (30 mL), acetic acid (0.75 mL), and water (0.5 mL). The reaction system was stirred at 25°C overnight. After the reaction was complete, the reaction solution was concentrated to dryness under reduced pressure, and the residue was separated and purified through a silica gel column to obtain **compound** 1C.
MS (ESI) m/z 236.0 $(M+H)^+$.

**Step 4:** preparation of (*S*)-3-((tert-butyldiphenylsilyl)oxy)-2-phenylpropionic acid **(compound 1D)**

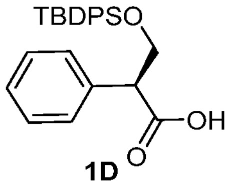

**1D**

(*S*)-3-hydroxy-2-phenylpropionic acid (900 mg, 5.4 mmol) and 4-dimethylaminopyridine (1.32 g, 10.8 mmol) were dissolved in dichloromethane (10 mL), and then added with tert-butylchlorodiphenylsilane (1.64 g, 6 mmol). The reaction system was stirred at 25°C overnight. After the reaction was complete, the reaction solution was concentrated to dryness, and the crude product was separated and purified through a C-18 column to obtain **compound 1D.**

MS (ESI) m/z 405.2 $(M+H)^+$.

**Step 5:** Preparation of benzyl 3-methyleneazetidine-1-carboxylate **(compound 1E)**

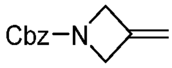

**1E**

Benzyl 3-oxoazetidine-1-carboxylate (86.6 g, 268 mmol) and methyltriphenylphosphonium bromide (50 g, 243.5 mmol) were dissolved in tetrahydrofuran (500 mL), and then added with potassium t-butoxide (40.9 g, 365.5 mmol). The mixture was stirred at 25°C overnight. After the reaction was complete, the reaction solution was concentrated to dryness, and the residue was separated and purified through a silica gel column to obtain **compound 1E.**

MS (ESI) m/z 204.1 $(M+H)^+$.

**Step 6:** preparation of 1-benzyl 1'-(tert-butyl)2*H*,2'*H*-[3,3'-diazetidinylidene]-1,1'(4*H*,4'*H*)-dicarboxylate **(compound 1F)**

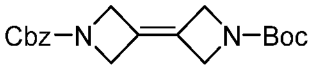

**1F**

Benzyl 3-methyleneazetidine-1-carboxylate (39 g, 191.9 mmol) and tert-butyl 3-methyleneazetidine-1-carboxylate (32.47 g, 191.9 mmol) were dissolved in dichloroethane (2000 mL), and then added with Grubb's 2 (16.29 g, 19.19 mmol), and the mixture was stirred at 50°C for 48 hours. After the reaction was complete, the reaction solution was concentrated to dryness, and the residue was separated and purified through a silica gel column to obtain **compound 1F.**

MS (ESI) m/z 345.2 $(M+H)^+$.

**Step 7:** preparation of benzyl 1',4'-dihydro-2*H*,2'*H*-[3,3'-diazetidinylidene]-1(4*H*)-carboxylate **(compound 1G)**

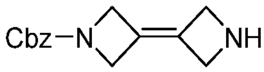

**1G**

1-benzyl 1'-(tert-butyl)2*H*,2'*H*-[3,3'-diazetidinylidene]-1,1'(4*H*,4'*H*)-dicarboxylate (3.2 g, 9.29 mmol) was dissolved in ethyl acetate (32 mL), and then added with p-tolu enesulfonic acid (1.6 g, 9.29 mmol), and the reaction system was stirred at 50°C f or 6 hours. After the reaction was complete, the reaction solution was concentrated to dryness, and the residue was separated and purified through a silica gel column t o obtain **compound 1G.**

MS (ESI) m/z 245.1 $(M+H)^+$.

**Step 8:** preparation of benzyl 1'-((2,3-dihydro-[1,4]dioxino[2,3-*b*]pyridin-7-yl) sulfonyl)-1',4'-dihydro-2*H*,2'*H*-[3,3'-diazetidinylidenel-1(4*H*)-carboxylate **(compound 1H)**

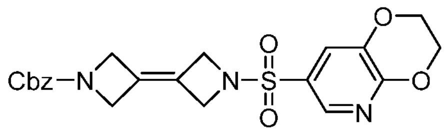

**1H**

Benzyl 1',4'-dihydro-2*H*,2'*H*-[3,3'-diazetidinylidene]-1(4*H*-carboxylate (100 mg, 0.4 1 mmol) and triethylamine (82.5 mg, 0.82 mmol) were dissolved in tetrahydrofuran (4 mL), and then added with 2,3-dihydro-[1,4]dioxino[2,3-b]pyridine-7-sulfonyl chl oride (96.5 mg, 0.41 mmol), and the mixture was stirred at 25°C for 2 hours. After the reaction was complete, the reaction solution was concentrated to dryness, and t he residue was separated and purified through a silica gel column to obtain compou nd **1H.**
MS (ESI) m/z 444.1 $(M+H)^+$.

**Step 9:** preparation of 7-((1',4'-dihydro-2*H*,2'*H*-[3,3'-diazetidinylidene]-1(4*H*-ylsulfonyl)-2,3-dihydro-[1,4] dioxino[2.3-*b*]pyridine) **(compound 11)**

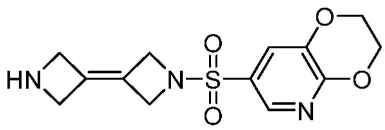

**1I**

Benzyl 1'-((2,3-dihydro-[1,4]dioxino[2,3-b]pyridin-7-yl)sulfonyl)-1',4'-dihydro-2*H*,2' *H*-[3,3'-diazetidinylidene]-1(4*H*)-carboxylate (120 mg, 0.27 mmol) was dissolved in trifluoroacetic acid (5 mL), and the reaction system was stirred at 60°C overnight. Af ter the reaction was complete, the reaction solution was concentrated to dryness, an d the residue was separated and purified through a silica gel column to obtain **com**

**pound 1I.**

MS (ESI) m/z 310.1 $(M+H)^+$.

**Step 10:** preparation of (*S*)-3-((tert-butyldiphenylsilyl)oxy)-1-(1'-((2,3-dihydro-[1,4]dioxino[2,3-*b*]pyridin-7-yl )sulfonyl)-1',4'-dihydro-2*H*,2'*H*-[3,3'-diazetidinylidenel-1(4*H*-yl)-2-phenylpropan-1-one **(compound 1J)**

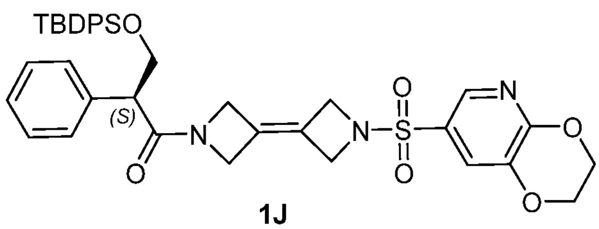

**1J**

(*S*)-3-((tert-butyldiphenylsilyl)oxy)-2-phenylpropionic acid (80 mg, 0.2 mmol) and 7-((1',4'-dihydro-2*H*,2'*H*-[3,3'-diazetidinylidene]-1(4*H*-ylsulfonyl)-2,3-dihydro-[1,4] dioxino[2,3-*b*]pyridine) (61 mg, 0.2 mmol) were dissolved in DMF (5 mL), and then added with HATU (112 mg, 0.3 mmol) and DIPEA (77 mg, 0.6 mmol), and the reaction system was stirred at 25°C for 3 hours. After the reaction was complete, the reaction solution was concentrated to dryness, and

subjected to reversed-phase high-pressure liquid chromatography to prepare **compound 1J.**

MS (ESI) m/z 695.3 (M+H)$^+$.

**Step 11:** preparation of (*S*)-1-(1'-((2,3-dihydro-[1,4]dioxino[2,3-b]pyridin-7-yl)sulfonyl)-1',4'-dihydro-2*H*,2' *H*-[3,3'-diazetidinylidene]-1(4*H*)-yl)-3-hydroxy-2-phenylpropan-1-one **(compound 1)**

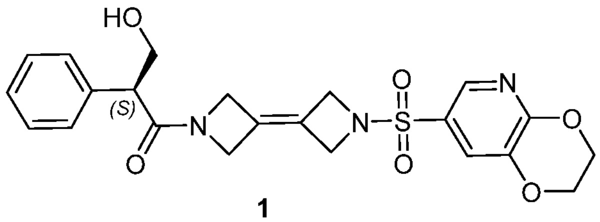

**1**

(*S*)-3-((tert-butyldiphenylsilyl)oxy)-1-(1'-((2,3-dihydro-[1,4]dioxino[2,3-*b*]pyridin-7-yl )sulfonyl)-1',4'-dihydro-2*H*,2'*H*-[3,3'-diazetidinylidene]-1(4*H*)-yl)-2-phenylpropan-1-one (50 mg, 0.087 mmol) was dissolved in tetrahydrofuran (5 mL), and then added with tetrabutylammonium fluoride (45.5 mg, 0.17 mmol), and the reaction system was stirred at 25°C for 3 hours. After the reaction was complete, the reaction solution was concentrated to dryness, and subjected to reversed-phase high-pressure liquid chromatography to prepare **compound** 1 of Example 1.

MS (ESI) m/z 458.2 (M+H)$^+$.

$^1$H NMR (400 MHz, DMSO-$d_6$) $\delta$ 8.23 (s, 1H), 7.71 (s, 1H), 7.51-7.20 (m, 5H), 4.87 (s, 1H), 4.80-4.72 (m, 1H), 4.60 (s, 2H), 4.50-4.30 (m, 8H), 4.30-4.20 (m, 1H), 3.96 (s, 1H), 3.75-3.55 (m, 2H).

**Example 2:** preparation of (*S*) -1-(1'-((2,3-dihydrobenzo[*b*][1,4]dioxin-6-yl)sulfonyl)-1',4'-dihydro-2*H*,2'*H*-[3,3'-di azetidinylidene]-1(4*H*)-yl)-3-hydroxy-2-phenylpropan-1-one **(compound 2):**

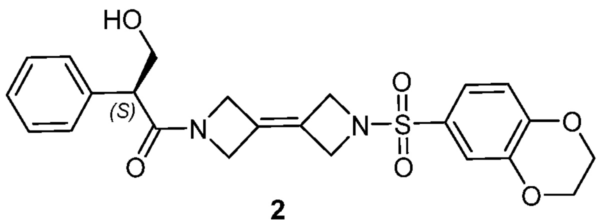

**2**

In step 8 of Example 1, 2,3-dihydro-[1 ,4]dioxino[2,3-*b*]pyridine-7-sulfonyl chloride was replaced by 2,3-dihydrobenzo[*b*][1,4]dioxin-6-sulfonyl chloride, and the same preparation method as that of Example 1 was adopted to obtain **compound 2** of Example 2.

MS (ESI) m/z 457.1 (M+H)$^+$.

$^1$H NMR (400 MHz, DMSO-$d_6$) $\delta$ 7.35 - 7.20 (m, 7H), 7.13 - 7.11 (m, 1H), 4.85- 4.7 (m, 2H), 4.40 - 4.20 (m, 11H), 3.95 - 3.85 (m, 1H), 3.75 - 3.70 (m, 1H), 3.50 - 3.40 (m, 1H).

**Example 3:** preparation of (*S*)-1-(1'-(benzo[*d*]thiazol-6-ylsulfonyl)-1',4'-dihydro-2*H*,2'*H*-[3,3'-diazetidinylidene] -1(4*H*)-yl)-3-hydroxy-2-phenylpropan-1-one **(compound 3):**

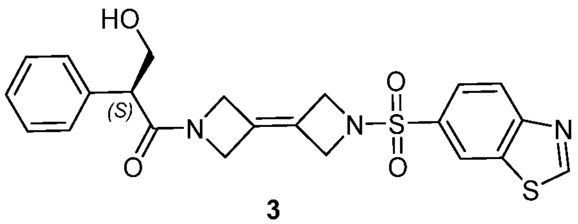

3

In step 8 of Example 1, 2,3-dihydro-[1,4]dioxino[2,3-*b*]pyridine-7-sulfonyl chloride was replaced by benzo[*d*]thiazole-6-sulfonyl chloride, and the same preparation method as that of Example 1 was adopted to obtain compound 3 of Example 3.

MS (ESI) m/z 456.1 $(M+H)^+$.

$^1H$ NMR (400 MHz, DMSO-$d_6$) $\delta$ 9.64 (s, 1H), 8.77 (s, 1H), 8.35-8.24 (m, 1H), 7.90 (d, *J* = 9.4 Hz, 1H), 7.21 (d, *J* = 14.2 Hz, 5H), 4.64 (d, *J* = 12.5 Hz, 2H), 4.34-4.10 (m, 7H), 3.81 (d, *J* = 10.0 Hz, 1H), 3.61-3.50 (m, 1H), 3.40 (d, J = 7.1 Hz, 1H).

**Example 4:** preparation of (*S*)-3-hydroxy-2-phenyl-1-(1'-(pyridin-2-ylsulfonyl)-1',4'-dihydro-2*H*,2'*H*-[3.3'-diaze tidinylidene]-1(4*H*)-yl)propan-1-one **(compound 4):**

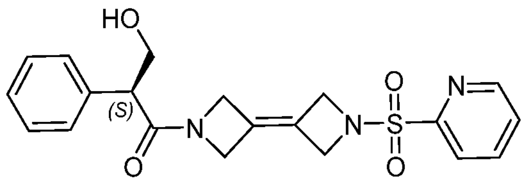

4

In step 8 of Example 1, 2,3-dihydro-[1,4]dioxino[2,3-*b*]pyridine-7-sulfonyl chloride was replaced by pyridine-2-sulfonyl chloride, and the same preparation method as that of Example 1 was adopted to obtain **compound 4** of Example 4.

MS (ESI) m/z 400.0$(M+H)^+$.

$^1H$ NMR (400 MHz, DMSO-$d_6$) $\delta$ 8.76 (s, 1H), 8.11 (d, *J* = 11.6 Hz, 1H), 7.95 (d, *J* = 5.3 Hz, 1H), 7.71 (s, 1H), 7.28-7.19 (m, 5H), 4.68 (d, *J* = 13.2 Hz, 1H), 4.50 (s, 4H), 4.37-4.23 (m, 2H), 4.17 (d, *J* = 14.6 Hz, 1H), 3.85 (q, *J* = 9.0 Hz, 1H), 3.58 (d, *J* = 10.4 Hz, 1H), 3.50-3.40 (m, 2H).

**Example 5:** preparation of (*S*)-3-hydroxy-1-(1'-((4-methoxyphenyl)sulfonyl)-1',4'-dihydro-2*H*,2'*H*-[3,3'-diazetidi nylidene]-1(4*H*)-yl)-2-phenylpropan-1-one **(compound 5):**

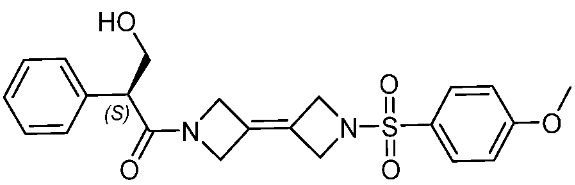

5

In step 8 of Example 1, 2,3-dihydro-[1 ,4]dioxino[2,3-*b*]pyridine-7-sulfonyl chloride was replaced by p-methoxybenzenesulfonyl chloride, and the same preparation method as that of Example 1 was adopted to obtain **compound 5** of Example 5.

MS (ESI) m/z 429.5$(M+H)^+$.

$^1H$ NMR (400 MHz, DMSO-$d_6$) $\delta$ 7.80-7.65 (m, 2H), 7.20 (m, 7H), 4.83-4.60 (m, 2H), 4.24 (d, *J* = 30.9 Hz, 7H), 3.82 (s, 4H), 3.60-3.54 (m, 1H), 3.41 (d, *J* = 10.1 Hz, 1H).

**Example 6:** preparation of 1-(1'-((4-(difluoromethoxy)phenyl)sulfonyl)-1',4'-dihydro-2*H*,2'*H*-[3,3'-diazetidinyliden e]-1(4*H*)-yl)-3-hydroxy-2-phenylpropan-1-one **(compound 6):**

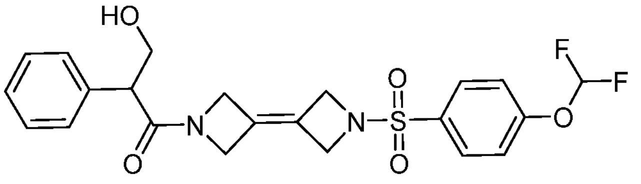

**6**

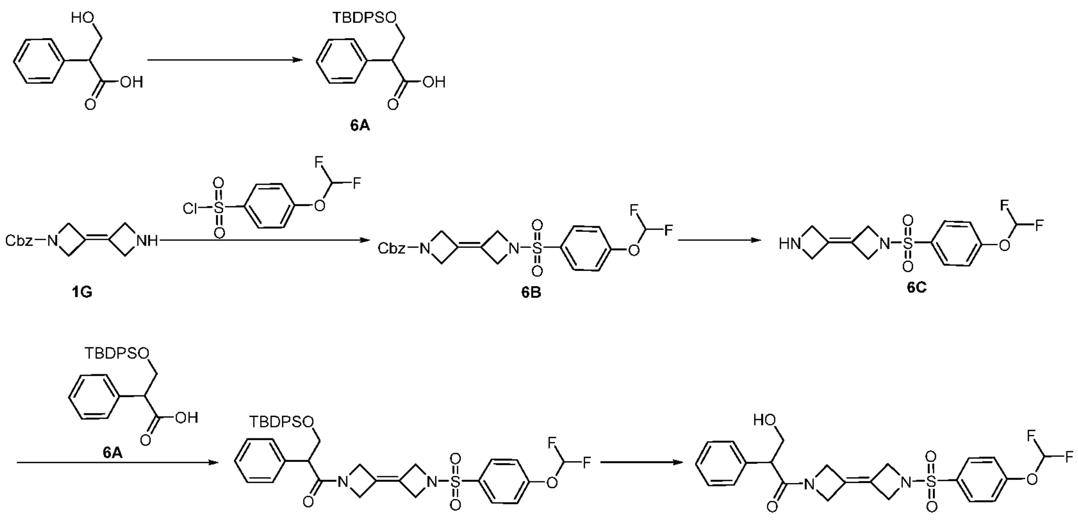

6A

1G 6B 6C

6A

6D 6

**Step 1:** preparation of 3-((tert-butyldiphenylsilyl)oxy)-2-phenylpropionic acid **(compound 6A)**

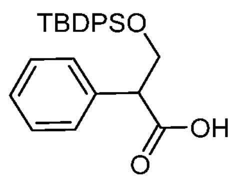

**6A**

3-hydroxy-2-phenylpropionic acid (1.0 g, 6.0 mmol) and 4-dimethylaminopyridine (1.47 g, 12 mmol) were dissolved in dichloromethane (10 mL), and then added with tert-butylchlorodiphenylsilane (1.54 g, 9 mmol), and the reaction system was stirred at 25°C overnight. After the reaction was complete, the reaction solution was concentrated to dryness, and separated and purified through a silica gel column to obtain **compound 6A.** MS (ESI) m/z 405.2 $(M+H)^+$.

**Step 2:** preparation of benzyl 1'-((4-(difluoromethoxy)phenyl)sulfonyl)-1',4'-dihydro-2*H*,2'*H*-[3,3'-diazetidinylidene]-1(4*H*)-carboxylate **(compound 6B)**

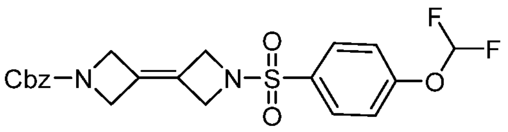

**6B**

Benzyl 3-(azetidin-3-ylidene)azetidine-1-carboxylate (500 mg, 2.05 mmol) and triethylamine (413 mg, 4.1 mmol) were dissolved in tetrahydrofuran solution (20 mL), and then added with 4-(difluoromethoxy)benzenesulfonyl chloride (500 mg, 2.05 mmol), and the reaction system was stirred at 25°C for 3 hours. After the reaction was complete, the reaction solution was concentrated to dryness under reduced pressure, and the residue was separated and purified through a silica gel column to obtain **compound 6B.**

MS (ESI) m/z 451.1 $(M+H)^+$.

**Step 3:** preparation of 1-((4-(difluoromethoxy)phenyl)sulfonyl)-1,1',4,4'-tetrahydro-2*H*,2'*H*-3,3'-diazetidinylid ene **(compound 6C)**

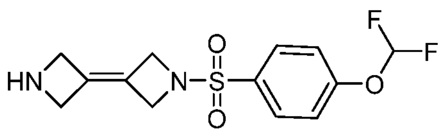

**6C**

Benzyl

1'-((4-(difluoromethoxy)phenyl)sulfonyl)-1',4'-dihydro-2*H*,2'*H*-[3,3'-diazetidinylidene]-1(4*H*)-carboxylate (750 mg, 1.66 mmol) was dissolved in trifluoroacetic acid (5 mL), and the mixture was stirred at 40°C for 16 hours. After the reaction was complete, the reaction solution was concentrated to dryness, and the residue was separated and purified through a silica gel column to obtain **compound 6C.**

MS (ESI) m/z 317.1 $(M+H)^+$.

**Step 4:** preparation of 3-((tert-butyldiphenylsilyl)oxy)-1-(1'-((4-(difluoromethoxy)phenyl)sulfonyl)-1',4'-dihydro -2*H*,2'*H*-[3,3'-diazetidinylidene]-1(4*H*)-yl)-2-phenylpropan-1-one **(compound 6D)**

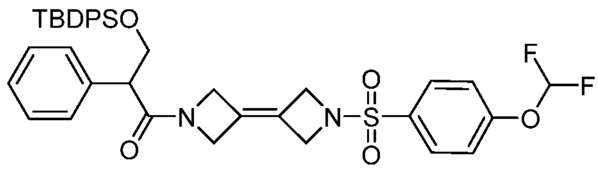

**6D**

3-((tert-butyldiphenylsilyl)oxy)-2-phenylpropionic acid (109 mg, 0.3 mmol) and 1-((4-(difluoromethoxy)phenyl)sulfonyl)-1 ,1',4,4'-tetrahydro-2*H*,2'*H*-3,3'-diazetidinylid ene (85 mg, 0.3 mmol) were dissolved in DMF (5 mL), and then added with HATU (157 mg, 0.4 mmol) and DIPEA (104 mg, 0.8 mmol), and the reaction system was stirred at 25°C for 3 hours. After the reaction was complete, the reaction solution was concentrated to dryness, and subjected to reversed-phase high-pressure liquid chromatography to prepare compound 6D.

MS (ESI) m/z 703.2 $(M+H)^+$.

**Step 5:** preparation of 1-(1'-((4-(difluoromethoxy)phenyl)sulfonyl)-1',4'-dihydro-2*H*,2'*H*-[3,3'-diazetidinyliden e]-1(4*H*)-yl)-3-hydroxy-2-phenylpropan-1-one **(compound 6)**

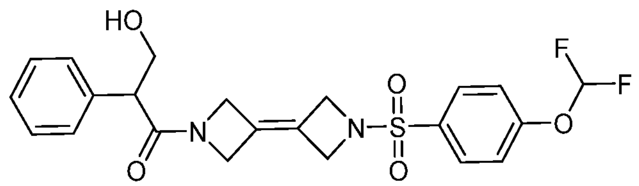

6

3-((tert-butyldiphenylsilyl)oxy)-1-(1'-((4-(difluoromethoxy)phenyl)sulfonyl)-1',4'-dihydro -2*H*,2'*H*-[3,3'-diazetidinylidene]-1(4*H*-yl)-2-phenylpropan-1-one (100 mg, 0.14 mmol) and tetrabutylammonium fluoride (37 mg, 0.14 mmol) were dissolved in tetrahydrofuran (5 mL), and the reaction system was stirred at 25°C for 3 hours. After the reaction was complete, the reaction solution was concentrated to dryness, and subjected to reversed-phase high-pressure liquid chromatography to prepare compound 6 of Example 6.

MS (ESI) m/z 465.2$(M+H)^+$.

$^1$H NMR (400MHz, DMSO-$d_6$) $\delta$ 7.91 - 7.88 (m, 2H), 7.45- 7.43 (m, 2H), 7.30 - 7.20 (m, 5H), 4.72 - 4.69 (m, 1H), 4.60 (s, 2H), 4.36 - 4.15 (m, 7H), 4.95 - 4.85 (m, 1H), 3.65 - 3.60 (m, 1H), 3.50 - 3.40 (m, 1H).

**Example 7:** preparation of (*S*)-1-(1'-((4-(difluoromethoxy)phenyl)sulfonyl)-1',4'-dihydro-2*H*,2'*H*-[3,3'-diazetidinyl idene]-1(4*H*)-yl)-3-hydroxy-2-phenylpropan-1-one **(compound 7):**

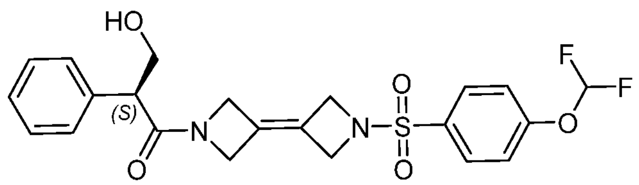

7

In step 4 of Example 6, 3-((tert-butyldiphenylsilyl)oxy)-2-phenylpropionic acid was replaced by (S)-3-((tert-butyldiphenylsilyl)oxy)-2-phenylpropionic acid, and the same preparation method as that of Example 6 was adopted to obtain **compound 7** of Example 7.

MS (ESI) m/z 465.1$(M+H)^+$.

$^1$H NMR (400MHz, DMSO-$d_6$) $\delta$ 7.91 - 7.88 (m, 2H), 7.45- 7.43 (m, 2H), 7.30 - 7.20 (m, 5H), 6.06 (s, 1H), 4.72 - 4.69 (m, 1H), 4.36 - 4.15 (m, 7H), 4.95 - 4.85 (m, 1H), 3.65 - 3.60 (m, 1H), 3.50 - 3.40 (m, 2H).

**Example 8:** preparation of

1-(1'-((4-(difluoromethoxy)phenyl)sulfonyl)-1',4'-dihydro-2*H*,2'*H*-[3,3'-diazetidinyliden e]-1(4*H*-yl)-2-hydroxy-2-phenylethan-1-one **(compound 8):**

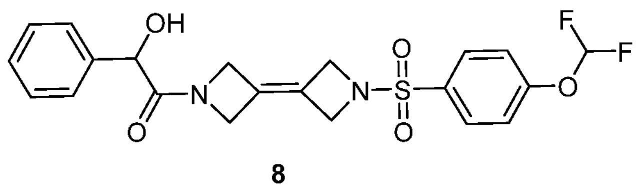

8

In step 4 of Example 6, 3-((tert-butyldiphenylsilyl)oxy)-2-phenylpropionic acid was replaced by 2-hydroxy-2-phenylacetic acid, and the same preparation method as that of Example 6 was adopted to obtain **compound 8** of Example 8.

MS (ESI) m/z 451.1$(M+H)^+$.

$^1$H NMR (400 MHz, DMSO-$d_6$) $\delta$ 8.47 (s, 1H), 7.90 (m, 2H), 7.45 (s, 2H), 7.30 (d, *J* = 21.5 Hz, 5H), 5.85 (s,

1H), 5.04 (s, 1H), 4.65 (d, *J* = 13.9 Hz, 1H), 4.52 (d, *J* = 14.1 Hz, 1H), 4.29 (s, 6H).

**Example 9:** preparation of (*S*)-3-hydroxy-2-phenyl-1-(1'-((4-(trifluoromethoxy)phenyl)sulfonyl)-1',4'-dihydro-2*H,* 2'*H*-[3,3'-diazetidinylidene]-1(4*H*)-yl)propan-1-one **(compound 9):**

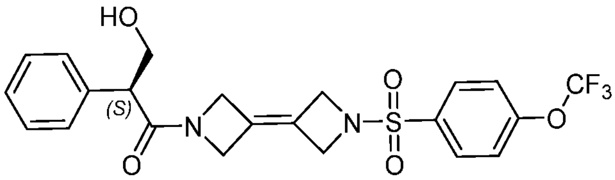

**9**

In step 8 of Example 1, 2,3-dihydro-[1,4]dioxino[2,3-*b*]pyridine-7-sulfonyl chloride was replaced by 4-(trifluoromethoxy)benzenesulfonyl chloride, and the same preparation method as that of Example 1 was adopted to obtain **compound 9** of Example 9.

MS (ESI) m/z 483.1 $(M+H)^+$.

$^1H$ NMR (400 MHz, DMSO-$d_6$) $\delta$7.99-7.96(m, 2H), 7.67-7.65 (d, *J* = 0.8 Hz, 2H), 7.30-7.22 (m, 5H), 4.81-4.69 (m, 2H), 4.36-4.18 (m, 7H), 3.90-3.86 (t, *J*=0.8 Hz, 1H), 3.63-3.60 (m, 1H), 3.47-3.43 (m, 1H).

**Example 10:** preparation of (*S*)-1-(1'-(4-fluorophenyl)sulfonyl)-1',4'-dihydro-2*H*,2'*H*-[3,3'-diazetidinylidene]-1(4*H* )-yl)-3-hydroxy-2-phenylpropan-1-one **(compound 10):**

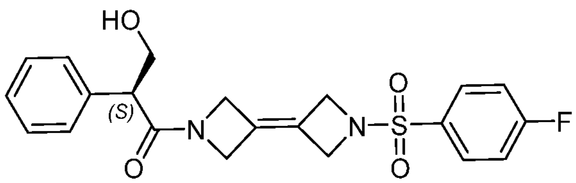

**10**

In step 8 of Example 1, 2,3-dihydro-[1,4]dioxino[2,3-*b*]pyridine-7-sulfonyl chloride was replaced by 4-fluorobenzenesulfonyl chloride, and the same preparation method as that of Example 1 was adopted to obtain **compound 10** of Example 10.

MS (ESI) m/z 417.1$(M+H)^+$.

$^1H$ NMR (400 MHz, DMSO-$d_6$) $\delta$7.99-7.96(m, 2H), 7.67-7.65 (d, *J* = 0.8 Hz, 2H), 7.30-7.22 (m, 5H), 4.81-4.69 (m, 2H), 4.36-4.18 (m, 7H), 3.90-3.86 (t, *J*=0.8 Hz, 1H), 3.63-3.60 (m, 1H), 3.47-3.43 (m, 1H).

**Example 11:** preparation of (*S*)-1-(1'-(3-fluoro-4-difluoromethoxyphenyl)sulfonyl)-1',4'-dihydro-2*H*,2'*H*-[3,3'-diaz etidinylidene]-1(4*H*)-yl)-3-hydroxy-2-phenylpropan-1-one **(compound 11):**

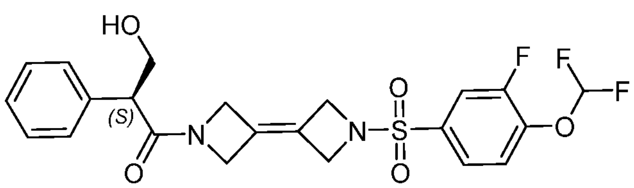

**11**

In step 8 of Example 1, 2,3-dihydro-[1,4]dioxino[2,3-*b*]pyridine-7-sulfonyl chloride was replaced by 3-fluoro-4-difluoromethoxybenzenesulfonyl chloride, and the same preparation method as that of Example 1 was adopted to obtain **compound 11** of Example 11.

MS (ESI) m/z 483.1(M+H)$^+$.

$^1$H NMR (400 MHz, DMSO-$d_6$) $\delta$ 7.92-7.89 (m, 1H), 7.89-7.62 (m, 2H), 7.44-7.22 (m, 6H), 4.82-4.79 (m, 1H), 4.72-4.69 (d, *J* = 28.0 Hz, 1H), 4.34-4.20 (m, 7H), 3.89-3.84 (m, 1H), 3.64-3.58 (m, 1H), 3.47-3.43 (m, 1H).

**Example 12:** preparation of (*S*)-1-(1'-((3,4-dihydro-2*H*-benzo[*b*][1,4]oxazin-6-yl)sulfonyl)-1',4'-dihydro-2*H*,2'*H*-[3,3'-diazetidinylidene]-1(4*H*)-yl)-3-hydroxy-2-phenylpropan-1-one **(compound 12):**

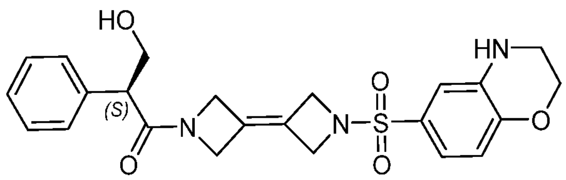

**12**

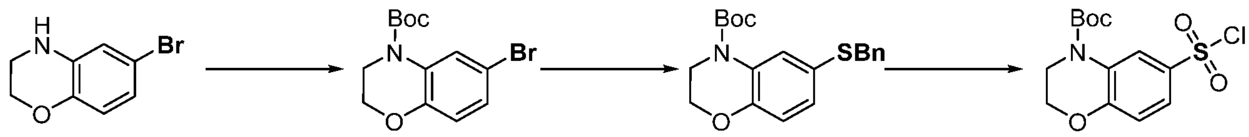

**12A** **12B** **12C** **12D**

Step 1: Preparation of tert-butyl 6-bromo-2,3-dihydro-4*H*-benzo[*b*][1,4]oxazine-4-carboxylate **(compound 12B)**

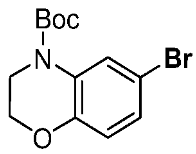

**12B**

6-bromo-3,4-dihydro-2*H*-1,4-benzoxazine (2 g, 9.34 mmol), 4-dimethylaminopyridine (114 mg, 0.93 mmol) and triethylamine (1.42 g, 14 mmol) were dissolved in tetrahydrofuran (40 mL), then added with di-tert-butyl dicarbonate (2 g, 9.34 mmol), and the reaction system was stirred at 40°C for 16 hours. After the reaction was complete, the reaction solution was poured into water, and extracted with ethyl acetate (60 mL*3), and the organic phases were combined, washed with a saturated brine, and dried over anhydrous sodium sulfate, and separated and purified through a silica gel column to obtain **compound 12B.**

MS (ESI) m/z 314.0 (M+H)$^+$.

**Step 2:** preparation of tert-butyl 6-(benzylthio)-2,3-dihydro-4*H*-benzo[*b*][1,4]oxazine-4-carboxylate **(compound 12C)**

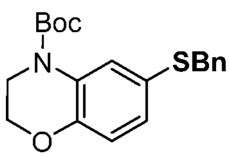

**12C**

Tert-butyl 6-bromo-2,3-dihydro-4*H*-benzo[*b*][1,4]oxazine-4-carboxylate (500 mg, 1.59 mmol), benzyl mercaptan (236.8 mg, 1.91 mmol), 4,5-bis(diphenylphosphino)-9,9-dimethylxanthene (92 mg, 0.16 mmol), tris(dibenzylideneacetone)dipalladium (73 mg, 0.07 mmol) and *N,N*-diisopropylethylamine (617 mg, 4.77 mmol) were dissolved in 1,4-dioxane (10 mL), and the reaction system was placed and stirred at 40°C for 16 hours. After the reaction was complete, the reaction solution was poured into water, the aqueous phase was extracted with ethyl acetate (40 mL*3), and the organic phases were combined, washed with a saturated brine, and then dried over anhydrous sodium sulfate. After the organic phase was concentrated to dryness under reduced pressure, the residue was separated and purified through a silica gel column to obtain **compound 12C.**

MS (ESI) m/z 358.1 $(M+H)^+$.

**Step 3:** preparation of tert-butyl 6-(chlorosulfonyl)-2,3-dihydro-4*H*-benzo[*b*][1,4]oxazine-4-carboxylate **(compound 12D)**

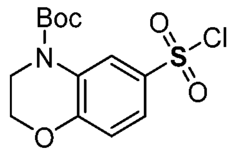

**12D**

Tert-butyl 6-(benzylthio)-2,3-dihydro-4*H*-benzo[*b*][1,4]oxazine-4-carboxylate (500 mg, 1.39 mmol) was dissolved in a mixed solvent (acetonitrile: acetic acid: water = 40:1.5:1, 25 mL), and added with 1,3-dichloro-5,5-dimethylhydantoin (547.6 mg, 2.78 mmol) with stirring at 0°C. Then stirring was conducted at room temperature for 3 hours. After the reaction was complete, the reaction solution was poured into water, and extracted with ethyl acetate (60 mL*3), and the organic phases were combined, washed with a saturated brine, and then dried over anhydrous sodium sulfate. The organic phase was concentrated to dryness, and the residue was separated and purified through a silica gel column to obtain **compound 12D.**

In step 8 of Example 1, 2,3-dihydro-[1 ,4]dioxino[2,3-*b*]pyridine-7-sulfonyl chloride was replaced by tert-butyl 6-(chlorosulfonyl)-2,3-dihydro-4*H*-benzo[*b*][1,4]oxazine-4-carboxylate, and the same preparation method as that of Example 1 was adopted to obtain **compound 12** of Example 12.

MS (ESI) m/z 456.1$(M+H)^+$.

$^1H$ NMR (400 MHz, DMSO-$d_6$) $\delta$ 7.34-7.21 (m, 6H), 7.0 (s, 1H), 6.89-6.84 (m, 2H), 6.33 (s, 1H), 4.82-4.79 (t, *J* = 0.8 Hz, 1H), 4.73-4.70 (d, *J* = 1.2 Hz, 1H), 4.38-4.21 (m, 10H), 3.91-3.85 (m, 1H), 3.63-3.59 (m, 1H), 3.47-3.43 (m, 1H).

**Example 13:** preparation of 1-(1'-((4-(difluoromethoxy)phenyl)sulfonyl)-1',4'-dihydro-2*H*,2'*H*-[3,3'-diazetidinyliden e]-1(4*H*)-yl)-2-(3,4-dihydro-2*H*-benzo[*b*][1,4]oxazin-6-yl)-2-hydroxyethan-1-one **(compound 13):**

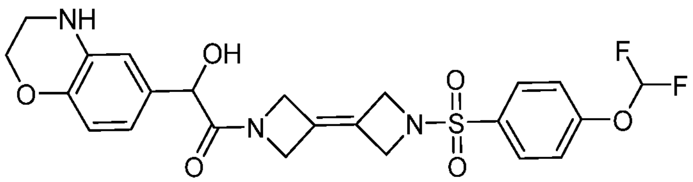

**13**

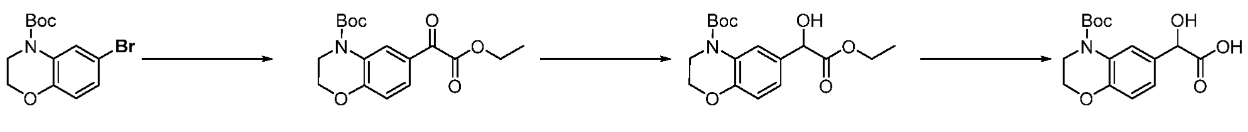

**12B** **13A** **13B** **13C**

Step 1: preparation of tert-butyl 6-(2-ethoxy-2-oxoacetyl)-2,3-dihydro-4*H*-benzo[*b*][1,4]oxazine-4-carboxylate (compound 13A)

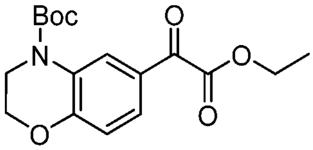

**13A**

Tert-butyl 6-bromo-2*H*-benzo[*b*][1,4]oxazine-4(3*H*)-carboxylate (500 mg, 1.59 mmol) was added into dry tetrahydrofuran (10 mL). The reaction system was replaced with nitrogen for three times and then maintained as a nitrogen system, cooled to -78°C, slowly added dropwise with n-butyllithium (122 mg, 1.91 mmol), and then stirred at -78°C for 0.5 hours. Diethyl oxalate (256 mg, 1.75 mmol) was added dropwise into the reaction solution, then heated to room temperature after the dropwise addition, and stirred for 1 hour. After the reaction was complete, the reaction solution was poured into water, the aqueous phase was extracted with ethyl acetate (40 mL*3), and the organic phases were combined, washed with a saturated brine, and then dried over anhydrous sodium sulfate. The organic phase was concentrated to dryness and then separated and purified through a silica gel column to obtain **compound 13A.**

MS (ESI) m/z 336.1 $(M+H)^+$.

**Step 2:** preparation of tert-butyl 6-(2-ethoxy-1-hydroxy-2-oxoethyl)-2,3-dihydro-4*H*-benzo[*b*][1,4]oxazine-4-carboxyl ate **(compound 13B)**

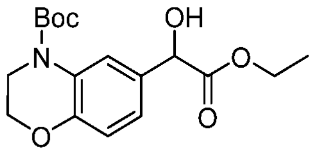

**13B**

Tert-butyl

6-(2-ethoxy-2-oxoacetyl)-2,3-dihydro-4*H*-benzo[*b*][1,4]oxazine-4-carboxylate (170 mg, 0.50 mmol) was added into tetrahydrofuran (8 mL), and added with sodium borohydride (29 mg, 0.76 mmol) with stirring at 0°C. Then stirring was conducted at room temperature for 0.5 hours. After the reaction was complete, the reaction solution was poured into water, and extracted with ethyl acetate (30 mL*3), and the organic phases were combined, washed with a saturated brine, and then dried over anhydrous sodium sulfate. After the organic phase was concentrated to dryness under reduced pressure, the residue was separated and purified through a silica gel column to obtain **compound 13B.**

MS (ESI) m/z 338.1 $(M+H)^+$.

**Step 3:** preparation of 2-(4-(tert-butoxycarbonyl)-3,4-dihydro-2*H*-benzo[*b*][1,4]oxazin-6-yl)-2-hydroxyaceti c acid **(compound 13C)**

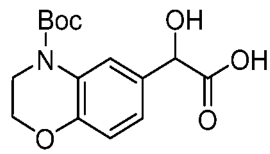

**13C**

Tert-butyl

6-(2-ethoxy-1-hydroxy-2-oxoethyl)-2,3-dihydro-4*H*-benzo[*b*][1,4]oxazine-4-carboxyl ate (170 mg, crude product) and lithium hydroxide hydrate (106 mg, 2.52 mmol) were added into a mixed solution of tetrahydrofuran (3 mL) and water (3 mL), and stirred at room temperature for 16 hours. After the reaction was complete, the reaction solution was adjusted to pH 4-5 with 2N hydrochloric acid solution in ice bath, diluted with water, and then extracted with ethyl acetate (20 mL*3). The organic phases were combined, washed with a saturated brine, and dried over anhydrous sodium sulfate. The organic phase was concentrated to dryness, and the residue was separated and purified through a silica gel column to obtain **compound 13C.**

MS (ESI) m/z 310.1 $(M+H)^+$.

In step 4 of Example 6, 3-((tert-butyldiphenylsilyl)oxy)-2-phenylpropionic acid was replaced by 2-(4-(tert-butoxycarbonyl)-3,4-dihydro-2*H*-benzo[*b*][1,4]oxazin-6-yl)-2-hydroxyaceti c acid, and the same preparation method as that of Example 6 was adopted to obtain **compound 13** of Example 13.

MS (ESI) m/z 508.1$(M+H)^+$.

$^1H$ NMR (400 MHz, DMSO-$d_6$) $\delta$ 7.95-7.88 (d, *J* = 8 Hz, 2H), 7.49-7.43 (d, *J* = 8 Hz, 3H), 6.56-6.51 (m, 2H), 6.41-6.39 (m, 1H), 5.76 (s, 1H), 5.49-5.48 (d, *J* = 4 Hz, 1H), 4.81-4.79 (d, *J* = 8 Hz, 1H), 4.61-4.57 (d, *J* = 16 Hz, 1H), 4.42-4.38 (d, *J* = 16 Hz, 1H), 4.27 (s, 6H), 4.07-4.05 (m, 2H), 3.22 (s, 2H).

**Example 14:** preparation of (1'-((4-(difluoromethoxy)phenyl)sulfonyl)-1',4'-dihydro-2*H*,2'*H*-[3,3'-diazetidinylidene] -1(4*H*)-yl)(4-phenyltetrahydro-2*H*-pyran-4-yl)methanone **(compound 14):**

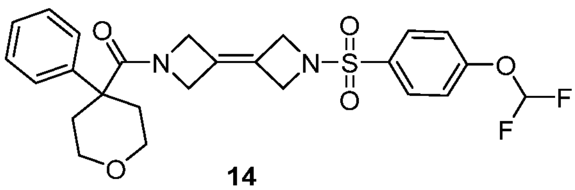

**14**

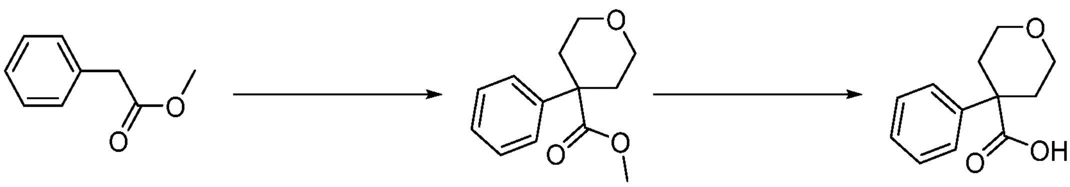

**14A** **14B** **14C**

**Step 1:** preparation of methyl 4-phenyltetrahydro-2*H*-pyran-4-carboxylate (compound 14B)

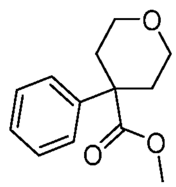

**14B**

Methyl 2-phenyl acetate (2.0 g, 13.32 mmol) was dissolved in tetrahydrofuran (40.0 mL), the temperature of the reaction system was reduced to 0°C, and then the reaction system was slowly added with sodium hydride (1.06 g, 26.64 mmol) in portions. The reaction solution was stirred at 0°C for 30 minutes, and then added dropwise with 2-iodoethyl ether (3.47 g, 10.65 mmol). After the dropwise addition was completed, the reaction system was stirred at 0°C for 10 minutes and then slowly heated to room temperature. The reaction system was then heated to 50°C and stirred for 18 hours. After the reaction was complete, water was added to quench the reaction. The aqueous phase was extracted with ethyl acetate (50 mL*3). The organic phases were combined, washed with a saturated brine, dried over anhydrous sodium sulfate, and separated and purified through a silica gel column to obtain **compound 14B.**

MS (ESI) m/z 221.1 $(M+H)^+$.

**Step 2:** preparation of 4-phenyltetrahydro-2*H*-pyran-4-carboxylic acid **(compound 14C)**

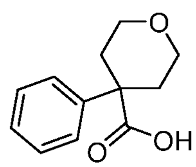

**14C**

Methyl 4-phenyltetrahydro-2*H*-pyran-4-carboxylate (1.0 g, 4.54 mmol) was dissolved in methanol (10.0 mL) and water (10.0 mL), and then added with sodium hydroxide (544.8 mg, 13.62 mmol), and the reaction solution was stirred at 50°C for 3 hours. After the reaction was complete, the reaction system was adjusted to pH 2 with 1N hydrochloric acid, and extracted with dichloromethane (50 mL*3), and the organic phases were combined, washed with a saturated brine, and dried over anhydrous sodium sulfate. After the organic phase was concentrated to dryness under reduced pressure, the residue was separated and purified through a silica gel column to obtain **compound 14C.**

MS (ESI) m/z 338.1 $(M+H)^+$.

In step 4 of Example 6, 3-((tert-butyldiphenylsilyl)oxy)-2-phenylpropionic acid was replaced by 4-phenyltetrahydro-2*H*-pyran-4-carboxylic acid, and the same preparation method as that of Example 6 was adopted to obtain **compound 14** of Example 14.

MS (ESI) m/z 505.1$(M+H)^+$.

$^1$H NMR (400 MHz, DMSO-*d*6) $\delta$7.88-7.84 (m, 2H), 7.46-7.23 (m, 8H), 4.30-4.10 (m, 6H), 3.97 (s, 2H), 3.78-3.63 (m, 2H), 3.52 (t, *J* = 11.0 Hz, 2H), 2.15 (d, *J* = 13.5 Hz, 2H), 1.91-1.72 (m, 2H).

**Example 15:** preparation of 1-(1'-((2,3-dihydrobenzo[*b*][1,4]dioxin-6-yl)sulfonyl)-1',4'-dihydro-2*H*,2'*H*-[3,3'-dia zetidinylidene]-1(4*H*-yl)-2-(3-methoxyphenyl)-3-(methylamino)propan-1-one (compound 15):

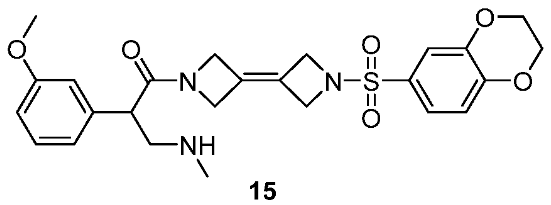

**15**

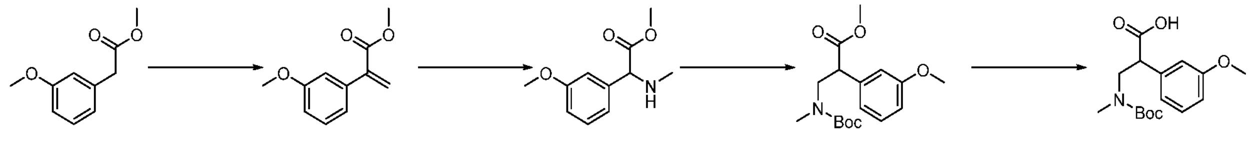

15A 15B 15C 15D 15E

**Step 1:** preparation of methyl 2-(3-methoxyphenyl)acrylate (compound 15B)

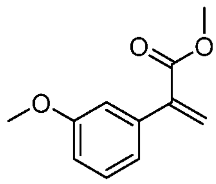

**15B**

Methyl 2-(3-methoxyphenyl)acetate (2.00 g, 11.10mmol) was added into *N*,*N*-dimethyl formamide (20.0 mL), and then sequentially added with potassium carbonate (3.83 g, 27.75 mmol), trioxymethylene (1.19 g, 13.32mmol) and tetrabutylammonium iodide (410.0 mg, 1.11 mmol). The reaction solution was reacted at 80°C for half an hour. After the reaction was complete, it was separated and conducted through a silica gel column to obtain **compound 15B.**
MS (ESI) m/z 193.1 $(M+H)^+$.

**Step 2:** Preparation of methyl 2-(3-methoxyphenyl)-2-(methylamino)acetate **(compound 15C)**

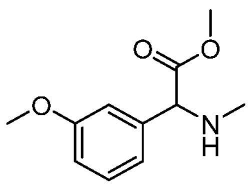

**15C**

Methyl 2-(3-methoxyphenyl)acrylate (2.02 g, 10.5 mmol) was added into methylamine tetrahydrofuran solution (20.0 mL), and the reaction was carried out at room temperature for 4 hours, separated and purified through a silica gel column to obtain compound 15C. MS (ESI) m/z 224.1 $(M+H)^+$.

**Step 3:** preparation of methyl 3-((tert-butoxycarbonyl)(methyl)amino)-2-(3-methoxyphenyl)propionate **(compound 15D)**

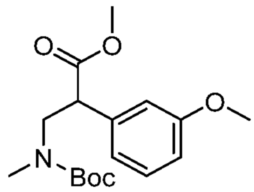

**15D**

Methyl 2-(3-methoxyphenyl)-2-(methylamino)acetate (2.00 g, 8.96 mmol) and 4-dimethylaminopyridine were dissolved in dichloromethane (10.0 mL), and added with triethylamine (1.81 g, 17.92 mmol) and di-tert-butyl dicarbonate (1.95 g, 8.96 mmol). Reaction was conducted at room temperature for two hours. After the reaction was complete, the reaction system was added with water to quench the reaction, and adjusted to pH 5-6 with 2N hydrochloric acid aqueous solution. Then the reaction system was extracted with ethyl acetate (20.0 mL*3). The organic layers were combined, washed with saturated sodium chloride (20.0 mL), and dried over anhydrous sodium sulfate. After the organic phase was concentrated to dryness under reduced pressure, the residue was separated and purified through a silica gel column to obtain **compound 15D.**

MS (ESI) m/z 324.2 $(M+H)^+$.

**Step 4:** preparation of 3-((tert-butoxycarbonyl)(methyl)amino)-2-(3-methoxyphenyl)propionic acid **(compound 15E)**

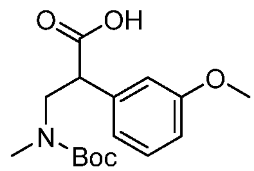

**15E**

Methyl 3-((tert-butoxycarbonyl)(methyl)amino)-2-(3-methoxyphenyl)propionate (669.0 mg, 2.07 mmol) was dissolved in tetrahydrofuran (5.0 mL) and water (5.0 mL), and added with lithium hydroxide (99.1 mg, 4.14 mmol). The reaction was carried out at room temperature for 16 hours. After the reaction was complete, the organic phase was concentrated to dryness under reduced pressure, and the residue was separated and purified through a silica gel column to obtain **compound 15E.**

MS (ESI) m/z 310.2 $(M+H)^+$.

In step 8 of Example 1, 2,3-dihydro-[1 ,4]dioxino[2,3-*b*]pyridine-7-sulfonyl chloride was replaced by 2,3-dihydrobenzo[b][1,4]dioxin-6-sulfonyl chloride, and in step 10, (S)-3-((tert-butyldiphenylsilyl)oxy)-2-phenylpropionic acid was replaced by 3-((tert-butoxycarbonyl)(methyl)amino)-2-(3-methoxyphenyl)propionic acid, and the same preparation method as that of Example 1 was adopted to obtain **compound 15** of Example 15.

MS (ESI) m/z 500.2 $(M+H)^+$.

$^1H$ NMR (400MHz,DMSO-$d_6$) $\delta$ 7.28-7.22 (m,3H),7.06-7.04 (m,1H), 6.85-6.81 (m,3H), 4.34-4.30 (m,6H), 4.10-3.81 (m,9H), 3.71 (s,3H), 3.15-3.04 (m,1H), 2.28 (s,3H).

**Example 16:** preparation of 1-(1'-((4-(difluoromethoxy)phenyl)sulfonyl)-1',4'-dihydro-2*H*,2'*H*-[3,3'-diazetidinyliden e]-1(4*H*)-yl)-2-(4-fluorophenyl)-3-hydroxypropan-1-one **(compound 16):**

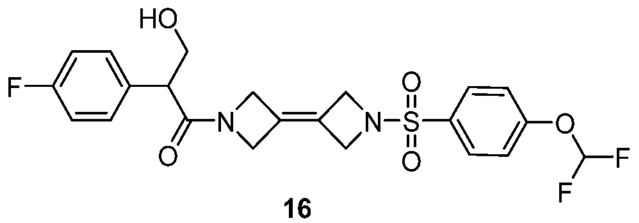

**16**

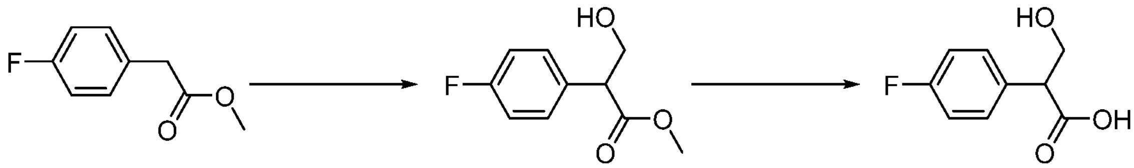

**16A** **16B** **16C**

**Step 1:** preparation of methyl 2-(4-fluorophenyl)-3-hydroxypropionate **(compound 16B)**

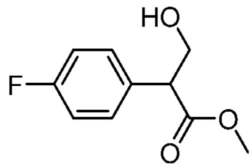

**16B**

Methyl 4-fluorophenylacetate (1.0 g, 5.95 mmol) and paraformaldehyde (562.5 mg, 6.24 mmol) were dissolved in dimethyl sulfoxide (40.0 mL), and then added with sodium methoxide (16.1 mg, 0.30 mmol). The reaction solution was reacted at room temperature for 4 hours. When the reaction was complete, the reaction system was added with water to quench the reaction. The aqueous phase was extracted with ethyl acetate (50 mL*3). The organic phases were combined, washed with a saturated brine, and dried over anhydrous sodium sulfate. After the organic phase was concentrated to dryness under reduced pressure, the residue was separated and purified through a silica gel column to obtain **compound 16B.**

MS (ESI) m/z 199.1 $(M+H)^+$.

**Step 2:** preparation of 2-(4-fluorophenyl)-3-hydroxypropionic acid **(compound 16C)**

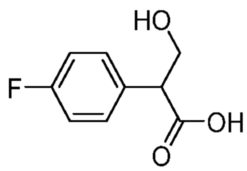

**16C**

Methyl 2-(4-fluorophenyl)-3-hydroxypropionate (600.0 mg, 3.03 mmol) was dissolved in methanol (9.0 mL) and water (6.0 mL), and then added with sodium hydroxide (242.2 mg, 6.05 mmol), and the reaction solution was stirred at 50°C for 3 hours. After the reaction was complete, the reaction solution was adjusted to pH = 2 with 1N hydrochloric acid aqueous solution, and extracted with dichloromethane (50 mL*3), and the organic phases were combined, washed with a saturated brine, and dried over anhydrous sodium sulfate. It was concentrated to obtain a white solid compound, and the residue was separated and purified through a silica gel column to obtain **compound 16C.**

MS (ESI) m/z 185.1 $(M+H)^+$.

In step 4 of Example 6, 3-((tert-butyldiphenylsilyl)oxy)-2-phenylpropionic acid was replaced by 2-(4-fluorophenyl)-3-hydroxypropionic acid, and the same preparation method as that of Example 6 was adopted to obtain **compound**

**16** of Example 16.

MS (ESI) m/z 483.1 (M+H)+.

$^1H$ NMR (400 MHz, DMSO-$d_6$) δ 7.91-7.89 (d, *J* = 7.7 Hz, 2H), 7.63-7.29 (m, 5H), 7.12-7.09 (m, 2H), 4.85 (s, 1H), 4.70 (d, *J* = 12.7 Hz, 1H), 4.39-4.22 (m, 7H), 3.85-3.83 (m, 1H), 3.70-3.64 (m, 1H), 3.51-3.41 (m, 1H).

**Example 17:** preparation of 1-(1'-((4-(difluoromethoxy)phenyl)sulfonyl)-1',4'-dihydro-2*H*,2'*H*-[3,3'-diazetidinyliden e]-1(4*H*)-yl)-3-hydroxy-3-methyl-2-phenylbutan-1-one **(compound 17):**

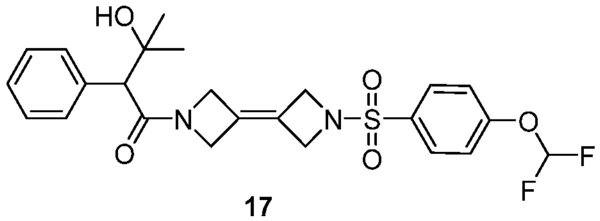

**17**

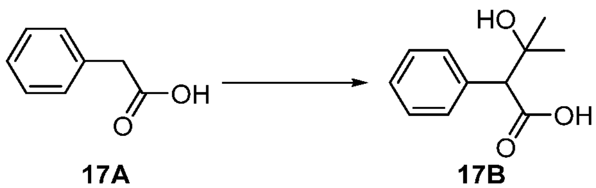

**17A** **17B**

**Step 1:** preparation of 3-hydroxy-3-methyl-2-phenylbutyric acid **(compound 17B)**

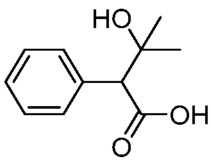

**17B**

2-phenylacetic acid (0.7 g, 5.14 mmol) was dissolved in tetrahydrofuran (11.0 mL), and added dropwise with isopropylmagnesium chloride (1.06 g, 10.28 mmol, 2M) at room temperature, and the reaction solution was stirred at 40°C for 1 hour after the dropwise addition. Then acetone (328.5 mg, 5.66 mmol) was added, and the reaction solution was continued to be stirred at 40°C for 1 hour. The reaction solution was adjusted to pH = 2 with 3N hydrochloric acid, the aqueous phase was extracted with dichloromethane (50 mL*3), and the organic phases were combined, washed with a saturated brine, and then dried over anhydrous sodium sulfate. It was concentrated to obtain a white solid compound, and the residue was separated and purified through a silica gel column to obtain **compound 17B.** MS (ESI) m/z 195.1 (M+H)+.

In step 4 of Example 6, 3-((tert-butyldiphenylsilyl)oxy)-2-phenylpropionic acid was replaced by 3-hydroxy-3-methyl-2-phenylbutyric acid, and the same preparation method as that of Example 6 was adopted to obtain **compound 17** of Example 17.

MS (ESI) m/z 493.2 (M+H)+.

$^1H$ NMR (400 MHz, DMSO-$d_6$) δ 7.90-7.88 (m, 2H), 7.63-7.19 (m, 8H), 4.91 (s, 1H), 4.75-4.72 (d, *J* = 13.3 Hz, 1H), 4.28-4.22 (m, 7H), 3.50 (s, 1H), 1.13 (s, 3H), 0.98 (s, 3H).

**Example 18:** preparation of 1-(1'-((2,3-dihydrobenzo[*b*][1,4]dioxin-6-yl)sulfonyl)-1',4'-dihydro-2*H*,2'*H*-[3,3'-dia zetidinylidene]-1(4*H*)-yl)-2-(4-fluorophenyl)-3-hydroxypropan-1-one (compound 18):

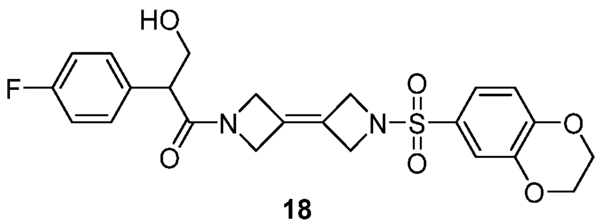

**18**

In step 10 of Example 1, (*S*)-3-((tert-butyldiphenylsilyl)oxy)-2-phenylpropionic acid was replaced by 3-((tert-butoxycarbonyl)(methyl)amino)-2-(3-methoxyphenyl)propionic acid, and the same preparation method as that of Example 1 was adopted to obtain **compound 18** of Example 15.

MS (ESI) m/z 475.1 $(M+H)^+$.

$^1$H NMR (400 MHz, DMSO-$d_6$) $\delta$ 7.34-7.24 (m, 4H), 7.14-7.10 (m, 2H), 7.06 (d, *J* = 8.4 Hz, 1H), 4.83-4.81 (m, 1H), 4.31-4.30 (m, 5H), 4.00-3.85 (m, 8H), 3.52-3.39 (m, 2H).

**Example 19:** preparation of 1-(1'-((2,3-dihydro-[1,4]dioxolane[2,3-*b*]pyridin-7-yl)sulfonyl)-1',4'-dihydro-2*H*,2'*H*-[3,3'-diazetidinylidene]-1(4*H*)-yl)-2-(4-fluorophenyl)-3-hydroxypropan-1-one **(compound 19):**

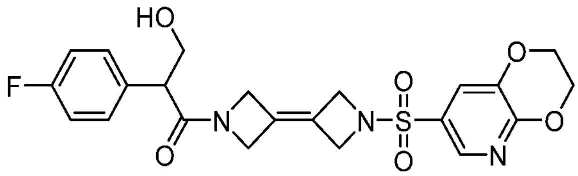

**19**

In step 10 of Example 1, 3-((tert-butyldiphenylsilyl)oxy)-2-phenylpropionic acid was replaced by 2-(4-fluorophenyl)-3-hydroxypropionic acid, and the same preparation method as that of Example 6 was adopted to obtain **compound 19** of Example 19.

MS (ESI) m/z 476.1 $(M+H)^+$.

$^1$H NMR (400 MHz, DMSO-$d_6$) $\delta$ 8.28(s, 1H), 7.66(s, 1H), 7.33-7.27 (m, 2H), 7.17-7.10 (m, 2H), 4.87 - 4.85 (m,1H), 4.75 - 4.68 (m, 1H), 4.55-4.53 (m, 2H), 4.40 - 4.21 (m, 9H), 3.85-3.80 (m, 1H), 3.70 - 3.55 (m, 2H).

**Example 20:** preparation of (*S*)-1-(1'-((8-fluoro-2,3-dihydrobenzo[*b*][1,4]dioxin-6-yl)sulfonyl)-1',4'-dihydro-2*H*,2'*H*-[3,3'-diazetidinylidene]-1(4*H*-yl)-3-hydroxy-2-phenylpropan-1-one **(compound 20):**

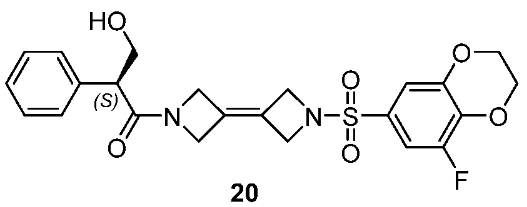

**20**

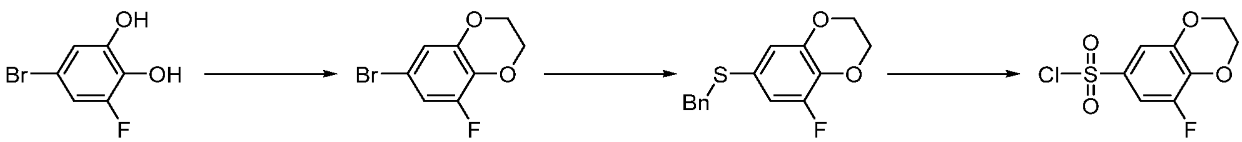

**20A** **20B** **20C** **20D**

**Step** 1: preparation of 7-bromo-5-fluoro-2,3-dihydrobenzo[*b*][1,4]dioxin **(compound 20B)**

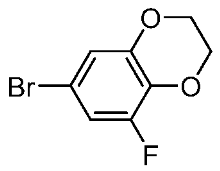

**20B**

5-bromo-3-fluorobenzene-1,2-diol (500 mg, 2.42 mmol), 1,2-dibromoethane (680.67 mg, 3.62 mmol), and potassium carbonate (1.0 g, 7.25 mmol) were added into *N*,*N*-dimethylformamide (5 mL), and finally the reaction system was stirred at 100°C for 16 hours. After the reaction was complete, the reaction solution was concentrated to dryness under reduced pressure, and the residue was separated and purified through a silica gel column to obtain **compound 20B.**
MS (ESI) m/z 233.0 $(M+H)^+$.

**Step 2:** preparation of 7-(benzylthio)-5-fluoro-2,3-dihydrobenzo[*b*][1,4]dioxin **(compound 20C)**

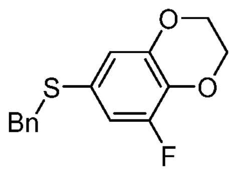

**20C**

7-bromo-5-fluoro-2,3-dihydrobenzo[*b*][1,4]dioxin (330 mg, 1.42 mmol), benzyl mercaptan (175.88 mg, 1.42 mmol), *N*,*N*-diisopropylethylamine (365.35 mg, 2.83 mmol), tris(dibenzylideneacetone)dipalladium-chloroform adduct (64.84 mg, 70.81 μmol), and 4,5-bis(diphenylphosphino)-9,9-dimethylxanthene (81.94 mg, 141.61 μmol) were added into 1 ,4-dioxane (6 mL), and finally the reaction system was placed and stirred at 80°C for 16 hours. After the reaction was complete, the reaction solution was concentrated to dryness under reduced pressure, and the residue was separated and purified through a silica gel column to obtain **compound 20C.**
MS (ESI) m/z 277.1 $(M+H)^+$.

**Step 3:** preparation of 8-fluoro-2,3-dihydrobenzo[*b*][1,4]dioxin-6-sulfonyl chloride **(compound 20D)**

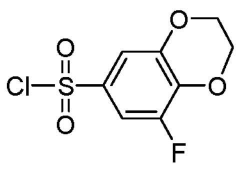

**20D**

7-(benzylthio)-5-fluoro-2,3-dihydrobenzo[*b*][1,4]dioxin (200 mg, 723.78 μmol) and 1,3-dichloro-5,5-dimethylhydantoin (285.20 mg, 1.45 mmol) were added into acetonitrile (7.5 mL), acetic acid (0.19 mL) and water (0.13 mL), and then the reaction system was stirred at 25°C for 16 hours. After the reaction was complete, the reaction solution was concentrated to dryness under reduced pressure, and the residue was separated and purified through a silica gel column to obtain **compound 20D.**
MS (ESI) m/z 253.0 $(M+H)^+$.

In step 8 of Example 1, 2,3-dihydro-[1 ,4]dioxino[2,3-*b*]pyridine-7-sulfonyl chloride was replaced by 8-fluoro-2,3-dihydrobenzo[*b*][1,4]dioxin-6-sulfonyl chloride, and the same preparation method as that of Example 1 was adopted to obtain **compound 20** of Example 20.

MS (ESI) m/z 475.1 $(M+H)^+$.

$^1H$ NMR (400 MHz, DMSO-$d_6$) $\delta$ 7.31 - 7.15 (m, 7H), 4.73- 4.70 (d, *J* = 13.0 Hz, 1H), 4.42 -4.30 (m, 12H), 3.90-3.87 (m, 1H), 3.47-3.44 (m, 1H), 3.33-3.31 (m, 1H).

**Example 21:** preparation of 2-amino-1-(1'-((4-(difluoromethoxy)phenyl)sulfonyl)-1',4'-dihydro-2*H*,2'*H*-[3,3'-diaze tidinylidene]-1(4*H*-yl)-2-phenylethan-1-one **(compound 21):**

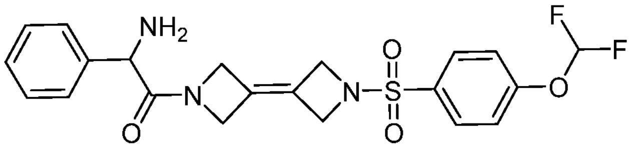

**21**

In step 4 of Example 6, 3-((tert-butyldiphenylsilyl)oxy)-2-phenylpropionic acid was replaced by 2-((tert-butoxycarbonyl)amino)-2-phenylacetic acid, and the same preparation method as that of Example 6 was adopted to obtain **compound 21** of Example 21.

MS (ESI) m/z 450.1 $(M+H)^+$.

$^1H$-NMR (400 MHz, DMSO-$d_6$) $\delta$ 7.91-7.89 (m, 2H), 7.45-7.43 (m, 3H), 7.32-7.22 (m, 5H), 4.72-4.69 (d, *J* = 10.4 Hz, 1H), 4.37-4.19 (m, 8H), 2.12 (s, 2H).

**Example 22:** preparation of 2-amino-1-(1'-((4-(difluoromethoxy)phenyl)sulfonyl)-1',4'-dihydro-2*H*,2'*H*-[3,3'-diaze tidinylidene]-1(4*H*)-yl)-2-(4-fluorophenyl)ethan-1-one **(compound 22):**

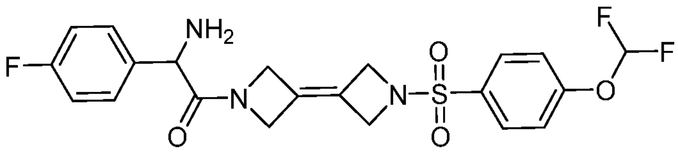

**22**

In step 4 of Example 6, 3-((tert-butyldiphenylsilyl)oxy)-2-phenylpropionic acid was replaced by 2-((tert-butoxycarbonyl)amino)-2-(4-fluorophenyl)acetic acid, and the same preparation method as that of Example 6 was adopted to obtain **compound 22** of Example 22.

MS (ESI) m/z 468.1 $(M+H)^+$.

$^1H$-NMR (400 MHz, DMSO-$d_6$) $\delta$ 7.91-7.89 (d, J = 8.8 Hz, 2H), 7.46-7.27 (m, 5H), 7.14-7.10 (m, 2H), 4.69 (s, 1H), 4.40-4.22 (m, 8H), 2.34-2.32 (m, 2H).

**Example 23:** preparation of 2-amino-1-(1'-((4-(difluoromethoxy)phenyl)sulfonyl)-1',4'-dihydro-2*H*,2'*H*-[3,3'-diaze tidinylidene]-1(4*H*)-yl)-2-(3-(trifluoromethyl)phenyl)ethan-1-one **(compound 23):**

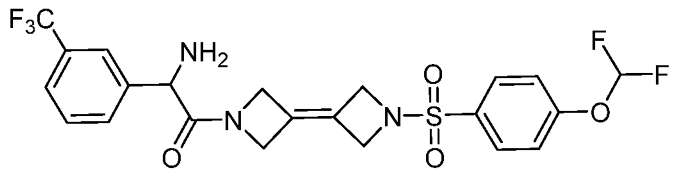

23

In step 4 of Example 6, 3-((tert-butyldiphenylsilyl)oxy)-2-phenylpropionic acid was replaced by 2-((tert-butoxycarbonyl)amino)-2-(3-(trifluoromethyl)phenyl)acetic acid, and the same preparation method as that of Example 6 was adopted to obtain **compound 23** of Example 23.

MS (ESI) m/z 518.1 $(M+H)^+$.

$^1$H-NMR (400 MHz, DMSO-$d_6$) δ 7.92-7.90 (d, *J* = 8.8 Hz, 2H), 7.67-7.51 (m, 4H), 7.46-7.44 (d, *J* = 8.5 Hz, 2H), 4.80-4.75 (d, *J* = 13.7 Hz, 1H), 4.53-4.21 (m, 8H), 2.33-2.27 (d, *J* = 24.7 Hz, 2H).

**Example 24:** preparation of 1-(1'-((4-(difluoromethoxy)phenyl) sulfonyl)-1',4'-dihydro-2*H*,2'*H*-[3,3'-diazetidinylidene]-1(4*H*-yl)-2-hydroxy-2-(2-met hylbenzo[*d*]thiazol-4-yl)ethan-1-one **(compound 24):**

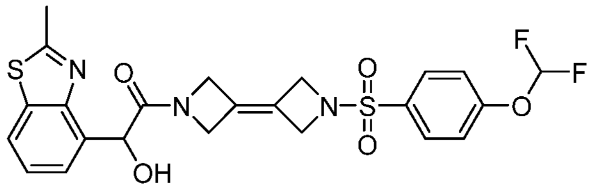

24

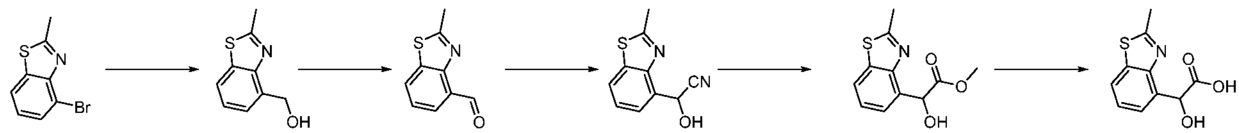

24A 24B 24C 24D 24E 24F

**Step 1:** preparation of 2-methyl-4-hydroxymethylbenzo[*d*]thiazole **(compound 24B):**

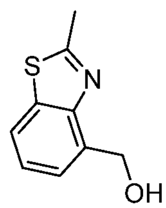

24B

4-bromo-2-methyl-1,3-benzothiazole (200 mg, 876.77 μmol) and tributylstannyl methanol (430 mg, 1.34 mmol) were dissolved in 1,4-dioxane (5 mL), and added with tetrakis(triphenylphosphine)palladium (100 mg, 86.58 μmol) at room temperature, and the resulting mixture was stirred at 100°C for 16 hours under the protection of nitrogen. After being cooled to room temperature, the reaction mixture was quenched with saturated ammonium chloride aqueous solution, the aqueous phase was extracted with ethyl acetate (10 mL*3), and the organic phases were combined, washed with a saturated brine, and dried over anhydrous sodium sulfate, and separated and purified through a silica gel column to obtain **compound 24B.**

MS (ESI) m/z 180.0 $(M+H)^+$.

**Step 2:** preparation of 2-methylbenzo[*d*]thiazole-4-carbaldehyde **(compound 24C):**

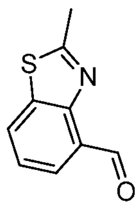

**24C**

At -78°C, oxalyl chloride (70 mg, 551.50 μmol) was slowly added dropwise into a solution of dimethyl sulfoxide (65 mg, 831.91 μmol) in dichloromethane (3 mL), and the resulting mixture was stirred at -78°C under the protection of nitrogen for 0.5 hours. Then a solution of 2-methyl-4-hydroxymethylbenzo[*d*]thiazole (50 mg, 278.96 μmol) in dichloromethane (2 mL) was added at the same temperature, the resulting mixture was stirred at this temperature for 1 hour and then added with triethylamine (0.3 mL), and the resulting mixture was continuously stirred for 0.5 hours, heated to room temperature, and then stirred for further 0.5 hours. After the reaction was complete, the reaction mixture was quenched with 10 mL brine. The aqueous phase was extracted with ethyl acetate (10 mL*3), and the organic phases were combined, washed with a saturated brine, and then dried over anhydrous sodium sulfate, and separated and purified through a silica gel column to obtain **compound 24C.**

MS (ESI) m/z 178.0(M+H)$^+$.

**Step 3:** preparation of 2-hydroxy-2-(2-methylbenzo[*d*]thiazol-4-yl)acetonitrile **(compound 24D):**

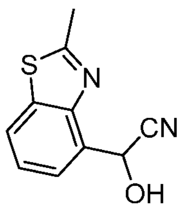

**24D**

2-methyl-1,3-benzothiazole-4-carbaldehyde (110 mg, 620.69 μmol) was dissolved in dichloromethane (3 mL), and added with trimethylsilyl cyanide (180 mg, 1.82 mmol) and zinc iodide (100 mg, 313.48 μmol) at room temperature. The resulting mixture was stirred at room temperature under the protection of nitrogen for 2 hours. The reaction mixture was quenched with 10 mL of brine. The aqueous phase was extracted with ethyl acetate (10 mL*3), and the organic phases were combined, washed with a saturated brine, and then dried over anhydrous sodium sulfate, and separated and purified through a silica gel column to obtain **compound 24D.**

MS (ESI) m/z 205.1 (M+H)$^+$.

**Step 4:** preparation of methyl 2-hydroxy-2-(2-methylbenzo[c/]thiazol-4-yl)acetate **(compound 24E):**

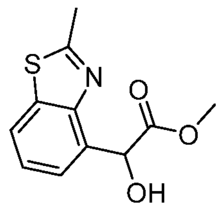

**24E**

2-hydroxy-2-(2-methyl-1,3-benzothiazol-4-yl)acetonitrile (110 mg, 538.56 μmol) was dissolved in methanol (3 mL), and then added dropwise with concentrated hydrochloric acid (3 mL), and the resulting mixture was stirred at 60°C for 2 hours under the protection of nitrogen. After the reaction was ended, the reaction mixture was concentrated and the pH value of the solution was adjusted to 7-8 with saturated sodium bicarbonate aqueous solution. The aqueous phase was extracted with ethyl acetate (10 mL*3), and the organic phases were combined, washed with a saturated brine, and then dried over anhydrous sodium sulfate, and separated and purified through a silica gel column to obtain

**compound 24E.**

MS (ESI) m/z 238.0 $(M+H)^+$.

**Step 5:** preparation of 2-hydroxy-2-(2-methylbenzo[*d*]thiazol-4-yl)acetic acid **(compound 24F):**

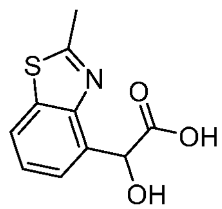

**24F**

Methyl 2-hydroxy-2-(2-methyl-1,3-benzothiazol-4-yl)acetate (125 mg, 526.82 μmol) was dissolved in a mixed solution of tetrahydrofuran (3 mL) and water (3 mL), and then added with lithium hydroxide monohydrate (100 mg, 2.44 mmol), and the resulting mixture was stirred at room temperature for 16 hours. The reaction mixture was concentrated, and the pH value of the solution was adjusted to 5-6 with 2N diluted hydrochloric acid. The aqueous phase was extracted with ethyl acetate (10 mL*3), and the organic phases were combined, washed with a saturated brine, and then dried over anhydrous sodium sulfate, and separated and purified through a silica gel column to obtain **compound 24F.**
MS (ESI) m/z 224.1 $(M+H)^+$.
In step 4 of Example 6, 3-((tert-butyldiphenylsilyl)oxy)-2-phenylpropionic acid was replaced by 2-hydroxy-2-(2-methylbenzo[*d*]thiazol-4-yl)acetic acid, and the same preparation method as that of Example 6 was adopted to obtain **compound 24** of Example 24.
MS (ESI) m/z 522.1 $(M+H)^+$.
$^1$H-NMR (400 MHz, DMSO-$d_6$) $\delta$ 7.96 - 7.90 (m, 3H), 7.65 - 7.29 (m, 5H), 5.88 - 5.78 (m, 2H), 4.85 - 4.59 (m, 2H), 4.31 - 4.23 (m, 6H), 2.77 (s, 3H).

**Example 25:** preparation of (*S*)-1-(1'-(benzo[*d*]thiazol-6-ylsulfonyl)-1',4'-dihydro-2*H*,2'*H*-[3,3'-diazetidinylidene] -1(4*H*)-yl)-2-(4-fluorophenyl)-3-hydroxypropan-1-one **(compound 25):**

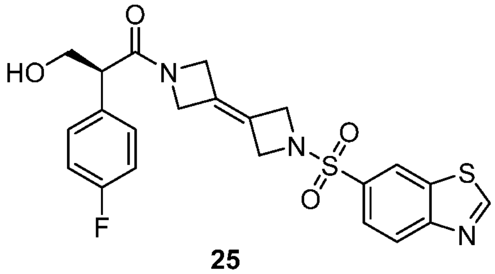

**25**

In step 8 of Example 1, 2,3-dihydro-[1,4]dioxino[2,3-*b*]pyridine-7-sulfonyl chloride was replaced by benzo[d]thiazole-6-sulfonyl chloride, and in step 10, (S)-3-((tert-butyldiphenylsilyl)oxy)-2-phenylpropionic acid was replaced by 2-(4-fluorophenyl)-3-hydroxypropionic acid, and the same preparation method as that of Example 1 was adopted to obtain **compound 25** of Example 25.
MS (ESI) m/z 474.1 $(M+H)^+$.
$^1$H-NMR (400 MHz, DMSO-$d_6$) δ 9.69 (s, 1H), 8.82 (s, 1H), 8.35-8.33 (d, *J* = 8.0 Hz, 1H), 7.96-7.94 (d, *J* = 8.0 Hz, 1H), 7.30-7.27 (m, 2H), 7.13-7.08 (m, 2H), 4.82 (s, 1H), 4.69-4.66 (d, *J* = 12.0 Hz, 1H), 4.35-4.16 (m, 8H), 3.83 (s, 1H), 3.64-3.61 (m, 1H).

**Example 26:** preparation of 2-(3-chlorophenyl)-1-(1'-((2,3-dihydrobenzo[b] [1 ,4]dioxin-6-yl)sulfonyl)-1',4'-dihydr o-2*H*,2'*H*-[3,3'-diazetidinylidene]-1(4*H*)-yl)-3-(methylamino)propan-1-one **(compound 26):**

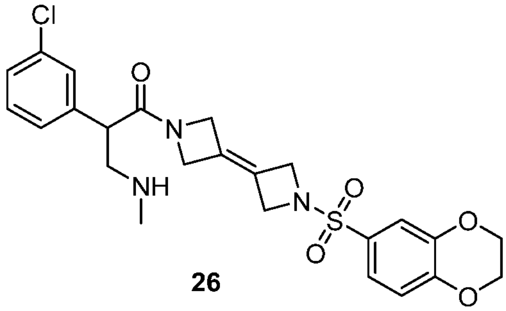

**26**

In step 10 of Example 1, 3-((tert-butyldiphenylsilyl)oxy)-2-phenylpropionic acid was replaced by 3-((tert-butoxycarbonyl)(methyl)amino)-2-(3-chlorophenyl)propionic acid, and the same preparation method as that of Example 6 was adopted to obtain compound **26** of Example 26.
MS (ESI) m/z 504.1 $(M+H)^+$.
$^1$H-NMR (400 MHz, DMSO-$d_6$) *δ* 7.36-7.24 (m, 6H), 7.07-7.05 (d, *J* = 26.1, 1H), 4.40-4.30 (m, 4H), 4.02-3.92 (m, 8H), 3.34 (s, 5H), 3.07-3.05 (m, 1H), 1.23 (s, 1H).

**Example 27:** preparation of (*S*)-1-(1'-((3-fluoro-4-(oxazol-2-yl)phenyl)sulfonyl)-1',4'-dihydro-2*H*,2'*H*-[3,3'-diazet idinylidene]-1(4*H*)-yl)-3-hydroxy-2-phenylpropan-1-one **(compound 27):**

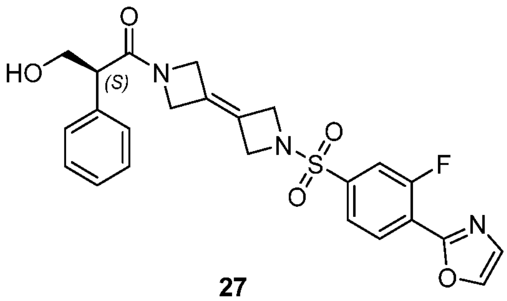

**27**

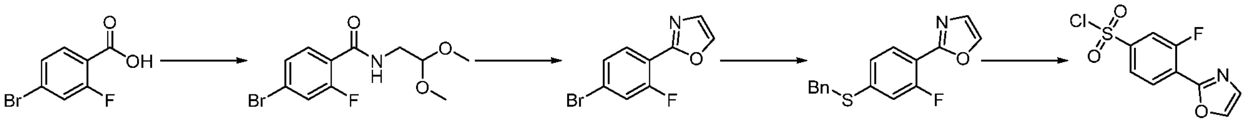

**27A** **27B** **27C** **27D** **27E**

**Step 1:** preparation of 4-bromo-*N*-(2,2-dimethoxyethyl)-2-fluorobenzamide **(compound 27B):**

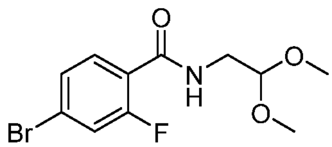

**27B**

4-bromo-2-fluoro-benzoic acid (3 g, 13.70 mmol) was dissolved in dichloromethane solution (40 mL), and then added with *N*,*N*-diisopropylethylamine (3.53 g, 27.40 mmol) and *N*,*N*-carbonyl diimidazole (2.17 g, 15.07 mmol). The reaction was stirred at 25°C for 30 minutes. The aforementioned mixture was added with 2,2-dimethoxyethylamine (1.58 g, 15.07 mmol), and stirred at 25°C for another 4 hours. After the reaction was complete, it was separated and purified through a silica gel column to obtain **compound 27B.**

MS (ESI) m/z 306.1 $(M+H)^+$.

**Step 2:** preparation of 2-(4-bromo-2-fluorophenyl)oxazole **(compound 27C):**

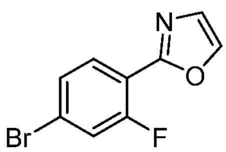

**27C**

4-bromo-*N*-(2,2-dimethoxyethyl)-2-fluoro-benzamide (2.1 g, 6.86 mmol) was added into polyphosphoric acid (20 mL) and reacted at 90°C for 5 hours. After the reaction was complete, it was separated and purified through a silica gel column to obtain **compound 27C.**

MS (ESI) m/z 243.0 $(M+H)^+$.

**Step 3:** preparation of 2-(4-(benzylthio)-2-fluorophenyl)oxazole **(compound 27D):**

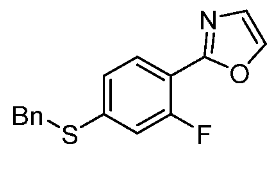

**27D**

Benzyl mercaptan (410.51 mg, 3.31 mmol), *N,N*-diisopropylethylamine (1.07 g, 8.26 mmol), tris(dibenzylideneacetone)dipalladium (342.09 mg, 330.52 μmol) and 4,5-bis(diphenylphosphino)-9,9-dimethylxanthene (382.74 mg, 661.04 μmol) were added into a solution of 2-(4-bromo-2-fluorophenyl)oxazole (0.8 g, 3.31 mmol) in 1,4-dioxane (10 mL), which were stirred at 90°C under the protection of nitrogen for 3 hours. After the reaction was complete, it was separated and purified through a silica gel column to obtain **compound 27D.**

MS (ESI)m/z 286.3 $(M+H)^+$.

**Step 4:** preparation of 3-fluoro-4-(oxazol-2-yl)benzenesulfonyl chloride **(compound 27E):**

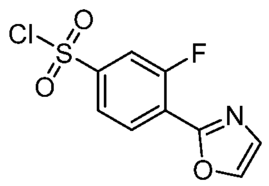

**27E**

2-(4-benzylsulfanyl-2-fluoro-phenyl)oxazole (0.7 g, 2.45 mmol) was dissolved in a mixed solution of dichloromethane (10 mL), water (176.63 mg, 9.81 mmol) and glacial acetic acid (735.98 mg, 12.27 mmol), and then added dropwise with sulfonyl chloride (1.32 g, 9.81 mmol) at 0°C. The resulting mixed system was stirred at 20°C for 5 hours. After the reaction was complete, it was separated and purified through a silica gel column to obtain **compound 27E.**

MS (ESI) m/z 262.0 $(M+H)^+$.

In step 8 of Example 1, 2,3-dihydro-[1 ,4]dioxino[2,3-*b*]pyridine-7-sulfonyl chloride was replaced by 3-fluoro-4-(oxazol-2-yl)benzenesulfonyl chloride, and the same preparation method as that of Example 1 was adopted to obtain **compound 27** of Example 27.

MS (ESI) m/z 484.1 $(M+H)^+$.

$^1H$ NMR (400 MHz, DMSO-$d_6$) δ 8.41 (s, 1H), 8.37-8.35 (m, 1H), 8.02-8.01 (m, 1H), 7.74-7.73 (m, 1H), 7.53 (s, 1H), 7.27-7.21 (m, 5H), 4.81-4.79 (t, *J* = 0.8 Hz, 1H), 4.71-4.68 (d, *J* = 1.2 Hz, 1H), 4.35-4.21 (m, 7H), 3.88-3.87 (m, 1H), 3.62-3.58 (m, 1H), 3.48-3.42 (m, 1H).

**Example 28:** preparation of 2-(3-chloro-4-fluoro-2-methoxyphenyl)-1-(1'-(2,3-dihydrobenzo[*b*][1,4]dioxin-6-yl)s ulfonyl)-1',4'-dihydro-2*H*,2'*H*-[3,3'-diazetidinylidene]-1(4*H*-yl)-3-(methylamino)prop an-1-one **(compound 28):**

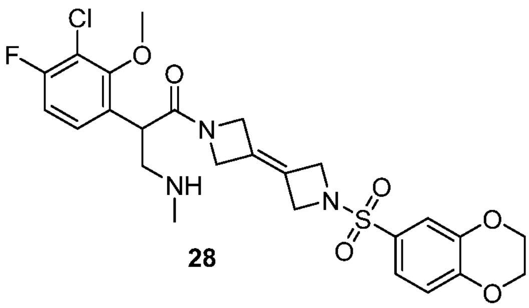

28

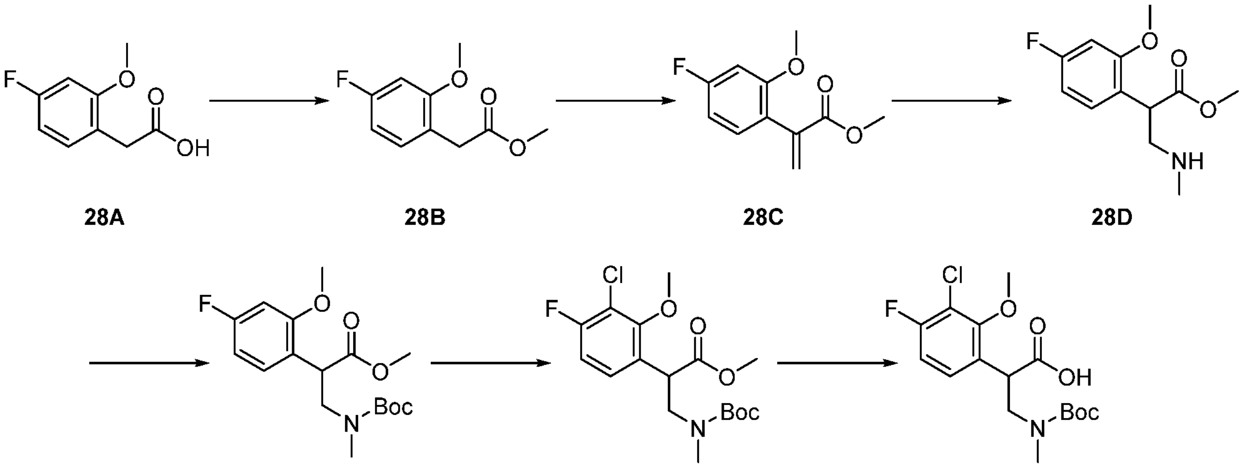

28A 28B 28C 28D

28E 28F 28G

**Step** 1: preparation of methyl 2-(4-fluoro-2-methoxyphenyl)acetate **(compound 28B):**

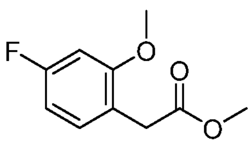

**28B**

2-(4-fluoro-2-methoxyphenyl)acetic acid (1.0 g, 5.43 mmol) was dissolved in anhydrous methanol (100 mL), and then slowly added with thionyl chloride (1.29 g, 10.8 mmol). The reaction solution was reacted at 70°C for 3 hours. After the reaction was complete, it was separated and purified through a silica gel column to obtain **compound 28B.**

MS (ESI) m/z 199.1 $(M+H)^+$.

**Step 2:** preparation of methyl 2-(4-fluoro-2-methoxyphenyl)acrylate **(compound 28C):**

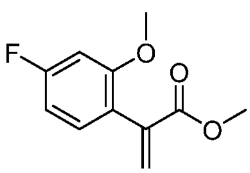

**28C**

Methyl 2-(4-fluoro-2-methoxyphenyl)acetate (900.0 mg, 4.54 mmol) was dissolved in *N*,*N*-dimethylformamide (100.0 mL), and then added with potassium carbonate (1.57 g, 11.4 mmol), paraformaldehyde (518.3 mg, 5.76 mmol) and tetrabutylammonium bromide (167.7 mg, 0.45 mmol), and the reaction solution was stirred at 60°C for 1 hour. After the reaction was complete, it was separated and purified through a silica gel column to obtain

**compound 28C.**

MS (ESI) m/z 211.1 $(M+H)^+$.

**Step 3:** preparation of methyl 2-(4-fluoro-2-methoxyphenyl)-3-(methylamino)propionate **(compound 28D):**

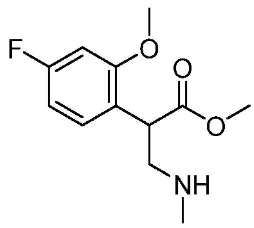

**28D**

Methyl 2-(4-fluoro-2-methoxyphenyl)acrylate (515.0 mg, 2.45 mmol) was dissolved in a solution of methylamine in tetrahydrofuran (20 mL), and the reaction solution was stirred at room temperature for 16 hours. After the reaction was complete, it was separated and purified through a silica gel column to obtain **compound 28D.**

MS (ESI) m/z 242.1 $(M+H)^+$.

**Step 4:** preparation of methyl 3-(tert-butoxycarbonyl)(methyl)amino)-2-(4-fluoro-2-methoxyphenyl)propionate **(compound 28E):**

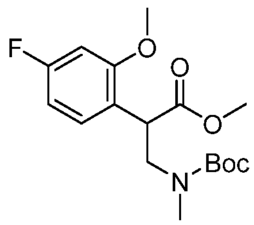

**28E**

Methyl 2-(4-fluoro-2-methoxyphenyl)-3-(methylamino)propionate (406 mg, 1.68 mmol) was dissolved in dichloromethane (50 mL), and then added with triethylamine (340 mg, 3.37 mmol). The temperature of the reaction system was reduced to 0°C, the reaction system was slowly added with di-tert-butyl dicarbonate (440 mg, 2.02 mmol), and the reaction solution was stirred at room temperature for 16 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 28E.**

MS (ESI) m/z 342.1 $(M+H)^+$.

**Step 5:** preparation of methyl 3-(tert-butoxycarbonyl)(methyl)amino)-2-(3-chloro-4-fluoro-2-methoxyphenyl) propion ate **(compound 28F):**

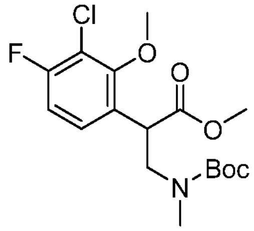

**28F**

Methyl 3-((tert-butoxycarbonyl)(methyl)amino)-2-(4-fluoro-2-methoxyphenyl)propionate (393 mg, 1.15 mmol) was dissolved in *N*,*N*-dimethylformamide (100.0 mL), and then added with *N*-chlorosuccinimide (461.3 mg, 3.45 mmol). The reaction solution was reacted at room temperature for 16 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 28F.**

MS (ESI) m/z 376.1 $(M+H)^+$.

**Step 6:** preparation of 3-((tert-butoxycarbonyl)(methyl)amino)-2-(3-chloro-4-fluoro-2-methoxyphenyl) propion ic acid **(compound 28G):**

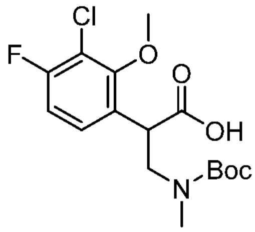

**28G**

Methyl

3-((tert-butoxycarbonyl) (m ethyl)am ino)-2-(3-chloro-4-fluoro-2-m ethoxyphenyl) propion ate (302 mg, 0.81 mmol) was dissolved in tetrahydrofuran (50 mL) and water (50 mL), and then added with lithium hydroxide (38.6 g, 1.61 mmol). The reaction solution was reacted at room temperature for 16 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 28G.**

MS (ESI) m/z 362.1 $(M+H)^+$.

In step 8 of Example 1, 2,3-dihydro-[1,4]dioxino[2,3-*b*]pyridine-7-sulfonyl chloride was replaced by 2,3-dihydrobenzo[*b*][1,4]dioxin-6-sulfonyl chloride, and in step 10, (S)-3-((tert-butyldiphenylsilyl)oxy)-2-phenylpropionic acid was replaced by 3-((tert-butoxycarbonyl)(methyl)amino)-2-(3-chloro-4-fluoro-2-methoxyphenyl) propion ic acid, and the same preparation method as that of Example 1 was adopted to obtain **compound 28** of Example 28.

MS (ESI) m/z 552.1 $(M+H)^+$.

$^1$H-NMR (400 MHz, DMSO-$d_6$) δ 7.25-7.21 (m, 3H), 7.13-7.10 (d, *J* = 8.0 Hz, 1H), 7.04-7.02 (d, *J* = 8.0 Hz, 1H), 4.45-4.15 (m, 6H), 3.98 (s, 6H), 3.78 (s, 3H), 2.96-2.94 (d, *J* = 8.0 Hz, 1H), 2.20 (s, 2H), 1.41-0.82 (m, 4H).

**Example 29:** preparation of (1'-(benzo[*d*]thiazol-6-ylsulfonyl)-1',4'-dihydro-2*H*,2'*H*-[3.3'-diazetidinylidene]-1(4*H*) -yl)(4-phenyltetrahydro-2*H*-pyran-4-yl)methanone **(compound 29):**

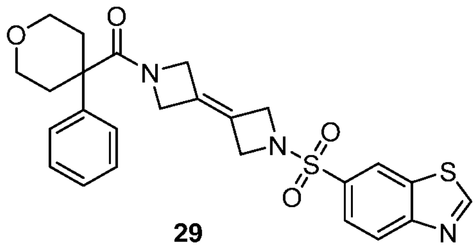

**29**

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 4-phenyltetrahydro-2*H*-pyran-4-carboxylic acid, and the same preparation method as that of Example 1 was adopted to obtain **compound 29** of Example 29.

MS (ESI) m/z 496.2 $(M+H)^+$.

$^1H$-NMR (400 MHz, DMSO-$d_6$) δ 9.68 (s, 1H), 8.78-8.75 (d, *J* = 1.9 Hz, 1H), 8.32-8.29 (d, *J* = 8.5 Hz, 1H), 7.92-7.87 (m, 1H), 7.37-7.31 (m, 2H), 7.28-7.19 (m, 3H), 4.31-4.16 (m, 6H), 3.97-3.87 (m, 2H), 3.71-3.67 (m, 2H), 3.56-3.44 (m, 2H), 2.17-2.07 (m, 2H), 1.83-1.76 (m, 2H).

**Example 30:** preparation of (*S*)-1-(1'-((2,3-dihydrobenzo[b][1,4]dioxin-6-yl)sulfonyl)-1',4'-dihydro-2*H*,2'*H*-[3,3'-diazetidinylidene]-1(4*H*)-yl)-2-(4-fluorophenyl)-3-hydroxypropan-1-one **(compound 30):**

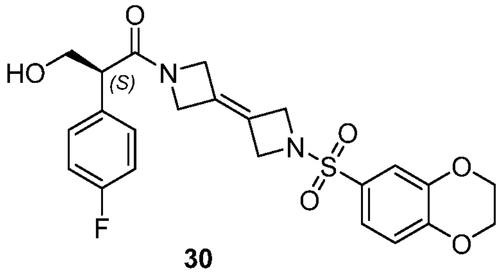

**30**

In step 8 of Example 1, 2,3-dihydro-[1 ,4]dioxino[2,3-*b*]pyridine-7-sulfonyl chloride was replaced by 2,3-dihydrobenzo[*b*][1,4]dioxin-6-sulfonyl chloride, and in step 10, (*S*)-3-((tert-butyldiphenylsilyl)oxy)-2-phenylpropionic acid was replaced by (*S*)-2-(4-fluorophenyl)-3-hydroxypropionic acid, and the same preparation method as that of Example 1 was adopted to obtain **compound 30** of Example 30.

MS (ESI) m/z 475.1 $(M+H)^+$.

$^1H$ NMR (400 MHz, DMSO-$d_6$) δ 7.34-7.24 (m, 4H), 7.14-7.10 (m, 2H), 7.06 (d, *J* = 8.4 Hz, 1H), 4.83-4.81 (m, 1H), 4.31-4.30 (m, 5H), 4.00-3.85 (m, 8H), 3.52-3.39 (m, 2H).

**Example 31:** preparation of (*R*)-1-(1'-((2,3-dihydrobenzo[*b*][1,4]dioxin-6-yl)sulfonyl)-1',4'-dihydro-2*H*,2'*H*-[3,3'-diazetidinylidene]-1(4*H*)-yl)-2-(4-fluorophenyl)-3-hydroxypropan-1-one **(compound 31):**

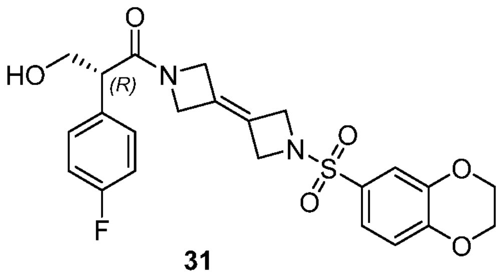

**31**

In step 8 of Example 1, 2,3-dihydro-[1 ,4]dioxino[2,3-*b*]pyridine-7-sulfonyl chloride was replaced by 2,3-dihydrobenzo[b][1,4]dioxin-6-sulfonyl chloride, and in step 10, (S)-3-((tert-butyldiphenylsilyl)oxy)-2-phenylpropionic acid was

replaced by (R)-2-(4-fluorophenyl)-3-hydroxypropionic acid, and the same preparation method as that of Example 1 was adopted to obtain **compound 31** of Example 31.

MS (ESI) m/z 475.1 $(M+H)^+$.

$^1H$ NMR (400 MHz, DMSO-$d_6$) $\delta$ 7.34-7.24 (m, 4H), 7.14-7.10 (m, 2H), 7.06 (d, *J* = 8.4 Hz, 1H), 4.83-4.81 (m, 1H), 4.31-4.30 (m, 5H), 4.00-3.85 (m, 8H), 3.52-3.39 (m, 2H). **Example 32:** preparation of 1-(1'-(benzo[*d*]thiazol-6-ylsulfonyl)-1',4'-dihydro-2*H*,2'*H*-[3,3'-diazetidinylidene]-1(4 *H*)-yl)-3-hydroxy-2-(naphthalene-2-yl)propan-1-one **(compound 32):**

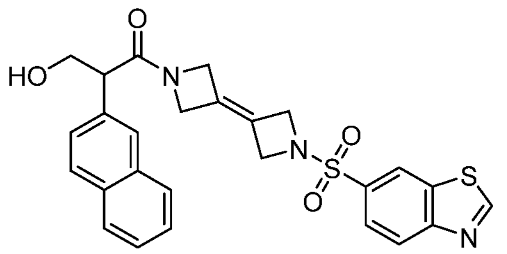

**32**

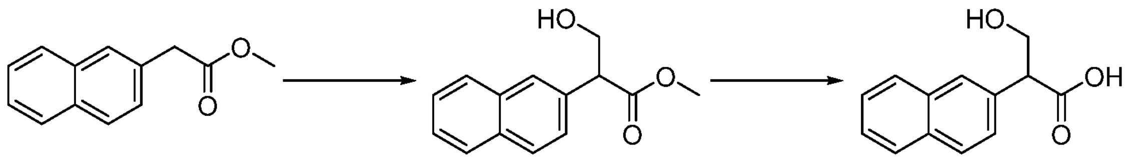

**32A** **32B** **32C**

**Step** 1: preparation of methyl 3-hydroxy-2-(naphthalene-2-yl)propionate **(compound 32B):**

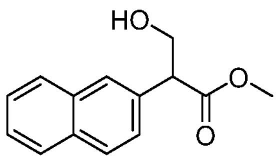

**32B**

Methyl 2-(naphthalene-2-yl)acetate (1.0 g, 4.99 mmol) and paraformaldehyde (449.9 mg, 4.99 mmol) were dissolved in dimethyl sulfoxide (40.0 mL), and then added with sodium methoxide (27.0 mg, 0.50 mmol). The reaction solution was reacted at room temperature for 4 hours. After the reaction was complete, the reaction solution was diluted by adding water. The aqueous phase was extracted with ethyl acetate (10 mL*3). The organic phases were combined, washed with a saturated brine, and dried over anhydrous sodium sulfate, and separated and purified through a silica gel column to obtain **compound 32B.**

MS (ESI) m/z 231.1 $(M+H)^+$.

**Step 2:** preparation of 3-hydroxy-2-(naphthalene-2-yl)propionic acid **(compound 32C):**

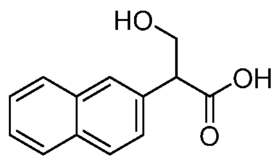

**32C**

Methyl 3-hydroxy-2-(naphthalene-2-yl)propionate (260.0 mg, 1.13 mmol) was dissolved in a mixed solution of tetrahydrofuran (60.0 mL) and water (40.0 mL), and then added with sodium hydroxide (45.2 mg, 1.13 mmol), and the reaction solution was stirred at room temperature for 5 hours. After the reaction was complete, the reaction solution was adjusted to pH 2 with 1N hydrochloric acid, and extracted with dichloromethane (100 mL*3), and the organic phases were combined, washed with a saturated brine, and dried over anhydrous sodium sulfate. After concentration, it was directly used for the next step of reaction.

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-hydroxy-2-(naphthalene-2-yl)propionic acid, and the same preparation method as that of Example 1 was adopted to obtain **compound 32** of Example 32.

MS (ESI) m/z 506.1 $(M+H)^+$.

$^1$H-NMR (400 MHz, DMSO-$d_6$) δ 9.64 (s, 1H), 8.78-8.77 (d, J = 4.0 Hz, 1H), 8.31-8.29 (d, J = 8.0 Hz, 1H), 7.92-7.90 (m, 1H), 7.85-7.80 (m, 3H), 7.72 (s, 1H), 7.47-7.44 (m, 2H), 7.40-7.33 (m, 1H), 4.80-4.78 (m, 1H), 4.72 (s, 1H), 4.34-4.26 (m, 6H), 4.19 (s, 1H), 3.95-3.92 (m, 1H), 3.77-3.74 (m, 1H), 3.57-3.53 (m, 1H).

**Example 33:** preparation of 1-(1'-(benzo[*d*]thiazol-6-ylsulfonyl(1',4'-dihydro-2*H*,2'*H*-[3,3'-diazetidinylidene]-1(4*H* )-yl)-3-hydroxy-2-(3-(4-methylpiperazin-1-yl)phenyl)propan-1-one **(compound 33):**

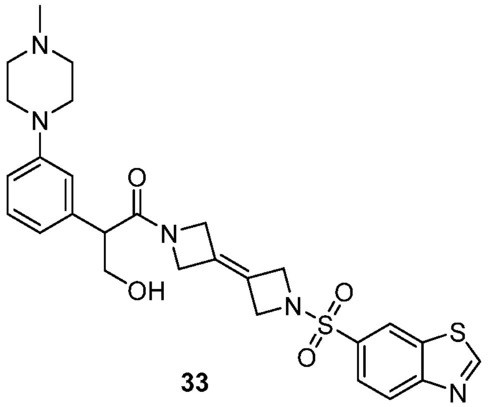

33

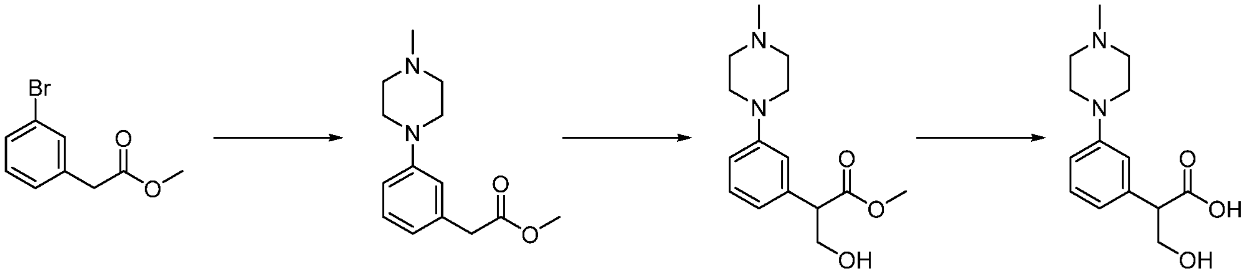

33A 33B 33C 33D

**Step** 1: preparation of methyl 2-(3-(4-methylpiperazin-1-yl)phenyl)acetate **(compound 33B)**

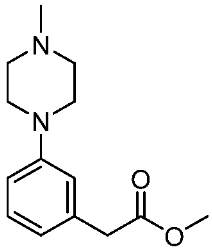

**33B**

Methyl 2-(3-bromophenyl)acetate (5.0 g, 21.83 mmol), 1-methylpiperazine (3.3 g, 32.74 mmol), 4,5-bis(diphenylphosphino)-9,9-dimethylxanthene (1.3 g, 2.18 mmol) and cesium carbonate (10.7 g, 32.74 mmol) were dissolved in toluene (100.0 mL), and then added with tris(dibenzylideneacetone)dipalladium (399.8 mg, 0.44 mmol). After the addition was completed, the reaction system was placed under a nitrogen protection environment, heated to 110°C, and stirred for 16 hours. After the reaction was complete, the reaction solution was diluted by adding water. The aqueous phase was extracted with ethyl acetate (150.0 mL*3). The organic phases were combined, washed with a saturated brine, and dried over anhydrous sodium sulfate, and separated and purified through a silica gel column to obtain **compound 33B.**

MS (ESI) m/z 249.2 $(M+H)^+$.

**Step 2:** preparation of methyl 3-hydroxy-2-(3-(4-methylpiperazin-1-yl)phenyl)propionate **(compound 33C)**

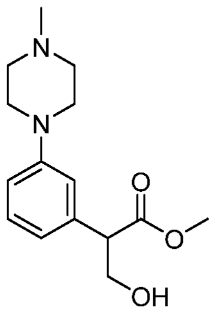

**33C**

Methyl 2-(3-(4-methylpiperazin-1-yl)phenyl)acetate (1.8 g, 7.25 mmol) and paraformaldehyde (685.6 mg, 7.61 mmol) were dissolved in dimethyl sulfoxide (90.0 mL), and then added with sodium methoxide (39.2 mg, 0.72 mmol). The reaction solution was reacted at 50°C for 4 hours. The reaction solution was diluted by adding water. The aqueous phase was extracted with dichloromethane (100 mL*3). The organic phases were combined, washed with a saturated brine, and dried over anhydrous sodium sulfate, and separated and purified through a silica gel column to obtain **compound 33C.**

MS (ESI) m/z 279.2 $(M+H)^+$

**Step 2:** preparation of 3-hydroxy-2-(3-(4-methylpiperazin-1-yl)phenyl)propionic acid **(compound 33D)**

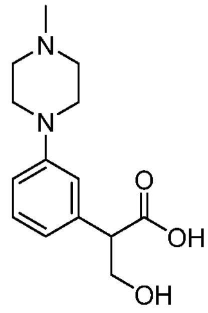

**33D**

Methyl 3-hydroxy-2-(3-(4-methylpiperazin-1-yl)phenyl)propionate (90.0 mg, 0.32 mmol) was dissolved in methanol (2.5 mL) and water (2.5 mL), and then added with sodium hydroxide (25.9 mg, 0.64 mmol), and the reaction solution was stirred at room temperature for 3 hours. After the reaction was complete, the reaction solution was adjusted to pH 2 with 1N hydrochloric acid, concentrated, and then used directly in the next step of reaction.

MS (ESI) m/z 265.2 $(M+H)^+$.

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (S)-3-((tert-butyldiphenylsilyl)oxy)-2-phenylpropionic acid was replaced by 3-hydroxy-2-(3-(4-methylpiperazin-1-yl)phenyl)propionic acid, and the same preparation method as that of Example 1 was adopted to obtain compound 33 of Example 33.

MS (ESI) m/z 554.2 $(M+H)^+$.

$^1$H NMR (400 MHz, DMSO-$d_6$) $\delta$ 9.68 (s, 1H), 8.81-8.80 (d, *J* = 1.7 Hz, 1H), 8.33-8.31 (d, *J* = 8.6 Hz, 1H), 7.5-7.94 (m, 1H), 7.11-7.07 (m, 1H), 6.78-6.76 (m, 2H), 6.64-6.62 (d, *J* = 7.6 Hz, 1H), 4.71-4.63 (m, 2H), 4.42-4.11 (m, 7H), 3.88-3.81 (m, 1H), 3.54-3.49 (m, 1H), 3.44-3.38 (m, 1H), 3.17-2.97 (m, 4H), 2.47-2.38 (m, 4H), 2.21 (s, 3H).

**Example 34:** preparation of 1-(1'-(benzo[*d*]thiazol-6-ylsulfonyl)-1',4'-dihydro-2*H*,2'*H*-[3,3'-diazetidinylidene]-1(4 *H*)-yl)-3-hydroxy-2-(4-(1-methyl-1*H*-pyrazol-4-yl) phenyl)propan-1-one **(compound 34):**

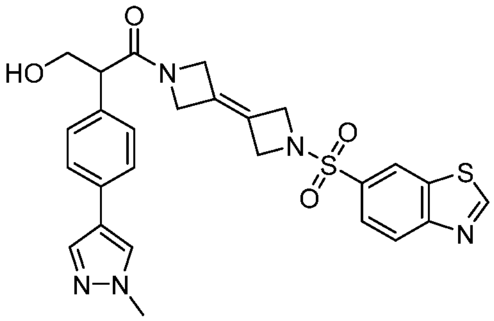

**34**

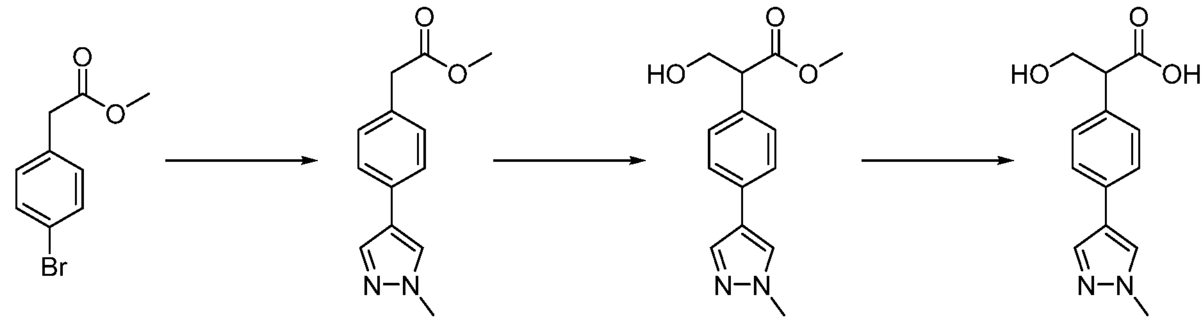

**34A** **34B** **34C** **34D**

**Step** 1: preparation of methyl 2-(4-(1-methyl-1*H*-pyrazol-4-yl)phenyl)acetate **(compound 34B)**

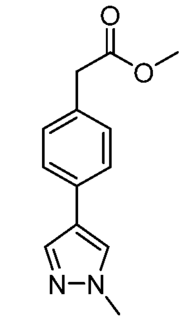

**34B**

Methyl 2-(4-bromophenyl)acetate (1.0 g, 4.37 mmol), (1-methyl-1*H*-pyrazol-4-yl)boronic acid (604.7 mg, 4.80 mmol) and potassium carbonate (1.2 g, 8.74 mmol) were dissolved in a mixed solution of dioxane and water (4:1, 20.0 mL), and then added with tetrakis(triphenylphosphine)palladium (504.5 mg, 0.44 mmol). After the addition was completed, the reaction system was placed under a nitrogenprotection environment, heated to 80°C, and stirred for 16 hours. After the reaction was complete, the reaction solution was diluted by adding water. The aqueous phase was extracted with ethyl acetate (50.0 mL*3). The organic phases were combined, washed with a saturated brine, and dried over anhydrous sodium sulfate, and separated and purified through a silica gel column to obtain **compound 34B.**

MS (ESI) m/z 231.2 $(M+H)^+$.

**Step 2:** preparation of methyl 3-hydroxy-2-(4-(1-methyl-1*H*-pyrazol-4-yl)phenyl)propionate (compound 34C)

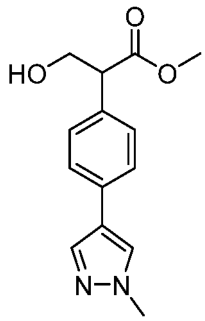

**34C**

Methyl 2-(4-(1-methyl-1*H*-pyrazol-4-yl)phenyl)acetate (800.0 mg, 3.47 mmol) and paraformaldehyde (313.0 mg, 3.47 mmol) were dissolved in dimethyl sulfoxide (50.0 mL), and then added with sodium methoxide (62.6 mg, 0.35 mmol). The reaction solution was reacted at room temperature for 4 hours. After the reaction was complete, the reaction solution was diluted by adding water. The aqueous phase was extracted with ethyl acetate (50 mL*3). The organic phases were combined, washed with a saturated brine, and dried over anhydrous sodium sulfate, and separated and purified through a silica gel column to obtain **compound 34C.**

MS (ESI) m/z 261.2 $(M+H)^+$.

**Step 3:** preparation of 3-hydroxy-2-(4-(1-methyl-1*H*-pyrazol-4-yl)phenyl)propionic acid **(compound 34D)**

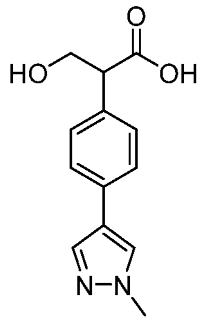

**34D**

Methyl 3-hydroxy-2-(4-(1-methyl-1*H*-pyrazol-4-yl)phenyl)propionate (200.0 mg, 0.77 mmol) was dissolved in a mixed solution of methanol (6.0 mL) and water (4.0 mL), and then added with sodium hydroxide (61.5 mg, 1.54 mmol), and the reaction solution was stirred at room temperature for 16 hours. After the reaction was complete, the reaction solution was adjusted to pH 2 with 1N hydrochloric acid, concentrated, and then used directly in the next step of reaction.

MS (ESI) m/z 267.1 $(M+H)^+$.

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-hydroxy-2-(4-(1-methyl-1*H*-pyrazol-4-yl)phenyl)propionic acid, and the same preparation method as that of Example 1 was adopted to obtain **compound 34** of Example 34.

MS (ESI) m/z 536.1 $(M+H)^+$.

$^1$H NMR (400 MHz, DMSO-$d_6$) $\delta$ 9.67 (s, 1H), 8.81-8.80 (d, *J* = 1.6 Hz, 1H), 8.32-8.31 (d, *J* = 8.6 Hz, 1H), 8.06 (s, 1H), 7.95-7.92 (d, *J* = 8.6, 1H), 7.79 (s, 1H), 7.45-7.43 (d, *J* = 8.2 Hz, 2H), 7.20-7.18 (d, *J* = 8.2 Hz, 2H), 4.77-4.74 (t, *J* = 5.3 Hz, 1H), 4.68-4.65 (m, 1H), 4.44-4.12 (m, 7H), 3.90-3.81 (m, 4H), 3.60-3.54 (m, 1H), 3.49-3.40 (m, 1H).

**Example 35:** preparation of (*S*)-3-hydroxy-1-(1'-((2-methylbenzo[*d*]thiazol-6-yl)sulfonyl)-1',4'-dihydro-2*H*,2'*H*-[3 ,3'-diazetidinylidene]-1(4*H*)-yl)-2-phenylpropan-1-one **(compound 35):**

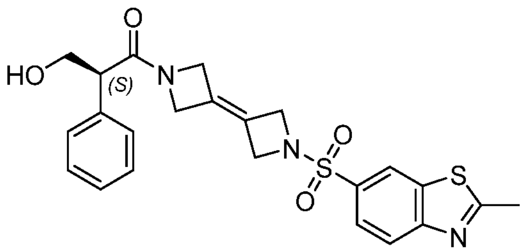

**35**

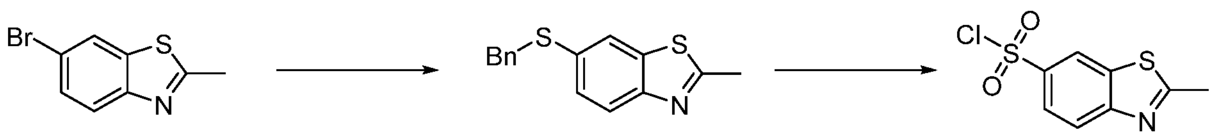

**35A** **35B** **35C**

**Step 1:** preparation of 6-(benzylthio)-2-methylbenzo[d]thiazole **(compound 35B)**

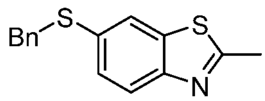

**35B**

6-bromo-2-methylbenzo[*d*]thiazole (2.0 g, 8.77 mmol), benzyl mercaptan (1.3 g, 10.52mmol), 4,5-bis(diphenylphosphino)-9,9-dimethylxanthene (0.5 g, 0.88 mmol) and *N*,*N*-diisopropylethylamine (3.4 g, 26.30 mmol) were dissolved in 1,4-dioxane (50.0 mL), and then added with tris(dibenzylideneacetone)dipalladium (401.4 mg, 0.44 mmol). After the addition was completed, the reaction system was placed under a nitrogen protection environment, heated to 100°C, and stirred for 16 hours. After the reaction was complete, the reaction solution was diluted by adding water. The aqueous phase was extracted with ethyl acetate (150.0 mL*3). The organic phases were combined, washed with a saturated brine, and dried over anhydrous sodium sulfate, and separated and purified through a silica gel column to obtain compound **35B.**

MS (ESI) m/z 272.1 $(M+H)^+$.

**Step 2:** preparation of 2-methylbenzo[*d*]thiazole-6-sulfonyl chloride **(compound 35C)**

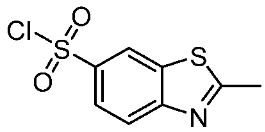

**35C**

6-(benzylthio)-2-methylbenzo[*d*]thiazole (1.0 g, 3.68 mmol) was dissolved in a mixed solution of acetonitrile (40.0 mL), acetic acid (1.5 mL) and water (1.0 mL), and then the reaction system was added with 1,3-dichloro-5,5-dimethylhydantoin (1.5 g, 7.37 mmol) at 0°C. After the addition was complete, the reaction solution was reacted at room temperature for 4 hours. After the reaction was complete, the reaction solution was diluted by adding water. The aqueous phase was extracted with ethyl acetate (50 mL*3). The organic phases were combined, washed with a saturated brine, and dried over anhydrous sodium sulfate, and separated and purified through a silica gel column to obtain **compound 35C.**

MS (ESI) m/z 248.0 $(M+H)^+$.

In step 8 of Example 1, 2,3-dihydro-[1 ,4]dioxino[2,3-b]pyridine-7-sulfonyl chloride was replaced by 2-methylbenzo[*d*]thiazole-6-sulfonyl chloride, and the same preparation method as that of Example 1 was adopted to obtain **compound 35** of Example 35.

MS (ESI) m/z 470.1 $(M+H)^+$.

$^1$H NMR (400 MHz, DMSO-$d_6$) $\delta$ 8.66-8.65 (d, *J* = 1.6 Hz, 1H), 8.15-8.13 (d, *J* = 8.5 Hz, 1H), 7.89-7.86 (d, *J* = 8.5, 1H), 7.29-7.19 (m, 5H), 4.79-4.76 (t, *J* = 5.2 Hz, 1H), 4.69-4.65 (m, 1H), 4.33-4.15 (m, 7H), 3.90-3.84 (m, 1H), 3.61-3.57 (m, 1H), 3.47-3.42 (m, 1H), 2.87 (s, 3H).

**Example 36:** preparation of 1-(1'-((2,2-difluorobenzo[*d*][1,3]dioxolan-5-yl)sulfonyl)-1',4'-dihydro-2*H*,2'*H*-[3,3'-d iazetidinylidene]-1(4*H*-yl)-2-(4-fluorophenyl)-3-hydroxypropan-1-one **(compound 36):**

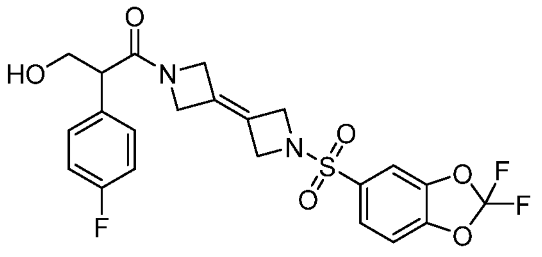

**36**

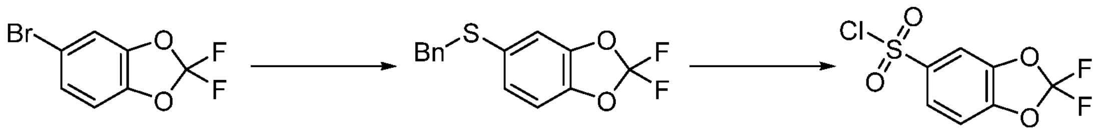

**36A** **36B** **36C**

**Step** 1: preparation of 5-(benzylthio)-2,2-difluorobenzo[*d*][1,3]dioxolane **(compound 36B)**

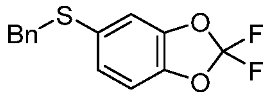

**36B**

5-bromo-2,2-difluoro-1,3-benzodioxolane (400 mg, 1.69 mmol) and benzyl mercaptan (280 mg, 2.25 mmol) were dissolved in dioxane (10 mL), and then added with sodium tert-butoxide (400.00 mg, 4.16 mmol), palladium acetate (40.00 mg, 178.57 μmol) and 1,1'-binaphthyl-2,2'-bis(diphenylphosphine) (100 mg, 160.60 μmol). After the addition was completed, the reaction system was placed under a nitrogen atmosphere and stirred at 100°C for 16 hours, and cooled to room temperature after the reaction was ended. The reaction solution was diluted by adding water, the aqueous phase was extracted with ethyl acetate (30.0 mL*3), and the organic phases were combined, washed with a saturated brine, and dried over anhydrous sodium sulfate, and separated and purified through a silica gel column to obtain **compound 36B.**

MS (ESI) m/z 280.1 $(M+H)^+$.

**Step 2:** preparation of 2,2-difluorobenzo[*d*][1,3]dioxolane-5-sulfonyl chloride **(compound 36C)**

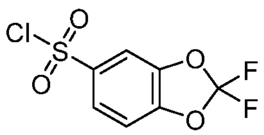

**36C**

2,2-difluoro-5-phenylthio-1,3-benzodioxolane (270 mg, 963.29 μmol) was dissolved in acetonitrile (20 mL), acetic acid (0.75 mL) and water (0.5 mL), and added with 5-dimethyl-imidazolidine-2,4-dione (380 mg, 1.93 mmol) at 0°C. After the addition was completed, the reaction system was placed under nitrogen atmosphere and stirred at room temperature for 2 hours. After the reaction was ended, the reaction solution was concentrated and adjusted to the pH value of 6-7 with a saturated aqueous sodium bicarbonate solution. The aqueous phase was extracted with ethyl acetate (30.0 mL*3). The organic phases were combined, washed with a saturated brine, and dried over anhydrous sodium sulfate, and separated and purified through a silica gel column to obtain **compound 36C.**

MS (ESI) m/z 257.0$(M+H)^+$.

In step 8 of Example 1, 2,3-dihydro-[1 ,4]dioxino[2,3-*b*]pyridine-7-sulfonyl chloride was replaced by 2,2-difluorobenzo[*d*][1,3]dioxolane-5-sulfonyl chloride, and in step 10, (*S*)-3-((tert-butyldiphenylsilyl)oxy)-2-phenylpropionic acid was replaced by 2-(4-fluorophenyl)-3-hydroxypropionic acid, and the same preparation method as that of Example 1 was adopted to obtain **compound 36** of Example 36.

MS (ESI) m/z 497.1 $(M+H)^+$.

$^1H$-NMR (400 MHz, DMSO- $d_6$) $\delta$ 7.92 (s, 1H), 7.74 - 7.68 (m, 2H), 7.31-7.28 (m, 2H), 7.11 (t, *J* = 8.8 Hz, 2H), 4.83 - 4.80 (m, 1H), 4.70 (d, *J* = 12.4 Hz, 1H), 4.38 - 4.19 (m, 7H), 3.87 - 3.81 (m, 1H), 3.66 - 3.63 (m, 1H), 3.47 - 3.42 (m, 1H).

**Example 37:** preparation of (1'-((4-(difluoromethoxy)phenyl)sulfonyl)-1',4'-dihydro-2*H*,2'*H*-[3,3'-diazetidinylidene] -1(4*H*)-yl)(3-phenyltetrahydrofuran-3-yl)methanone (**compound 37**):

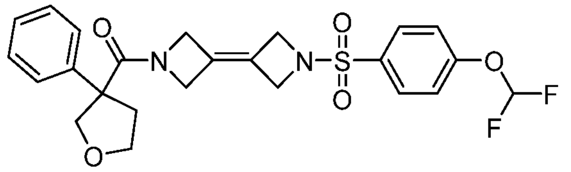

**37**

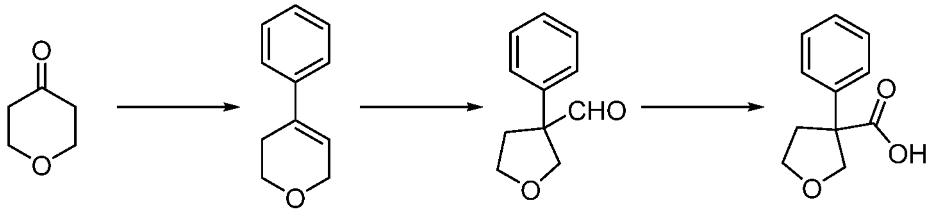

**37A** **37B** **37C** **37D**

**Step 1:** preparation of 4-phenyl-3,6-dihydro-2*H*-pyran (**compound 37B**)

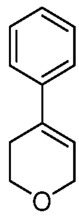

**37B**

An arylmagnesium bromide solution (2.8 N, 12.86 mL, 36 mmol) was slowly added into a solution of tetrahydro-4*H*-pyran-4-one (3.0 g, 29.97 mmol) in tetrahydrofuran (300 mL) at -78°C. The reaction mixture was stirred at room temperature under nitrogen atmosphere for 2 hours, and separated and purified through a silica gel column to obtain **compound 37B.**

MS (ESI) m/z 161.1 $(M+H)^+$.

**Step 2:** preparation of 3-phenyltetrahydrofuran-3-carboxaldehyde (**compound 37C**)

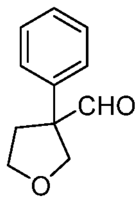

**37C**

4-phenyl-3,6-dihydro-2*H*-pyran (1 g, 6.24 mmol) and sodium carbonate (1.7 g, 16.04 mmol) were dissolved in dichloromethane (150 mL), added with m-chloroperoxybenzoic acid (2.4 g, 13.95 mmol) at 0°C, and then stirred at room temperature under nitrogen atmosphere for 3 hours. After the reaction was complete, the system was added with a saturated aqueous sodium carbonate solution (50 mL), and added with ethyl acetate (50 mL*3) until the reaction mixture is separated from the organic layer. The organic extractwas dried over anhydrous magnesium sulfate, and separated and purified through a silica gel column to obtain **compound 37C.**
MS (ESI) m/z 177.1 $(M+H)^+$.

**Step 3:** preparation of 3-phenyltetrahydrofuran-3-carboxylic acid (**compound 37D**)

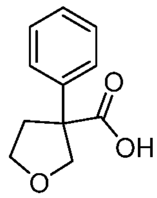

**37D**

3-phenyltetrahydrofuran-3-carboxaldehyde (250 mg, 1.42 mmol) and 2-methylbut-2-ene (2.25 mL) were dissolved in n-butanol (45 mL) and 2-methylbut-2-ene (2.25 mL), and added with sodium chlorite (520 mg, 4.62 mmol) and potassium dihydrogen phosphate (440 mg, 3.24 mmol) at room temperature, and the resulting mixture was stirred at room temperature under nitrogen atmosphere for 0.5 hours. After the reaction was complete, it was extracted with ethyl acetate (10 mL*3), and the organic phases were combined. It was separated and purified through a silica gel column to obtain **compound 37D.**
MS (ESI) m/z 193.1 $(M+H)^+$.
In step 4 of Example 6, 3-((tert-butyldiphenylsilyl)oxy)-2-phenylpropionic acid was replaced by 3-phenyltetrahydrofuran-3-carboxylic acid, and the same preparation method as that of Example 6 was adopted to obtain **compound 37** of Example 37.
MS (ESI) m/z 491.1$(M+H)^+$.
$^1$H-NMR (400 MHz, DMSO-$d_6$) $\delta$ 7.88 - 7.84 (m, 2H), 7.63 - 7.21 (m, 8H), 4.27 - 4.17 (m, 7H), 3.99 (s, 2H), 3.83 - 3.77 (m, 3H), 2.60 - 2.55 (m, 1H), 2.23 - 2.16 (m, 1H).

**Example 38:** preparation of 1-(1'-(benzo[*d*]thiazol-6-ylsulfonyl)-1',4'-dihydro-2*H*,2'*H*-[3,3'-diazetidinylidene]-1(4 *H*)-yl)-2-(3-chloro-4-fluorophenyl)-3-(methylamino)propan-1-one (**compound 38**):

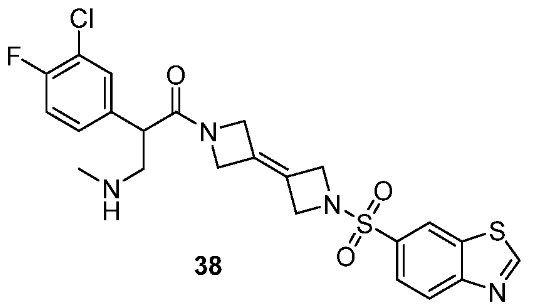

38

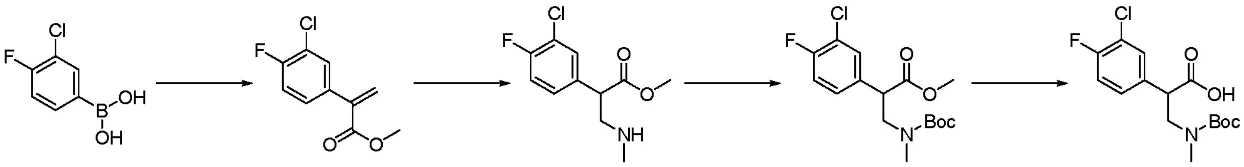

38A 38B 38C 38D 38E

**Step 1:** preparation of methyl 2-(3-chloro-4-fluorophenyl)acrylate (**compound 38B**)

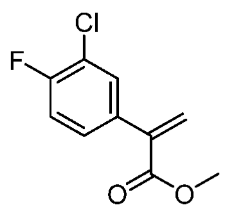

**38B**

(3-chloro-4-fluorophenyl)boronic acid (1.0 g, 5.74 mmol), methyl 2-bromoacrylate (860.2 mg, 32.74mmol), and potassium phosphate (2.2 g, 10.43 mmol) were dissolved in a mixed solution (25.0 mL) of dioxane and water (4:1), and then added with [1,1'-bis(diphenylphosphino)ferrocene]dichloropalladium (378.5 mg, 0.52 mmol). After the addition was completed, the reaction system was placed under a nitrogen protection environment, heated to 80°C, and stirred for 16 hours. After the reaction was complete, the reaction solution was diluted with water. The aqueous phase was extracted with ethyl acetate (150.0 mL*3). The organic phases were combined, washed with a saturated brine, and dried over anhydrous sodium sulfate, and separated and purified through a silica gel column to obtain **compound 38B.**

MS (ESI) m/z 215.2 $(M+H)^+$.

**Step 2:** preparation of methyl 2-(3-chloro-4-fluorophenyl)-3-(methylamino)propionate (**compound 38C**)

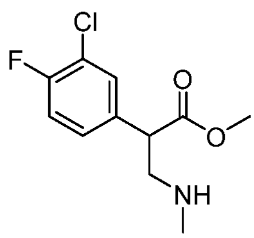

**38C**

Methyl 2-(3-chloro-4-fluorophenyl)acrylate (395.0 mg, 1.84 mmol) was dissolved in a solution of methylamine in tetrahydrofuran (8.0 mL). The reaction solution was reacted at room temperature for 16 hours. After the reaction was complete, it was separated and purified through a silica gel column to obtain **compound 38C.**

MS (ESI) m/z 246.1 $(M+H)^+$.

**Step 3:** preparation of methyl 3-(tert-butoxycarbonyl)(methyl)amino)-2-(3-chloro-4-fluorophenyl)propionate (**compound 38D**)

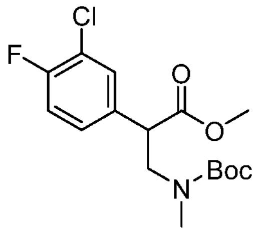

**38D**

2-(3-chloro-4-fluorophenyl)-3-(methylamino)propionate (276.0 mg, 1.12 mmol), *N*,*N*-dimethylamino pyridine (13.7 mg, 0.11 mmol) and triethylamine (227.4 mg, 2.25 mmol) were dissolved in dichloromethane (5 mL), and then added with di-tert-butyl dicarbonate (244.9 mg, 1.12 mmol), and the reaction solution was stirred at room temperature for 16 hours. After the reaction was complete, it was separated and purified through a silica gel column to obtain **compound 38D.**

MS (ESI) m/z 346.1 $(M+H)^+$.

**Step 4:** preparation of 3-((tert-butoxycarbonyl)(methyl)amino)-2-(3-chloro-4-fluorophenyl)propionic acid (**compound 38E**)

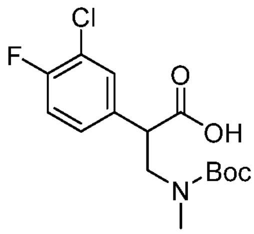

**38E**

Methyl 3-(tert-butoxycarbonyl)(methyl)amino)-2-(3-chloro-4-fluorophenyl)propionate (245.0 mg, 0.71 mmol) was dissolved in a mixed solution of methanol (5.0 mL) and water (5.0 mL), and then added with lithium hydroxide monohydrate (59.5 mg, 1.42 mmol). The reaction solution was stirred at room temperature for 3 hours. After the reaction was complete, the reaction solution was adjusted to pH 2 with 1N hydrochloric acid, and extracted with dichloromethane (100 mL*3), and the organic phases were combined, washed with a saturated brine, and dried over anhydrous sodium sulfate. it was concentrated to obtain a white solid compound, which was directly used in the next step of reaction.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-((tert-butoxycarbonyl)(methyl)amino)-2-(3-chloro-4-fluorophenyl)propionic acid, and the same preparation method as that of Example 1 was adopted to obtain **compound 38** of Example 38.

MS (ESI) m/z 521.1 $(M+H)^+$.

$^1$H NMR (400 MHz, DMSO-$d_6$) $\delta$ 9.69 (s, 1H), 8.82 (s, 1H), 8.37-8.31 (d, *J* = 8.5 Hz, 1H), 7.98-7.91 (d, *J* = 8.5 Hz, 1H), 7.49-7.42 (d, *J* = 8.5 Hz, 1H), 7.37-7.21 (m, 2H), 4.70-4.61 (m, 1H), 4.43-4.13 (m, 7H), 3.73-3.64 (t, *J* = 7.0 Hz, 1H), 3.00-2.89 (m, 1H), 2.64-2.55 (m, 2H), 2.21 (s, 3H).

**Example 39:** preparation of (*S*)-3-hydroxy-1-(1'-((4-methyl-3,4-dihydro-2H-benzo[*b*][1,4]oxazin-6-yl)sulfonyl)-1',4'-dihydro-2*H*,2'*H*-[3,3'-diazetidinylidene]-1(4*H*-yl)-2-phenylpropan-1-one (**compound 39**):

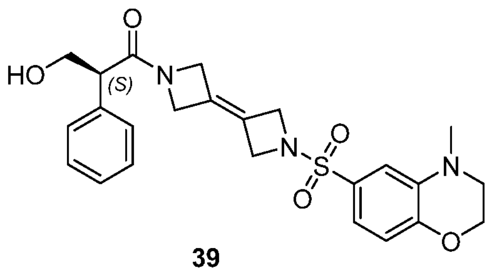

**39**

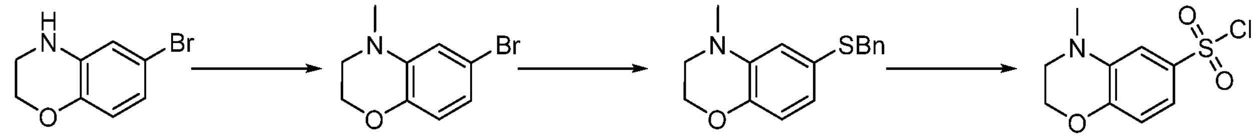

**39A** **39B** **39C** **39D**

**Step** 1: preparation of 6-bromo-4-methyl-3,4-dihydro-2*H*-benzo[*b*][1,4]oxazine (**compound 39B**)

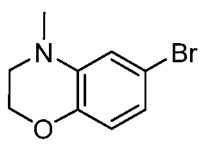

**39B**

6-bromo-3,4-dihydro-2*H*-benzo[*b*][1,4]oxazine (0.5 g, 2.34 mmol), paraformaldehyde (140.3 mg, 4.67 mmol) and sodium cyanoborohydride (234.8 mg, 3.74 mmol) were dissolved in acetonitrile solution (30 mL). After the addition was completed, the reaction system was placed and stirred at room temperature for 0.5 hours, and then added with glacial acetic acid (0.5 mL). After the reaction was complete, the reaction solution was diluted by adding water. The aqueous phase was extracted with ethyl acetate (60 mL*3). The organic phases were combined, washed with a saturated brine, and dried over anhydrous sodium sulfate, and separated and purified through a silica gel column to obtain **compound 39B.**

MS (ESI) m/z 228.0 $(M+H)^+$.

**Step 2:** preparation of 6-(benzylthio)-4-methyl-3,4-dihydro-2*H*-benzo[*b*][1,4]oxazine (**compound 39C**)

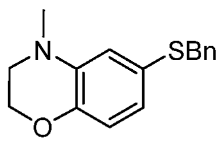

**39C**

6-bromo-4-methyl-3,4-dihydro-2*H*-benzo[*b*][1,4]oxazine (0.45 g, 1.77 mmol), benzyl mercaptan (0.26 g, 3.54 mmol), tris(dibenzylideneacetone)dipalladium (0.09 g, 0.09 mmol), 4,5-bis(diphenylphosphino)-9,9-dimethylxanthene (0.1 g, 0.18 mmol) and *N*,*N*-diisopropylethylamine (686 mg, 5.31 mmol) were dissolved in a mixed solution of methanol (7.0 mL) and water (3.0 mL). After the addition was completed, the reaction system was placed and stirred at room temperature overnight. After the reaction was complete, it was separated and purified through a silica gel column to obtain **compound 39C.**

MS (ESI)m/z 272.3$(M+H)^+$.

**Step 3:** preparation of 4-methyl-3,4-dihydro-2*H*-benzo[*b*][1,4]oxazine-6-sulfonyl chloride (**compound 39D**)

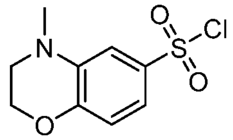

**39D**

6-(benzylthio)-4-methyl-3,4-dihydro-2*H*-benzo[*b*][1,4]oxazine (375 mg, 1.38 mmol) was added into a mixed solvent (acetonitrile:acetic acid:water = 40:1.5:1) (25 mL), and added with 1,3-dichloro-5,5-dimethylhydantoin (545 mg, 2.76 mmol) under stirring at 0°C. Then it was stirred at room temperature for 3 hours. After the reaction was complete, it was separated and purified through a silica gel column to obtain **compound 39D.**

In step 8 of Example 1, 2,3-dihydro-[1 ,4]dioxino[2,3-*b*]pyridine-7-sulfonyl chloride was replaced by 4-methyl-3,4-dihydro-2*H*-benzo[*b*][1,4]oxazine-6-sulfonyl chloride, and the same preparation method as that of Example 1 was adopted to obtain **compound 39** of Example 39.

MS (ESI) m/z 470.2$(M+H)^+$.

$^1H$ NMR (400 MHz, DMSO-$d_6$) $\delta$ 7.29-7.21 (m, 5H), 7.01-6.99 (m, 1H), 6.92-6.87 (m, 2H), 4.79-4.76 (t, *J* = 10.8 Hz, 1H), 4.73-4.70 (t, *J* = 13.2 Hz, 1H), 4.31-4.24 (m, 9H), 3.89-3.88 (m, 1H), 3.62-3.60 (m, 1H), 3.48-3.47 (m, 1H), 3.28 (s, 2H), 2.89 (s, 3H).

**Example 40:** preparation of (*S*)-6-((1'-(3-hydroxy-2-phenylpropionyl)-1',4'-dihydro-2*H*,2'*H*-[3,3'-diazetidinylidene ]-1(4*H*)-yl)sulfonyl)-2*H*-benzo[*b*][1,4]oxazine-3(4*H*)-one (**compound 40**):

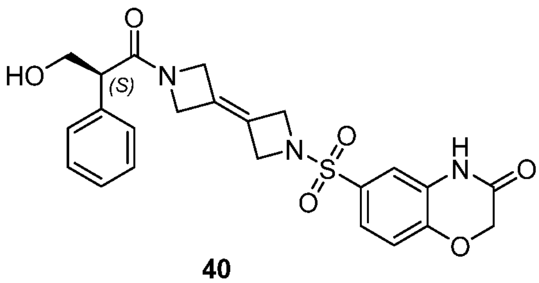

**40**

In step 8 of Example 1, 2,3-dihydro-[1 ,4]dioxino[2,3-*b*]pyridine-7-sulfonyl chloride was replaced by 3-oxo-3,4-dihydro-2*H*-benzo[*b*][1,4]oxazine-6-sulfonyl chloride, and the same preparation method as that of Example 1 was adopted to obtain **compound 40** of Example 40.

MS (ESI) m/z 470.1$(M+H)^+$.

$^1H$ NMR (400 MHz, DMSO-$d_6$) $\delta$ 10.92 (s, 1H), 7.38-7.17 (m, 8H), 4.81-4.79 (m, 1H), 4.78-4.70 (m, 3H), 4.29-4.26 (m, 7H), 3.91-3.87 (m, 1 H), 3.63-3.61 (m, 1H), 3.46-3.42 (m, 1H).

**Example 41:** preparation of (*S*)-6-((1'-(3-hydroxy-2-phenylpropionyl)-1',4'-dihydro-2*H*,2'*H*-[3,3'-diazetidinylidene ]-1(4*H*)-yl)sulfonyl)-4-methyl-2*H*-benzo[*b*][1,4]oxazine-3(4*H*)-one (**compound 41**):

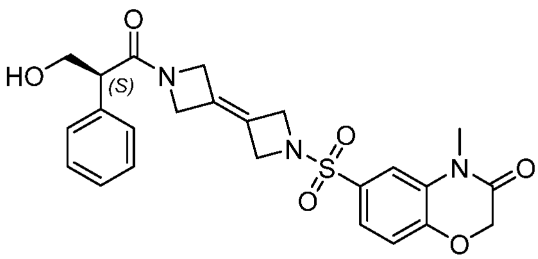

**41**

In step 8 of Example 1, 2,3-dihydro-[1,4]dioxino[2,3-*b*]pyridine-7-sulfonyl chloride was replaced by 4-methyl-3-oxo-3,4-dihydro-2*H*-benzo[*b*][1,4]oxazine-6-sulfonyl chloride, and the same preparation method as that of Example 1 was adopted to obtain **compound 41** of Example 41.

MS (ESI) m/z 484.2(M+H)$^+$.

$^1$H-NMR (400 MHz, DMSO-$d_6$) $\delta$7.48-7.46 (d, 2H), 7.39 (s, 1H), 7.30-7.23 (m, 6 H), 4.81-4.70 (m, 4H), 4.31-4.19 (m, 8H), 3.91-3.85 (m, 1H), 3.63-3.60 (m, 1H), 3.51-3.43 (m, 2H).

**Example 42:** preparation of 1-(1'-(benzo[*d*]thiazol-6-ylsulfonyl)-1',4'-dihydro-2*H*,2'*H*-[3,3'-diazetidinylidene]-1(4 *H*)-yl)-2-(3-chloro-4-fluorophenyl)-3-(dimethylamino)propan-1-one (**compound 42**):

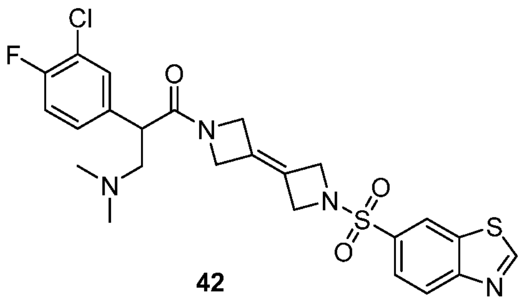

**42**

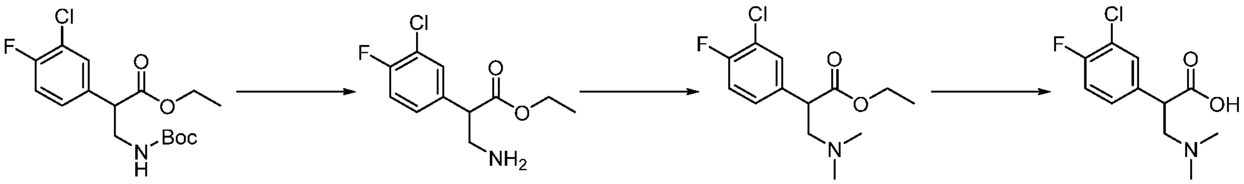

**42A** **42B** **42C** **42D**

**Step 1:** preparation of ethyl 3-amino-2-(3-chloro-4-fluorophenyl)propionate (**compound 42B**)

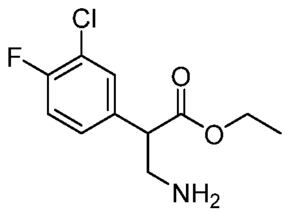

**42B**

Ethyl 3-(tert-butoxycarbonylamino)-2-(3-chloro-4-fluorophenyl)propionate (1 g, 2.89 mmol) was dissolved in hydrochloric acid-1 ,4-dioxane (200 mL), and then the reaction system was placed and stirred at room temperature

under nitrogen atmosphere for 6 hours. After the reaction was complete, it was separated and purified through a silica gel column to obtain **compound 42B.**

MS (ESI) m/z 246.1 $(M+H)^+$.

**Step 2:** preparation of ethyl 2-(3-chloro-4-fluorophenyl)-3-(dimethylamino)propionate (**compound 42C**)

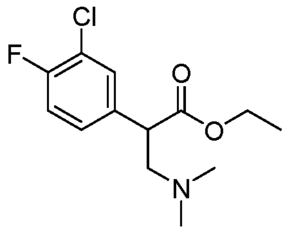

**42C**

Ethyl 3-amino-2-(3-chloro-4-fluoro-phenyl)propionate (801 mg, 3.26 mmol) was dissolved in methanol (30 mL), and added with *N*,*N*-diisopropylethylamine (842.74 mg, 6.52 mmol) and paraformaldehyde (587.39 mg, 6.52 mmol) at room temperature. After the addition was completed, the reaction system was placed under nitrogen atmosphere and stirred at 25°C for 6 hours. After the reaction was ended, it was separated and purified through a silica gel column to obtain **compound 42C.**

MS (ESI) m/z 274.1 $(M+H)^+$.

**Step 3:** preparation of 2-(3-chloro-4-fluorophenyl)-3-(dimethylamino)propionic acid (**compound 42D**)

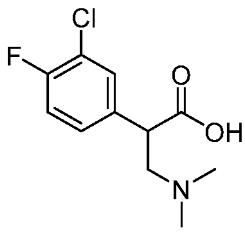

**42D**

Ethyl 2-(3-chloro-4-fluoro-phenyl)-3-(dimethylamino)propionate (391 mg, 1.43 mmol) was dissolved in ethanol (7 mL) and water (7 mL), and added with lithium hydroxide (119.99 mg, 2.86 mmol) at room temperature. After the addition was completed, the reaction system was placed and stirred at 50°C under nitrogen atmosphere for 16 hours. After the reaction was ended, it was separated and purified through a silica gel column to obtain **compound 42D.**

MS (ESI) m/z 246.2 $(M+H)^+$.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-(3-chloro-4-fluorophenyl)-3-(dimethylamino)propionic acid, and the same preparation method as that of Example 1 was adopted to obtain **compound 42** of Example 42.

MS (ESI) m/z 535.1 $(M+H)^+$.

$^1$H-NMR (400 MHz, DMSO-$d_6$) δ 9.68 (s, 1H), 8.82 (s, 1H), 8.35-8.33 (d, *J* = 8.5 Hz, 1H), 7.96-7.94 (d, *J* = 8.5 Hz, 1H), 7.49-7.47 (d, *J* = 7.1 Hz, 1H), 7.33-7.29 (m, 2H), 4.68-4.65 (d, *J* = 13.2 Hz, 1H), 4.48 - 4.15 (m, 7H), 3.74 - 3.71 (m, 1H), 2.78 - 2.67 (m, 1H), 2.37-2.33 (m, 1H), 2.09 (s, 6H).

**Example 43:** preparation of 1-(1'-(benzo[*d*]thiazol-6-ylsulfonyl)-1',4'-dihydro-2*H*,2'*H*-[3,3'-diazetidinylidene]-1(4 *H*)-yl)-2-hydroxy-2-(3-(4-methylpiperazin-1-yl)phenyl)ethan-1-one (**compound 43**):

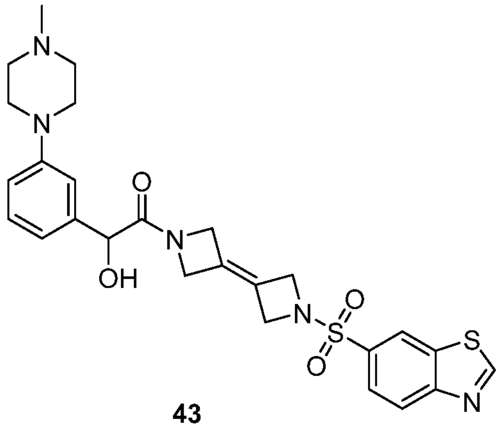

**43**

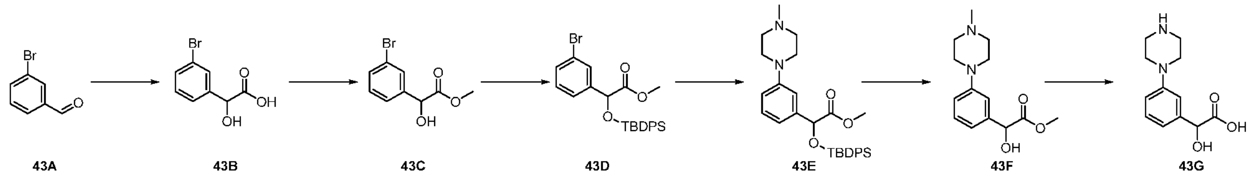

43A 43B 43C 43D 43E 43F 43G

**Step 1:** preparation of 2-(3-bromophenyl)-2-hydroxyacetic acid (**compound 43B**)

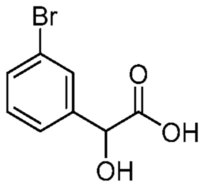

**43B**

3-bromobenzaldehyde (1.85 g, 10.0 mmol) was added into tetrahydrofuran (40 mL), and the solution was added with trimethylsilyl cyanide (1.1 g, 11.0 mmol) and added dropwise with 1 drop of 1N solution of tetrabutylammonium fluoride in tetrahydrofuran. The reaction solution was stirred at room temperature for 1 hour, and then subjected to rotary evaporation under reduced pressure to remove the solvent. The residual oil was added with 6N hydrochloric acid aqueous solution (10 mL), and heated to reflux for 3 hours. The reaction mixture was cooled to 0°C and added dropwise with 10N sodium hydroxide solution to adjust pH to 1 . After the reaction was complete, the mixture was extracted with ethyl acetate (60 mL*3), and the organic phases were combined, washed with a saturated brine, and dried over anhydrous sodium sulfate, and separated and purified through a silica gel column to obtain **compound 43B.**

MS (ESI)m/z 230.1 (M-H)$^+$.

**Step 2:** preparation of methyl 2-(3-bromophenyl)-2-hydroxyacetate (**compound 43C**)

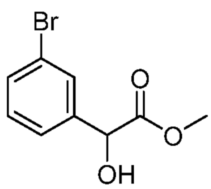

**43C**

(3-bromophenyl)glycolic acid (500 mg, 2.16 mmol) was added into methanol (10 mL), thionyl chloride (773 mg, 6.49 mmol) was added dropwise into the reaction solution at room temperature, and after the dropwise addition was completed, the reaction system was then placed and stirred at 60°C for 3 hours. After the reaction was complete, it was separated and purified through a silica gel column to obtain **compound 43C.**

MS (ESI)m/z 268.1 $(M+Na)^+$.

**Step 3:** preparation of methyl 2-(3-bromophenyl)-2-((tert-butyldiphenylsilyl)oxy)acetate (**compound 43D**)

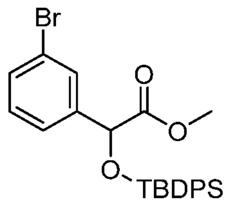

**43D**

Methyl 2-(3-bromophenyl)-2-hydroxyacetate (400 mg, 1.63 mmol), tert-butylchlorodiphenylsilane (539 mg, 1.96 mmol), and imidazole (278 mg, 4.08 mmol) were added into tetrahydrofuran (8 mL), and stirred at 40°C for 16 hours. After the reaction was complete, it was separated and purified through a silica gel column to obtain **compound 43D.**

MS (ESI)m/z 507.2 $(M+Na)^+$.

**Step 4:** preparation of methyl 2-((tert-butyldiphenylsilyl)oxy)-2-(3-(4-methylpiperazin-1-yl)phenyl)acetate (**compound 43E**)

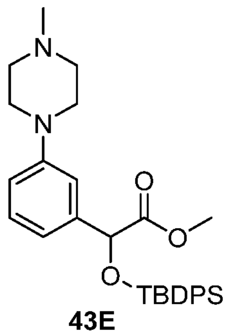

**43E**

Methyl 2-(3-bromophenyl)-2-((tert-butyldiphenylsilyl)oxy)acetate (400 mg, 0.83 mmol), 1-methylpiperazine (124 mg, 1.24 mmol), 4,5-bis(diphenylphosphino)-9,9-dimethylxanthene (48 mg, 0.08 mmol), tris(dibenzylideneacetone)dipalladium (43 mg, 0.04 mmol) and cesium carbonate (540 mg, 1.65 mmol) were added into 1,4-dioxane (10 mL), and after being replaced with nitrogen for three times, it was stirred at 100°C overnight. After the reaction was complete, it was separated and purified through a silica gel column to obtain **compound 43E.**

MS (ESI)m/z 542.6 $(M+Na)^+$.

**Step 5:** preparation of methyl 2-hydroxy-2-(3-(4-methylpiperazin-1-yl)phenyl)acetate (**compound 43F**)

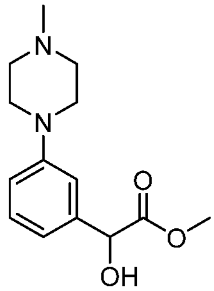

**43F**

Methyl 2-((tert-butyldiphenylsilyl)oxy)-2-(3-(4-methylpiperazin-1-yl)phenyl)acetate (350 mg, 0.74 mmol) was dissolved in tetrahydrofuran (10 mL), and added with tetrabutylammonium fluoride (386 mg, 1.48 mmol), and the reaction system was placed and stirred at 50°C overnight. After the reaction was complete, the product was diluted by adding 50 mL of water, and extracted with ethyl acetate (50 mL*3) and water, and the organic phases were combined, dried over anhydrous sodium sulfate, and separated and purified through a silica gel column to obtain **compound 43F.**
MS (ESI)m/z 256.3 $(M+H)^+$.

**Step 6:** preparation of 2-hydroxy-2-(3-(4-methylpiperazin-1-yl)phenyl)acetic acid (**compound 43G**)

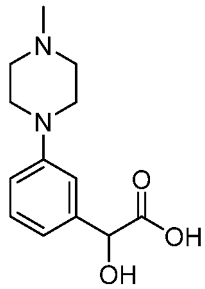

**43G**

Methyl 2-hydroxy-2-(3-(4-methylpiperazin-1-yl)phenyl)acetate (150 mg, 0.56 mmol) and sodium hydroxide (45 mg, 1.12 mmol) were dissolved in a mixed solution of methanol (3 mL) and water (3 mL), and then the reaction system was placed and stirred at 25°C for 3 hours. After the reaction was complete, it was separated and purified through a silica gel column to obtain **compound 43G.**
MS (ESI) m/z 249.2$(M-H)^+$.
In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-hydroxy-2-(3-(4-methylpiperazin-1-yl)phenyl)acetic acid, and the same preparation method as that of Example 1 was adopted to obtain **compound 43** of Example 43.
MS (ESI) m/z 540.2$(M+H)^+$.
$^1H$ NMR (400 MHz, DMSO-$d_6$) $\delta$ 9.68 (s, 1H), 8.80 - 8.79 (d, *J* = 1.6 Hz, 1H), 8.34-8.32 (d, *J* = 8.4 Hz, 1H), 7.95-7.92 (m, 1H), 7.14-7.10 (t, *J* = 15.6 Hz, 1H), 6.86-6.69 (m, 3H), 5.69-5.67 (d, *J* = 5.2 Hz, 1H), 4.93-4.92 (d, *J* = 5.6 Hz, 1H), 4.62-4.22 (m , 8H), 3.09-3.06 (t, *J* = 10 Hz, 4H), 2.43-2.41 (t, *J* = 9.6 Hz, 4H), 2.21 (s , 3H).

**Example 44:** preparation of 1-(1'-(benzo[*d*]thiazol-6-ylsulfonyl)-1',4'-dihydro-2*H*,2'*H*-[3,3'-diazetidinylidene]-1(4 *H*)-yl)-3-hydroxy-2-(4-methoxyphenyl)propan-1-one (**compound 44**):

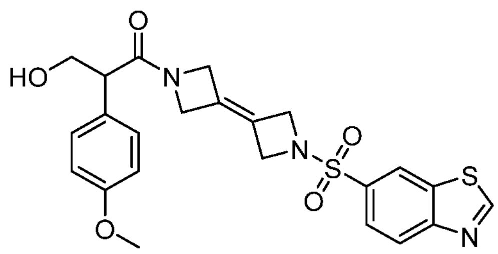

**44**

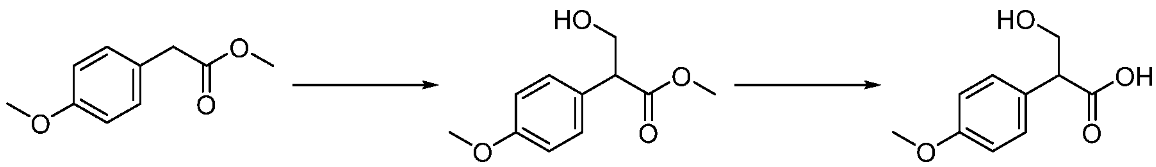

**44A** **44B** **44C**

**Step 1:** preparation of methyl 3-hydroxy-2-(4-methoxyphenyl)propionate (**compound 44B**)

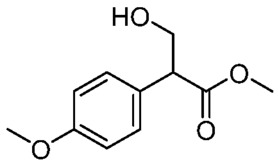

**44B**

Methyl 2-(4-methoxyphenyl)acetate (1.0 g, 5.55 mmol), paraformaldehyde (524.4 mg, 5.83 mmol) and sodium methoxide (15 mg, 0.28 mmol) were dissolved in a dimethyl sulfoxide solution (10 mL). After the addition was completed, the reaction system was placed and stirred at room temperature for 3 hours. After the reaction was complete, it was separated and purified through a silica gel column to obtain **compound 44B.**

MS (ESI) m/z 211.0 $(M+H)^+$.

**Step 2:** preparation of 3-hydroxy-2-(4-methoxyphenyl)propionic acid (**compound 44C**)

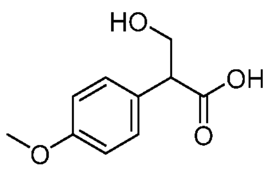

**44C**

Methyl 3-hydroxy-2-(4-methoxyphenyl)propionate (0.49 g, 2.33 mmol) and lithium hydroxide monohydrate (0.2 g, 4.66 mmol) were dissolved in a mixed solution of methanol (7.0 mL) and water (3.0 mL). After the addition was completed, the reaction system was placed and stirred at room temperature overnight. After the reaction was complete, it was separated and purified through a silica gel column to obtain **compound 44C.**

MS (ESI) m/z 197.0$(M+H)^+$.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-hydroxy-2-(4-methoxyphenyl)propionic acid, and the same preparation method as that of Example 1 was adopted to

obtain **compound 44** of Example 44.

MS (ESI) m/z 486.1 $(M+H)^+$.

$^1H$ NMR (400 MHz, DMSO-$d_6$) $\delta$9.67 (s, 1H), 8.81-8.80 (d, *J* = 2 Hz, 1H), 8.34-8.32 (d, *J* = 8.8 Hz, 1H), 7.95-7.92 (m, 1H), 7.14-7.12 (d, *J* = 8.8 Hz, 2H), 6.83-6.81 (d, *J* = 8.8 Hz, 2H), 4.73-4.63 (m, 2H), 4.34-4.23 (m, 6H), 3.83-3.80 (m, 1H), 3.69 (s, 3H), 3.54-3.50 (m, 1H), 3.41-3.37 (m, 2H).

**Example 45:** preparation of 1-(1'-(benzo[*d*]thiazol-6-ylsulfonyl)-1',4'-dihydro-2*H*,2'*H*-[3,3'-diazetidinylidene]-1(4 *H*)-yl)-3-hydroxy-2-(p-tolyl)propan-1-one (**compound 45**):

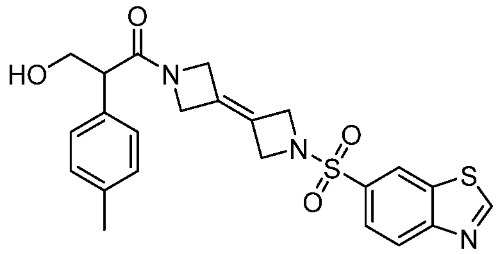

**45**

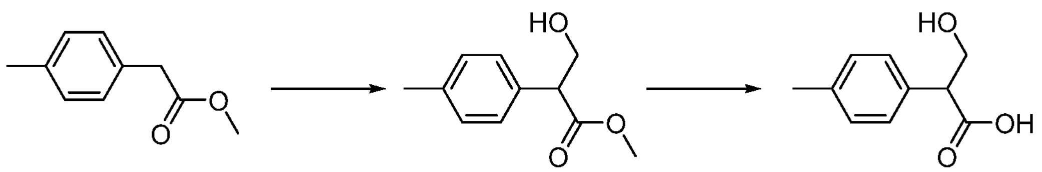

**45A** **45B** **45C**

**Step 1:** preparation of methyl 3-hydroxy-2-(p-tolyl)propionate (**compound 45B**)

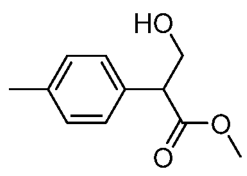

**45B**

Methyl 2-(p-tolyl)acetate (0.5 g, 3.05 mmol) and paraformaldehyde (287.8 mg, 3.20 mmol) were dissolved in dimethyl sulfoxide (2.0 mL), and then added with sodium methoxide (8.2 mg, 0.31 mmol). The reaction solution was reacted at room temperature for 4 hours. After the reaction was complete, it was separated and purified through a silica gel column to obtain **compound 45B.**

MS (ESI) m/z 195.1 $(M+H)^+$.

**Step 2:** preparation of 3-hydroxy-2-(p-tolyl)propionic acid (**compound 45C**)

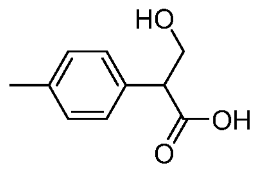

**45C**

Methyl 2-(p-tolyl)acetate (380.0 mg, 1.96 mmol) was dissolved in a mixed solution of methanol (9.0 mL) and water (6.0 mL), and then added with sodium hydroxide (156.5 mg, 3.91 mmol), and the reaction solution was stirred at 25°C for 16 hours. After the reaction was complete, the reaction solution was adjusted to pH 2 with 1N hydrochloric acid, and extracted with dichloromethane (50 mL*3), and the organic phases were combined, washed with a saturated brine, and dried over anhydrous sodium sulfate. It was concentrated, and directly used in the next step of reaction.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-hydroxy-2-(p-tolyl)propionic acid, and the same preparation method as that of Example 1 was adopted to obtain **compound 45** of Example 45.

MS (ESI) m/z 470.1(M+H)$^+$.

$^1$H NMR (400 MHz, DMSO-*d*$_6$) δ9.68 (s, 1H), 8.81 (s, 1H), 8.34-8.32 (d, *J* = 9.1 Hz, 1H), 7.95-7.93 (d, *J* = 8.4 Hz, 1H), 7.09-7.08 (m, 4H), 4.73 -4.60 (m, 2H), 4.34 - 4.07 (m, 7H), 3.85-3.84(m, 1H), 3.54-3.52 (m, 1 H), 3.40- 3.38 (m, 1H), 2.23 (s, 3H).

**Example 46:** preparation of 1-(1'-(benzo[*d*]thiazol-6-ylsulfonyl)-1',4'-dihydro-2*H*,2'*H*-[3,3'-diazetidinylidene]-1(4 *H*)-yl)-2-(3-fluorophenyl)-3-hydroxypropan-1-one (**compound 46**):

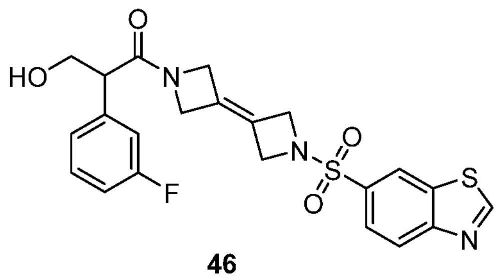

**46**

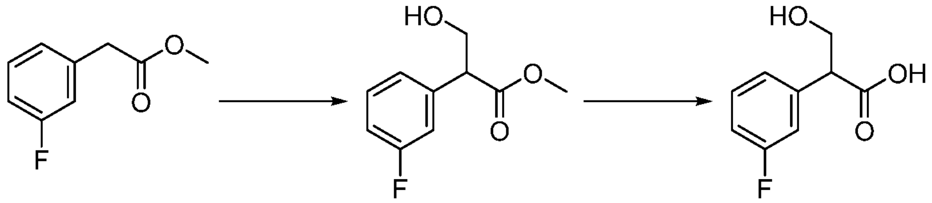

**46A** **46B** **46C**

**Step 1:** preparation of methyl 2-(3-fluorophenyl)-3-hydroxypropionate (**compound 46B**)

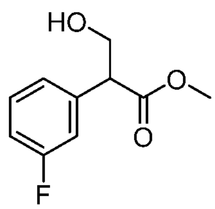

**46B**

Methyl 2-(3-fluorophenyl)acetate (1.0 g, 5.95 mmol) was dissolved in dimethyl sulfoxide (10 mL), and then added with sodium methoxide (100 mg, 1.85 mmol) and paraformaldehyde (535 mg, 5.94 mmol) at room temperature. After the addition was completed, the reaction system was placed and stirred at room temperature under nitrogen atmosphere for 3 hours. The reaction solution was diluted by adding water, the aqueous phase was extracted with ethyl acetate (10.0 mL*3), and the organic phases were combined, washed with a saturated brine, and then dried over anhydrous sodium sulfate, and separated and purified through a silica gel column to obtain **compound 46B.**
MS (ESI) m/z 199.2 $(M+H)^+$.

**Step 2:** preparation of 2-(3-fluorophenyl)-3-hydroxypropionic acid (**compound 46C**)

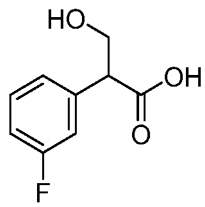

**46C**

Methyl 2-(3-fluorophenyl)-3-hydroxypropionate (610 mg, 3.08 mmol) was dissolved in methanol (6 mL) and water (6 mL), and added with lithium hydroxide (650 mg, 27.14 mmol) at room temperature. After the addition was completed, the reaction system was placed and stirred at 60°C under nitrogen atmosphere for 3 hours. After the reaction was completed, the reaction solution was concentrated and adjusted to the pH value of 3-4 with 1N hydrochloric acid solution. The aqueous phase was extracted with ethyl acetate (10.0 mL*3), and the organic phases were combined, washed with a saturated brine, and then dried over anhydrous sodium sulfate. It was separated and purified through a silica gel column to obtain **compound 46C.**
MS (ESI) m/z 183.1 $(M-H)^-$.
In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-(3-fluorophenyl)-3-hydroxypropionic acid, and the same preparation method as that of Example 1 was adopted to obtain **compound 46** of Example 46.
MS (ESI) m/z 474.1$(M+H)^+$.
$^1$H-NMR (400 MHz, DMSO-$d_6$) $\delta$ 9.68 (s, 1H), 8.81-8.80 (m, 1H), 8.34 - 8.32 (m, 1H), 7.95-7.93 (m, 1H), 7.34-7.28 (m, 1H), 7.09-7.03 (m, 3H), 4.38-4.16 (m, 8H), 3.86 - 3.81 (m, 1H), 3.66-3.58 (m, 2H), 3.51- 3.44 (m, 1H).

**Example 47:** preparation of 1-(1'-(benzo[*d*]thiazol-6-ylsulfonyl)-1',4'-dihydro-2*H*,2'*H*-[3,3'-diazetidinylidene]-1(4 *H*)-yl)-2-phenylethan-1-one (**compound 47**):

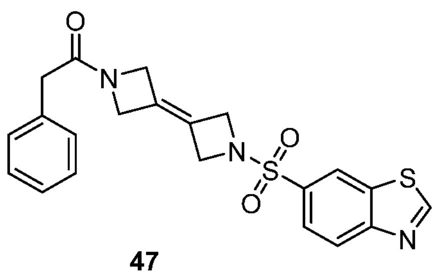

**47**

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by phenylacetic acid, and the same preparation method as that of Example 1 was adopted to obtain **compound 47** of Example 47.
MS (ESI) m/z 426.1$(M+H)^+$.
$^1$H NMR (400 MHz, DMSO-$d_6$) $\delta$ 9.69 (s, 1H), 8.82 (s, 1H), 8.35-8.33 (d, *J*=8.0 Hz, 1H), 7.96-7.94 (d, *J*=8.0

Hz, 1H), 7.27-7.15 (m, 5H), 4.54-4.22 (m, 8H), 3.44 (s, 2H).

**Example 48:** preparation of 1-(1'-(benzo[*d*]thiazol-6-ylsulfonyl)-1',4'-dihydro-2*H*,2'*H*-[3,3'-diazetidinylidene]-1(4 *H*)-yl)-3-hydroxy-2-(pyridin-3-yl)propan-1-one (**compound 48**):

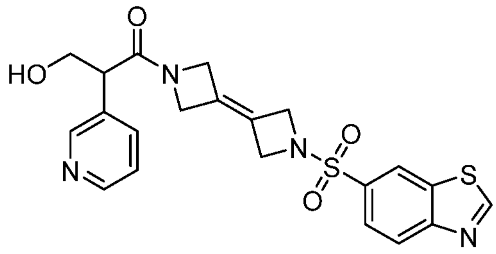

**48**

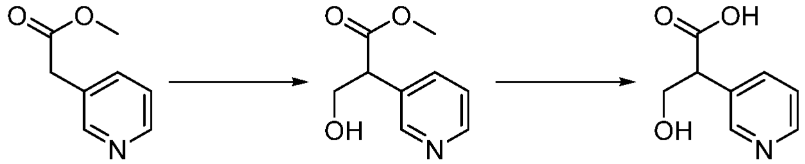

**48A** **48B** **48C**

**Step 1:** preparation of methyl 3-hydroxy-2-(pyridin-3-yl)propionate (**compound 48B**)

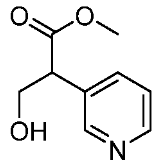

**48B**

Methyl 2-(3-pyridyl)acetate (1.0 g, 6.62 mmol) was dissolved in dimethyl sulfoxide (10 mL), and then added with sodium methoxide (120 mg, 666.67 μmol) and paraformaldehyde (600 mg, 6.67 mmol) at room temperature. After the addition was completed, the reaction system was placed and stirred at room temperature under nitrogen atmosphere for 3 hours. The reaction solution was diluted by adding water. The aqueous phase was extracted with ethyl acetate (30.0 mL*3), and the organic phases were combined, washed with a saturated brine, and then dried over anhydrous sodium sulfate. It was separated and purified through a silica gel column to obtain **compound 48B.**

MS (ESI) m/z 182.1 $(M+H)^+$.

**Step 2:** preparation of 3-hydroxy-2-(pyridin-3-yl)propionic acid (**compound 48C**)

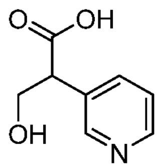

**48C**

Methyl 3-hydroxy-2-(2-pyridyl)propionate (140 mg, 772.68 μmol) was dissolved in a mixed solution of methanol

(2 mL), water (2 mL) and tetrahydrofuran (2 mL), and added with lithium hydroxide (350 mg, 8.33 mmol) at room temperature. After the addition was completed, the reaction system was placed and stirred at 60°C under nitrogen atmosphere for 3 hours. After the reaction was ended, the reaction solution was concentrated and adjusted to pH 3-4 with 1N hydrochloric acid solution, the aqueous phase was extracted with ethyl acetate (10.0 mL*3), and the organic phases were combined, washed with a saturated brine, and then dried over anhydrous sodium sulfate. It was separated and purified through a silica gel column to obtain **compound 48C.**

MS (ESI) m/z 168.1 $(M+H)^+$.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 3-hydroxy-2-(pyridin-3-yl)propionic acid, and the same preparation method as that of Example 1 was adopted to obtain **compound 48** of Example 48.

MS (ESI) m/z 457.1$(M+H)^+$.

$^1$H-NMR (400 MHz, DMSO-$d_6$) $\delta$ 9.67 (s, 1H), 8.81- 8.79 (m, 1H), 8.43- 8.42(m, 2H), 8.37-8.32 (m, 1H), 7.96 - 7.93 (m, 1H), 7.68 -7.66 (m, 1H), 7.32 - 7.28 (m, 1H), 4.91 - 4.88 (m, 1H), 4.75 - 4.67 (m, 1H), 4.44 - 4.17 (m, 7H), 3.88 - 3.82 (m, 1H), 3.68 - 3.64 (m, 1H), 3.53 - 3.48 (m, 1H).

**Example 49:** preparation of (*S*)-1-(1'-((2,3-dihydro-[1,4]dioxino[2,3-*b*]pyridin-6-yl)sulfonyl)-1',4'-dihydro-2*H*,2' *H*-[3,3'-diazetidinylidene]-1(4*H*)-yl)-3-hydroxy-2-phenylpropan-1-one (**compound 49**):

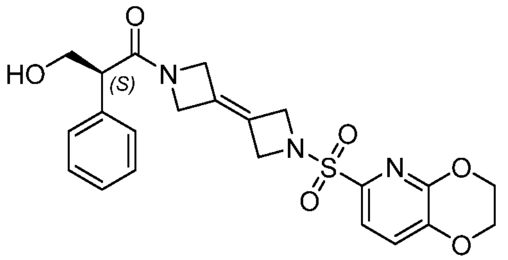

49

In step 8 of Example 1, 2,3-dihydro-[1,4]dioxino[2,3-*b*]pyridine-7-sulfonyl chloride was replaced by 2,3-dihydro-[1,4]dioxino[2,3-*b*]pyridine-6-sulfonyl chloride (the synthesis method was the same as that in Example **20D**), and the same preparation method as that of Example 1 was adopted to obtain **compound 49** of Example 49.

MS (ESI) m/z 458.1 $(M+H)^+$.

$^1$H NMR (400 MHz, DMSO-$d_6$) $\delta$ 7.54-7.50 (t, *J* = 1.6 Hz, 2H), 7.32-7.21 (m, 5H), 4.50-4.46 (m, 1H), 4.37-4.30 (m, 11H), 3.91-3.86 (t, *J* = 1.0 Hz, 1H), 3.64-3.61 (m, 2H), 3.48-3.44 (m, 1H).

**Example 50:** preparation of (*S*)-1-(1'-(benzo[*d*]thiazol-6-ylsulfonyl)-1',4'-dihydro-2*H*,2'*H*-[3,3'-diazetidinylidene] -1(4*H*)-yl)-2-(4-fluorophenyl)-3-hydroxypropan-1-one (**compound 50**):

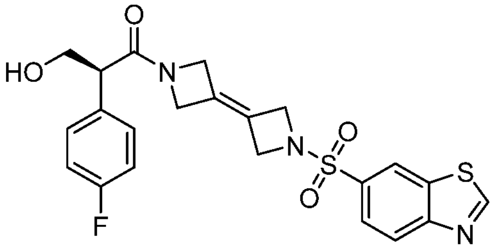

**50**

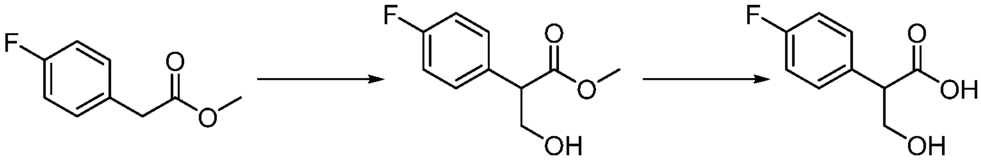

**50A** **50B** **50C**

**Step 1:** preparation of methyl 2-(4-fluorophenyl)-3-hydroxypropionate (**compound 50B**)

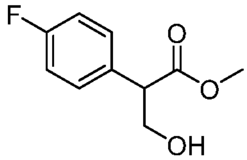

**50B**

Methyl 2-(4-fluorophenyl)acetate (2.0 g, 11.89 mmol) was dissolved in dimethyl sulfoxide (20.0 mL), and then added with paraformaldehyde (1.2 g, 12.49 mmol), and then slowly added with sodium methoxide solution (32.1 mg, 594.66 μmol). The reaction solution was reacted at room temperature for 4 hours. After the reaction was complete, the reaction solution was diluted by adding water. The aqueous phase was extracted with ethyl acetate (50.0 mL*3). The organic phases were combined, washed with a saturated brine, and dried over anhydrous sodium sulfate, and separated and purified through a silica gel column to obtain **compound 50B.**

MS (ESI) m/z 199.0 $(M+H)^+$.

**Step 2:** preparation of 2-(4-fluorophenyl)-3-hydroxypropionic acid (**compound 50C**)

Methyl 2-(4-fluorophenyl)-3-hydroxypropionate (1.5 g, 7.47 mmol) was dissolved in a mixed solution of methanol (15.0 mL) and water (10.0 mL), and then added with sodium hydroxide (597.4 mg, 14.94 mmol). The reaction solution was reacted at 50°C for 3 hours. TLC showed that the reaction was complete. The reaction solution was adjusted to an acidic pH with 1N hydrochloric acid. The aqueous phase was extracted with ethyl acetate (20mL*3). The organic phases were combined, washed with a saturated brine, and then dried over anhydrous sodium sulfate, and separated and purified through a silica gel column to obtain **compound 50C.**

MS (ESI) m/z 185.0 $(M+H)^+$.

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-(4-fluorophenyl)-3-hydroxypropionic acid, the same preparation method as that of Example 1 was adopted, and then the product was subjected to chiral separation to obtain **compound 50** of Example 50.

MS (ESI) m/z 474.1$(M+H)^+$.

$^1H$ NMR (400 MHz, DMSO-($d_6$) $\delta$9.68 (s, 1H), 8.81-8.80 (d, *J*=4.0 Hz, 1H), 8.34-8.32 (d, *J*=8.0 Hz, 1H), 7.95-7.92 (m, 1H), 7.29-7.25 (m, 2H), 7.11-7.07 (m, 2H), 4.68-4.65 (m, 1H), 4.34-4.25 (m, 7H), 3.82-3.79 (m, 1H), 3.62-3.60 (m, 1H), 3.44-3.40 (m, 2H).

**Example 51:** preparation of (*R*)-1-(1'-(benzo[*d*]thiazol-6-ylsulfonyl)-1',4'-dihydro-2*H*,2'*H*-[3,3'-diazetidinylidene] -1(4*H*)-yl)-2-(4-fluorophenyl)-3-hydroxypropan-1-one (**compound 51**):

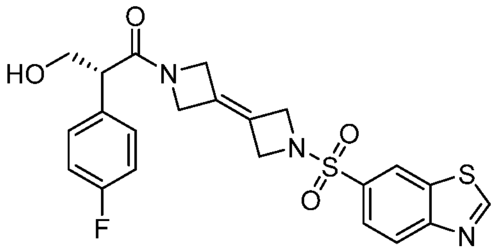

**51**

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-(4-fluorophenyl)-3-hydroxypropionic acid, the same preparation method as that of Example 1 was adopted, and then the product was subjected to chiral separation to obtain **compound 51** of Example 51.

MS (ESI) m/z 474.1$(M+H)^+$.

$^1$H NMR (400 MHz, DMSO-$d_6$) $\delta$9.68 (s, 1H), 8.81-8.80 (d, *J*=4.0 Hz, 1H), 8.34-8.32 (d, *J*=8.0 Hz, 1H), 7.95-7.92 (m, 1H), 7.29-7.25 (m, 2H), 7.11-7.07 (m, 2H), 4.68-4.65 (m, 1H), 4.34-4.25 (m, 7H), 3.82-3.79 (m, 1H), 3.62-3.60 (m, 1H), 3.44-3.40 (m, 2H).

**Example 52:** preparation of (1'-(benzo[*d*]thiazol-6-ylsulfonyl)-1',4'-dihydro-2*H*,2'*H*-[3,3'-diazetidinylidene]-1(4*H*) -yl)(1*H*-indol-3-yl)methanone (**compound 52**):

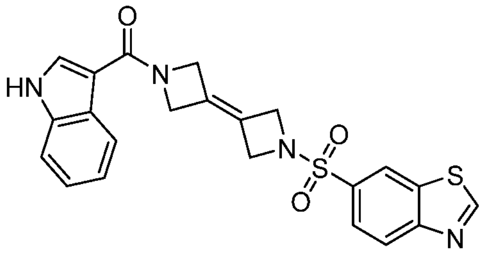

**52**

In step 8 of Example 1, 2,3-dihydro-[1 ,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 1*H*-indole-3-carboxylic acid, and the same preparation method as that of Example 1 was adopted to obtain **compound 52** of Example 52.

MS (ESI) m/z 451.1$(M+H)^+$.

$^1$H NMR (400 MHz, DMSO-$d_6$) $\delta$ 11.68 (s, 1H), 9.73-9.63 (t, *J* = 4 Hz, 1H), 8.85-8.84 (d, *J* = 0.4 Hz, 1H), 8.36-8.34 (d, *J* = 0.8 Hz, 1H), 8.05-8.03 (d, *J* = 0.8 Hz, 1H), 7.99-7.96 (m, 1H), 7.69-7.68 (d, *J* = 0.4 Hz, 1H), 7.42-7.40 (d, *J* = 0.8 Hz, 1H), 7.16-7.06 (m, 2H), 59-4.40 (m, 8H).

**Example 53:** preparation of 3-hydroxy-2-(4-methoxyphenyl)-1-(1'-(pyridin-2-ylsulfonyl)-1',4'-dihydro-2*H*,2'*H*-[3, 3'-diazetidinylidene]-1(4*H*)-yl)propan-1-one (**compound 53**):

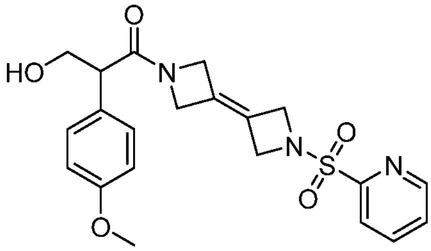

**53**

In step 8 of Example 1, 2,3-dihydro-[1,4]dioxino[2,3-*b*]pyridine-7-sulfonyl chloride was replaced by pyridine-2-sulfonyl chloride, and in step 10, (S)-3-((tert-butyldiphenylsilyl)oxy)-2-phenylpropionic acid was replaced by 3-hydroxy-2-(4-methoxyphenyl)propionic acid, and the same preparation method as that of Example 1 was adopted to obtain **compound 53** of Example 53.

MS (ESI) m/z 430.1$(M+H)^+$.

$^1$H-NMR (400 MHz, DMSO-$d_6$) δ 8.81-8.79 (m, 1H), 8.17-8.13 (m, 1H), 7.99-7.97 (d, *J* =8.0 Hz, 1H), 7.76-7.73 (m, 1H), 7.18-7.15 (m, 2H), 6.87-6.83 (m, 2H), 4.76-4.68 (m, 2H), 4.53 (s, 4H), 4.35-4.18 (m, 3H), 3.89-3.81 (m, 1H), 3.71 (s, 3H), 3.57-3.53 (m, 1H), 3.42-3.37 (m, 1H).

**Example 54:** preparation of (*S*)-2-(4-fluorophenyl)-3-hydroxy-1-(1'-(pyridin-2-ylsulfonyl)-1',4'-dihydro-2*H*,2'*H*[3 ,3'-diazetidinylidene]-1(4*H*)-yl)propan-1-one (**compound 54**):

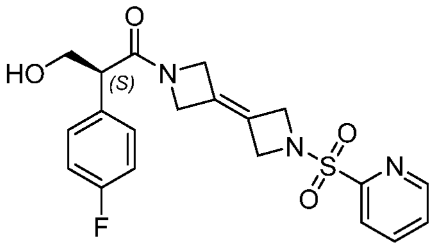

**54**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(4-fluorophenyl)-3-hydroxypropionic acid, the same preparation method as that of Example 1 was adopted, and then the product was subjected to chiral separation to obtain **compound 54** of Example 54.

MS (ESI) m/z 418.1$(M+H)^+$.

$^1$H-NMR (400 MHz, DMSO-$d_6$) δ 8.81-8.80 (d, *J* = 4.7 Hz, 1H), 8.17-8.13 (m, 1H), 7.99-7.97 (d, *J* = 7.7 Hz, 1H), 7.77-7.73 (m, 1H), 7.32-7.29 (m, 2H), 7.14-7.09 (m, 2H), 4.83-4.70 (m, 2H), 4.54-4.19 (m, 7H), 3.87-3.82 (m, 1H), 3.66-3.63 (m, 1H), 3.46-3.42 (m, 1H).

**Example 55:** preparation of (*R*)-2-(4-fluorophenyl)-3-hydroxy-1-(1'-(pyridin-2-ylsulfonyl)-1',4'-dihydro-2*H*,2'*H*-[ 3,3'-diazetidinylidene]-1(4*H*)-yl)propan-1-one (**compound 55**):

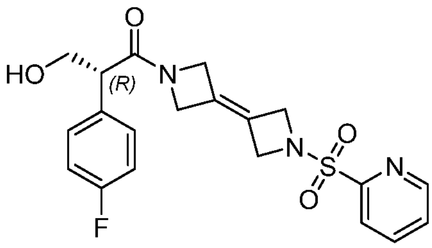

**55**

In step 8 of Example 1, 2,3-dihydro-[1,4]dioxino[2,3-*b*]pyridine-7-sulfonyl chloride was replaced by pyridine-2-sulfonyl chloride, and in step 10, (S)-3-((tert-butyldiphenylsilyl)oxy)-2-phenylpropionic acid was replaced by 2-(4-fluorophenyl)-3-hydroxypropionic acid, the same preparation method as that of Example 1 was adopted, and then the product was subjected to chiral separation to obtain **compound 55** of Example 55.

MS (ESI) m/z 418.1$(M+H)^+$.

$^1$H-NMR (400 MHz, DMSO-$d_6$) δ 8.81-8.80 (d, *J* = 4.7 Hz, 1H), 8.17-8.13 (m, 1H), 7.99-7.97 (d, *J* = 7.7 Hz, 1H), 7.77-7.73 (m, 1H), 7.32-7.29 (m, 2H), 7.14-7.09 (m, 2H), 4.83-4.70 (m, 2H), 4.54-4.19 (m, 7H), 3.87-3.82 (m, 1H), 3.66-3.63 (m, 1H), 3.46-3.42 (m, 1H).

**Example 56:** preparation of (1*H*-indol-3-yl)(1'-(pyridin-2-ylsulfonyl)-1',4'-dihydro-2*H*,2'*H*-[3,3'-diazetidinylidene] -1(4*H*)-yl)methanone (**compound 56**):

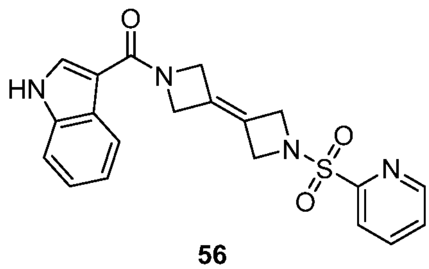

**56**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 1*H*-indole-3-carboxylic acid, and the same preparation method as that of Example 1 was adopted to obtain **compound 56** of Example 56.

MS (ESI) m/z 395.1$(M+H)^+$.

$^1$H NMR (400 MHz, DMSO-$d_6$) δ 11.71 (s, 1H), 8.84 (s, 1H), 8.19-8.16 (t, *J* = 1.2 Hz, 1H), 8.08-8.06 (d, *J* = 0.8 Hz, 1H), 8.02-8.00 (d, *J* = 0.8 Hz, 1H), 7.77-7.73 (d, *J* = 1.6 Hz, 2H), 7.43-7.41 (d, *J* = 0.8 Hz, 1H), 7.17-7.08 (m, 2H), 4.60(s, 8H).

**Example 57:** preparation of 3-hydroxy-1-(1'-(pyridin-2-ylsulfonyl)-1',4'-dihydro-2*H*,2'*H*-[3,3'-diazetidinylidene]-1 (4*H*)-yl)-2-(p-tolyl)propan-1-one (**compound 57**):

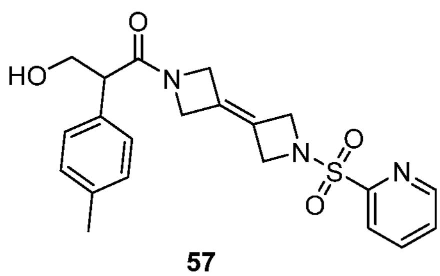

**57**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 3-hydroxy-2-(p-tolyl)propionic acid, and the same preparation method as that of Example 1 was adopted to obtain **compound 57** of Example 57.

MS (ESI) m/z 414.1$(M+H)^+$.

$^1$H-NMR (400 MHz, DMSO-$d_6$) δ 8.80-8.79 (m, 1H), 8.17-8.13 (m, 1H), 7.99-7.96 (m, 1H), 7.76-7.73 (m, 1H), 7.14-7.08 (m, 4H), 4.77-4.68 (m, 2H), 4.53 (s, 4H), 4.35-4.18 (m, 3H), 3.89-3.83 (m, 1H), 3.58-3.55 (m, 1H), 3.44-3.39 (m, 1H), 2.25 (s, 3H).

**Example 58:** preparation of (4-phenyltetrahydro-2*H*-pyran-4-yl)(1'-(pyridin-2-ylsulfonyl-(1',4'-dihydro-2*H*,2'*H*-[3, 3'-diazetidinylidene]-1(4*H*)-yl)methanone (**compound 58**):

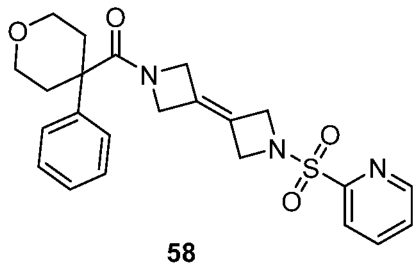

**58**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 4-phenyltetrahydro-2*H*-pyran-4-carboxylic acid, and the same preparation method as that of Example 1 was adopted to obtain **compound 58** of Example 58.

MS (ESI) m/z 440.2$(M+H)^+$.

$^1$H-NMR (400 MHz, DMSO-$d_6$) δ 8.78-8.76 (m, 1H), 8.16-8.11 (m, 1H), 7.95-7.93 (d, *J* =8.0 Hz, 1H), 7.76-7.73 (m, 1H), 7.40-7.25 (m, 5H), 4.46-3.99 (m, 8H), 3.73-3.70 (m, 2H), 3.56-3.51 (m, 2H), 2.18-2.15 (d, *J* = 13.5 Hz, 2H), 1.85-1.78 (m, 2H).

**Example 59:** preparation of 2-(3-chloro-4-fluorophenyl)-3-hydroxy-1-(1'-(pyridin-2-ylsulfonyl)-1',4'-dihydro-2*H*,2'*H*-[3,3'-diazetidinylidene]-1(4*H*-yl)propan-1-one (**compound 59**):

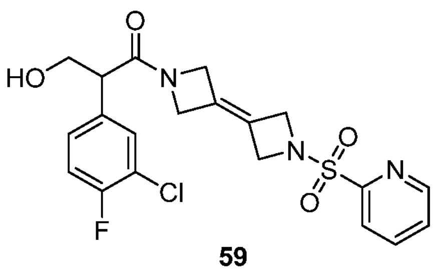

**59**

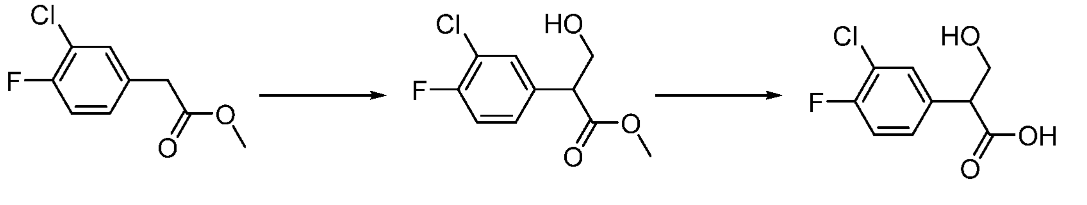

**59A** **59B** **59C**

**Step 1:** preparation of methyl 2-(3-chloro-4-fluorophenyl)-3-hydroxypropionate (**compound 59B**)

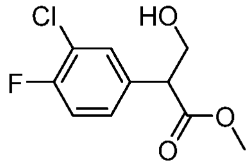

**59B**

Methyl 2-(3-chloro-4-fluorophenyl)acetate (2.2 g, 10.61 mmol) and paraformaldehyde (477.9 mg, 5.31 mmol) were dissolved in dimethyl sulfoxide (40.0 mL), and then added with sodium methoxide (191.1 mg, 1.06 mmol, 30%). The reaction solution was reacted at room temperature for 4 hours. After the reaction was complete, it was separated and purified through a silica gel column to obtain **compound 59B.**

MS (ESI) m/z 233.0 $(M+H)^+$.

**Step 2:** preparation of 2-(3-chloro-4-fluorophenyl)-3-hydroxypropionic acid (**compound 59C**)

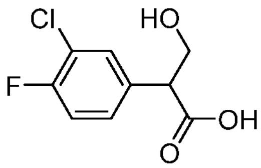

**59C**

Methyl 2-(3-chloro-4-fluorophenyl)-3-hydroxypropionate (2.0 g, 8.60 mmol) was dissolved in tetrahydrofuran (60.0 mL) and water (40.0 mL), and then added with sodium hydroxide (687.8 mg, 17.19 mmol), and the reaction solution was stirred at room temperature for 5 hours. After the reaction was complete, the reaction solution was adjusted to pH 2 with 1N hydrochloric acid, and extracted with dichloromethane (100 mL*3), and the organic phases were combined, washed with a saturated brine, and dried over anhydrous sodium sulfate. It was concentrated to obtain a yellow oily compound, which was directly used in the next step of reaction.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(3-chloro-4-fluorophenyl)-3-hydroxypropionic acid, and the same preparation method as that of Example 1 was adopted to obtain **compound 59** of Example 59.

MS (ESI) m/z 452.1 $(M+H)^+$.

$^1H$ NMR (400 MHz, DMSO-$d_6$) δ 8.82-8.81 (m, 1H), 8.19-8.14 (m, 1H), 8.00-7.97 (m, 1H), 7.78-7.74 (m, 1H), 7.51-7.49 (m, 1H), 7.37-7.31 (m, 2H), 4.94-4.87 (m, 1H), 4.78-4.40 (m, 6H), 4.38-4.19 (m, 2H), 3.87-3.77 (m, 2H), 3.55-3.43 (m, 1H).

**Example 60:** preparation of 3-hydroxy-1-(1'-(pyridin-2-ylsulfonyl)-1',4'-dihydro-2*H*,2'*H*-[3,3'-diazetidinylidene]-1 (4*H*-yl)-2-(pyridin-3-yl)propan-1-one (**compound 60**):

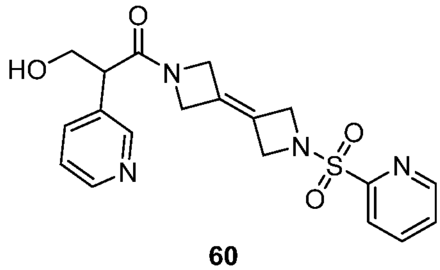

60

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 3-hydroxy-2-(pyridin-3-yl)propionic acid, and the same preparation method as that of Example 1 was adopted to obtain **compound 60** of Example 60.

MS (ESI) m/z 401.1$(M+H)^+$.

$^1$H-NMR (400 MHz, DMSO-$d_6$) $\delta$ 8.81-8.79 (m, 1H), 8.47-8.44 (m, 2H), 8.18-8.13 (m, 1H), 7.99-7.97 (d, *J* = 8.0 Hz, 1H), 7.77-7.69 (m, 2H), 7.34-7.31 (m, 1H), 4.94-4.92 (m, 1H), 4.76-4.73 (d, *J* = 12.6 Hz, 1H), 4.55-4.46 (m, 5H), 4.35-4.21 (m, 2H), 3.91-3.85 (m, 1H), 3.70 (m, 1H), 3.55-3.50 (m, 1H)

**Example 61:** preparation of 2-(3,4-difluorophenyl)-3-hydroxy-1-(1'-(pyridin-2-ylsulfonyl)-1',4'-dihydro-2*H*,2'*H*[3 ,3'-diazetidinylidene]-1(4*H*)-yl)propan-1-one (**compound 61**):

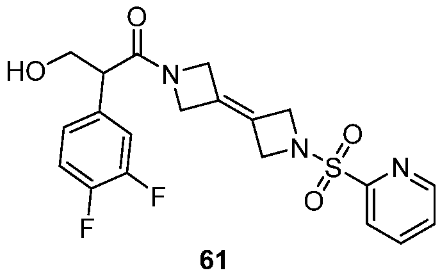

61

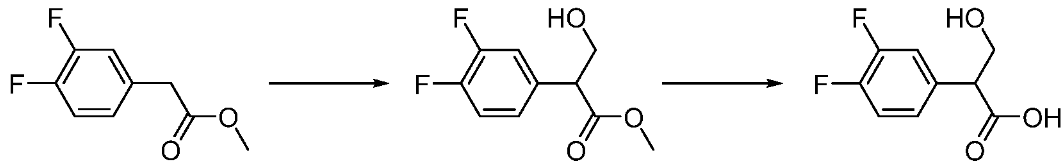

61A 61B 61C

**Step 1:** preparation of methyl 2-(3,4-difluorophenyl)-3-hydroxypropionate (**compound 61B**)

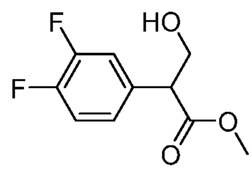

61B

Methyl 2-(3,4-difluorophenyl)acetate (0.5 g, 2.69 mmol) and paraformaldehyde (241.9 mg, 2.69 mmol) were dissolved in dimethyl sulfoxide (10.0 mL), and then added with sodium methoxide (48.4 mg, 0.27 mmol, 30%). The reaction solution was reacted at room temperature for 4 hours. After the reaction was complete, it was separated and

purified through a silica gel column to obtain **compound 50C.**

MS (ESI) m/z 217.2 $(M+H)^+$.

**Step 2:** preparation of 2-(3,4-difluorophenyl)-3-hydroxypropionic acid (**compound 61C**)

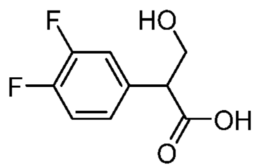

**61C**

Methyl 2-(3,4-difluorophenyl)-3-hydroxypropionate (320.0 mg, 1.48 mmol) was dissolved in methanol (15.0 mL) and water (10.0 mL), and then added with sodium hydroxide (118.4 mg, 2.96 mmol). The reaction solution was stirred at 50°C for 3 hours. After the reaction was complete, the reaction solution was adjusted to pH 2 with 1N hydrochloric acid, and extracted with dichloromethane (50 mL*3), and the organic phases were combined, washed with a saturated brine, and dried over anhydrous sodium sulfate. After concentration, it was directly used in the next step of reaction.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(3,4-difluorophenyl)-3-hydroxypropionic acid, and the same preparation method as that of Example 1 was adopted to obtain **compound 61** of Example 61.

MS (ESI) m/z 436.1 $(M+H)^+$.

$^1H$ NMR (400 MHz, DMSO-$d_6$) $\delta$ 8.82-8.78 (m, 1H), 8.18-8.14 (m, 1H), 8.00-7.95 (m, 1H), 7.79-7.74 (m, 1H), 7.39-7.32 (m, 2H), 7.15-7.11 (m, 1H), 4.90-4.87 (m, 1H), 4.73-4.69 (m, 1H), 4.55-4.44 (m, 5H), 4.34-4.21 (m, 2H), 3.85-3.80 (m, 1H), 3.69-3.65 (m, 1H), 3.50-3.45 (m, 1H).

**Example 62:** preparation of 3-hydroxy-3-methyl-2-phenyl-1-(1'-(pyridin-2-ylsulfonyl)-1',4'-dihydro-2*H*,2'*H*[3,3'-diazetidinylidene]-1(4*H*)-yl)butan-1-one (**compound 62**):

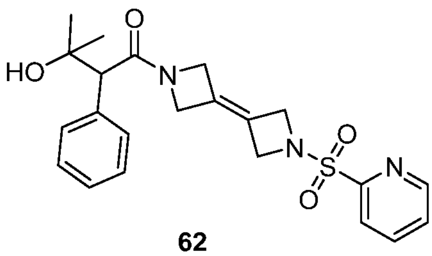

**62**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 3-hydroxy-3-methyl-2-phenylbutyric acid, and the same preparation method as that of Example 1 was adopted to obtain **compound 62** of Example 62.

MS (ESI) m/z 428.2$(M+H)^+$.

$^1H$ NMR (400 MHz, DMSO-$d_6$) $\delta$ 8.79-8.77 (m, 1H), 8.17-8.12 (m, 1H), 7.98-7.96 (d, *J* = 7.7 Hz, 1H), 7.75-7.72 (m, 1H), 7.38-7.22 (m, 5H), 4.89 (brs, 1H), 4.77-4.73 (m, 1H), 4.60-4.45 (m, 4H), 4.35-4.19 (m, 3H), 3.51 (s, 1H), 1.15 (s, 3H), 0.98 (s, 3H).

**Example 63:** preparation of (5)-3-hydroxy-1-(1'-((1-methyl-1*H*-benzimidazol-6-yl)sulfonyl)-1',4'-dihydro-2*H*,2'*H* -[3,3'-diazetidinylidene]-1(4*H*)-yl)-2-phenylpropan-1-one (**compound 63**):

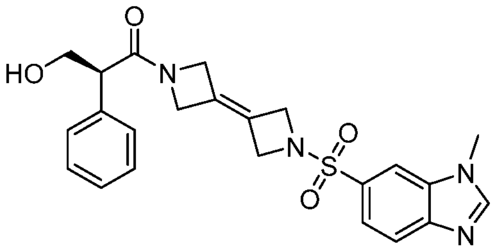

**63**

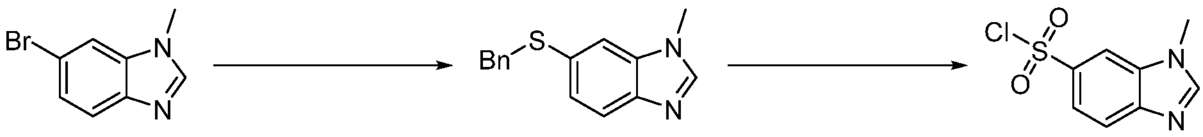

**63A** **63B** **63C**

**Step 1:** preparation of 6-(benzylthio)-1-methyl-1*H*-benzo[*d*]imidazole (**compound 63B**)

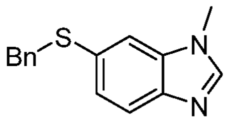

**63B**

6-bromo-1-methyl-1*H*-benzo[*d*]imidazole (1 g, 4.74 mmol) was dissolved in 1,4-dioxane solution, and then sequentially added with benzyl mercaptan (588 mg, 4.74 mmol), DIPEA (1.22 g, 9.48 mmol), $Pd_2(dba)_3CHCl_3$ (485 mg, 0.47 mmol) and xantphos (548 mg, 0.95 mmol). After the addition was completed, the reaction was replaced under nitrogen environment for 3 times, then heated to 80°C, and stirred for 16 hours. After the reaction was complete, it was separated and purified through a silica gel column to obtain **compound 63B.**

MS (ESI) m/z 255.1 $(M+H)^+$.

**Step 2:** preparation of 1-methyl-1*H*-benzo[*d*]imidazole-6-sulfonyl chloride (**compound 63C**)

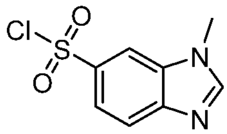

**63C**

6-(benzylthio)-1-methyl-1*H*-benzo[*d*]imidazole (560 mg, 2.20 mmol) was dissolved in acetonitrile. Then it was sequentially added with acetic acid (661 mg, 11.01 mmol), water (397 mg, 22.02 mmol) and 1,3-dichloro-5,5-dimethylimidazolidine-2,4-dione (868 mg, 4.40 mmol). After the addition was completed, the reaction was stirred at room temperature for 16 hours. After the reaction was complete, it was separated and purified through a silica gel column to obtain **compound 63C.**

MS (ESI) m/z 231.0 $(M+H)^+$.

In step 8 of Example 1, 2,3-dihydro-[1 ,4]dioxino[2,3-*b*]pyridine-7-sulfonyl chloride was replaced by 1-methyl-1*H*-benzo[*d*]imidazole-6-sulfonyl chloride, and the same preparation method as that of Example 1 was adopted to obtain **compound 63** of Example 63.

MS (ESI) m/z 453.2$(M+H)^+$.

$^1$H NMR (400 MHz, DMSO-$d_6$) $\delta$ 8.48 (s, 1H), 8.17-8.04 (m, 1H), 7.90 (d, *J* = 8.5 Hz, 1H), 7.70 - 7.59 (m, 1H), 7.25 (dt, *J* = 13.8, 7.2 Hz, 5H), 4.77 (t, *J* = 5.2 Hz, 1H), 4.67 (d, J = 12.5 Hz, 1H), 4.28 (d, *J* = 21 .7 Hz, 6H), 4.17 (d, *J*

= 14.6 Hz, 1H), 3.96 (s, 3H), 3.86 (td, *J* = 9.4, 5.5 Hz, 1H), 3.59 (dd, *J* = 8.8, 5.3 Hz, 1H), 3.45 (dd, *J* = 9.8, 4.9 Hz, 1H).

**Example 64:** preparation of (1'-(benzothiazol-6-ylsulfonyl)-1',4'-dihydro-2*H*,2'*H*-[3,3'-diazetidinylidene]-1(4*H*)-yl) (isoindolin-1-yl)methanone (**compound 64**):

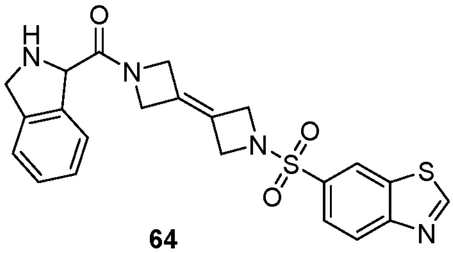

**64**

In step 8 of Example 1, 2,3-dihydro-[1,4]dioxino[2,3-*b*]pyridine-7-sulfonyl chloride was replaced by benzo[*d*]thiazole-6-sulfonyl chloride, and in step 10, (*S*)-3-((tert-butyldiphenylsilyl)oxy)-2-phenylpropionic acid was replaced by 2-(tert-butoxycarbonyl)isoindoline-1-carboxylic acid, and the same preparation method as that of Example 1 was adopted to obtain **compound 64** of Example 64.
MS (ESI) m/z 453.1(M+H)$^+$.
$^1$H NMR (400 MHz, DMSO-$d_6$) $\delta$ 9.69 (s, 1H), 8.82 (d, *J* = 1.8 Hz, 1H), 8.35 (d, *J* = 8.6 Hz, 1H), 7.96 (dd, *J* = 8.6, 1.9 Hz, 1H), 7.27-7.17 (m, 4H), 4.82 (m, 2H), 4.49 (m, 1H), 4.32 (m, 7H), 4.20 - 4.04 (m, 2H).

**Example 65:** preparation of (*S*)-4-(1'-(3-hydroxy-2-phenylpropionyl)-1',4'-dihydro-2*H*,2'*H*[3,3'-diazetidinylidene] -1(4*H*-ylsulfonyl)benzonitrile (**compound 65**):

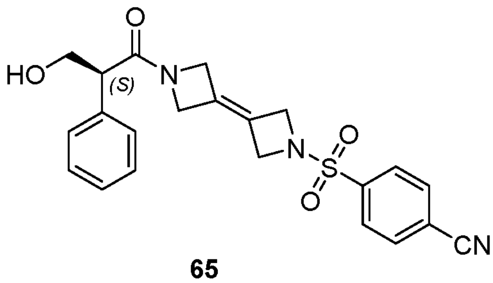

**65**

In step 8 of Example 1, 2,3-dihydro-[1,4]dioxino[2,3-*b*]pyridine-7-sulfonyl chloride was replaced by 4-cyanobenzenesulfonyl chloride, and the same preparation method as that of Example 1 was adopted to obtain **compound 65** of Example 65.
MS (ESI) m/z 424.1(M+H)$^+$.
$^1$H NMR (400 MHz, DMSO-$d_6$) $\delta$ 8.19-8.13 (m, 2H), 8.03-7.98 (m, 2H), 7.32-7.18 (m, 5H), 4.79 (t, *J* = 5.3 Hz, 1H), 4.70 (d, *J* = 12.5 Hz, 1H), 4.33 (d, *J* = 15.0 Hz, 6H), 4.19 (d, *J* = 14.4 Hz, 1H), 3.88 (m, 1H), 3.61 (dd, *J* = 8.8, 5.5 Hz, 1H), 3.45 (m, 1H).

**Example 66:** preparation of (*S*)-1-(1'-(5-chloropyridin-2-yl)sulfonyl)-1',4'-dihydro-2*H*,2'*H*[3,3'-diazetidinylidene] -1(4*H*)-yl)-3-hydroxy-2-phenylpropan-1-one (**compound 66**):

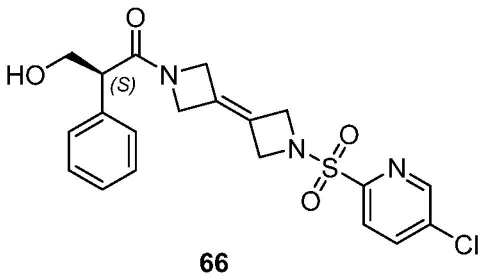

**66**

In step 8 of Example 1, 2,3-dihydro-[1,4]dioxino[2,3-*b*]pyridine-7-sulfonyl chloride was replaced by 5-chloropyridine-2-sulfonyl chloride, and the same preparation method as that of Example 1 was adopted to obtain **compound 66** of Example 66.

MS (ESI) m/z 434.1 $(M+H)^+$.

$^1$H NMR (400 MHz, DMSO-$d_6$) $\delta$ 8.93 - 8.84 (m, 1H), 8.28 (dd, *J* = 8.4, 2.4 Hz, 1H), 7.99 (dd, *J* = 8.4, 0.7 Hz, 1H), 7.31 - 7.23 (m, 5H), 4.82 - 4.67 (m, 2H), 4.54 (d, *J* = 2.8 Hz, 4H), 4.39 - 4.21 (m, 3H), 3.89 (m, 1H), 3.63 (dd, *J* = 8.9, 5.4 Hz, 1H), 3.46 (m, 1H).

**Example 67:** preparation of (*S*)-3-hydroxy-1-(1'-((5-methoxypyridin-2-yl)sulfonyl)-1',4'-dihydro-2*H*,2'*H*-[3,3'-dia zetidinylidene]-1(4*H*)-yl)-2-phenylpropan-1-one (**compound 67**):

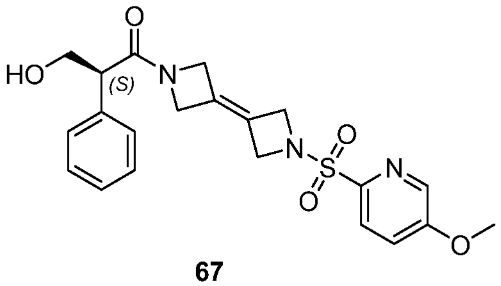

**67**

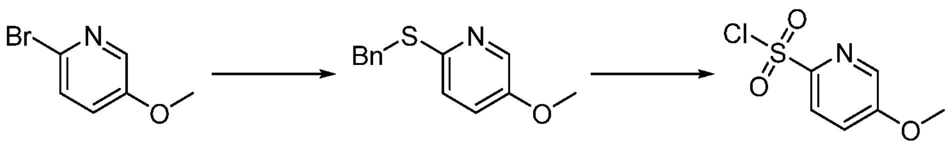

**67A** **67B** **67C**

**Step 1:** preparation of 2-(benzylthio)-5-methoxypyridine (**compound 67B**)

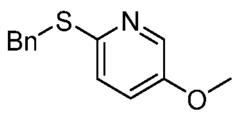

**67B**

2-bromo-5-methoxypyridine (1 g, 5.32 mmol) was dissolved in 1,4-dioxane solution, and then sequentially added with benzyl mercaptan (660 mg, 5.32 mmol), DIPEA (1.37 g, 10.64 mmol), $Pd_2(dba)_3CHCl_3$ (544 mg, 0.53 mmol) and xantphos (615 mg, 1.06 mmol). After the addition was completed, the reaction was replaced under nitrogen environment for 3 times, then heated to 80°C, and stirred for 16 hours. After the reaction was complete, it was separated and purified through a silica gel column to obtain **compound 67B.**

MS (ESI) m/z 232.1 $(M+H)^+$.

**Step 2:** preparation of 5-methoxypyridine-2-sulfonyl chloride (**compound 67C**)

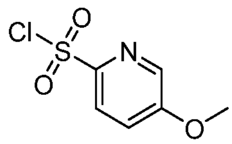

**67C**

2-(benzylthio)-5-methoxypyridine (1.3 g, 5.62 mmol) was dissolved in acetonitrile, and then sequentially added with acetic acid (1.69 g, 28.1 mmol), water (1.01 g, 56.2 mmol) and NCS (1.5 g, 11.24 mmol). After the addition was completed, the reaction was stirred at room temperature for 16 hours. After the reaction was complete, it was separated and purified through a silica gel column to obtain **compound 67C.**

MS (ESI) m/z 208.0 $(M+H)^+$.

In step 8 of Example 1, 2,3-dihydro-[1,4]dioxino[2,3-*b*]pyridine-7-sulfonyl chloride was replaced by 5-methoxypyridine-2-sulfonyl chloride, and the same preparation method as that of Example 1 was adopted to obtain **compound 67** of Example 67.

MS (ESI) m/z 430.1$(M+H)^+$.

$^1$H NMR (400 MHz, DMSO-$d_6$) $\delta$ 8.48 (d, *J* = 2.9 Hz, 1H), 7.94 (d, *J* = 8.7 Hz, 1H), 7.65 (m, 1H), 7.33 - 7.20 (m, 5H), 4.80 (t, *J* = 5.3 Hz, 1H), 4.72 (d, *J* = 12.8 Hz, 1H), 4.54 - 4.42 (m, 4H), 4.41 -4.27 (m, 2H), 4.25-4.16 (m, 1H), 3.93 (s, 4H), 3.62 (m, 1H), 3.46 (m, 1H).

**Example 68:** preparation of (*S*)-3-hydroxy-2-phenyl-1-(1'-(pyridin-3-ylsulfonyl)-1',4'-dihydro-2*H*,2'*H*-[3,3'-diaze tidinylidene]-1(4*H*)-yl)propane-1-one (**compound 68**):

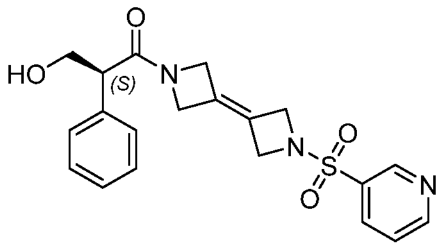

**68**

In step 8 of Example 1, 2,3-dihydro-[1,4]dioxino[2,3-*b*]pyridine-7-sulfonyl chloride was replaced by pyridine-3-sulfonyl chloride, and the same preparation method as that of Example 1 was adopted to obtain **compound 68** of Example 68.

MS (ESI) m/z 400.1$(M+H)^+$.

$^1$H NMR (400 MHz, DMSO-$d_6$) $\delta$ 8.99 (d, *J* = 2.3 Hz, 1H), 8.92 (dd, *J* = 4.8, 1.6 Hz, 1H), 8.26 (m, 1H), 7.72 (dd, *J* = 8.0, 4.9 Hz, 1H), 7.33 - 7.18 (m, 5H), 4.76 - 4.64 (m, 1H), 4.44 - 4.25 (m, 6H), 4.19 (d, *J* = 13.4 Hz, 1H), 3.88 (t, *J* = 9.5 Hz, 1H), 3.61 (dd, *J* = 8.8, 5.5 Hz, 1H), 3.45 (dd, *J* = 10.1, 5.5 Hz, 2H).

**Example 69:** preparation of (*S*)-3-hydroxy-2-phenyl-1-(1'-(quinolin-8-ylsulfonyl)-1',4'-dihydro-2*H*,2'*H*[3,3'-diaz etidinylidene]-1(4*H*)-yl)propan-1 -one (**compound 69**):

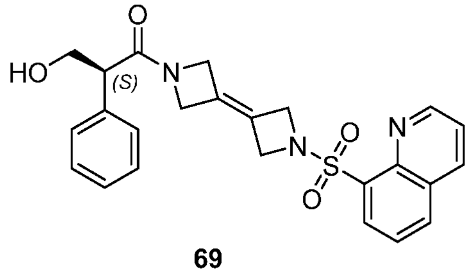

**69**

In step 8 of Example 1,2,3-dihydro-[1,4]dioxino[2,3-*b*]pyridine-7-sulfonyl chloride was replaced by quinoline-8-sulfonyl chloride, and the same preparation method as that of Example 1 was adopted to obtain **compound 69** of Example 69.

MS (ESI) m/z 450.1(M+H)$^+$.

$^1$H NMR (400 MHz, DMSO-$d_6$) $\delta$ 9.08 (dd, *J* = 4.2, 1.8 Hz, 1H), 8.56 (dd, *J* = 8.4, 1.8 Hz, 1H), 8.35 (m, 2H), 7.78 (t, *J* = 7.8 Hz, 1H), 7.71 (dd, *J* = 8.3, 4.2 Hz, 1H), 7.33-7.19 (m, 5H), 4.92-4.59 (m, 6H), 4.40-4.28 (m, 2H), 4.22 (m, 1H), 3.89 (t, *J* = 9.5 Hz, 1H), 3.62 (dd, *J* = 8.8, 5.5 Hz, 1H), 3.46 (dd, *J* = 10.1, 5.5 Hz, 1H).

**Example 70:** preparation of (*S*)-6-((1'-(3-hydroxy-2-phenylpropionyl)-1',4'-dihydro-2*H*,2'*H*-[3,3'-diazetidinylidene ]-1(4*H*-yl)sulfonyl)nicotinonitrile (**compound 70**):

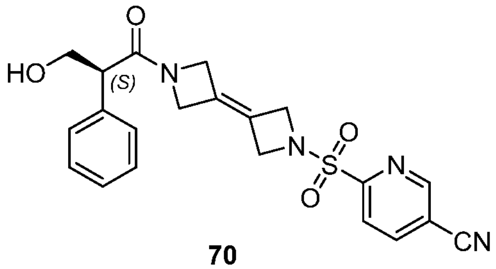

**70**

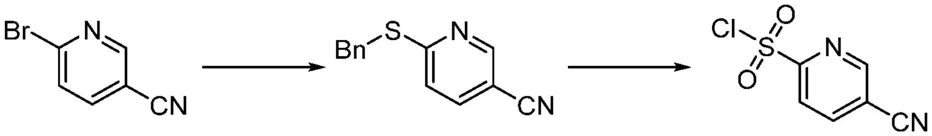

**70A** **70B** **70C**

**Step 1:** preparation of 6-(benzylthio)nicotinonitrile (**compound 70B**)

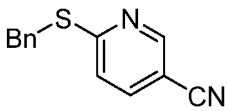

**70B**

2-bromo-5-cyanopyridine (2 g, 14.44 mmol) was dissolved in DMF, and then sequentially added with benzylthiol (1.79 g, 14.44 mmol) and cesium carbonate (5.65 g, 17.33 mmol). After the addition was completed, the reaction was heated to 60°C and stirred for 6 hours. After the reaction was complete, it was separated and purified through a silica gel column to obtain **compound 70B.**

MS (ESI) m/z 227.1 (M+H)$^+$

**Step 2:** preparation of 5-cyanopyridine-2-sulfonyl chloride (**compound 70C**):

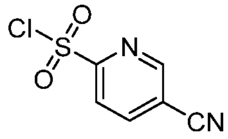

**70C**

6-(benzylthio)nicotinonitrile (1 g, 4.42 mmol) was dissolved in a mixed solution of dichloromethane and water. The reaction system was cooled to 0°C, and then slowly added dropwise with concentrated hydrochloric acid. After the dropwise addition was completed, the reaction was stirred at 0°C for 10 minutes, and then slowly added dropwise with a sodium hypochlorite aqueous solution. After the dropwise addition was completed, the reaction system was kept at 0°C and stirred for 10 minutes, and then subjected to quick liquid separation. The organic phase was directly used in the next step of reaction.

In step 8 of Example 1, 2,3-dihydro-[1,4]dioxino[2,3-*b*]pyridine-7-sulfonyl chloride was replaced by 5-cyanopyridine-2-sulfonyl chloride, and the same preparation method as that of Example 1 was adopted to obtain **compound 70** of Example 70.

MS (ESI) m/z 425.1 $(M+H)^+$.

$^1$H NMR (400 MHz, DMSO-$d_6$) $\delta$ 9.25 (dd, *J*= 2.1, 0.9 Hz, 1H), 8.67 (dd, *J* = 8.2, 2.1 Hz, 1H), 8.14 (dd, *J* = 8.1, 0.9 Hz, 1H), 7.33-7.20 (m, 5H), 4.80 (t, *J* = 5.3 Hz, 1H), 4.73 (m, 1H), 4.63-4.52 (m, 4H), 4.34 (dd, *J* = 21.7, 8.2 Hz, 2H), 4.27-4.18 (m, 1H), 3.90 (m, 1H), 3.63 (dd, *J* = 8.9, 5.5 Hz, 1H), 3.47 (m, 1H).

**Example 71:** preparation of 2-(4-(difluoromethyl) phenyl)-3-hydroxy-1-(1'-(pyridin-2-ylsulfonyl)-1',4'-dihydro-2*H*,2'*H*[3,3'-diazetidinyli dene]-1(4*H*)-yl)propan-1-one (**compound 71**):

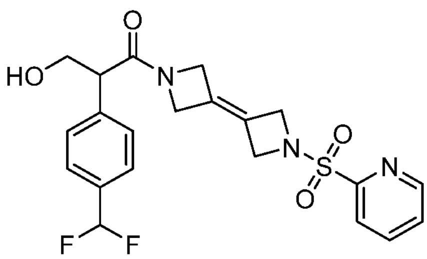

**71**

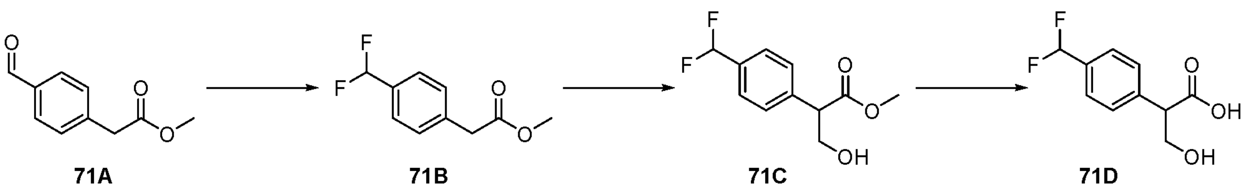

**71A** **71B** **71C** **71D**

**Step 1:** preparation of methyl 2-(4-(difluoromethyl)phenyl)acetate (**compound 71B**)

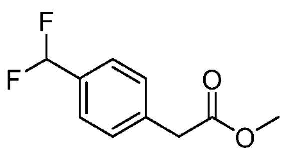

**71B**

Diethylaminosulfur trifluoride (3.62 g, 22.45 mmol) was added into the solution of methyl 2-(4-formylphenyl)acetate (2.0 g, 11.22 mmol) in dichloromethane (10.0 mL). The mixture was stirred at 50°C under the protection of nitrogen for 3 hours. After the reaction was complete, it was separated and purified through a silica gel column to obtain **compound 71B.**
MS (ESI) m/z 201.0 $(M+H)^+$.

**Step 2:** preparation of methyl 2-(4-(difluoromethyl)phenyl)-3-hydroxypropionate (**compound 71C**)

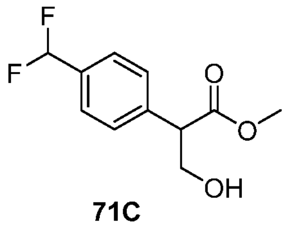

**71C**

Methyl 2-(4-(difluoromethyl)phenyl)acetate (1.5 g, 7.49 mmol) was dissolved in dimethyl sulfoxide (10.0 mL), then added with paraformaldehyde (681.13 mg, 7.57 mmol), and then slowly added with a sodium methoxide solution (136.23 mg, 756.81 μmol, purity of 30%). The reaction solution was reacted at room temperature for 3 hours. After the reaction was complete, it was separated and purified through a silica gel column to obtain **compound 71C.**
MS (ESI) m/z 231.0 $(M+H)^+$.

**Step 3:** preparation of 2-(4-(difluoromethyl)phenyl)-3-hydroxypropionic acid (**compound 71D**)

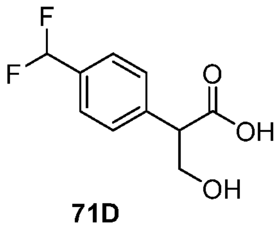

**71D**

Methyl 2-[4-(difluoromethyl)phenyl]-3-hydroxy-propionate (300 mg, 1.30 mmol) was dissolved in a mixed solution of water (3.0 mL), methanol (3.0 mL) and tetrahydrofuran (3.0 mL), and then added with lithium hydroxide (156.05 mg, 6.52 mmol). The mixture was stirred at 50°C for 3 hours. After the reaction was complete, it was separated and purified through a silica gel column to obtain **compound 71D**.
MS (ESI) m/z 217.0 $(M+H)^+$.
In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(4-(difluoromethyl)phenyl)-3-hydroxypropionic acid, and the same preparation method as that of Example 1 was adopted to obtain **compound 71** of Example 71.
MS (ESI) m/z 450.0 $(M+H)^+$.
$^1H$ NMR (400 MHz, DMSO-$d_6$) δ 8.80 (s, 1H), 8.17-8.13 (t, 1H), 7.98-7.97 (m, 1H), 7.76-7.72 (m, 1H), 7.51-7.49 (d, *J*=6.8 Hz, 2H), 7.42-7.40 (m, *J*=6.8 Hz, 2H), 7.113-6.85 (t, 1 H), 4.87 (s, 1H), 4.75-4.72 (d, *J*=12.0 Hz, 1H), 4.54 (s, 4H), 4.40-4.19 (m, 3 H), 3.88 (s, 1H), 3.71 (s, 1H), 3.49 (s, 1H).

**Example 72:** preparation of (*S*)-1-(1'-((5-ethoxypyridin-2-yl)sulfonyl)-1',4'-dihydro-2*H*,2*H*[3,3'-diazetidinylidene ]-1(4*H*)-yl)-3-hydroxy-2-phenylpropan-1-one (**compound 72**):

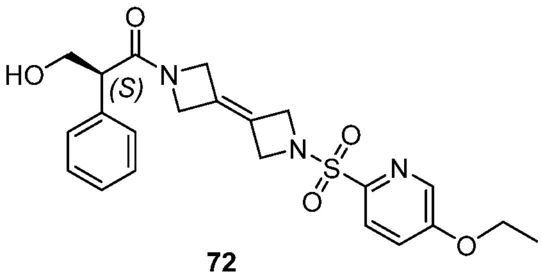

72

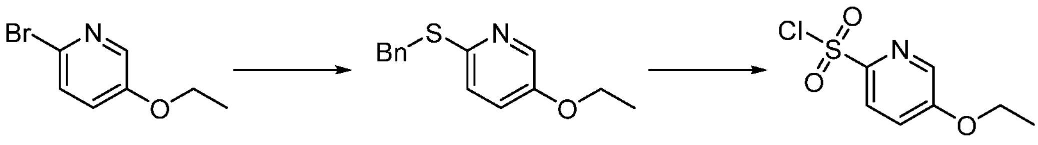

72A 72B 72C

**Step 1:** preparation of 2-(benzylthio)-5-ethoxypyridine (**compound 72B**)

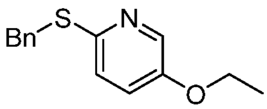

72B

2-bromo-5-ethoxypyridine (2 g, 9.9 mmol) was dissolved in 1,4-dioxane solution, and then sequentially added with benzyl mercaptan (1.23 g, 9.9mmol), DIPEA (3.28 ml, 19.8 mmol), $Pd_2(dba)_3CHCl_3$ (1.02 g, 0.99 mmol) and xantphos (1.15 g, 1.98 mmol). After the addition was completed, the reaction was replaced under nitrogen environment for 3 times, then heated to 80°C, and stirred for 16 hours. After the reaction was complete, it was separated and purified through a silica gel column to obtain **compound 72B.**

MS (ESI) m/z 246.1 $(M+H)^+$.

**Step 2:** preparation of 5-ethoxypyridine-2-sulfonyl chloride (**compound 72C**)

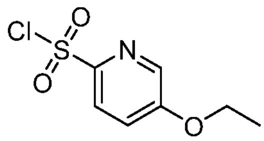

72C

2-(benzylthio)-5-ethoxypyridine (2.3 g, 9.37 mmol) was dissolved in acetonitrile, into which acetic acid (2.81 g, 46.85 mmol), water (1.69 g, 93.7 mmol) and 1,3-dichloroimidazolidine-2,4-dione (3.69 g, 18.74 mmol) were sequentially added. After the addition was completed, the reaction was stirred at room temperature for 16 hours. After the reaction was complete, it was separated and purified through a silica gel column to obtain **compound 72C.**

MS (ESI) m/z 221.9 $(M+H)^+$.

In step 8 of Example 1, 2,3-dihydro-[1,4]dioxino[2,3-*b*]pyridine-7-sulfonyl chloride was replaced by 5-ethoxypyridine-2-sulfonyl chloride, and the same preparation method as that of Example 1 was adopted to obtain **compound 72** of Example 72.

MS (ESI) m/z 444.1$(M+H)^+$.

$^1H$ NMR (400 MHz, DMSO-$d_6$) δ 8.46 (d, *J* = 2.8 Hz, 1H), 7.92 (d, *J* = 8.7 Hz, 1H), 7.63 (dd, *J* = 8.8, 2.9 Hz, 1H), 7.39-7.11 (m, 5H), 4.80 (t, *J* = 5.3 Hz, 1H), 4.72 (d, *J*= 13.4 Hz, 1H), 4.47 (s, 4H), 4.40 - 4.26 (m, 2H), 4.22 (q, *J*= 7.0 Hz, 3H), 3.89 (m, 1H), 3.62 (dd, *J*= 8.9, 5.5 Hz, 1H), 3.46 (m, 1H), 1.37 (t, J = 6.9 Hz, 3H).

**Example 73:** preparation of 3-fluoro-2-phenyl-1-(1'-(pyridin-2-ylsulfonyl)-1',4'-dihydro-2*H*,2'*H*[3,3'-diazetidinyli dene]-1(4*H*)-yl)propan-1-one (**compound 73**):

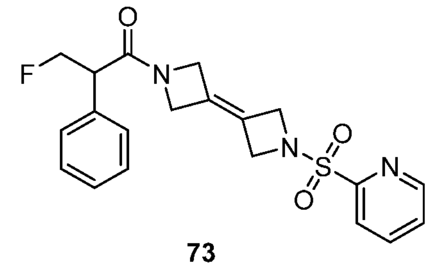

**73**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 3-fluoro-2-phenylpropionic acid, and the same preparation method as that of Example 1 was adopted to obtain **compound 73** of Example 73.

MS (ESI) m/z 402.1 $(M+H)^+$.

$^1$H-NMR (400 MHz, DMSO-$d_6$) δ 8.79-8.78 (d, *J*= 8.0 Hz, 1H), 8.17-8.12 (m, 1H), 7.98-7.96 (d, *J*= 8.0 Hz, 1H), 7.76-7.72 (m, 1H), 7.37-7.33 (m, 2H), 7.31-7.23 (m, 3H), 4.89-4.72 (m, 2H), 4.56-4.52 (m, 4H), 4.44-4.21 (m, 4H), 4.06-3.99 (m, 1H).

**Example 74:** preparation of 2-(2,4-difluorophenyl)-3-hydroxy-1-(1'-(pyridin-2-ylsulfonyl)-1',4'-dihydro-2*H*,2'*H*[3 ,3'-diazetidinylidene]-1(4*H*)-yl)propan-1-one (**compound 74**):

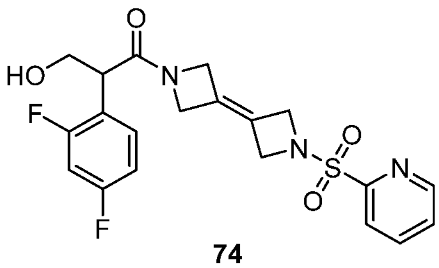

**74**

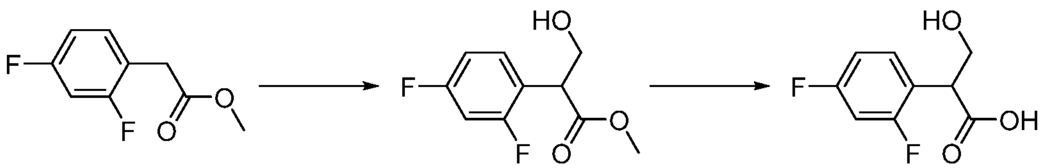

**74A** **74B** **74C**

**Step 1**: preparation of methyl 2-(2,4-difluorophenyl)-3-hydroxypropionate (**compound 74B**)

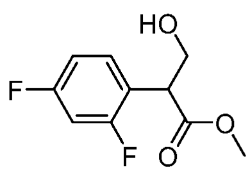

**74B**

Methyl 2-(2,4-difluorophenyl)acetate (0.8 g, 4.30 mmol) and paraformaldehyde (387.1 mg, 4.30 mmol) were dissolved in dimethyl sulfoxide (5.0 mL), and then added with sodium methoxide (77.4 mg, 0.43 mmol, 30%). The reaction solution was reacted at room temperature for 4 hours. After the reaction was complete, it was separated and purified through a silica gel column to obtain **compound 74B.**

MS (ESI) m/z 217.2 $(M+H)^+$.

**Step 2:** preparation of 2-(2,4-difluorophenyl)-3-hydroxypropionic acid (**compound 74C**)

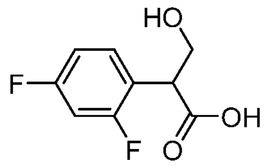

**74C**

Methyl 2-(2,4-difluorophenyl)-3-hydroxypropionate (440.0 mg, 2.04 mmol) was dissolved in methanol (15.0 mL) and water (10.0 mL), and then added with sodium hydroxide (162.8 mg, 4.07 mmol). The reaction solution was stirred at 50°C for 3 hours. After the reaction was complete, the reaction solution was adjusted to pH 2 with 1N hydrochloric acid, and extracted with dichloromethane (50 mL*3), and the organic phases were combined, washed with a saturated brine, and dried over anhydrous sodium sulfate. After concentration, it was directly used in the next step of reaction.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(2,4-difluorophenyl)-3-hydroxypropionic acid, and the same preparation method as that of Example 1 was adopted to obtain **compound 74** of Example 74.

MS (ESI) m/z 436.1 $(M+H)^+$.

$^1H$ NMR (400 MHz, DMSO-$d_6$) $\delta$ 8.81-8.80 (d, *J* = 3.8 Hz, 1H), 8.18-8.13 (t, *J* = 7.7 Hz, 1H), 7.99-7.97 (d, *J* = 7.7 Hz, 1H), 7.77-7.74 (t, *J* = 5.9 Hz, 1H), 7.45-7.39 (q, *J* = 7.7 Hz, 1H), 7.23-7.17 (t, *J* = 9.8 Hz, 1H), 7.07-7.02 (t, *J* = 8.4 Hz, 1H), 4.92-4.89 (m, 1H), 4.74-4.71 (m, 1H), 4.60-4.48 (m, 4H), 4.40-4.19 (m, 3H), 3.92-3.82 (m, 2H), 3.55-3.48 (m, 1H).

**Example 75:** preparation of 2-(4-chlorophenyl)-3-hydroxy-1-(1'-(pyridin-2-ylsulfonyl)-1',4'-dihydro-2*H*,2'*H*-[3,3' -diazetidinylidene]-1(4*H*)-yl)propan-1-one (**compound 75**):

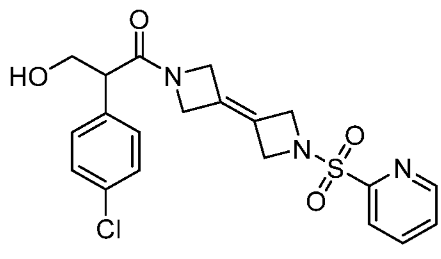

**75**

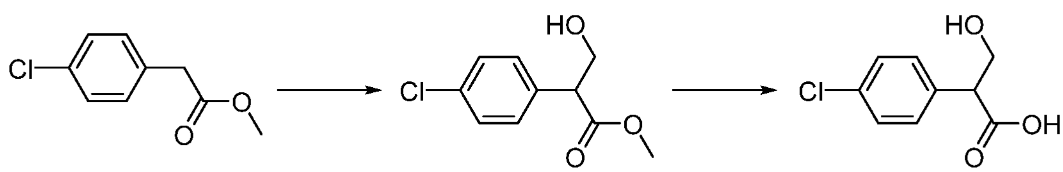

**75A** **75B** **75C**

**Step 1:** preparation of methyl 2-(4-chlorophenyl)-3-hydroxypropionate (**compound 75B**)

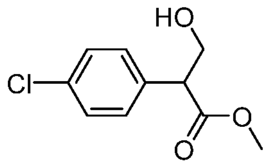

**75B**

Methyl 2-(4-chlorophenyl)acetate (2.0 g, 10.83 mmol) and paraformaldehyde (975.9 mg, 10.83 mmol) were dissolved in dimethyl sulfoxide (50.0 mL), and then added with sodium methoxide (195.1 mg, 1.08 mmol, 30%). The reaction solution was reacted at room temperature for 4 hours. After the reaction was complete, the reaction solution was diluted with water. The aqueous phase was extracted with ethyl acetate (50 mL*3). The organic phases were combined, washed with a saturated brine, and dried over anhydrous sodium sulfate. After the reaction was complete, it was separated and purified through a silica gel column to obtain **compound 75B.**

MS (ESI) m/z 215.1 $(M+H)^+$

**Step 2:** preparation of 2-(4-chlorophenyl)-3-hydroxypropionic acid (**compound 75C**)

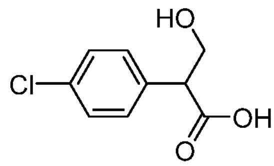

**75C**

Methyl 2-(4-chlorophenyl)-3-hydroxypropionate (1.3 g, 6.06 mmol) was dissolved in methanol (45.0 mL) and water (30.0 mL), and then added with sodium hydroxide (484.5 mg, 12.11 mmol). The reaction solution was stirred at 50°C for 3 hours. After the reaction was complete, the reaction solution was adjusted to pH 2 with 1N hydrochloric acid, and extracted with ethyl acetate (50 mL*3), and the organic phases were combined, washed with a saturated brine, and dried over anhydrous sodium sulfate. After concentration, it was directly used in the next step of reaction.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(4-chlorophenyl)-3-hydroxypropionic acid, and the same preparation method as that of Example 1 was adopted to obtain **compound 75** of Example 75.

MS (ESI) m/z 434.1$(M+H)^+$.

$^1$H NMR (400 MHz, DMSO-$d_6$) $\delta$ 8.81-8.80 (m, 1H), 8.17-8.12 (m, 1H), 7.99-7.97 (d, *J* = 7.7 Hz, 1H), 7.77-7.73 (m, 1H), 7.38-7.32 (m, 2H), 7.32-7.27 (m, 2H), 4.86-4.83 (t, *J= 5.2* Hz, 1H), 4.73-4.20 (m, 8H), 3.88-3.82 (m, 1H), 3.67-3.63 (m, 1H), 3.49-3.44 (m, 1H).

**Example 76:** preparation of (*R*)-2-(3-chloro-4-fluorophenyl)-3-hydroxy-1-(1'-(pyridin-2-ylsulfonyl)-1',4'-dihydro-2*H*,2'*H*-[3,3'-diazetidinylidene]-1(4*H*)-yl)propan-1-one (**compound 76**):

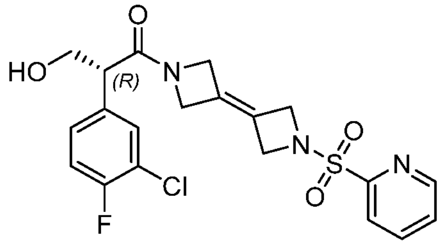

**76**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(3-chloro-4-fluorophenyl)-3-hydroxypropionic acid, the same preparation method as that of Example 1 was adopted, and then the product was subjected to chiral separation to obtain **compound 76** of Example 76.

MS (ESI) m/z 452.1 $(M+H)^+$.

$^1H$ NMR (400 MHz, DMSO-$d_6$) δ 8.82-8.81 (m, 1H), 8.19-8.14 (m, 1H), 8.00-7.97 (m, 1H), 7.78-7.74 (m, 1H), 7.51-7.49 (m, 1H), 7.37-7.31 (m, 2H), 4.94-4.87 (m, 1H), 4.78-4.40 (m, 6H), 4.38-4.19 (m, 2H), 3.87-3.77 (m, 2H), 3.55-3.43 (m, 1H).

**Example 77:** preparation of (*S*)-2-(3-chloro-4-fluorophenyl)-3-hydroxy-1-(1'-(pyridin-2-ylsulfonyl)-1',4'-dihydro-2*H*,2'*H*-[3,3'-diazetidinylidene]-1(4*H*)-yl)propan-1-one (**compound 77**):

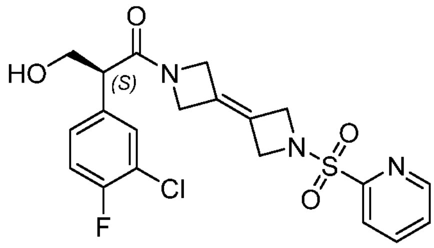

**77**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(3-chloro-4-fluorophenyl)-3-hydroxypropionic acid, the same preparation method as that of Example 1 was adopted, and then the product was subjected to chiral separation to obtain **compound 77** of Example 77.

MS (ESI) m/z 452.1 $(M+H)^+$.

$^1H$ NMR (400 MHz, DMSO-$d_6$) δ 8.82-8.81 (m, 1H), 8.19-8.14 (m, 1H), 8.00-7.97 (m, 1H), 7.78-7.74 (m, 1H), 7.51-7.49 (m, 1H), 7.37-7.31 (m, 2H), 4.94-4.87 (m, 1H), 4.78-4.40 (m, 6H), 4.38-4.19 (m, 2H), 3.87-3.77 (m, 2H), 3.55-3.43 (m, 1H).

**Example 78:** preparation of 3-hydroxy-1-(1'-(pyridin-2-ylsulfonyl)-1',4'-dihydro-2*H*,2'*H*[3,3'-diazetidinylidene]-1 (4*H*)-yl)-2-(4-(trifluoromethyl)phenyl)propan-1-one (**compound 78**):

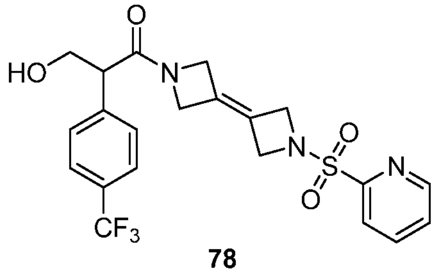

**78**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 3-hydroxy-2-(4-(trifluoromethyl)phenyl)propionic acid, and the same preparation method as that of Example 1 was adopted to obtain **compound 78** of Example 78.

MS (ESI) m/z 468.1$(M+H)^+$.

$^1$H-NMR (400 MHz, DMSO-$d_6$) δ 8.81-8.79 (d, *J* = 4.7 Hz, 1H), 8.18-8.13 (m, 1H), 7.99-7.97 (d, *J* = 8.0 Hz, 1H), 7.76-7.73 (m, 1H), 7.67-7.65 (d, *J* = 8.2 Hz, 2H), 7.52-7.50 (d, *J* = 8.2 Hz, 2H), 4.76-4.73 (d, *J* = 12.4 Hz, 1H), 4.54 (s, 4H), 4.43-4.39 (d, *J*= 13.2 Hz, 1H), 4.34-4.31 (d, *J* = 13.7 Hz, 1H), 4.24-4.21 (d, *J* = 12.9 Hz, 1H), 3.91-3.87 (m, 1H), 3.78-3.75 (m, 1H), 3.54-3.50(m, 1H), 2.67-2.65 (d, *J* = 7.4 Hz, 1H).

**Example 79:** preparation of 2-(3,4-dihydro-2*H*-benzo[*b*][1,4]oxazin-6-yl)-3-hydroxy-1-(1'-(pyridin-2-ylsulfonyl)-1',4'-dihydro-2*H*,2'*H*-[3,3'-diazetidinylidene]-1(4*H*)-yl)propan -1-one (**compound 79**):

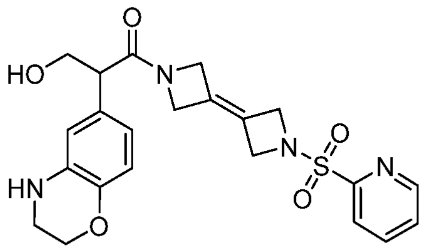

**79**

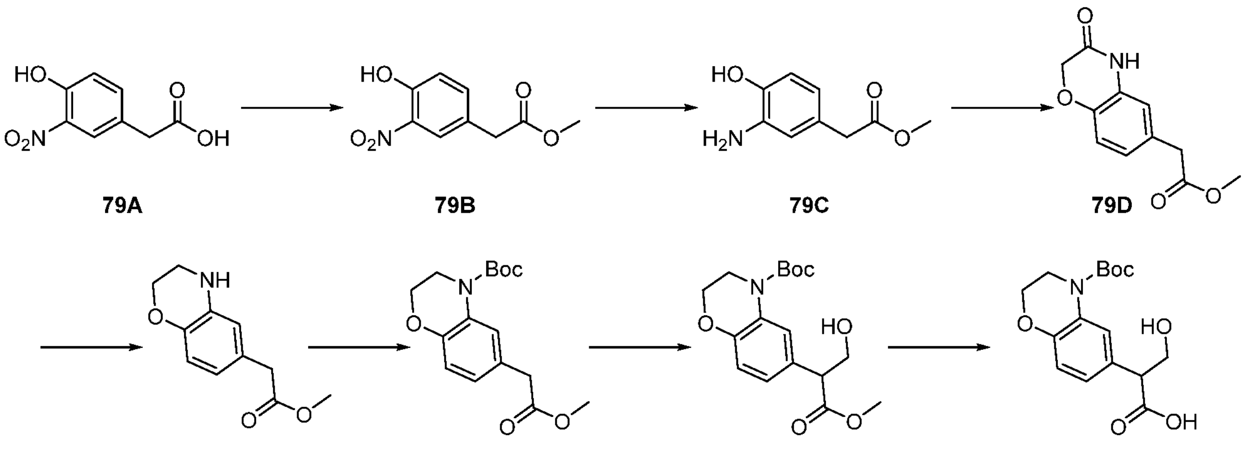

79A 79B 79C 79D

79E 79F 79G 79H

**Step 1:** preparation of methyl 2-(4-hydroxy-3-nitrophenyl)acetate (**compound 79B**)

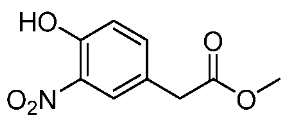

**79B**

2-(4-hydroxy-3-nitrophenyl)acetic acid (5.0 g, 25.36 mmol) was dissolved in anhydrous methanol (100 mL), and then slowly added with thionyl chloride (6.03 g, 50.72 mmol). The reaction solution was reacted at room temperature for 3 hours. After the reaction was complete, the reaction solution was diluted with water. The aqueous phase was extracted with ethyl acetate (100 mL*3). The organic phases were combined, washed with a saturated brine, and dried

over anhydrous sodium sulfate, and separated and purified through a silica gel column to obtain **compound 79B.**
MS (ESI) m/z 212.1 $(M+H)^+$

**Step 2:** preparation of methyl 2-(3-amino-4-hydroxyphenyl)acetate (**compound 79C**)

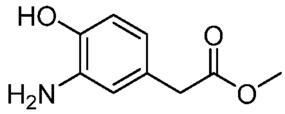

**79C**

Methyl 2-(4-hydroxy-3-nitrophenyl)acetate (4.7 g, 22.3 mmol) was dissolved in absolute ethanol (100.0 mL), and then added with palladium on carbon (470 mg, 2.23 mmol). The reaction solution was stirred at room temperature for 16 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 79C.**
MS (ESI) m/z 182.1 $(M+H)^+$.

**Step 3:** preparation of methyl 2-(3-oxo-3,4-dihydro-2*H*-benzo[*b*][1,4]oxazin-6-yl)acetate (**compound 79D**)

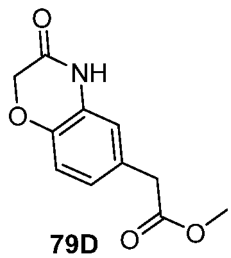

**79D**

Methyl 2-(4-fluoro-2-methoxyphenyl)acrylate (3.5 g, 19.3 mmol) was dissolved in ethyl acetate (100 mL) and water (100 mL), and then added with sodium carbonate (4.19 g, 38.6 mmol) and 2-chloroacetyl chloride (2.16 g, 19.3 mmol). The reaction solution was stirred at room temperature for 4 hours. After the reaction was complete, the product was extracted with ethyl acetate (200 mL) for 3 times, and the organic phases were combined, and separated and purified through a silica gel column to obtain **compound 79D.**
MS (ESI) m/z 222.1 $(M+H)^+$.

**Step 4:** preparation of methyl 2-(3,4-dihydro-2*H*-benzo[*b*][1,4]oxazin-6-yl)acetate (**compound 79E**)

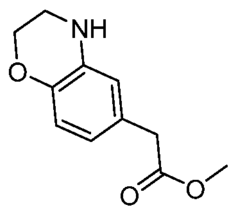

**79E**

Methyl 2-(3-oxo-3,4-dihydro-2*H*-benzo[*b*][1,4]oxazin-6-yl)acetate (2.4 g, 10.9 mmol) was dissolved in tetrahydrofuran (100.0 mL), and then added with the solution of borane in tetrahydrofuran (1.87 g, 21.7 mmol). The reaction solution was stirred at 60°C for 12 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 79E.**

MS (ESI) m/z 208.1 $(M+H)^+$.

**Step 5:** preparation of tert-butyl 6-(2-methoxy-2-oxoethyl)-2,3-dihydro-4*H*-benzo[b][1,4]oxazine-4-carboxylate (**compound 79F**)

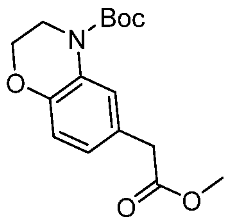

**79F**

Methyl 2-(3,4-dihydro-2*H*-benzo[*b*][1,4]oxazin-6-yl)acetate (1.2 g, 5.45 mmol) was dissolved in dichloromethane (100 mL), and then added with triethylamine (1.18 g, 10.9 mmol) and 4-dimethylaminopyridine (662.7 mg, 5.45 mmol). The reaction system was cooled to 0°C, and then slowly added with di-tert-butyl dicarbonate (1.77 g, 8.14 mmol), and the reaction solution was stirred at room temperature for 16 hours. After the reaction was complete, the reaction solution was diluted by adding water. The aqueous phase was extracted with ethyl acetate (100 mL*3). The organic phases were combined, washed with a saturated brine, and dried over anhydrous sodium sulfate, and separated and purified through a silica gel column to obtain **compound 79F.**

MS (ESI) m/z 308.1 $(M+H)^+$.

**Step 6:** preparation of tert-butyl 6-(3-hydroxy-1-methoxy-1-oxopropan-2-yl)-2,3-dihydro-4*H*-benzo[*b*][1,4]oxazine-4-carboxylate (**compound 79G**)

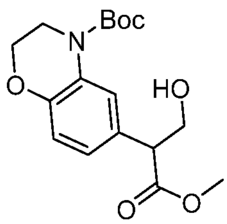

**79G**

Tert-butyl 6-(2-methoxy-2-oxoethyl)-2/*H*-benzo[*b*][1,4]oxazine-4(3*H*)-carboxylate (576 mg, 1.87 mmol) and paraformaldehyde (168.9 mg, 1.87mmol) were dissolved in dimethyl sulfoxide (50.0 mL), and then added with sodium methoxide (10.1 mg, 0.19 mmol). The reaction solution was reacted at room temperature for 4 hours. After the reaction was complete, the reaction solution was diluted by adding water. The aqueous phase was extracted with ethyl acetate (10 mL*3). The organic phases were combined, washed with a saturated brine, and dried over anhydrous sodium sulfate, and separated and purified through a silica gel column to obtain **compound 79F.**

MS (ESI) m/z 338.4 $(M+H)^+$.

**Step 7:** preparation of 2-(4-(tert-butoxycarbonyl)-3,4-dihydro-2*H*-benzo[*b*][1,4]oxazin-6-yl)-3-hydroxypropi onic acid (**compound 79H**)

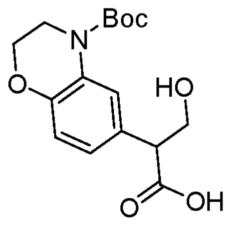

**79H**

Methyl 3-hydroxy-2-(naphthalene-2-yl)propionate (235.0 mg, 0.69 mmol) was dissolved in anhydrous methanol (60.0 mL) and water (40.0 mL), and then added with sodium hydroxide (27.8 mg, 0.69 mmol). The reaction solution was stirred at room temperature for 5 hours. After the reaction was complete, the reaction solution was adjusted to pH 2 with 1N hydrochloric acid, and extracted with dichloromethane (100 mL*3), and the organic phases were combined, washed with a saturated brine, and dried over anhydrous sodium sulfate. It was concentrated to obtain a yellow oily compound, which was directly used in the next step of reaction.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(4-(tert-butoxycarbonyl)-3,4-dihydro-2*H*-benzo[*b*][1,4]oxazin-6-yl)-3-hydroxypropi onic acid, and the same preparation method as that of Example 1 was adopted to obtain **compound 79** of Example 79.

MS (ESI) m/z 457.2(M+H)$^+$.

$^1$H-NMR (400 MHz, DMSO-$d_6$) δ 8.82-8.81 (m, 1H), 8.17-8.14(m, 1H), 8.00-7.98 (d, *J* = 8.0 Hz, 1H), 7.78-7.74 (m, 1H), 6.55-6.31 (m, 3H), 5.75 (s, 1H), 4.70-4.67 (m, 2H), 4.55 (s, 4H), 4.33-4.20 (m, 3H), 4.08-4.06 (m, 2H), 3.87-3.80 (m, 1H), 3.41-3.38 (m, 2H), 3.24 (s, 2H).

**Example 80:** preparation of (*S*)-1-(1'-((5-(difluoromethoxy)pyridin-2-yl)sulfonyl)-1',4'-dihydro-2*H*,2'*H*-[3,3'-diaze tidinylidene]-1(4*H*)-yl)-3-hydroxy-2-phenylpropan-1-one (**compound 80**):

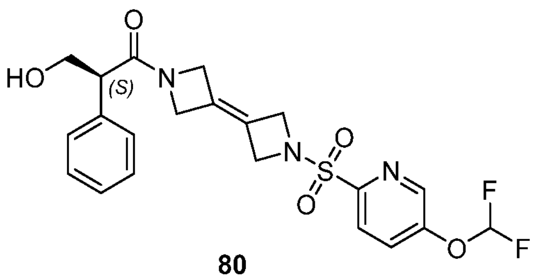

**80**

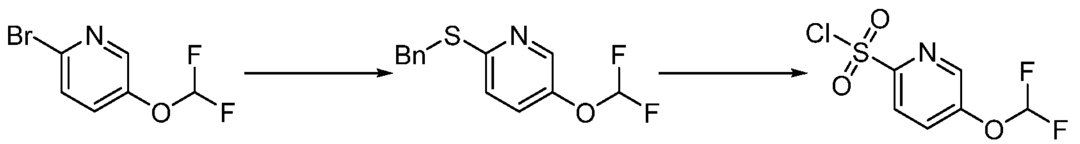

**80A** **80B** **80C**

**Step 1:** preparation of 2-(benzylthio)-5-(difluoromethoxy)pyridine (**compound 80B**)

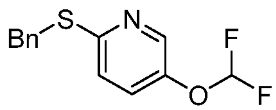

**80B**

2-bromo-5-difluoromethoxypyridine (2 g, 8.93 mmol) was dissolved in 1,4-dioxane solution, and then sequentially added with benzyl mercaptan (1.11 g, 8.93 mmol), DIPEA (2.31 g, 17.86 mmol), $Pd_2(dba)_3CHCl_3$ (913 mg, 0.89 mmol) and xantphos (1.03 g, 1.79 mmol). After the addition was completed, the reaction was replaced under nitrogen environment for 3 times, then heated to 80°C, and stirred for 16 hours. After the reaction was complete, it was separated and purified through a silica gel column to obtain

**compound 80B.**

MS (ESI) m/z 268.1 $(M+H)^+$.

**Step 2:** preparation of 5-(difluoromethoxy)pyridine-2-sulfonyl chloride (**compound 80C**)

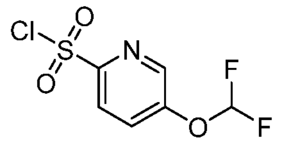

**80C**

2-(benzylthio)-5-difluoromethoxypyridine (1.0 g, 3.74 mmol) was dissolved in acetonitrile, into which acetic acid (1 .12 g, 18.71 mmol), water (674 mg, 37.41 mmol) and 1,3-dichloro-5,5-dimethylhydantoin (1.47 g, 7.48 mmol) were sequentially added. After the addition was completed, the reaction was stirred at room temperature for 16 hours. After the reaction was complete, it was separated and purified through a silica gel column to obtain **compound 80C.**

MS (ESI) m/z 244.0 (M+H)+.

In step 8 of Example 1, 2,3-dihydro-[1,4]dioxino[2,3-*b*]pyridine-7-sulfonyl chloride was replaced by 5-(difluoromethoxy)pyridine-2-sulfonyl chloride, and the same preparation method as that of Example 1 was adopted to obtain **compound 80** of Example 80.

MS (ESI) m/z 466.1$(M+H)^+$.

$^1H$ NMR (400 MHz, DMSO-$d_6$) δ 8.71 (d, *J* = 2.7 Hz, 1H), 8.07 (d, *J* = 8.7 Hz, 1H), 7.95 (dd, *J* = 8.7, 2.7 Hz, 1H), 7.50 (s, 1H), 7.34 -7.19 (m, 5H), 4.80 (t, *J* = 5.3 Hz, 1H), 4.73 (d, *J* = 13.3 Hz, 1H), 4.53 (s, 4H), 4.42 - 4.27 (m, 2H), 4.22 (d, *J* = 15.0 Hz, 1H), 3.89 (m, 1H), 3.63 (dd, *J* = 8.8, 5.5 Hz, 1H), 3.46 (m, 1H).

**Example 81:** preparation of (*S*)-3-hydroxy-1-(1'-((5-isopropoxypyridin-2-yl)sulfonyl)-1',4'-dihydro-2*H*,2'*H*-[3,3'-diazetidinylidene]-1(4*H*)-yl)-2-phenylpropan-1-one (**compound 81**):

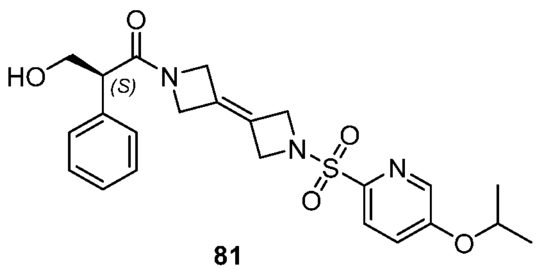

**81**

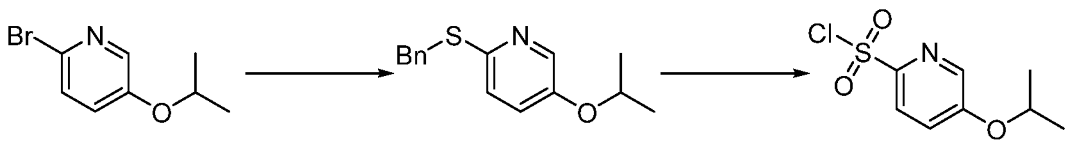

**81A** **81B** **81C**

**Step 1:** preparation of 2-(benzylthio)-5-isopropoxypyridine (**compound 81B**)

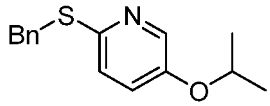

**81B**

2-bromo-5-isopropoxypyridine (2 g, 9.26 mmol) was dissolved in 1,4-dioxane solution, and then sequentially added with benzyl mercaptan (1.15 g, 9.26 mmol), DIPEA (2.39 g, 18.51 mmol), $Pd_2(dba)_3CHCl_3$ (947 mg, 0.93 mmol) and xantphos (1.07 g, 1.85 mmol). After the addition was completed, the raction was replaced under nitrogen environment for 3 times, then heated to 80°C, and stirred for 16 hours. After the reaction was complete, it was separated and purified through a silica gel column to obtain **compound 81B.**

MS (ESI) m/z 260.1$(M+H)^+$.

**Step 2:** preparation of 5-isopropoxypyridine-2-sulfonyl chloride (**compound 81C**)

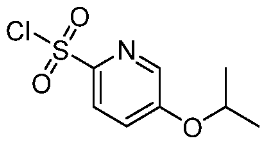

**81C**

2-(benzylthio)-5-isopropoxypyridine (1.0 g, 3.86 mmol) was dissolved in acetonitrile, Into which acetic acid (1.16 g, 19.28 mmol), water (695 mg, 38.56 mmol) and 1,3-dichloro-5,5-dimethylhydantoin (1.52 g, 7.71 mmol) were sequentially added. After the material addition was completed, the reaction was stirred at room temperature for 16 hours. After the reaction was complete, it was separated and purified through a silica gel column to obtain **compound 81C.**

MS (ESI) m/z 236.0$(M+H)^+$.

In step 8 of Example 1, 2,3-dihydro-[1,4]dioxino[2,3-*b*]pyridine-7-sulfonyl chloride was replaced by 5-isopropoxypyridine-2-sulfonyl chloride, and the same preparation method as that of Example 1 was adopted to obtain **compound 81** of Example 81.

MS (ESI) m/z 458.2$(M+H)^+$.

$^1H$ NMR (400 MHz, DMSO-$d_6$) δ 8.42 (d, *J* = 2.8 Hz, 1H), 7.90 (d, *J* = 8.8 Hz, 1H), 7.64 (dd, *J* = 8.8, 2.8 Hz, 1H), 7.26 (m, 5H), 4.82 (m, 2H), 4.72 (d, *J* = 13.3 Hz, 1H), 4.47 (s, 4H), 4.42 -4.26 (m, 2H), 4.21 (d, *J* = 14.6 Hz, 1H), 3.89 (m, 1H), 3.62 (dd, *J* = 8.9, 5.5 Hz, 1H), 3.46 (m, 1H), 1.32 (d, *J* = 6.0 Hz, 6H).

**Example 82:** preparation of (*S*)-1-(1'-((5-cyclopropoxypyridin-2-yl)sulfonyl)-1',4'-dihydro-2*H*,2'*H*[3,3'-diazetidin ylidene]-1(4*H*)-yl)-3-hydroxy-2-phenylpropan-1-one (**compound 82**):

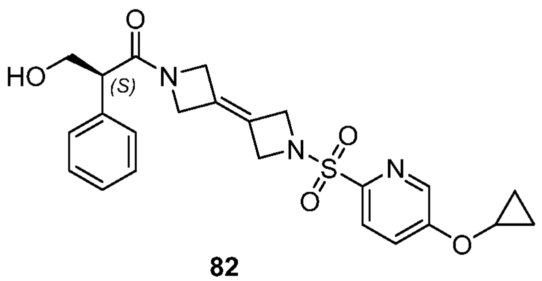

**82**

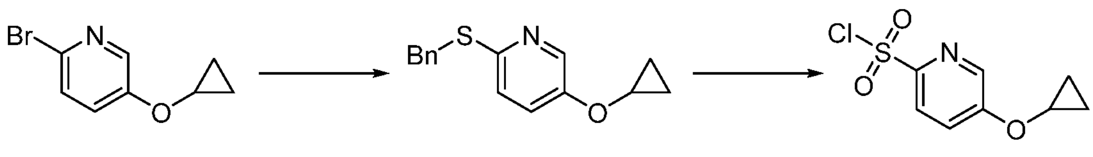

**82A** **82B** **82C**

**Step 1:** preparation of 2-(benzylthio)-5-cyclopropoxypyridine (**compound 82B**)

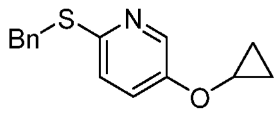

**82B**

2-bromo-5-cyclopropoxypyridine (3.98 g, 18.59 mmol) was dissolved in 1,4-dioxane solution, and then sequentially added with benzyl mercaptan (2.31 g, 18.59mmol), DIPEA (7.21 g, 55.77mmol), $Pd_2(dba)_3CHCl_3$ (1.92 g, 1.86 mmol) and xantphos (2.15 g, 3.72 mmol). After the addition was completed, the reaction was replaced under nitrogen environment for 3 times, then heated to 80°C, and stirred for 16 hours. After the reaction was complete, it was separated and purified through a silica gel column to obtain **compound 82B.**
MS (ESI) m/z 258.1 $(M+H)^+$.

**Step 2:** preparation of 5-cyclopropoxypyridine-2-sulfonyl chloride (**compound 82C**)

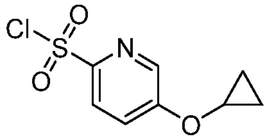

**82C**

2-(benzylthio)-5-cyclopropoxypyridine (2.0 g, 7.78 mmol) was dissolved in acetonitrile, into which acetic acid (2.33 g, 38.9 mmol), water (1.40 g, 77.8 mmol) and 1 ,3-dichloroimidazolidine-2,4-dione (3.06 g, 15.56 mmol) were sequentially added. After the addition was completed, the reaction was stirred at room temperature for 16 hours. After the reaction was complete, it was separated and purified through a silica gel column to obtain **compound 82C.**
MS (ESI) m/z 233.9 $(M+H)^+$.
In step 8 of Example 1, 2,3-dihydro-[1,4]dioxino[2,3-*b*]pyridine-7-sulfonyl chloride was replaced by 5-cyclopropoxypyridine-2-sulfonyl chloride, and the same preparation method as that of Example 1 was adopted to obtain **compound 82** of Example 82.
MS (ESI) m/z 456.2$(M+H)^+$.
$^1H$ NMR (400 MHz, Chloroform-*d*) δ 8.46 (d, *J* = 2.8 Hz, 1H), 7.93 (d, *J* = 8.7 Hz, 1H), 7.53 (dd, *J* = 8.6, 2.9 Hz, 1H), 7.38 -7.28 (m, 3H), 7.25 -7.19 (m, 2H), 4.50 (d, *J* = 29.8 Hz, 7H), 4.19 - 3.92 (m, 2H), 3.86 (m, *J* = 6.4, 2.9 Hz, 1H), 3.71 (dd, *J* = 11.2, 4.4 Hz, 1H), 3.65 (dd, *J* = 8.8, 4.4 Hz, 1H), 0.97 - 0.79 (m, 4H).

**Example 83:** preparation of (*S*)-2-(2,4-difluorophenyl)-3-hydroxy-1-(1'-(pyridin-2-ylsulfonyl)-1',4'-dihydro-2*H*,2' *H*-[3,3'-diazetidinylidene]-1(4*H*)-yl)propan-1-one (**compound 83**):

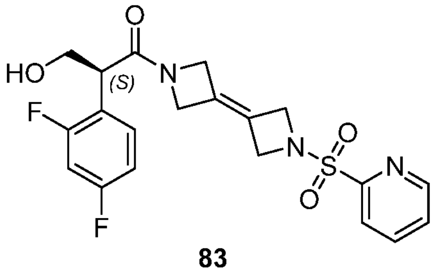

**83**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(2,4-difluorophenyl)-3-hydroxypropionic acid, the same preparation method as that of Example 1 was adopted for separation, and then the product was subjected to chiral separation to obtain **compound 83** of Example 83.

MS (ESI) m/z 436.1 $(M+H)^+$.

$^1H$ NMR (400 MHz, DMSO-$d_6$) $\delta$ 8.81-8.80 (d, *J* = 3.8 Hz, 1H), 8.18-8.13 (t, *J* = 7.7 Hz, 1H), 7.99-7.97 (d, *J*= 7.7 Hz, 1H), 7.77-7.74 (t, *J* = 5.9 Hz, 1H), 7.45-7.39 (q, *J*= 7.7 Hz, 1H), 7.23-7.17 (t, *J* = 9.8 Hz, 1H), 7.07-7.02 (t, *J* = 8.4 Hz, 1H), 4.92-4.89 (m, 1H), 4.74-4.71 (m, 1H), 4.60-4.48 (m, 4H), 4.40-4.19 (m, 3H), 3.92-3.82 (m, 2H), 3.55-3.48 (m, 1H).

**Example 84:** preparation of (*R*)-2-(2,4-difluorophenyl)-3-hydroxy-1 -(1'-(pyridin-2-ylsulfonyl)-1',4'-dihydro-2*H*,2' *H*-[3,3'-diazetidinylidene]-1(4*H*)-yl)propan-1-one (**compound 84**):

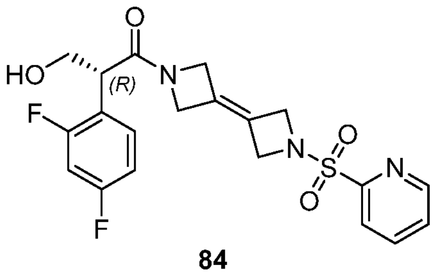

**84**

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(2,4-difluorophenyl)-3-hydroxypropionic acid, the same preparation method as that of Example 1 was adopted for separation, and then the product was subjected to chiral separation to obtain **compound 84** of Example 84.

MS (ESI) m/z 436.1 $(M+H)^+$.

$^1H$ NMR (400 MHz, DMSO-$d_6$) $\delta$ 8.81-8.80 (d, *J* = 3.8 Hz, 1H), 8.18-8.13 (t, *J* = 7.7 Hz, 1H), 7.99-7.97 (d, *J* = 7.7 Hz, 1H), 7.77-7.74 (t, *J* = 5.9 Hz, 1H), 7.45-7.39 (q, *J* = 7.7 Hz, 1H), 7.23-7.17 (t, *J* = 9.8 Hz, 1H), 7.07-7.02 (t, *J* = 8.4 Hz, 1H), 4.92-4.89 (m, 1H), 4.74-4.71 (m, 1H), 4.60-4.48 (m, 4H), 4.40-4.19 (m, 3H), 3.92-3.82 (m, 2H), 3.55-3.48 (m, 1H)

**Example 85:** preparation of 4-(3-hydroxy-1-oxo-1-(1'-(pyridin-2-ylsulfonyl)-1',4'-dihydro-2*H*,2'*H*[3,3'-diazetidin ylidene]-1(4*H*)-yl)propan-2-yl)benzonitrile (**compound 85**):

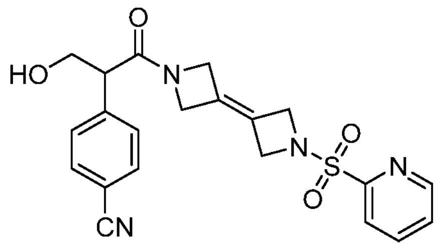

**85**

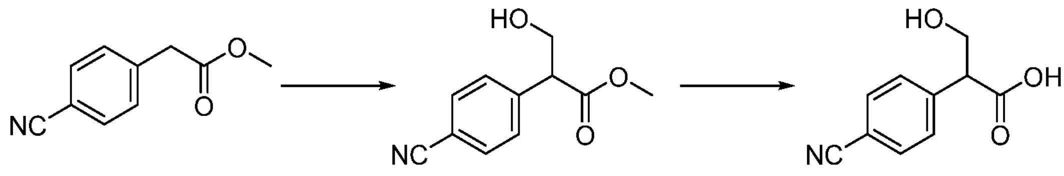

**85A** **85B** **85C**

**Step 1:** preparation of methyl 2-(4-cyanophenyl)-3-hydroxypropionate (**compound 85B**)

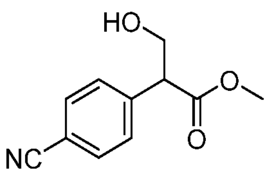

**85B**

Methyl 2-(4-cyanophenyl)acetate (1.0 g, 5.71 mmol), paraformaldehyde (257 mg, 2.85 mmol) and sodium bicarbonate (14 mg, 0.17 mmol) were dissolved in a dimethyl sulfoxide solution (10 mL). After the addition was completed, the reaction system was placed and stirred at room temperature for 3 hours. After the reaction was complete, it was separated and purified through a silica gel column to obtain **compound 85B.**

MS (ESI) m/z 206.1 $(M+H)^+$.

**Step 2:** preparation of 2-(4-cyanophenyl)-3-hydroxypropionic acid (**compound 85C**)

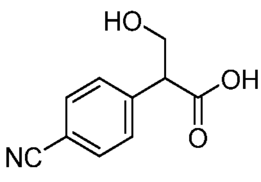

**85C**

Methyl 3-hydroxy-2-(4-cyanophenyl)propionate (0.2 g, 0.97 mmol) and lithium hydroxide monohydrate (82 mg, 1.95 mmol) were dissolved in a mixed solution of methanol (3.0 mL) and water (1 mL). After the addition was completed, the reaction system was placed and stirred at room temperature overnight. After the reaction was complete, it was separated and purified through a silica gel column to obtain **compound 85C.**

MS (ESI) m/z 192.2 $(M+H)^+$.

In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(4-cyanophenyl)-3-hydroxypropionic acid, and the same preparation method as that of Example 1 was adopted for separation to obtain **compound 85** of Example 85.

MS (ESI) m/z 425.1$(M+H)^+$.

$^{1}H$ NMR (400 MHz, DMSO-$d_6$) δ 8.82-8.81 (m, 1H), 8.17-8.14 (t, *J* = 1.2 Hz, 1H), 8.00-7.98 (d, *J* = 0.8Hz, 1H), 7.79-7.77 (m, 3H), 7.51-7.48 (d, *J* = 1.2 Hz, 2H), 4.94-4.92 (d, *J* = 0.8 Hz, 1H), 4.75-4.72 (d, *J* = 1.2 Hz, 1H), 4.55-4.25 (m, 6H), 3.91-3.81 (m, 1H), 3.79-3.77 (t, *J* = 0.8 Hz, 1H), 3.56-3.38 (m, 2H).

**Example 86:** preparation of 2-(3-chloro-5-fluorophenyl)-3-hydroxy-1-(1'-(pyridin-2-ylsulfonyl)-1',4'-dihydro-2*H*,2'*H*-[3,3'-diazetidinylidene]-1(4*H*)-yl)propan-1-one (**compound 86**):

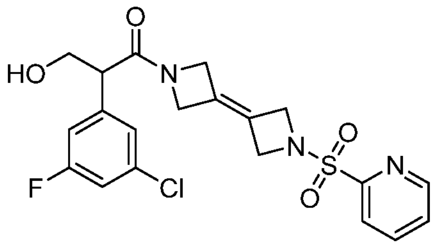

**86**

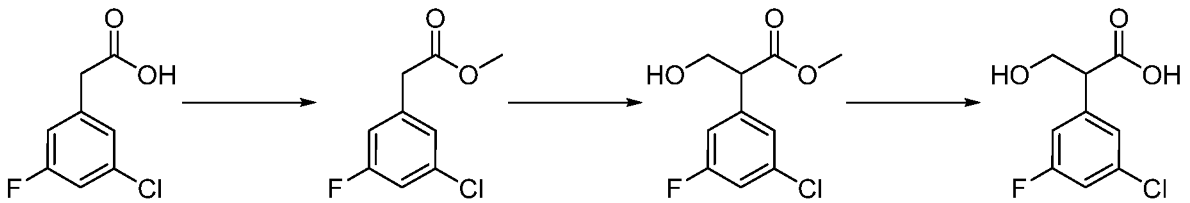

**86A** **86B** **86C** **86D**

**Step 1:** preparation of methyl 2-(3-chloro-5-fluorophenyl)acetate (**compound 86B**)

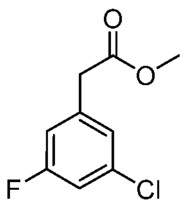

**86B**

2-(3-chloro-5-fluorophenyl)acetic acid (1 g, 5.30 mmol) was dissolved in methanol (10 mL), and then added with thionyl chloride (1.25 g, 10.59 mmol) at 0°C. After the addition was completed, the reaction system was placed and stirred at 60°C under nitrogen atmosphere for 3 hours. After the reaction was complete, it was separated and purified through a silica gel column to obtain **compound 86B.**

MS (ESI) m/z 201.2 (M-H)⁻.

**Step 2:** preparation of methyl 2-(3-chloro-5-fluorophenyl)-3-hydroxypropionate (**compound 86C**)

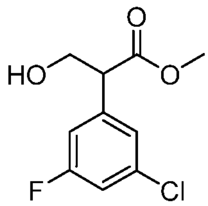

**86C**

Methyl 2-(3-chloro-5-fluorophenyl)acetate (1.01 g, 4.98 mmol) was dissolved in dimethyl sulfoxide (10 mL), and then added with sodium methoxide (90 mg, 500.00 μmol) and paraformaldehyde (220 mg, 2.44 mmol) at room temperature. After the addition was completed, the reaction system was placed and stirred at room temperature under nitrogen atmosphere for 1 hour. The reaction solution was diluted by adding water, the aqueous phase was extracted with ethyl acetate (10.0 mL*3), and the organic phases were combined, washed with a saturated brine, and then dried over anhydrous sodium sulfate. After the reaction was complete, it was separated and purified through a silica gel column to obtain **compound 86C.**
MS (ESI) m/z 233.1 $(M+H)^+$.

**Step 3:** preparation of 2-(3-chloro-5-fluorophenyl)-3-hydroxypropionic acid (**compound 86D**)

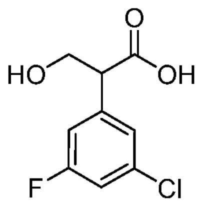

**86D**

Methyl 2-(3-chloro-5-fluorophenyl)-3-hydroxypropionate (200 mg, 859.71 μmol) was dissolved in tetrahydrofuran (3 mL) and water (3 mL), and added with lithium hydroxide (72 mg, 1.71 mmol) at room temperature. After the addition was completed, the reaction system was placed and stirred at room temperature under nitrogen atmosphere for 2 hours. After the reaction was completed, the reaction solution was concentrated and adjusted to the pH value of 3-4 with 1N hydrochloric acid solution. The aqueous phase was extracted with ethyl acetate (10.0 mL*3), and the organic phases were combined, washed with a saturated brine, and then dried over anhydrous sodium sulfate. After the reaction was complete, it was separated and purified through a silica gel column to obtain

**compound 86D.**

MS (ESI) m/z 217.1 $(M-H)^-$.
In step 8 of Example 1, 2,3-dihydro-[1,4]dioxino[2,3-*b*]pyridine-7-sulfonyl chloride was replaced by pyridine-2-sulfonyl chloride, and in step 10, (*S*)-3-((tert-butyldiphenylsilyl)oxy)-2-phenylpropionic acid was replaced by 2-(3-chloro-5-fluorophenyl)-3-hydroxypropionic acid, and the same preparation method as that of Example 1 was adopted for separation to obtain **compound 86** of Example 86. MS (ESI) m/z 452.1$(M+H)^+$.
$^1$H-NMR (400 MHz, DMSO-$d_6$) δ 8.82-8.81 (m, 1H), 8.16-8.14 (m, 1H), 8.00-7.98 (m, 1H), 7.77-7.74 (m, 1H), 7.34-7.30 (m, 1H), 7.24 (s, 1H), 7.17-7.14 (m, 1H), 4.94 (t, *J* = 5.2 Hz, 1H), 4.74-4.70 (m, 1H), 4.55-4.50 (m, 5H), 4.35-4.23 (m, 2H), 3.86- 3.80 (m, 1H), 3.73-3.70 (m, 1H), 3.54-3.49 (m, 1H).

**Example 87:** preparation of (*S*)-2-(2,4-difluorophenyl)-3-hydroxy-1-(1'-((5-methoxypyridin-2-yl)sulfonyl)-1',4'-di hydro-2*H*,2'*H*[3,3'-diazetidinylidene]-1(4*H*)-yl)propan-1-one (**compound 87**):

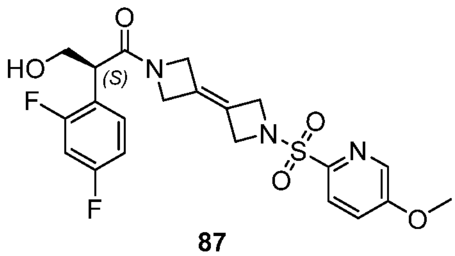

**87**

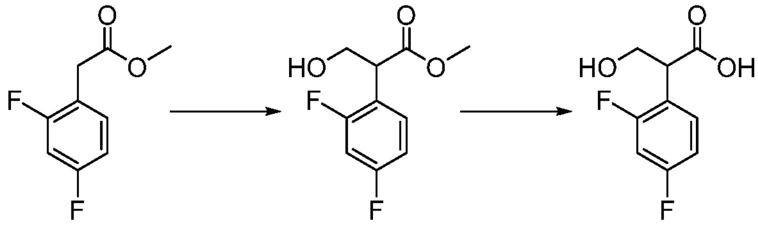

**87A** **87B** **87C**

Step 1: preparation of methyl 2-(2,4-difluorophenyl)-3-hydroxypropionate (compound 87B)

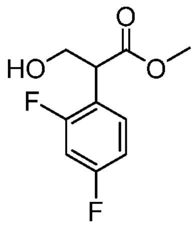

**87B**

Methyl 2-(2,4-difluorophenyl)acetate (820 mg, 4.40 mmol) was dissolved in dimethyl sulfoxide (8 mL), and then added with sodium methoxide (80 mg, 444.44 μmol, purity of 30%) and paraformaldehyde (200 mg, 2.22 mmol) at room temperature. After the addition was completed, the reaction system was placed and stirred at room temperature under nitrogen atmosphere for 3 hours. After the reaction was complete, the reaction solution was diluted by adding water. The aqueous phase was extracted with ethyl acetate (30.0 mL*3), and the organic phases were combined, washed with a saturated brine, and then dried over anhydrous sodium sulfate, and separated and purified through a silica gel column to obtain compound 87B.

MS (ESI) m/z 217.1 $(M+H)^+$.

Step 2: preparation of 2-(2,4-difluorophenyl)-3-hydroxypropionic acid (compound 87C)

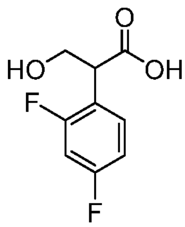

**87C**

Methyl 2-(2,4-difluorophenyl)-3-hydroxy-propionate (260 mg, 1.20 mmol) was dissolved in water (3 mL) and

tetrahydrofuran (3 mL), and added with lithium hydroxide (100 mg, 2.38 mmol) at room temperature. After the addition was completed, the reaction system was placed and stirred at 25°C under nitrogen atmosphere for 2 hours. After the reaction was complete, the product was separated and purified through a silica gel column to obtain compound 87C.

MS (ESI) m/z 201.0 $(M-H)^-$.

In step 8 of Example 1, 2,3-dihydro-[1 ,4]dioxino[2,3-b]pyridine-7-sulfonyl chloride was replaced by 5-methoxypyridine-2-sulfonyl chloride, and in step 10, (S)-3-((tert-butyldiphenylsilyl)oxy)-2-phenylpropionic acid was replaced by 2-(2,4-difluorophenyl)-3-hydroxypropionic acid, and the same preparation method as that of Example 1 was adopted for separation to obtain compound 87 of Example 87. MS (ESI) m/z 466.1$(M+H)^+$.

$^1$H-NMR (400 MHz, DMSO- $d_6$) δ 8.49-8.48 (d, *J* = 2.7 Hz, 1H), 7.96-7.34 (d, *J* = 8.8 Hz, 1H), 7.67-7.64 (m, 1H), 7.43-7.41 (m, 1H), 7.22-7.17 (m, 1H), 7.07-7.04 (m, 1H), 4.74-4.4.71 (d, *J* = 12.9 Hz, 1H), 4.48 (s, 4H), 4.36-4.21 (m, 3H), 3.94 (s, 3H), 3.89-3.83 (m, 2H), 3.55-3.48 (m, 1H).

Example 88: preparation of (*R*)-2-(2,4-difluorophenyl)-3-hydroxy-1-(1'-((5-methoxypyridin-2-yl)sulfonyl)-1',4'-di hydro-2*H*,2'*H*-[3,3'-diazetidinylidene]-1(4*H*)-yl)propan-1-one (compound 88):

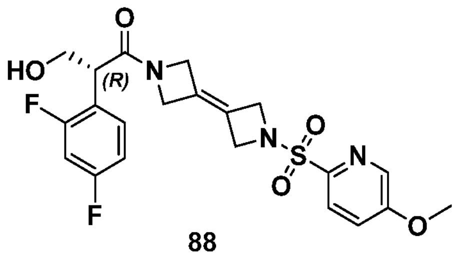

88

In step 8 of Example 1, 2,3-dihydro-[1 ,4]dioxino[2,3-*b*]pyridine-7-sulfonyl chloride was replaced by 5-methoxypyridine-2-sulfonyl chloride, and in step 10, (*S*)-3-((tert-butyldiphenylsilyl)oxy)-2-phenylpropionic acid was replaced by 2-(2,4-difluorophenyl)-3-hydroxypropionic acid, the same preparation method as that of Example 1 was adopted for separation, and then the product was subjected to chiral separation to obtain compound 88 of Example 88.

MS (ESI) m/z 466.1 $(M+H)^+$.

$^1$H-NMR (400 MHz, DMSO- $d_6$) δ 8.49-8.48 (d, *J* = 2.7 Hz, 1H), 7.96-7.34 (d, *J* = 8.8 Hz, 1H), 7.67-7.64 (m, 1H), 7.43-7.41 (m, 1H), 7.22-7.17 (m, 1H), 7.07-7.04 (m, 1H), 4.74-4.4.71 (d, *J* = 12.9 Hz, 1H), 4.48 (s, 4H), 4.36-4.21 (m, 3H), 3.94 (s, 3H), 3.89-3.83 (m, 2H), 3.55-3.48 (m, 1H).

Example 89: preparation of (3-phenyloxetan-3-yl)(1'-(pyridin-2-ylsulfonyl)-1',4'-dihydro-2*H*,2'*H*-[3,3'-diazetidinyli dene]-1(4*H*)-yl)methanone **(compound 89):**

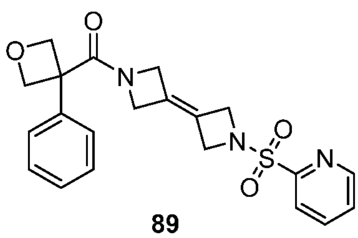

89

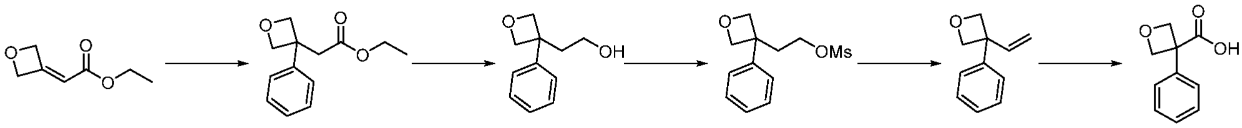

89A 89B 89C 89D 89E 89F

**Step 1:** preparation of ethyl 2-(3-phenyloxetan-3-yl)acetate (compound 89B)

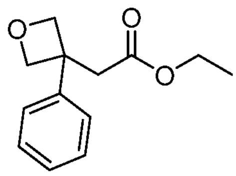

**89B**

Ethyl 2-(oxetan-3-ylidene)acetate (5 g, 35.17 mmol) was dissolved in 1,4-dioxane, and then sequentially added with an aqueous solution of potassium hydroxide (2.57 g, 1.5 N, 45.72 mmol), phenylboronic acid (6.43 g, 52.76 mmol) and chloro(1,5-cyclooctadiene)rhodium (I) dimer (1.73 g, 3.52 mmol). After the addition was completed, the reaction was stirred at room temperature for 16 hours. After the reaction was complete, it was separated and purified through a silica gel column to obtain

**compound 89B.**

MS (ESI) m/z 221.1 $(M+H)^+$.

**Step 2:** preparation of 2-(3-phenyloxetan-3-yl)ethan-1-ol **(compound 89C)**

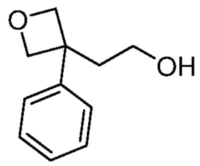

**89C**

Ethyl 2-(3-phenyloxetan-3-yl)acetate (3 g, 13.62 mmol) was dissolved in a tetrahydrofuran solution, and the reaction system was cooled to 0°C, and then slowly added with lithium aluminum hydride (0.52 g, 13.62 mmol). After the addition was completed, the reaction was naturally warmed to room temperature and stirred for 2 hours. After the reaction was complete, it was separated and purified through a silica gel column to obtain **compound 89C.**

MS (ESI) m/z 179.1 $(M+H)^+$.

**Step 3:** preparation of 2-(3-phenyloxetan-3-yl)ethyl methanesulfonate **(compound 89D)**

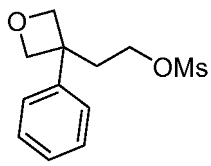

**89D**

2-(3-phenyloxetan-3-yl)ethan-1-ol (2.43 g, 13.63 mmol) was dissolved in dichloromethane. The reaction system was cooled to 0°C, then added with triethylamine (2.48 g, 24.54 mmol), and then slowly added dropwise with methylsulfonyl chloride (2.81 g, 24.54 mmol). After the addition was completed, the reaction was naturally warmed to room temperature and stirred for 2 hours. After the reaction was complete, it was separated and purified through a silica gel column to obtain **compound 89D.**

**Step 4:** preparation of 3-phenyl-3-vinyloxetane **(compound 89E)**

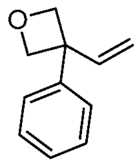

**89E**

2-(3-phenyloxetan-3-yl)ethyl methanesulfonate (3.4 g, 13.26 mmol) was dissolved in dimethyl sulfoxide, into which potassium *t*-butoxide (1.64 g, 14.59 mmol) was added in portions. After the addition was completed, the reaction system was stirred at room temperature for 16 hours. After the reaction was complete, the reaction system was added with a saturated aqueous sodium bicarbonate solution and extracted with ethyl acetate (200 mL*3). The organic phases were combined, and separated and purified through a silica gel column to obtain **compound 89E.**

MS (ESI) m/z 161.1 $(M+H)^+$.

Step 5: preparation of 3-phenyloxetane-3-carboxylic acid (compound 89F)

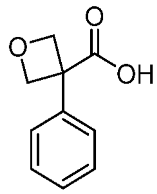

**89F**

3-phenyl-3-vinyloxetane (1.4 g, 8.74 mmol) was dissolved in a mixed solution of ethyl acetate, acetonitrile and water. The reaction system was cooled to 0°C and added with ruthenium trichloride (91 mg, 0.43 mmol). After the addition was completed, the reaction was stirred at 0°C for 10 minutes, and then slowly added with sodium periodate (7.17 g, 33.21 mmol) in portions. After the addition was completed, the reaction was kept at 0°C and stirred for 30 minutes. After the reaction was complete, the reaction system was filtered through diatomite, the aqueous phase was extracted with ethyl acetate (200 mL*3), and the organic phases were combined, concentrated, then added with a saturated aqueous sodium bicarbonate, and extracted once with ethyl acetate. The aqueous phase was slowly added dropwise with 1N hydrochloric acid aqueous solution to adjust pH to 1, and spinned to dry to obtain **compound 89F.**

In step 8 of Example 1, 2,3-dihydro-[1 ,4]dioxino[2,3-*b*]pyridine-7-sulfonyl chloride was replaced by pyridine-2-sulfonyl chloride, and in step 10, (S)-3-((tert-butyldiphenylsilyl)oxy)-2-phenylpropionic acid was replaced by 3-phenyloxetane-3-carboxylic acid, the same preparation method as that of Example 1 was adopted for separation, and then the product was subjected to chiral separation to obtain compound 89 of Example 89.

MS (ESI) m/z 412.1 $(M+H)^+$.

$^1$H NMR (400 MHz, DMSO-$d_6$) δ 8.79 (m, 1H), 8.14 (m, 1H), 7.96 (dd, *J* = 7.9, 1.2 Hz, 1H), 7.75 (m, 1H), 7.43 (dd, *J* = 8.2, 6.8 Hz, 2H), 7.35 (m, 3H), 5.09 (d, *J* = 6.2 Hz, 2H), 4.69 (d, *J* = 6.2 Hz, 2H), 4.48 (m, 4H), 4.41 - 4.33 (m, 2H), 4.12 - 4.03 (m, 2H).

Example 90: preparation of 2-cyclohexyl-3-hydroxy-1-(1'-(pyridin-2-ylsulfonyl)-1',4'-dihydro-2*H*,2'*H*-[3,3'-diazeti dinylidene]-1(4*H*)-yl)propan-1-one (compound 90):

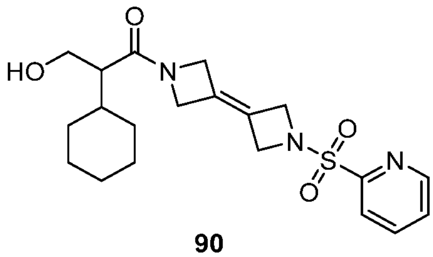

**90**

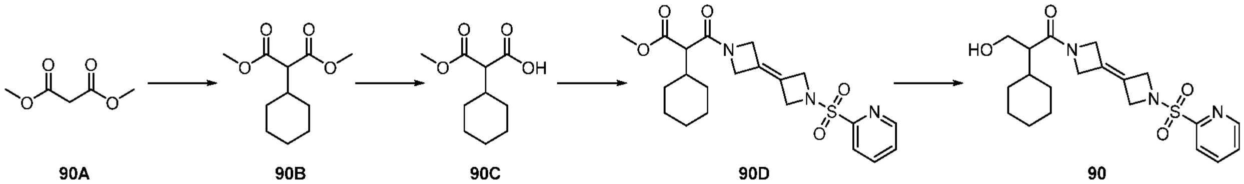

90A 90B 90C 90D 90

**Step** 1: preparation of dimethyl 2-cyclohexylmalonate (compound 90B)

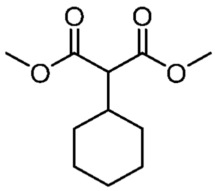

**90B**

Sodium (1.6 g, 71.41 mmol) was slowly added into anhydrous methanol (100.0 mL) in portions in ice bath. After the sodium wire was completely dissolved, dimethyl malonate (6.3 g, 47.61 mmol) and iodocyclohexane (10.0 g, 47.61 mmol) were added thereto. The reaction solution was reacted at 70°C for 18 hours. After the reaction was complete, it was separated and purified through a silica gel column to obtain compound 90B.

MS (ESI) m/z 215.3 $(M+H)^+$.

Step 2: preparation of 2-cyclohexyl-3-methoxy-3-oxopropionic acid (compound 90C)

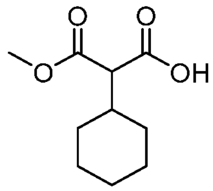

**90C**

Dimethyl 2-cyclohexylmalonate (100.0 mg, 0.47 mmol) was dissolved in methanol (3.0 mL) and water (1.0 mL), and then added with an aqueous solution (1.0 mL) of potassium hydroxide (26.2 mg, 0.47 mmol). The reaction solution was stirred at room temperature for 3 hours. LCMS showed that the reaction was substantially completed. The reaction solution was adjusted to pH of 5 with 1N hydrochloric acid, and extracted with ethyl acetate (50 mL*3). The organic phases were combined, washed with a saturated brine, and dried over anhydrous sodium sulfate. It was concentrated to obtain a yellow oily compound, which was directly used in the next step of reaction.

Step 3: preparation of methyl 2-cyclohexyl-3-oxo-3-(1(pyridin-2-ylsulfonyl)-1',4dihydro-2*H*,2'*H*-[3,3'-diazetidin ylidene]-1(4*H*)-yl)propionate (compound 90D)

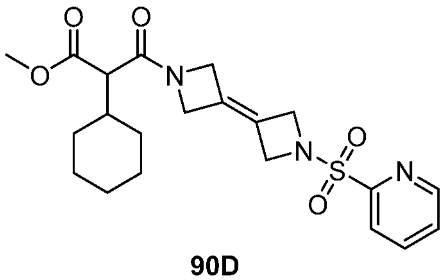

**90D**

2-cyclohexyl-3-methoxy-3-oxopropionic acid (93.4 mg, 0.46 mmol), 1-(pyridin-2-ylsulfonyl)-1',4'-dihydro-2*H*,2'*H*-3,3'-diazetidinylidene (94.78 mg, 0.26 mmol), HATU (197.3 mg, 0.52 mmol) and *N*,*N*-diisopropylethylamine (134.1 mg, 1.04 mmol) were dissolved in *N*,*N*-dimethylformamide (5.0 mL). The reaction solution was stirred at room temperature for 3 hours. After the reaction was complete, it was separated and purified through a silica gel column to obtain compound 90D.
MS (ESI) m/z 434.2 $(M+H)^+$.

Step 4: preparation of 2-cyclohexyl-3-hydroxy-1-(1'-(pyridin-2-ylsulfonyl)-1',4'-dihydro-2*H*,2'*H*-[3,3'-diazeti dinylidene]-1(4*H*)-yl)propan-1-one (compound 90):

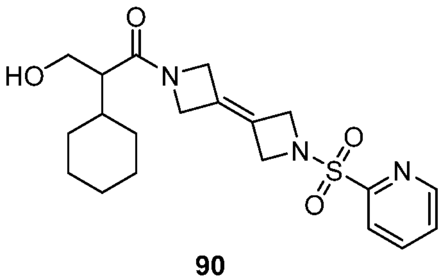

**90**

Methyl

2-cyclohexyl-3-oxo-3-(1'-(pyridin-2-ylsulfonyl)-1',4'-dihydro-2*H*,2'*H*-[3,3'-diazetidin ylidene]-1(4*H*)-yl)propionate (60.0 mg, 0.14 mmol) was dissolved in methanol (6.0 mL) and tetrahydrofuran (6.0 mL), and then added with sodium borohydride (52.4 mg, 1.38 mmol). The reaction solution was stirred at 80°C for 72 hours. After the reaction was complete, the product was separated and purified through reversed-phase high-pressure liquid chromatography to obtain compound 90.

MS (ESI) m/z 406.2 $(M+H)^+$.

$^1$H NMR (400 MHz, DMSO-$d_6$) δ 8.83-8.82 (d, *J* = 4.4 Hz, 1H), 8.19-8.17 (m, 1H), 8.00-7.98 (d, J = 8.0 Hz, 1H), 7.78-7.75 (m, 1H), 4.63-4.46 (m, 8H), 4.30-4.25 (m, 2H), 3.52-3.36 (m, 2H), 2.16-2.14 (m, 1H), 1.65-1.58 (m, 4H), 1.52-1.32 (m, 2H), 1.10-1.03 (m, 2H), 1.00-0.81 (m, 2H).

Example 91: preparation of (*S*)-3-hydroxy-1-(1'-((5-(methoxy-$d_3$)pyridin-2-yl)sulfonyl)-1',4'-dihydro-2H,2'*H*-[3,3 '-diazetidinylidene]-1(4*H*)-yl)-2-phenylpropan-1-one (compound 91):

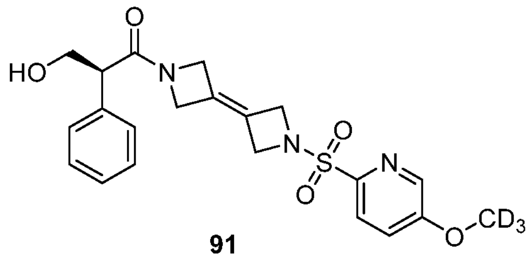

**91**

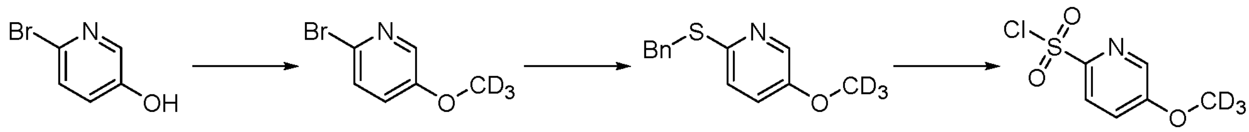

**91A** **91B** **91C** **91D**

**Step 1:** preparation of 2-bromo-5-(methoxy-$d_3$)pyridine (compound 91B)

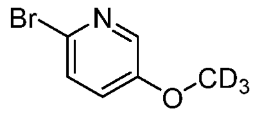

**91B**

2-bromo-5-hydroxypyridine (3 g, 17.24 mmol) was dissolved in 1,4-dioxane solution, and then sequentially added with deuterated methanol (3.11 g, 86.20 mmol), triphenylphosphine (4.97 g, 18.96 mmol) and DIAD (3.83 g, 18.96 mmol). After the addition was completed, the reaction was stirred at room temperature for 16 hours. After the reaction was complete, it was separated and purified through a silica gel column to obtain compound 91B.
MS (ESI) m/z 191.0 $(M+H)^+$.

Step 2: preparation of 2-(benzylthio)-5-(methoxy-$d_3$)pyridine (compound 91C)

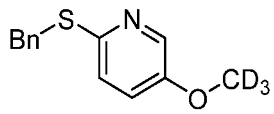

**91C**

2-bromo-5-(methoxy-$d_3$)pyridine (2.53 g, 13.24 mmol) was dissolved in 1,4-dioxane solution, and then sequentially added with benzyl mercaptan (1.64 g, 13.24 mmol), DIPEA (3.42 g, 26.49 mmol), $Pd_2(dba)_3CHCl_3$ (1.35 g, 1.32 mmol) and xantphos (1.53 g, 2.65 mmol). After the addition was completed, the reaction was replaced under nitrogen environment for 3 times, then heated to 80°C, and stirred for 16 hours. After the reaction was complete, it was separated and purified through a silica gel column to obtain **compound 91C.**
MS (ESI) m/z 235.1 $(M+H)^+$.

**Step 3:** preparation of 5-(methoxy-$d_3$)pyridine-2-sulfonyl chloride (compound 91D)

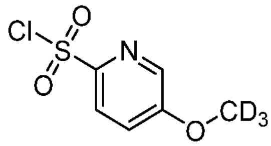

**91D**

2-(benzylthio)-5-(methoxy-$d_3$)pyridine (1.1 g, 4.69 mmol) was dissolved in acetonitrile, into which acetic acid (1.41 g, 23.47 mmol), water (846 mg, 46.94 mmol) and 1,3-dichloro-5,5-dimethylhydantoin (1.85 g, 9.39 mmol) were sequentially added. After the addition was completed, the reaction was stirred at room temperature for 16 hours. After the reaction was complete, it was separated and purified through a silica gel column to obtain **compound 91D.**

MS (ESI) m/z 211.0 $(M+H)^+$.

In step 8 of Example 1, 2,3-dihydro-[1 ,4]dioxino[2,3-b]pyridine-7-sulfonyl chloride was replaced by 5-(methoxy-$d_3$)pyridine-2-sulfonyl chloride, and the same preparation method as that of Example 1 was adopted for separation to obtain compound 91 of Example 91.

MS (ESI) m/z 433.2$(M+H)^+$.

$^1H$ NMR (400 MHz, DMSO-$d_6$) δ 8.48 (d, *J* = 2.9 Hz, 1H), 7.94 (d, *J* = 8.8 Hz, 1H), 7.64 (dd, *J* = 8.8, 3.0 Hz, 1H), 7.27 (m, 5H), 4.80 (t, *J* = 5.2 Hz, 1H), 4.72 (d, *J* = 13.4 Hz, 1H), 4.48 (s, 4H), 4.41 - 4.27 (m, 2H), 4.21 (d, *J* = 14.7 Hz, 1H), 3.89 (m, 1H), 3.62 (dd, *J* = 8.9, 5.4 Hz, 1H), 3.46 (m, 1H). **Biological test data**

Unless otherwise specified, all of the experimental materials, reagents, operations and methods used in the following activity examples are commercially available or can be easily known or prepared based on the prior art.

Test Example 1: pyruvate kinase (PKR) activity experiment

Reagents and consumables:

| Reagent Name | Supplier | Cat. No. |
|---|---|---|
| PKLR Var2 active human | Sigma-Aldrich | 9001-59-6 |
| DMSO | Xiya reagent | 20190701 |
| Pyruvate Kinase Assay Kit | Abcam | ab83432 |

Instrument:

| Instrument name | Factory | Model |
|---|---|---|
| Multifunctional microplate reader | BMG | PHERAstar® FSX |

**1. Test steps:**

1). A recombinant human PKR enzyme solution was formulated at a concentration of 60 ng/well (a 20 μL reaction system), and 5 μL of the solution was added into each well of a black transparent bottom 384-well plate.

2). Compound solutions were formulated at different concentrations, with the final concentrations of 1000/300/100 nM (containing 1% DMSO). Each 5 μL of the compound solution was added into the plate in item 3.1 and mixed well. The plate was incubated at room temperature for 10 min.

3). A reaction system was formulated according to the instructions of a kit (as shown in the table below), and 10 μL of the reaction system was added into each well of the 384-well plate and mixed uniformly with the solution in item 3.2.

| Item | μL |
|---|---|
| OxiRed™ Probe | 0.08 |

(continued)

| Item | μL |
|---|---|
| HRP Enzyme Mix (Lyophilized) | 0.2 |
| PK Substrate Mix (Lyophilized) | 0.4 |
| PK Assay Buffer | 9.32 |

4). The 384-well plate was placed on a microplate reader to detect the fluorescence value at 540/590 nm, where the value was read every 2 minutes and the detection was conducted continuously for 1 hour.
5). The Slope value within the detection time was calculated, and the 0.1% DMSO well was used as a control well to calculate a compound activation rate.

$$\text{Activation rate \%} = (\text{Slope of compound well/Slope of control well}) \times 100\%$$

6). Fitting dose-effect curve

With the log value of the compound concentration as the X axis and the percentage activation rate as the Y axis, a dose-effect curve was fitted with the log(agonist) vs. response-variable slope of the analysis software GraphPad Prism 5, and the $AC_{50}$ value of the activity of each compound against PKR was obtained.

$$\text{Calculation formula: } Y = \min + (\max-\min) / (1 + 10\text{^}((\text{Log}AC_{50}-X) \times \text{Hillslope})).$$

**2. Test results:**

The activation effects of the compounds of the present invention on PKR enzyme were determined through the aforementioned tests, and the determined $AC_{50}$ values were shown as follows (A represented 0-100 nM, B represented 101-300 nM, and C represented 300-1,000 nM).

| Compound | $AC_{50}$ | Compound | $AC_{50}$ | Compound | $AC_{50}$ |
|---|---|---|---|---|---|
| 1 | C | 32 | A | 63 | B |
| 2 | A | 33 | C | 64 | C |
| 3 | A | 34 | C | 65 | B |
| 4 | B | 35 | A | 66 | B |
| 5 | A | 36 | C | 67 | A |
| 6 | A | 37 | A | 68 | C |
| 7 | B | 38 | C | 69 | A |
| 8 | C | 39 | A | 70 | C |
| 9 | B | 40 | B | 71 | A |
| 10 | B | 41 | C | 72 | A |
| 11 | B | 42 | C | 73 | B |
| 12 | A | 43 | C | 74 | A |
| 13 | A | 44 | A | 75 | B |
| 14 | B | 45 | A | 76 | C |
| 15 | C | 46 | A | 77 | A |
| 16 | B | 47 | C | 78 | C |
| 17 | C | 48 | B | 79 | A |

(continued)

| Compound | $AC_{50}$ | Compound | $AC_{50}$ | Compound | $AC_{50}$ |
|---|---|---|---|---|---|
| 18 | A | 49 | A | 80 | A |
| 19 | B | 50 | A | 81 | B |
| 20 | B | 51 | C | 82 | A |
| 21 | C | 52 | C | 83 | A |
| 22 | C | 53 | C | 84 | C |
| 23 | C | 54 | A | 85 | C |
| 24 | C | 55 | C | 86 | C |
| 25 | A | 56 | C | 87 | A |
| 26 | A | 57 | B | 88 | C |
| 27 | C | 58 | B | 89 | B |
| 28 | C | 59 | B | 90 | B |
| 29 | A | 60 | C | 91 | A |
| 30 | A | 61 | C | | |
| 31 | C | 62 | C | | |

Test Example 2: USP9X enzyme activity experiment

Reagents and consumables:

| **Reagent Name** | **Supplier** | **Cat. No.** |
|---|---|---|
| USP9X, His-tag, Flag-tag | BPS | 100188 |
| Recombinant Human Ubiquitin Rhodamine 110 Protein, CF (Ub-Rho) | R&D | U-555-050 |
| 384-well plate | Perkin Elmer | 6007279 |

Instrument:

| **Instrument name** | **Factory** | **Model** |
|---|---|---|
| Multifunctional microplate reader | Molecular Devices | SpectraMax Paradigm |

**1. Test steps**

1). A 1× analysis buffer was formulated, consisting of a modified Tris buffer (pH 7.5).
2). Compound solutions of different concentrations were formulated with final concentrations of 3,000/1,000/300/100/30/10/3/1 nM (containing 1% DMSO), and added into a 384-well plate.
3). A USP9X enzyme solution was formulated with the 1× analysis buffer.
4). 10 μL of the USP9X enzyme solution was added into the 384-well plate, and incubated with the compounds at room temperature for 1 h.
5). Rhodamine 110 Protein was added into the 1× analysis buffer to formulate a substrate working solution.
6). 10 μL of the substrate solution was added into each reaction well, centrifuged for 30 s, and mixed uniformly for 30 s.

7). The 384-well plate was placed on a microplate reader for detection, with an excitation wavelength of 480 nm and an emission wavelength of 540 nm. Test was conducted for 30 minutes and the data was collected.
8. Fitting the dose-effect curve.

$$\text{Percentage inhibition rate Inh \%} = (\text{Max}-\text{Signal}) / (\text{Max}-\text{Min})*100.$$

With the log value of the compound concentration as the X axis and the percentage inhibition rate as the Y axis, a dose-effect curve was fitted with the log(inhibitor) vs. response-variable slope of the analysis software GraphPad Prism 5, and the $IC_{50}$ value of the activity of each compound against USP9X was obtained.

$$\text{Calculation formula: } Y = \min + (\max-\min) / (1 + 10\hat{}((\text{Log}IC_{50} - X) \times \text{Hillslope})).$$

**2. Test results**

The inhibition effects of the compounds of the present invention on USP9X enzymes were determined through the aforementioned tests, and the determined $IC_{50}$ values were shown as follows (A represented 0-100 nM, B represented 101-300 nM, and C represented 300-1,000 nM).

| Compound | $IC_{50}$ | Compound | $IC_{50}$ |
|---|---|---|---|
| 1 | A | 4 | B |
| 2 | A | 5 | A |
| 3 | C | 6 | B |

The present invention is not limited to the aforementioned optional embodiments, and anyone can derive various other forms of products under the motivation of the present invention. The aforementioned specific embodiments should not be understood as limiting the scope of the present invention. The protection scope of the present invention should be subjected to the definitions of the claims, and the specification can be used for interpreting the claims.

## Claims

**1.** A compound of formula I, or an isomer, pharmaceutically acceptable salt or solvate thereof:

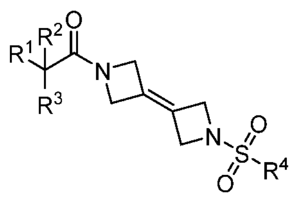

( I )

wherein,

$R^1$, $R^2$ and $R^3$ are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ or $-C(O)OR^g$, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ and $-C(O)OR^g$;
or, $R^1$ and $R^2$, $R^1$ and $R^3$, or $R^2$ and $R^3$ together with atoms to which they are attached optionally bind to form

$-(C_3-C_8)$cycloalkyl, 3-14 membered heterocyclyl containing 1-4 heteroatoms independently selected from O, N and S, $-(C_5-C_8)$spirocyclyl, 5-8 membered spiroheterocyclyl containing 1-4 heteroatoms independently selected from O, N and S, 7-14 membered fused ring group containing 0-4 heteroatoms independently selected from O, N and S, or 5-14 membered heteroaryl containing 1-4 heteroatoms independently selected from O, N and S;

$R^4$ is $-NH_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$, $-C(O)OR^g$ or $-NR^c(CR^hR^i)_t-R^a$, wherein each of the alkyl, alkenyl, alkynyl, cycloalkyl, cycloalkenyl, heterocyclyl, aryl, heteroaryl, or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ and $-C(O)OR^g$;

t is 0, 1, 2 or 3;

$R^a$, $R^b$, $R^c$, $R^d$, $R^e$, $R^f$, $R^g$, $R^h$ and $R^i$ at each occurrence are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H or -C(O)OH, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, $-(C_1-C_6)$alkyl, $-(C_3-C_8)$cycloalkyl, and 3-14 membered heterocyclyl containing 1-4 heteroatoms independently selected from O, N and S;

or, any two groups on adjacent atoms selected from $R^a$, $R^b$, $R^c$, $R^d$, $R^e$, $R^f$, $R^g$, $R^h$ and $R^i$ together with atoms to which they are attached optionally bind to form $C_6-C_{14}$ aryl, 5-14 membered heteroaryl containing 1-4 heteroatoms independently selected from O, N and S, $(C_3-C_8)$cycloalkyl, or 3-14 membered heterocyclyl containing 1-4 heteroatoms independently selected from O, N and S, which is optionally substituted by one or more $R^a$.

**2. A compound of formula I, or an isomer, pharmaceutically acceptable salt or solvate thereof:**

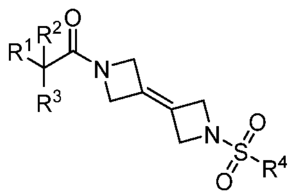

( I )

wherein,

$R^1$, $R^2$ and $R^3$ are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ or $-C(O)OR^9$, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ and $-C(O)OR^g$;

or, $R^1$ and $R^2$, $R^1$ and $R^3$, or $R^2$ and $R^3$ together with atoms to which they are attached optionally bind to form $-(C_3-C_8)$cycloalkyl, 3-14 membered heterocyclyl containing 1-4 heteroatoms independently selected from O, N and S, $-(C_5-C_8)$spirocyclyl, 5-8 membered spiroheterocyclyl containing 1-4 heteroatoms independently selected from O, N and S, 7-14 membered fused ring group containing 0-4 heteroatoms independently selected from O, N and S, or 5-14 membered heteroaryl containing 1-4 heteroatoms independently selected from O, N and S;

$R^4$ is $-NH_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered

fused ring group containing 0-4 heteroatoms independently selected from O, N and S, $-OR^b$, $-SR^b$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$, $-C(O)OR^g$ or $-NR^c(CR^hR^i)_t-R^a$, wherein each of the alkyl, alkenyl, alkynyl, cycloalkyl, cycloalkenyl, heterocyclyl, aryl, heteroaryl, or fused ring group is optionally substituted by one or more substituents selected from: =O, halogen, -CN, $-R^a$, $-OR^b$, $-SR^b$, $-NO_2$, $-NR^cR^d$, $-S(O)_2R^e$, $-S(O)_2NR^cR^d$, $-S(O)R^e$, $-S(O)NR^cR^d$, $-NR^cS(O)_2R^e$, $-NR^cS(O)R^e$, $-C(O)R^f$ and $-C(O)OR^g$;

t is 0, 1, 2 or 3;

$R^a$, $R^b$, $R^c$, $R^d$, $R^e$, $R^f$, $R^g$, $R^h$ and $R^i$ at each occurrence are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$, -NHS(O)OH, -C(O)H or -C(O)OH, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, $-(C_1-C_6)$alkyl, $-(C_3-C_8)$cycloalkyl, and 3-14 membered heterocyclyl containing 1-4 heteroatoms independently selected from O, N and S;

or, any two groups on adjacent atoms selected from $R^a$, $R^b$, $R^c$, $R^d$, $R^e$, $R^f$, $R^g$, $R^h$ and $R^i$ together with atoms to which they are attached optionally bind to form $C_6-C_{14}$ aryl, 5-14 membered heteroaryl containing 1-4 heteroatoms independently selected from O, N and S, $(C_3-C_8)$cycloalkyl, or 3-14 membered heterocyclyl containing 1-4 heteroatoms independently selected from O, N and S, which is optionally substituted by one or more $R^a$.

3. **The compound, or an isomer, pharmaceutically acceptable salt, or solvate thereof according to claim 1,** wherein the compound has formula I-1 or I-2:

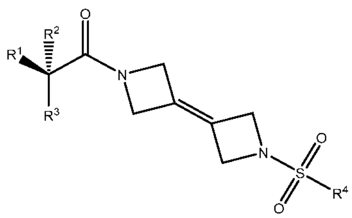 ( I -1),

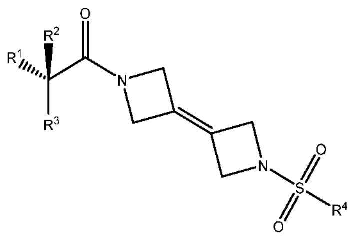 ( I -2).

4. **The compound, or an isomer, pharmaceutically acceptable salt, or solvate thereof according to claim 1 or 3,** wherein

$R^1$ and $R^2$ are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, $-OR^b$ or $-NR^cR^d$, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, $-R^a$, $-OR^b$, $-NO_2$, and $-NR^cR^d$;

or, $R^1$ and $R^2$ together with atoms to which they are attached optionally bind to form $-(C_3-C_8)$cycloalkyl or 3-14 membered heterocyclyl containing 1-4 heteroatoms independently selected from O, N and S;

$R^a$, $R^b$, $R^c$, and $R^d$ at each occurrence are independently -H, halogen, -OH, $-NH_2$, -CN, $-NO_2$, $-(C_1-C_6)$alkyl, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, $-NO_2$, $-NH_2$, $-(C_1-C_6)$alkyl, $-(C_3-C_8)$cycloalkyl, and 3-14 membered heterocyclyl containing 1-4 heteroatoms independently selected from O, N and S.

5. **The compound, or an isomer, pharmaceutically acceptable salt, or solvate thereof according to claim 4,** wherein

$R^1$ and $R^2$ are independently -H, halogen, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, 5-8 membered heteroaryl containing 1-4 heteroatoms independently selected from O, N and S, 7-14 membered fused ring group containing 0-4 heteroatoms independently selected from O, N and S, $-OR^b$ or $-NR^cR^d$, wherein each of the alkyl, cycloalkyl, heterocyclyl, aryl, heteroaryl or fused ring group is optionally substituted by one or more substituents selected from: =O, halogen, -CN, $-R^a$, $-OR^b$, $-NO_2$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;
$R^a$, $R^b$, $R^c$, and $R^d$ at each occurrence are independently -H, halogen, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, 5-8 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -OH, $-NO_2$, $-NH_2$, $-(C_1-C_6)$alkyl, $-(C_3-C_6)$cycloalkyl, and 3-6 membered heterocyclyl containing 1-4 heteroatoms independently selected from O, N and S.

6. **The compound, or an isomer, pharmaceutically acceptable salt, or solvate thereof according to claim 5,** wherein

$R^1$ and $R^2$ are independently -H, -F, -Cl, -Br, -OH, $-NH_2$, -CN, $-NO_2$, $-(C_1-C_6)$ linear alkyl, $-(C_1-C_6)$ branched alkyl, $-(C_3-C_6)$cycloalkyl, 3-6 membered heterocyclyl containing 1-4 heteroatoms independently selected from O, N and S, $C_6-C_8$ aryl, 7-14 membered bicyclic or tricyclic fused ring group containing 0-4 heteroatoms independently selected from O, N and S, $-OR^b$ or $-NR^cR^d$, wherein each of the alkyl, cycloalkyl, heterocyclyl, aryl, heteroaryl or fused ring group is optionally substituted by one or more substituents selected from: -F, -Cl, -Br, -CN, $-R^a$, $-OR^b$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;
$R^a$, $R^b$, $R^c$, and $R^d$ at each occurrence are independently H, -F, -Cl, -Br, -OH, $-NH_2$, -CN, $-NO_2$, $-(C_1-C_6)$alkyl, $-(C_3-C_6)$cycloalkyl, 3-6 membered heterocyclyl containing 1-4 heteroatoms independently selected from O, N and S, $C_6-C_8$ aryl, or 5-8 membered heteroaryl containing 1-4 heteroatoms independently selected from O, N and S, wherein each of the alkyl, cycloalkyl, heterocyclyl, aryl, heteroaryl or fused ring group is optionally substituted by one or more substituents selected from: -F, -Cl, -Br, -CN, -OH, $-NO_2$, $-NH_2$, and $-(C_1-C_6)$alkyl.

7. **The compound, or an isomer, pharmaceutically acceptable salt, or solvate thereof according to claim 6,** wherein

$R^1$ and $R^2$ are independently -H, -F, -Cl, -Br, -OH, $-NH_2$, -CN, $-NO_2$, $-(C_1-C_6)$ linear alkyl, $-(C_1-C_6)$ branched alkyl, $-(C_3-C_6)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, $-R^a$, $-OR^b$, and $-NR^cR^d$;
or, $R^1$ and $R^2$ together with atoms to which they are attached optionally bind to form $-(C_3-C_8)$cycloalkyl or 3-6 membered heterocyclyl containing 1-4 heteroatoms independently selected from O, N and S;
$R^a$, $R^b$, $R^c$, and $R^d$ at each occurrence are independently H, -F, -Cl, -Br, -OH, $-NH_2$, -CN, $-NO_2$, $-(C_1-C_6)$alkyl, $-(C_3-C_6)$cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, -OH, $-NO_2$, $-NH_2$, and $-(C_1-C_6)$alkyl.

8. **The compound, or an isomer, pharmaceutically acceptable salt, or solvate thereof according to claim 7,** wherein

$R^2$ is -H, -F, -Cl or -Br;
$R^1$ is -H, -F, -Cl, -Br, -OH, $-NH_2$, $-(CH_2)_qCH_3$, $-(CH_2)_qOH$, $-CH(OH)(CH_2)_qCH_3$, $-C(OH)((CH_2)_qCH_3)_2$,

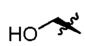 ,

$-(CH_2)_qNH_2$, $-(CH_2)_qNH(CH_2)_qCH_3$, $-(CH_2)_qN((CH_2)_qCH_3)_2$, 3-6 membered heterocycloalkyl containing 1 N atom, or $-(C_1-C_6)$alkyl substituted by 3-6 membered heterocycloalkyl containing 1 N atom;

q at each occurrence is independently 0, 1, 2, 3 or 4;
or, $R^1$ and $R^2$ together with atoms to which they are attached optionally bind to form a cyclopropane ring, a cyclobutane ring, a cyclopentane ring, a cyclohexane ring, tetrahydrofuran, tetrahydropyran, morpholine, dioxane, 2,3-dihydrobenzofuran ring, or tetrahydro-2H-pyran.

**9. The compound, or an isomer, pharmaceutically acceptable salt, or solvate thereof according to claim 4,** wherein

$R^2$ is -H, -F, -Cl or -Br;
$R^1$ is -H, -F, -Cl, -Br, -OH, $-NH_2$,

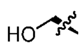, 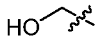, 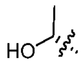, 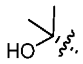, 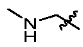, 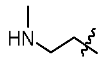, 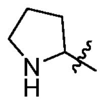

or

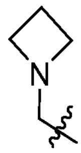;

or, $R^1$ and $R^2$ together with atoms to which they are attached optionally bind to form

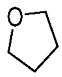

or

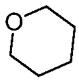.

**10. The compound, or an isomer, pharmaceutically acceptable salt, or solvate thereof according to claim 6,** wherein

$R^2$ is -H, -F, -Cl or -Br;
$R^1$ is -H, -F, -Cl, -Br, -OH, $-NH_2$, $-CH_3$, $-CH(OH)CH_3$, $-(CH_2)_qCH_3$, $-(CH_2)_qOH$, $-CH(OH)(CH_2)_qCH_3$, $-C(OH)((CH_2)_qCH_3)_2$, $-C(OH)((CH_2)_qCH_3)(CH_3)$, $-C(OH)(CH_3)_2$,

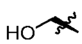, 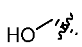,

$-CH_2F$, $-CHF_2$, $-(CH_2)_qCH_2F$, $-(CH_2)_qCHF_2$, $-CH_2Cl$, $-CHCl_2$, $-(CH_2)_qCH_2Cl$, $-(CH_2)_qCHCl_2$, $-(CH_2)_qNH_2$, $-NHCH_3$, $-NH(CH_2)_qCH_3$, $-(CH_2)_qNHCH_3$, $-(CH_2)_qNH(CH_2)_qCH_3$, $-N(CH_3)_2$, $-N((CH_2)_qCH_3)_2$, $-(CH_2)_qN(CH_3)((CH_2)_qCH_3)$, $-(CH_2)_q N(CH_3)_2$, $-(CH_2)_qN((CH_2)_qCH_3)_2$, 3-6 membered heterocycloalkyl containing 1 N atom, or $-(C_1-C_6)$alkyl substituted by 3-6 membered heterocycloalkyl containing 1 N atom;
q at each occurrence is independently 1, 2, 3 or 4;
or, $R^1$ and $R^2$ together with atoms to which they are attached optionally bind to form a cyclopropane ring, a cyclobutane ring, a cyclopentane ring, a cyclohexane ring, oxetane, tetrahydrofuran, tetrahydropyran, morpholine, dioxane, 2,3-dihydrobenzofuran ring, or tetrahydro-2H-pyran.

**11. The compound, or an isomer, pharmaceutically acceptable salt, or solvate thereof according to claim 4,** wherein

$R^2$ is -H, -F, -Cl or -Br;

$R^1$ is -H, -F, -Cl, -Br, -OH, $-NH_2$,

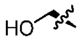, 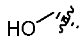, 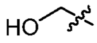, 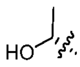, 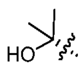, 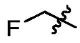, 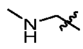,

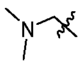, 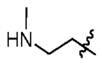, 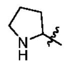 or 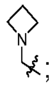;

or, $R^1$ and $R^2$ together with atoms to which they are attached optionally bind to form

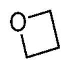,

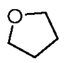 or 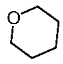.

12. **The compound, or an isomer, pharmaceutically acceptable salt, or solvate thereof according to any one of claims 1 and 3-9,** wherein

$R^3$ is -H, halogen, $-(C_1\text{-}C_6)$alkyl, $-(C_3\text{-}C_8)$cycloalkyl, 3-14 membered heterocyclyl containing 1-4 heteroatoms independently selected from O, N and S, $C_6\text{-}C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl , heteroaryl or fused ring group is optionally substituted by one or more substituents selected from: =O, halogen, -CN, $-R^a$, $-OR^b$, $-NO_2$, and $-NR^cR^d$;

$R^a$, $R^b$, $R^c$, and $R^d$ at each occurrence are independently -H, halogen, -OH, $-NH_2$, -CN, $-NO_2$, $-(C_1\text{-}C_6)$alkyl, $-(C_2\text{-}C_6)$alkenyl, $-(C_2\text{-}C_6)$alkynyl, $-(C_3\text{-}C_8)$cycloalkyl, $-(C_4\text{-}C_8)$cycloalkenyl, 3-14 membered heterocyclyl containing 1-4 heteroatoms independently selected from O, N and S, $C_6\text{-}C_{14}$ aryl, 5-14 membered heteroaryl containing 1-4 heteroatoms independently selected from O, N and S, or 7-14 membered fused ring group containing 0-4 heteroatoms independently selected from O, N and S, wherein each of the alkyl, alkenyl, alkynyl, cycloalkyl, cycloalkenyl, heterocyclyl, aryl, heteroaryl or fused ring group is optionally substituted by one or more substituents selected from: =O, halogen, -CN, -OH, $-NO_2$, $-NH_2$, $-(C_1\text{-}C_6)$alkyl, $-(C_3\text{-}C_8)$cycloalkyl, and 3-14 membered heterocyclyl containing 1-4 heteroatoms independently selected from O, N and S.

13. **The compound, or an isomer, pharmaceutically acceptable salt, or solvate thereof according to claim 12,** wherein

$R^3$ is -H, halogen, $-(C_1\text{-}C_6)$alkyl, $-(C_3\text{-}C_6)$cycloalkyl, 3-6 membered heterocyclyl containing 1-4 heteroatoms independently selected from O, N and S, $C_6\text{-}C_8$ aryl, 5-8 membered heteroaryl containing 1-4 heteroatoms independently selected from O, N and S, or 7-14 membered bicyclic or tricyclic fused ring group containing 0-4 heteroatoms independently selected from O, N and S, wherein each of the alkyl, cycloalkyl, heterocyclyl, aryl , heteroaryl or fused ring group is optionally substituted by one or more substituents selected from: =O, halogen, -CN, $-R^a$, $-OR^b$, $-NO_2$, and $-NR^cR^d$;

$R^a$, $R^b$, $R^c$, and $R^d$ at each occurrence are independently -H, halogen, -OH, $-NH_2$, -CN, $-NO_2$, $-(C_1\text{-}C_6)$alkyl, $-(C_3\text{-}C_8)$cycloalkyl, 3-6 membered heterocyclyl containing 1-4 heteroatoms independently selected from O, N and S, $C_6\text{-}C_8$ aryl, 5-8 membered heteroaryl containing 1-4 heteroatoms independently selected from O, N and S, or 7-14 membered fused ring group containing 0-4 heteroatoms independently selected from O, N and S, wherein each of the alkyl, cycloalkyl, heterocyclyl, aryl, heteroaryl or fused ring group is optionally substituted by one or more substituents selected from: =O, halogen, -CN, -OH, $-NO_2$, $-NH_2$, $-(C_1\text{-}C_6)$alkyl, $-(C_3\text{-}C_6)$cycloalkyl, and 3-6 membered heterocyclyl containing 1-4 heteroatoms independently selected from O, N and S.

14. **The compound, or an isomer, pharmaceutically acceptable salt, or solvate thereof according to claim 13,** wherein

$R^3$ is -H, -F, -Cl, -Br, -($C_1$-$C_6$)alkyl, -($C_3$-$C_6$)cycloalkyl, $C_6$-$C_8$ aryl, or 7-14 membered bicyclic fused ring group containing 0-4 heteroatoms independently selected from O, N and S, wherein each of the alkyl, cycloalkyl, aryl or fused ring group is optionally substituted by one or more substituents selected from: -F, -Cl, -Br, -CN, -$R^a$ and -$OR^b$; $R^a$ and $R^b$ at each occurrence are independently -H, halogen, -OH, -$NH_2$, -CN, -$NO_2$, -($C_1$-$C_6$)alkyl, -($C_3$-$C_8$)cycloalkyl, or 3-6 membered heterocyclyl containing 1-4 heteroatoms independently selected from O, N and S, wherein each of the alkyl, cycloalkyl or heterocyclyl is optionally substituted by one or more substituents selected from: -F, -Cl, -Br, -CN, -OH, -$NO_2$, -$NH_2$, and -($C_1$-$C_6$)alkyl.

**15. The compound, or an isomer, pharmaceutically acceptable salt, or solvate thereof according to claim 12,** wherein
$R^3$ is -H, -F, -Cl, -Br, -($C_1$-$C_6$)alkyl, -($C_3$-$C_6$)cycloalkyl, pyridyl, phenyl, benzothiazolyl, benzomorpholinyl, or benzopyrrolidinyl, wherein the pyridyl, phenyl, benzothiazolyl, benzomorpholinyl, or benzopyrrolidinyl is optionally substituted by one or more substituents selected from: -F, -Cl, -Br, -($C_1$-$C_6$)alkyl, -($C_3$-$C_6$)cycloalkyl, -O-($C_1$-$C_6$)alkyl, -O-($C_3$-$C_6$)cycloalkyl, -($C_1$-$C_6$)haloalkyl, -($C_3$-$C_6$)halocycloalkyl, -O-($C_1$-$C_6$)haloalkyl, -O-($C_3$-$C_6$)halocycloalkyl, piperazinyl, and piperazinyl substituted by any number of halogen or -($C_1$-$C_6$)alkyl.

**16. The compound, or an isomer, pharmaceutically acceptable salt, or solvate thereof according to claim 12,** wherein
$R^3$ is -H, -F, -Cl, -Br, -$CH_3$, -$CH_2CH_3$, pyridyl,

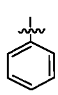, 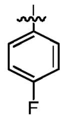, 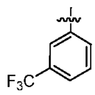, 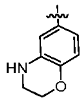, 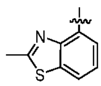,

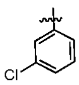, 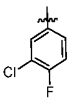, 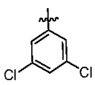, 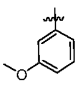, 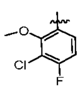, 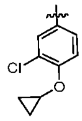, 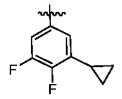, 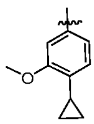, 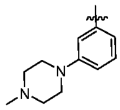, 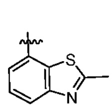,

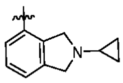, 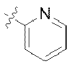, 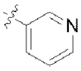, 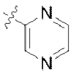, 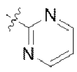, 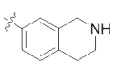, 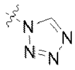, 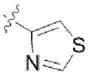, 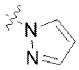, 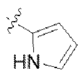, 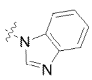,

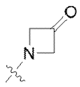, 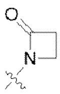, or 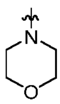.

**17.** The compound, or an isomer, pharmaceutically acceptable salt, or solvate thereof according to claim 12, wherein

$R^3$ is -H, -F, -Cl, -Br, -($C_1$-$C_6$)alkyl, -($C_3$-$C_6$)cycloalkyl, pyridyl, phenyl, naphthyl, benzothiazolyl, benzomorpholinyl, benzopyrrolidinyl or

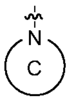,

wherein the pyridyl, phenyl, benzothiazolyl, benzomorpholinyl, benzopyrrolidinyl, or

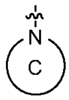

is optionally substituted by one or more substituents selected from: -F, -Cl, -Br, -CN, $-(C_1-C_6)$alkyl, $-(C_3-C_6)$cycloalkyl, $-O-(C_1-C_6)$alkyl, $-O-(C_3-C_6)$cycloalkyl, $-(C_1-C_6)$haloalkyl, $-(C_3-C_6)$halocycloalkyl, $-O-(C_1-C_6)$haloalkyl, $-O-(C_3-C_6)$halocycloalkyl, pyrazolyl, pyrazolyl substituted by any number of halogen or $-(C_1-C_6)$alkyl, piperazinyl, piperazinyl substituted by any number of halogen or $-(C_1-C_6)$alkyl;
the ring

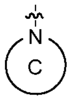

is 3-6 membered saturated or unsaturated N-containing heterocyclyl connected through an N atom, and in addition to the N atom, the heterocyclyl optionally contains 1-2 heteroatoms independently selected from O, N and S.

**18.** The compound, or an isomer, pharmaceutically acceptable salt, or solvate thereof according to claim 12, wherein $R^3$ is -H, -F, -Cl, -Br, $-CH_3$, $-CH_2CH_3$,

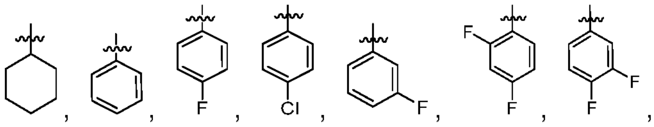

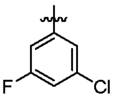, 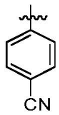, 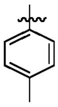, 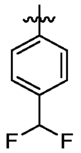, 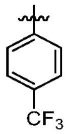, 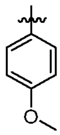, 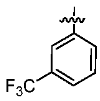, 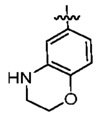, 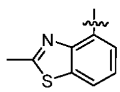, 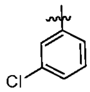, 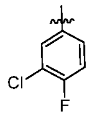,

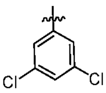, 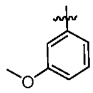, 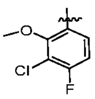, 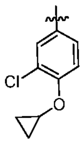, 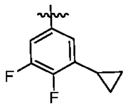, 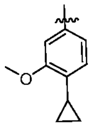, 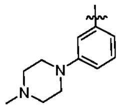, 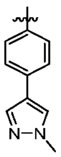, 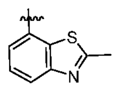, 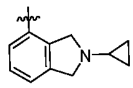,

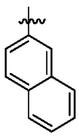, 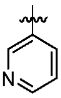, 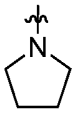, 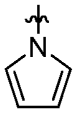, 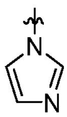, 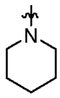, 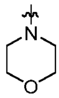, 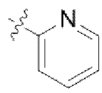, 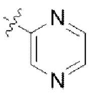, 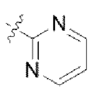, 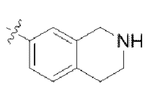,

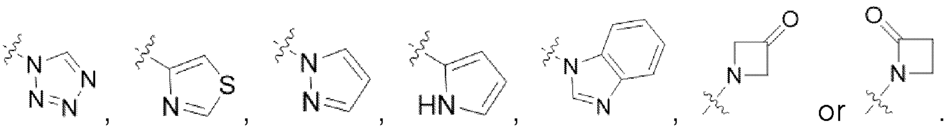

**19.** A compound of formula I, 1-1 or 1-2, or an isomer, pharmaceutically acceptable salt or solvate thereof:

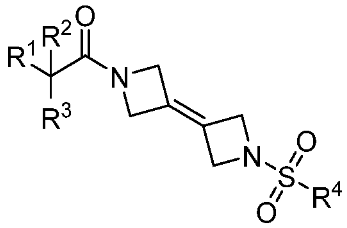

( I ),

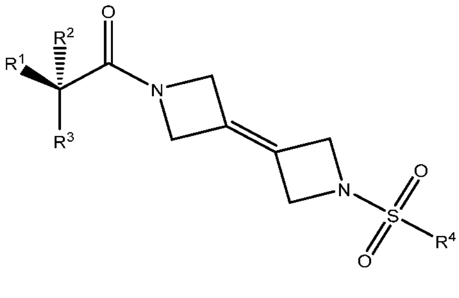

( I -1),

or

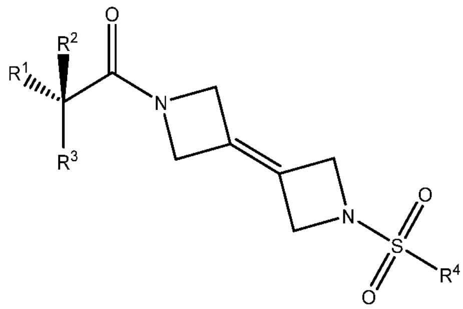

( I -2),

wherein

$R^4$ is

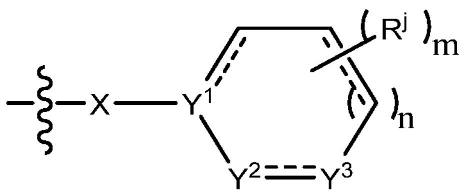

;

X is a chemical bond, $-(CR^hR^i)_t-$, $-NR^c(CR^hR^i)_t-$ or -O-;

------ represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H, or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: -H, =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, 3-14 membered heterocyclyl containing 1-4 heteroatoms independently selected from O, N and S,

$C_6$-$C_{14}$ aryl, or 5-14 membered heteroaryl containing 1-4 heteroatoms independently selected from O, N and S;
m is an integer selected from 0-6;
n is 0, 1 or 2,
$R^1$, $R^2$, $R^3$, $R^c$, $R^h$, $R^i$ and t are as defined in any one of claims 1, 3-9 or 12-16.

**20. The compound, or an isomer, pharmaceutically acceptable salt, or solvate thereof according to claim 19, wherein**

X is a chemical bond, $-CH_2-$, $-CH_2CH_2-$, $-NHCH_2-$, $-NHCH_2CH_2-$ or -O-;
- - - - - - represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, $-(C_1-C_6)$alkyl, $-(C_1-C_6)$alkoxy, $-(C_1-C_6)$alkyl or $-(C_1-C_6)$alkoxy substituted by any number of halogen, oxazolyl, thiazolyl and triazolyl;
m is an integer selected from 0-6;
n is 0, 1 or 2.

**21.** A compound of formula I**,** I-1 or 1-2, or an isomer, pharmaceutically acceptable salt or solvate thereof:

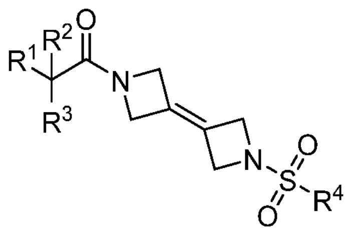 ( I ),

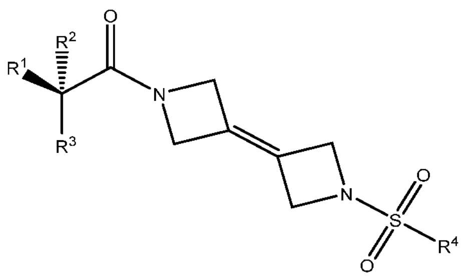 ( I -1),

or

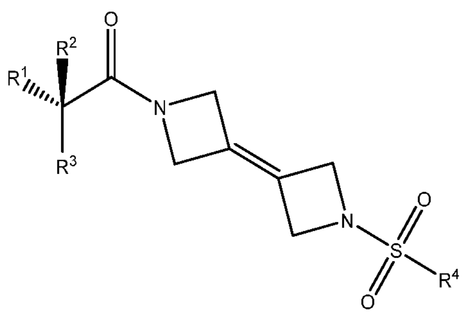 ( I -2),

wherein

$R^4$ is

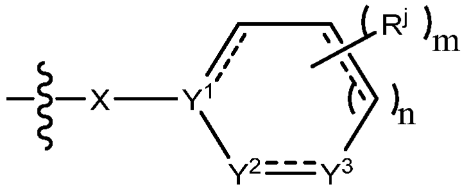

;

X is a chemical bond, $-(CR^hR^i)_t-$, $-NR^c(CR^hR^i)_t-$ or -O-;

------ represents a single bond or double bond;

$Y^1$ is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-O-(C_3-C_6)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$, -NHS(O)OH, -C(O)H, or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: -H, =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$, -NHS(O)OH, -C(O)H, -C(O)OH, 3-14 membered heterocyclyl containing 1-4 heteroatoms independently selected from O, N and S, $C_6-C_{14}$ aryl, or 5-14 membered heteroaryl containing 1-4 heteroatoms independently selected from O, N and S;

m is an integer selected from 0-6;

n is 0, 1 or 2,

$R^1$, $R^2$, $R^3$, $R^c$, $R^h$, $R^i$ and t are as defined in any one of claims 1-18.

**22. The compound, or an isomer, pharmaceutically acceptable salt, or solvate thereof according to claim 21, wherein**

X is a chemical bond, $-CH_2-$, $-CH_2CH_2-$, $-NHCH_2-$, $-NHCH_2CH_2-$ or -O-;

------ represents a single bond or double bond;

Y' is N, C or CH;

$Y^2$ and $Y^3$ are each independently N, $CH_2$ or CH, and $Y^2$ and $Y^3$ are not both N at the same time;

each $R^j$ is independently -H, halogen, -OH, $-NH_2$, -CN, $-(C_1-C_6)$alkyl, $-(C_3-C_6)$cycloalkyl, $-(C_1-C_6)$alkoxy, $-O-(C_3-C_6)$cycloalkyl, $-(C_1-C_6)$alkyl substituted by any number of halogen, $-(C_1-C_6)$alkoxy substituted by any number of halogen, $-(C_3-C_6)$cycloalkyl substituted by any number of halogen, $-O-(C_3-C_6)$cycloalkyl substituted by any number of halogen, oxazolyl, thiazolyl and triazolyl;

m is an integer selected from 0-6;

n is 0, 1 or 2.

**23. A compound of formula I, 1-1 or 1-2, or an isomer, pharmaceutically acceptable salt or solvate thereof:**

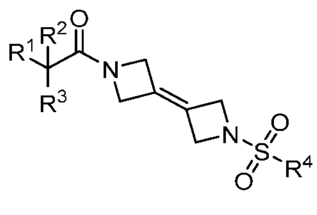

( I ),

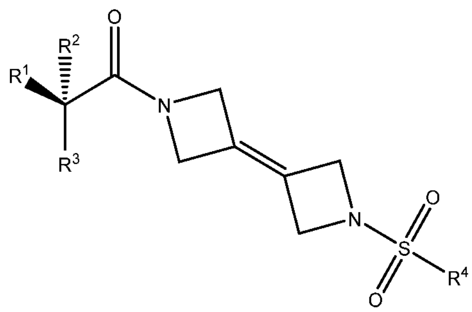

( I -1),

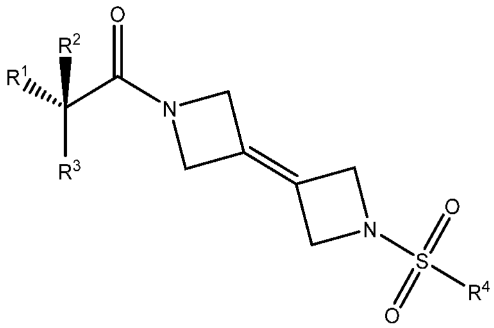

( I -2),

wherein

$R^4$ is

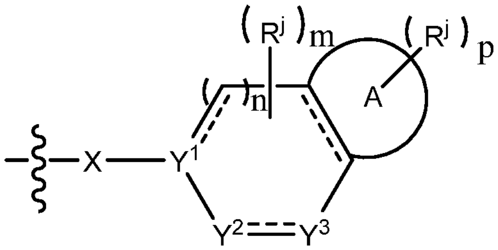

;

X is a chemical bond, $-(CR^hR^i)_t-$, $-NR^c(CR^hR^i)_t-$ or -O-;
------ represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, -C(O)H, or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, 3-14 membered heterocyclyl containing 1-4 heteroatoms independently selected from O, N and S, $C_6-C_{14}$ aryl, or 5-14 membered heteroaryl containing 1-4 heteroatoms independently selected from O, N and S;
m and p are independently integers selected from 0-6;
n is 0, 1 or 2;
ring A is $(C_3-C_8)$cycloalkyl, $(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S;

$R^1$, $R^2$, $R^3$, $R^c$, $R^h$, $R^i$ and t are as defined in any one of claims 1, 3-9 or 12-16.

**24. The compound, or an isomer, pharmaceutically acceptable salt, or solvate thereof according to claim 23, wherein**

X is a chemical bond, $-CH_2-$, $-CH_2CH_2-$, $-NHCH_2-$, $-NHCH_2CH_2-$ or -O-;
------ represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, $-(C_1-C_6)$alkyl, $-(C_1-C_6)$alkoxy, $-(C_1-C_6)$alkyl or $-(C_1-C_6)$alkoxy substituted by any number of halogen, oxazolyl, thiazolyl or triazolyl;
m and p are independently integers selected from 0-6;
n is 0, 1 or 2;
the ring A is furan, thiophene, oxazole, thiazole, triazole, piperidine, pyridine, pyran, thiopyran, morpholine, 1,4-dioxane, piperazine, pyrazine, or triazine.

**25. A compound of formula I, I-1 or 1-2, or an isomer, pharmaceutically acceptable salt or solvate thereof:**

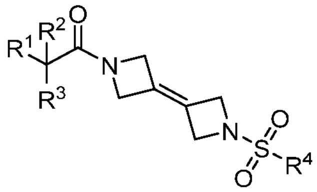

( I ),

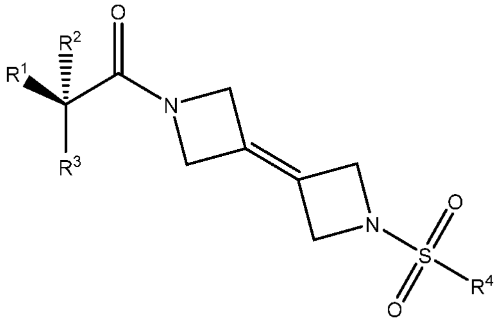

( I −1),

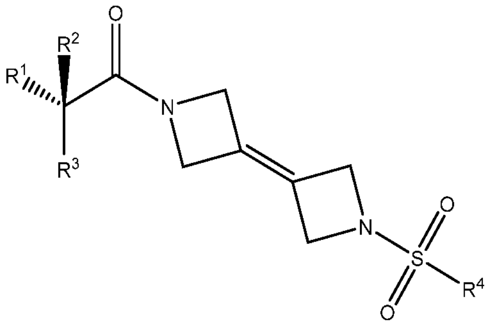

( I −2),

wherein

$R^4$ is

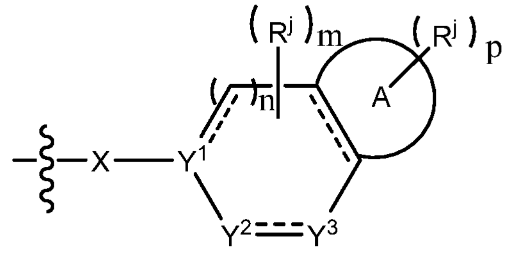 ;

X is a chemical bond, $-(CR^hR^i)_t-$, $-NR^c(CR^hR^i)_t-$ or -O-;
- - - - - - represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, =O, $-NH_2$, -CN, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$, -NHS(O)OH, -C(O)H, or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, -C(O)H, -C(O)OH, 3-14 membered heterocyclyl containing 1-4 heteroatoms independently selected from O, N and S, $C_6-C_{14}$ aryl, or 5-14 membered heteroaryl containing 1-4 heteroatoms independently selected from O, N and S;
m and p are independently integers selected from 0-6;
n is 0, 1 or 2;
ring A is $(C_3-C_8)$cycloalkyl, $(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S;
$R^1$, $R^2$, $R^3$, $R^c$, $R^h$, $R^i$ and t are as defined in any one of claims 1-18.

**26.** The compound, or an isomer, pharmaceutically acceptable salt, or solvate thereof according to claim 25, wherein

X is a chemical bond, $-CH_2-$, $-CH_2CH_2-$, $-NHCH_2-$, $-NHCH_2CH_2-$ or -O-;
- - - - - - represents a single bond or double bond;
$Y^1$ is N, C or CH;
$Y^2$ and $Y^3$ are each independently N, $CH_2$ or CH, and $Y^2$ and $Y^3$ are not both N at the same time;
each $R^j$ is independently -H, halogen, -OH, $-NH_2$, -CN, =O, $-(C_1-C_6)$alkyl, $-(C_1-C_6)$alkoxy, $-(C_1-C_6)$alkyl substituted by any number of halogen, $-(C_1-C_6)$alkoxy substituted by any number of halogen, oxazolyl, thiazolyl, or triazolyl;
m and p are independently integers selected from 0-6;
n is 0, 1 or 2;
the ring A is furan, thiophene, oxazole, thiazole, triazole, piperidine, pyridine, pyran, thiopyran, morpholine, 1,4-dioxane, piperazine, pyrazine, triazine, 4,5-dihydro-1H-imidazole, or 1,3-dioxolane.

**27.** The compound, or an isomer, pharmaceutically acceptable salt, or solvate thereof according to claim 1, wherein

$R^4$ is 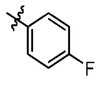, 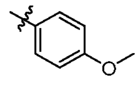, 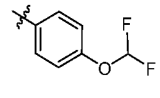, 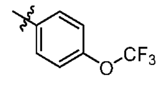, 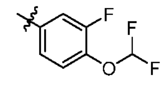, 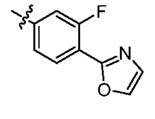, 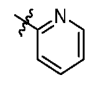,

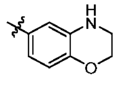, 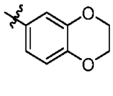, 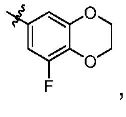, 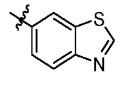, 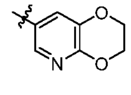, 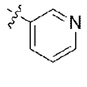, 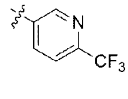, 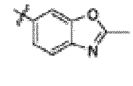, 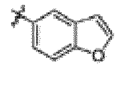,

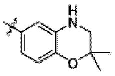, 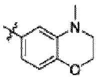, 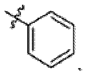, or 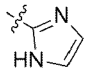.

**28.** The compound, or an isomer, pharmaceutically acceptable salt, or solvate thereof according to claim 1, wherein $R^4$ is

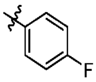, 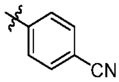, 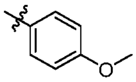, 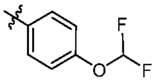, 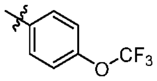, 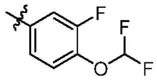, 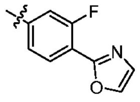,

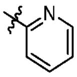, 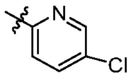, 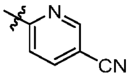, 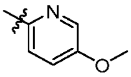, 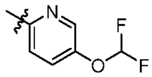, 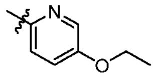, 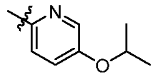,

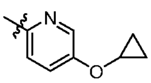, 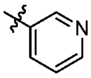, 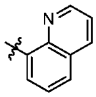, 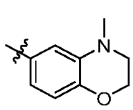, 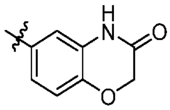, 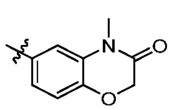, 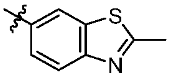,

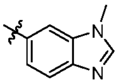, 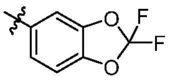, 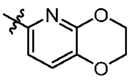, 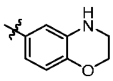, 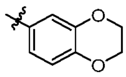, 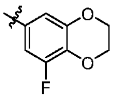, 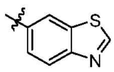, 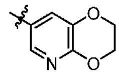,

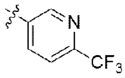, 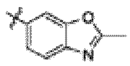, 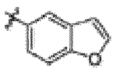, 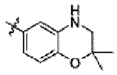, 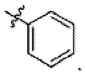, or 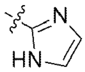.

**29.** The compound, or an isomer, pharmaceutically acceptable salt, or solvate thereof according to claim 1, wherein the compound is selected from:

| | |
|---|---|
| 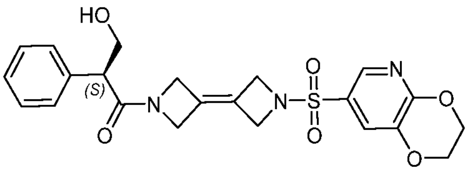 | |
| | |

| | |
|---|---|
| 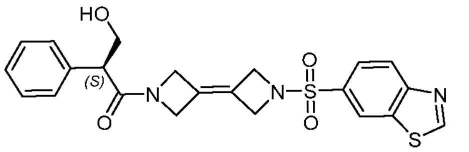 | 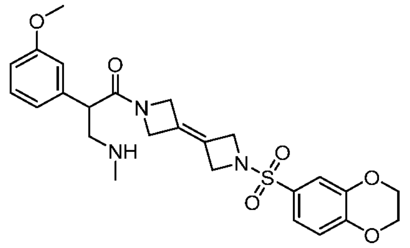 |
| 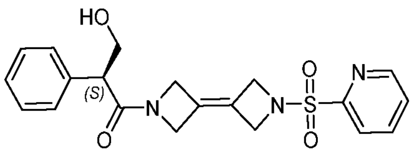 | 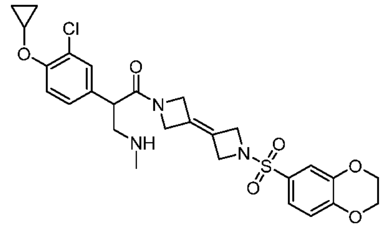 |
| 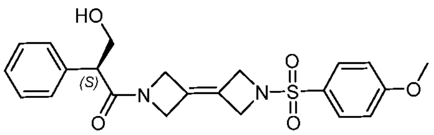 | 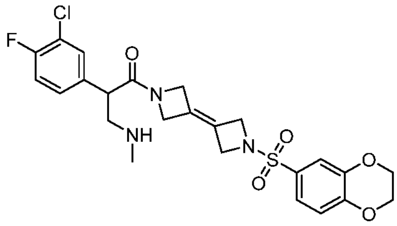 |
| 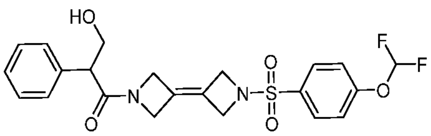 | 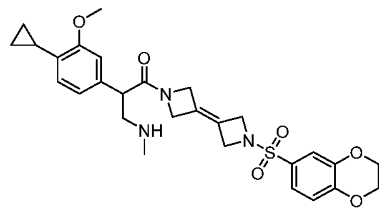 |
| 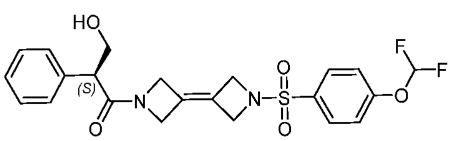 | 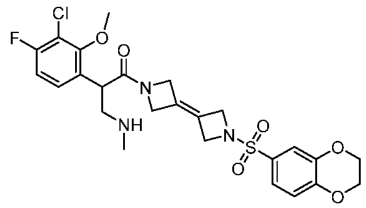 |
| 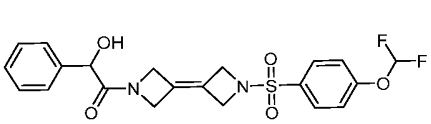 | 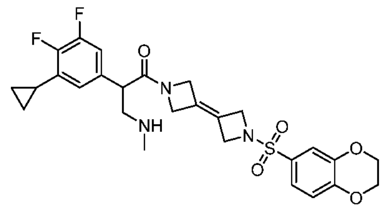 |
| 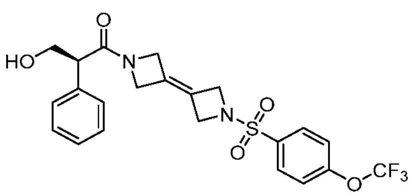 | 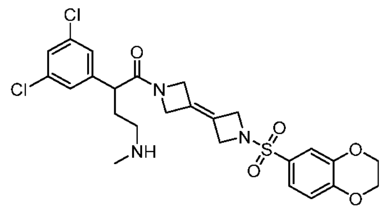 |

| | |
|---|---|
| 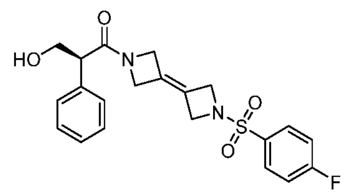 | 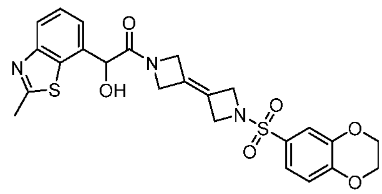 |
| 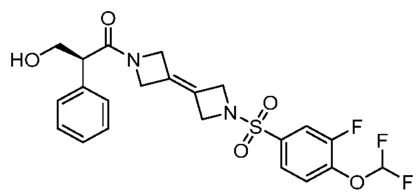 | 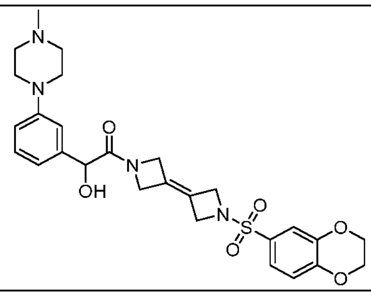 |
| 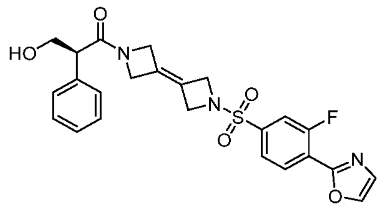 | 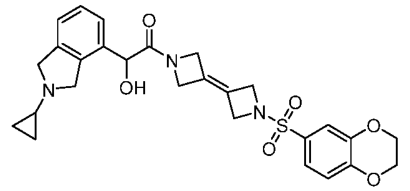 |
| 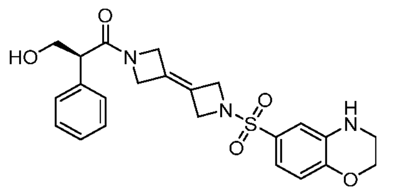 | 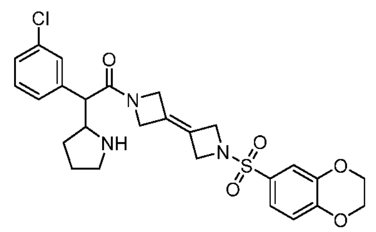 |
| 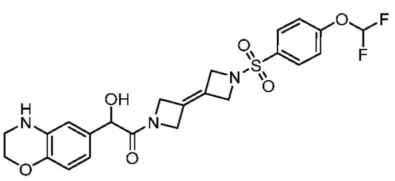 | 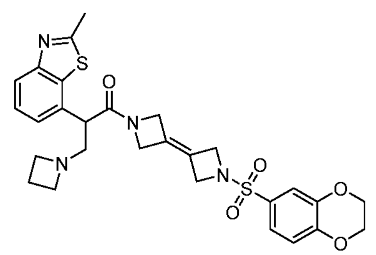 |
| 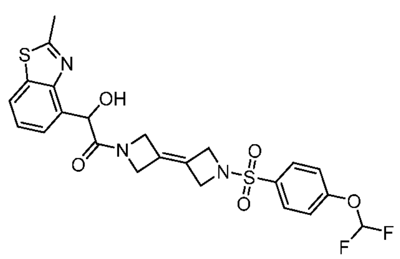 | 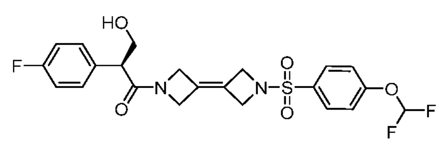 |
| 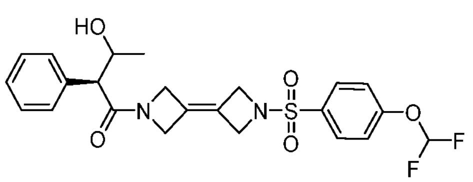 | 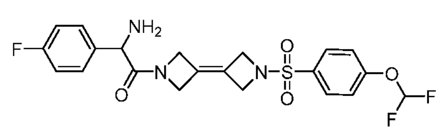 |

| | |
|---|---|
| 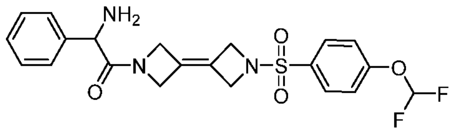 | 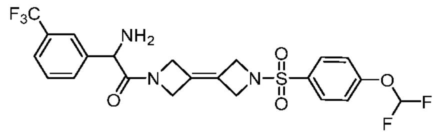 |
| 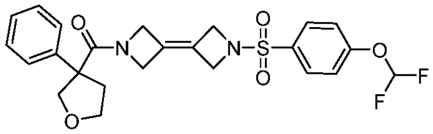 | 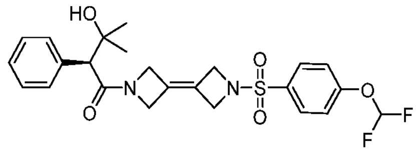 |

**30.** The compound, or an isomer, pharmaceutically acceptable salt, or solvate thereof according to claim 1, wherein the compound is selected from:

| | |
|---|---|
| 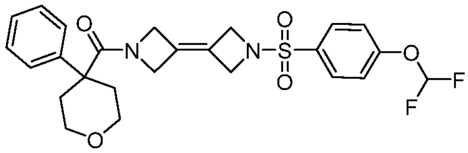 | 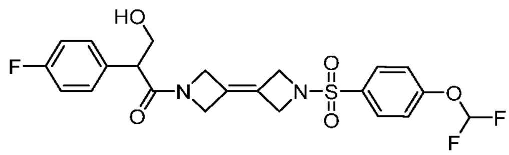 |
| 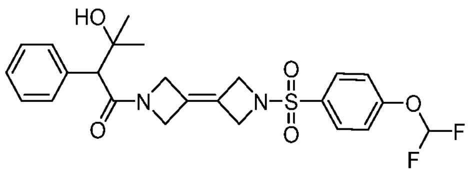 | 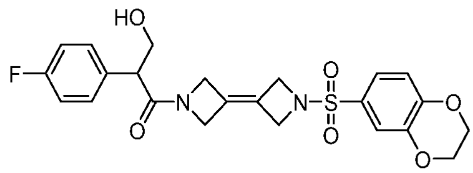 |
| 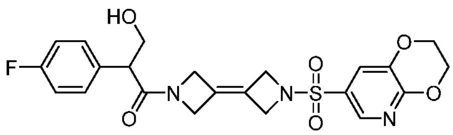 | 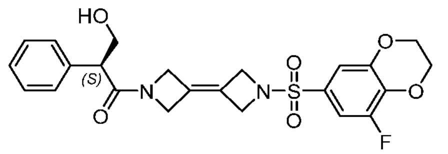 |

**31.** The compound, or an isomer, pharmaceutically acceptable salt, or solvate thereof according to claim 1 wherein the compound is selected from:

| | | |
|---|---|---|
| 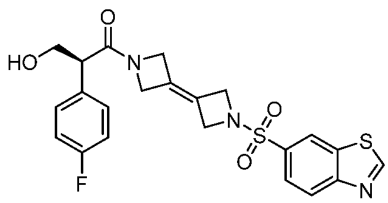 | 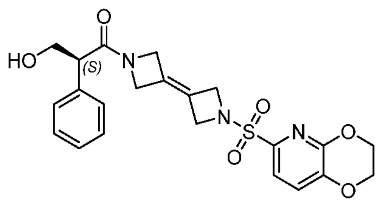 | 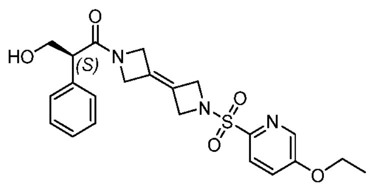 |
| 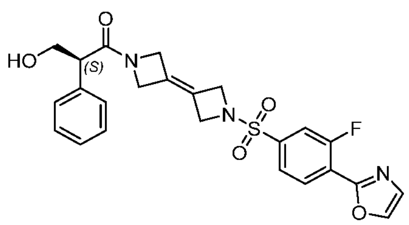 | 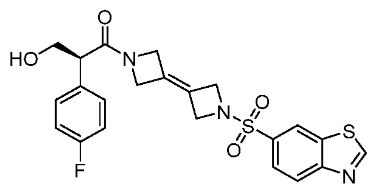 | 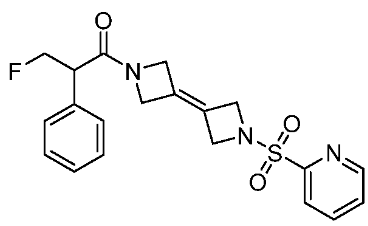 |

| | | |
|---|---|---|
| 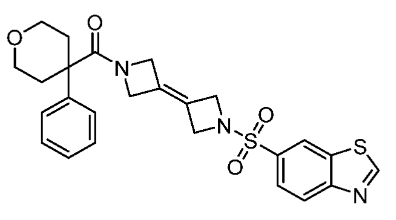 | 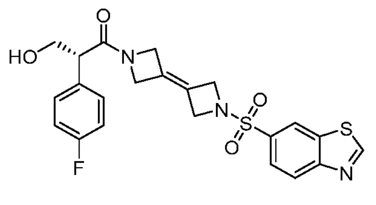 | 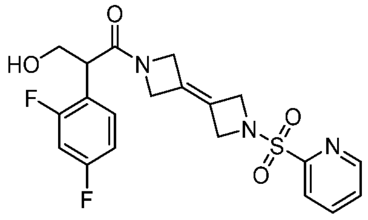 |
| 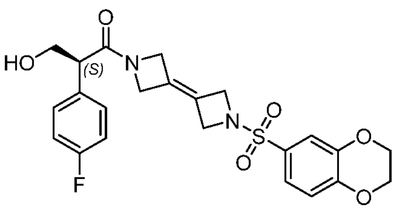 | 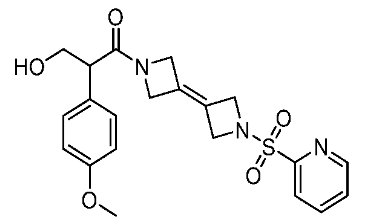 | 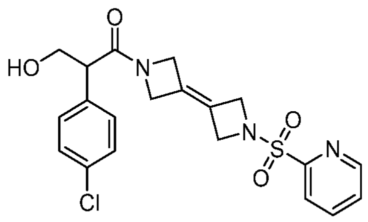 |
| 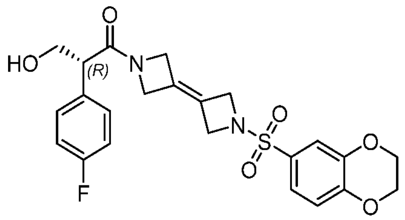 | 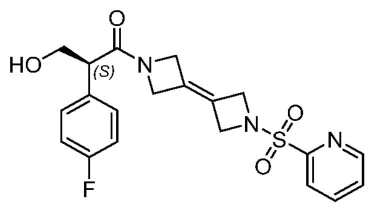 | 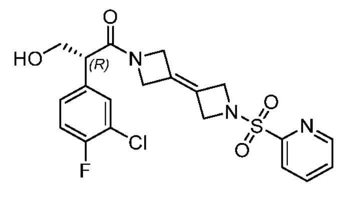 |
| 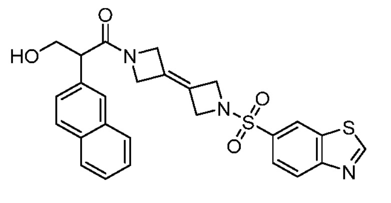 | 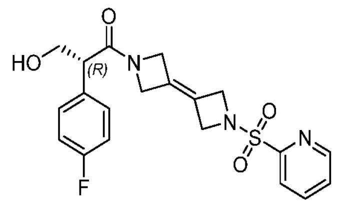 | 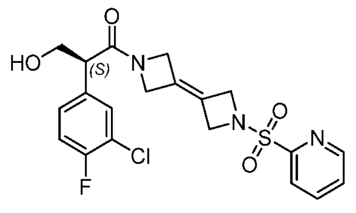 |
| 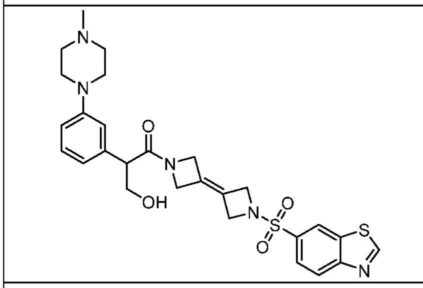 | 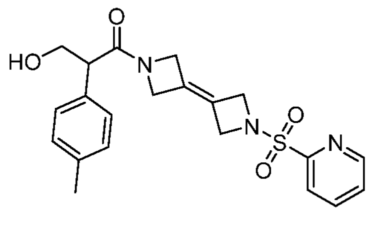 | 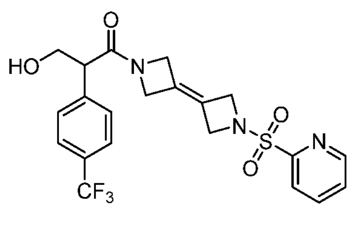 |
| 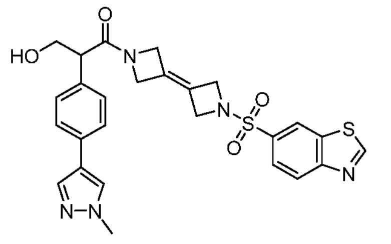 | 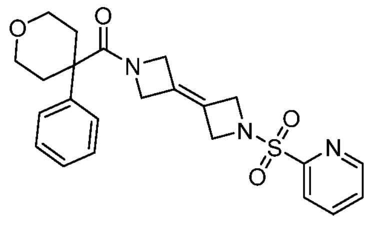 | 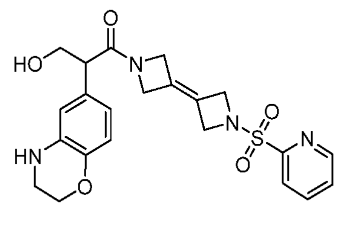 |
| 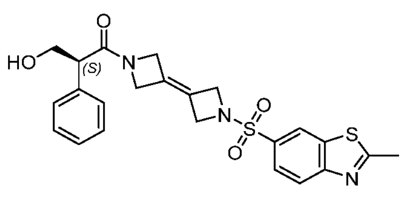 | 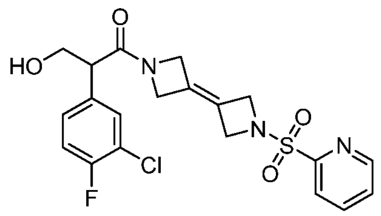 | 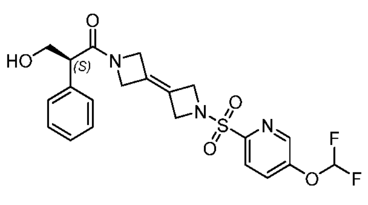 |

| | | |
|---|---|---|
| 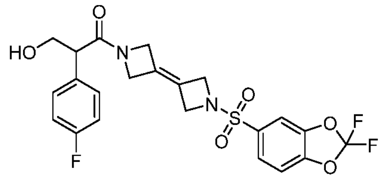 | 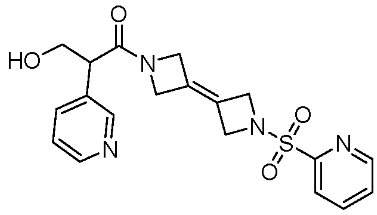 | 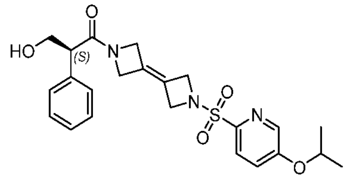 |
| 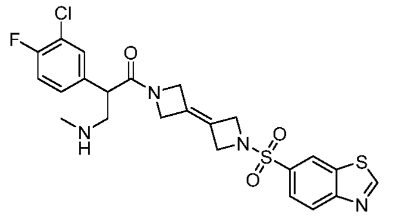 | 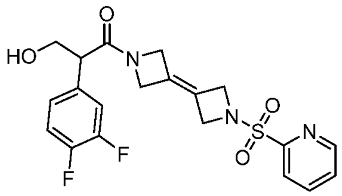 | 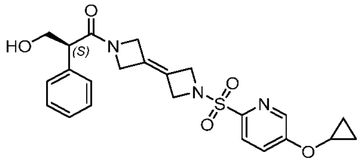 |
| 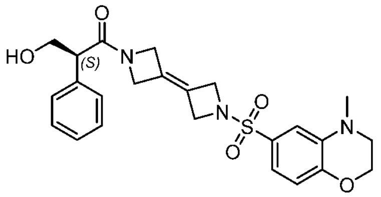 | 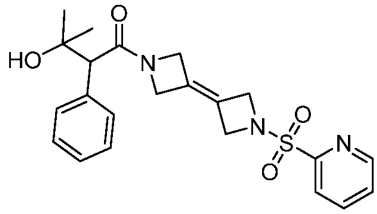 | 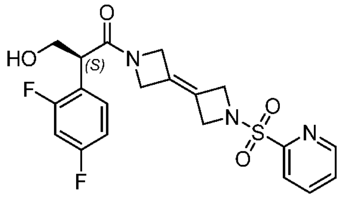 |
| 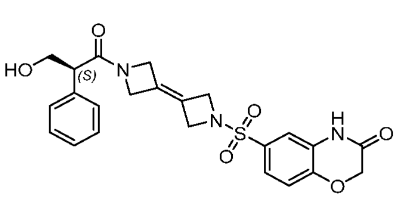 | 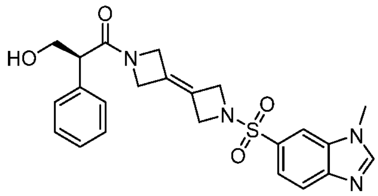 | 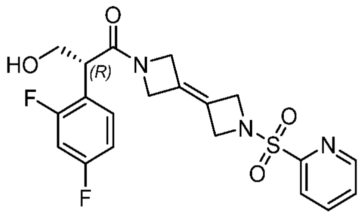 |
| 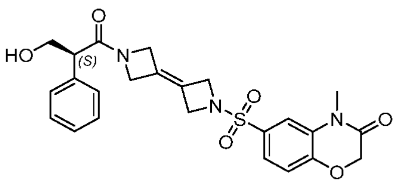 | 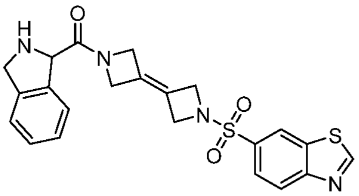 | 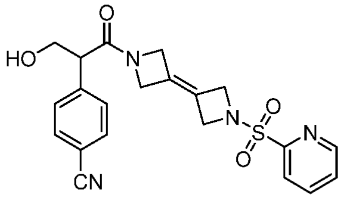 |
| 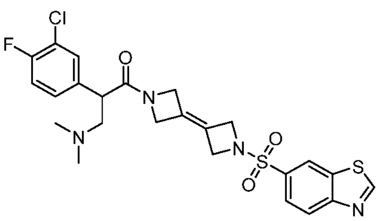 | 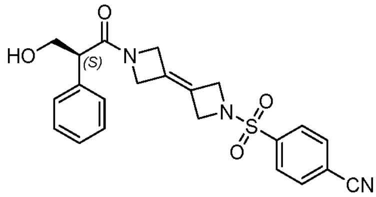 | 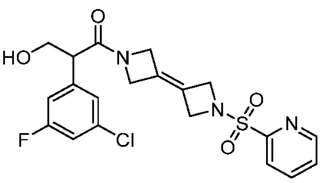 |
| 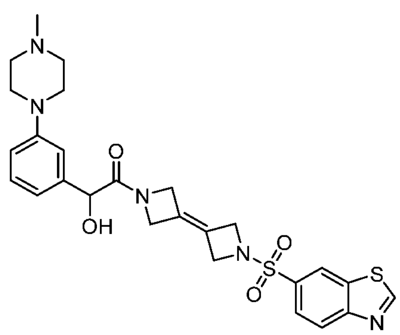 | 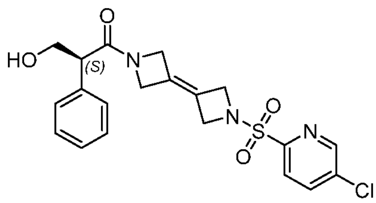 | 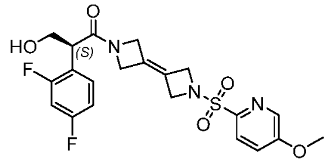 |

| | | |
|---|---|---|
| 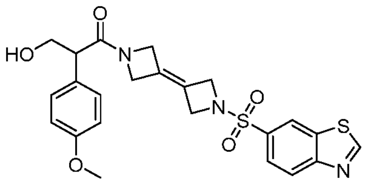 | 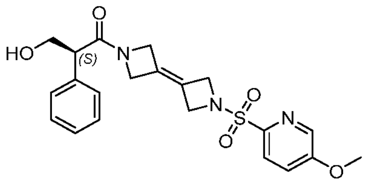 | 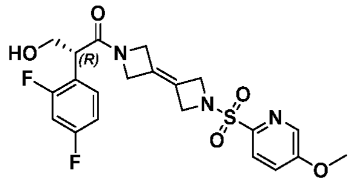 |
| 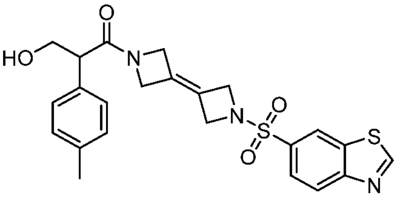 | 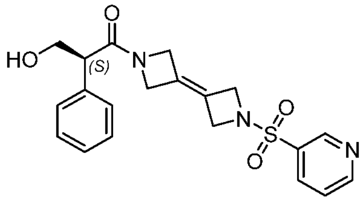 | 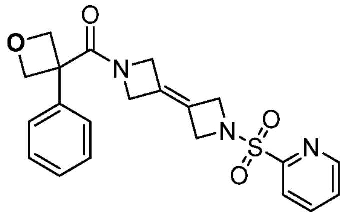 |
| 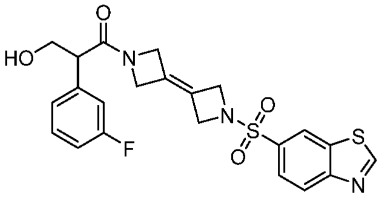 | 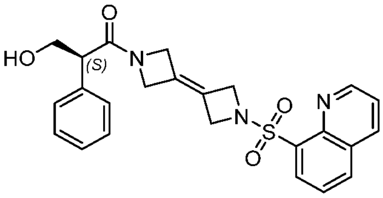 | 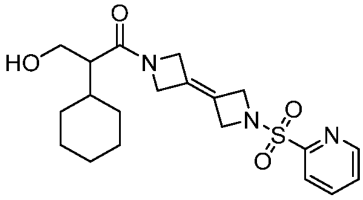 |
| 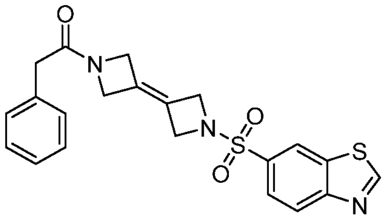 | 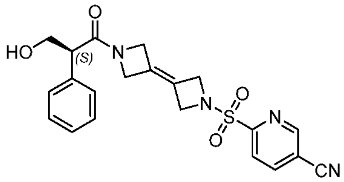 | |
| 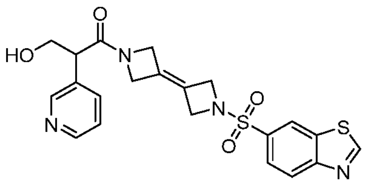 | 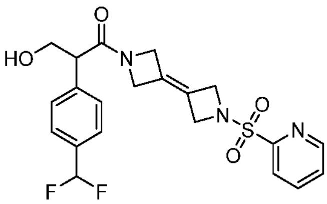 | |
| 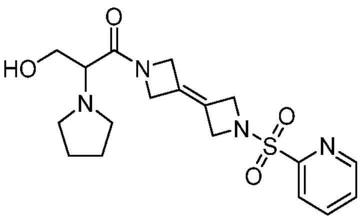 | 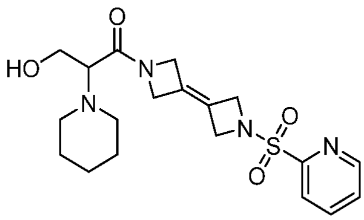 | 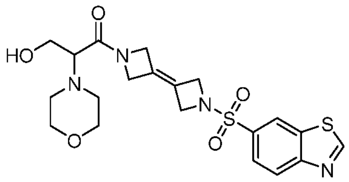 |
| 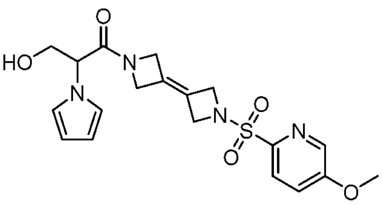 | 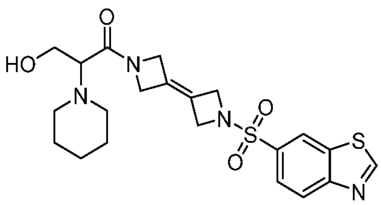 | 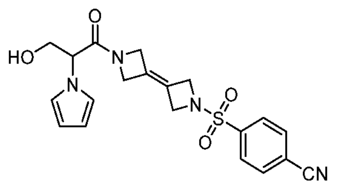 |

| | | |
|---|---|---|
| 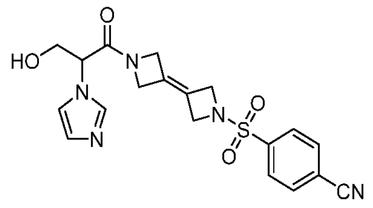 | 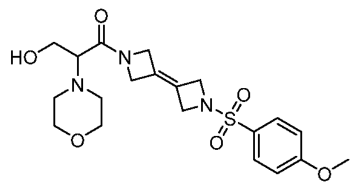 | 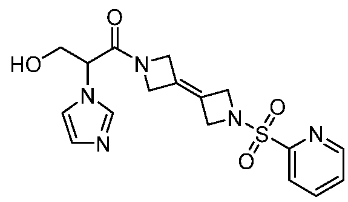 |

**32.** A compound of formula II , or an isomer, pharmaceutically acceptable salt or solvate thereof:

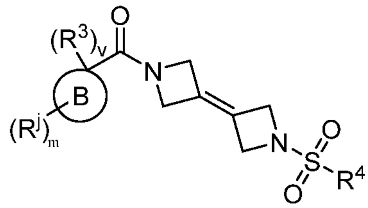 ( II ),

wherein

$R^3$ and $R^4$ are as defined in any one of claims 1-28;
each $R^j$ is independently -H, halogen, -OH, $-NH_2$, -CN, =O, $-NO_2$, $-(C_1-C_6)$alkyl, $-(C_1-C_6)$alkoxy, $-(C_2-C_6)$alkenyl, $-(C_2-C_6)$alkynyl, $-(C_3-C_8)$cycloalkyl, $-(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, 7-14 membered fused ring group containing 0-4 heteroatoms independently selected from O, N and S, -SH, $-S(O)_2H$, $-S(O)_2NH_2$, -S(O)H, $-S(O)NH_2$, $-NHS(O)_2H$, -NHS(O)H, $-S(O)_2OH$, -S(O)OH, $-NHS(O)_2OH$, -NHS(O)OH, -C(O)H, or -C(O)OH, wherein each of the alkyl, alkoxy, alkenyl, alkynyl, cycloalkyl, cycloalkenyl, heterocyclyl, aryl, heteroaryl or fused ring group is optionally substituted by one or more substituents selected from: -H, =O, halogen, -CN, -OH, -SH, $-NO_2$, $-NH_2$, $-S(O)_2H$, $-S(O)_2NH_2$, -S(O)H, $-S(O)NH_2$, $-NHS(O)_2H$, -NHS(O)H, $-S(O)_2OH$, $-S(O)_2OH$, $-NHS(O)_2OH$, -NHS(O)OH, -C(O)H, -C(O)OH, 3-14 membered heterocyclyl containing 1-4 heteroatoms independently selected from O, N and S, $C_6-C_{14}$ aryl, or 5-14 membered heteroaryl containing 1-4 heteroatoms independently selected from O, N and S;
m is independently an integer selected from 0-6;
v is 0 or 1;
ring B is $(C_3-C_8)$cycloalkyl, $(C_4-C_8)$cycloalkenyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered fused ring group containing 0-4 heteroatoms independently selected from O, N and S.

**33. The** compound, or an isomer, pharmaceutically acceptable salt, or solvate thereof according to claim 32, **wherein**

each $R^j$ is independently -H, halogen, -OH, $-NH_2$, -CN, =O, $-(C_1-C_6)$alkyl, $-(C_1-C_6)$alkoxy, $-(C_1-C_6)$alkyl substituted by any number of halogen, $-(C_1-C_6)$alkoxy substituted by any number of halogen, oxazolyl, thiazolyl, or triazolyl;
the ring B is $(C_3-C_8)$cycloalkyl, 3-14 membered heterocyclyl containing 1-4 heteroatoms independently selected from O, N and S, $C_6-C_{14}$ aryl, 5-14 membered heteroaryl containing 1-4 heteroatoms independently selected from O, N and S, or 7-14 membered bicyclic or tricyclic fused ring group containing 0-4 heteroatoms independently selected from O, N and S.

**34. The** compound, or an isomer, pharmaceutically acceptable salt, or solvate thereof according to claim 33, **wherein** the ring B is 3-7 membered oxacycloalkyl or 8-10 membered azabicyclic fused heteroaryl.

**35. The** compound, or an isomer, pharmaceutically acceptable salt, or solvate thereof according to claim 34, **wherein** the ring B is oxacycloheptane, oxacyclohexane, tetrahydrofuran, oxetane, oxacyclopropane, 1H-indole or isoindoline.

**36. The** compound, or an isomer, pharmaceutically acceptable salt, or solvate thereof according to claim 35, **wherein**

the ring B is

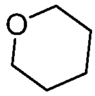, 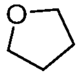, 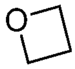, 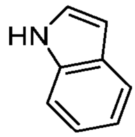 or 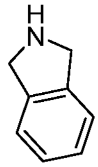.

**37.** The compound, or an isomer, pharmaceutically acceptable salt, or solvate thereof according to claim 32, wherein the compound is selected from:

| | |
|---|---|
| 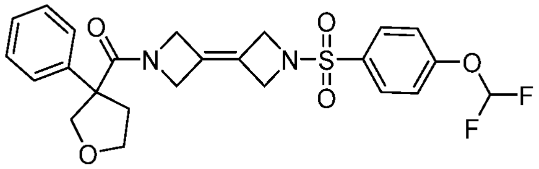 | 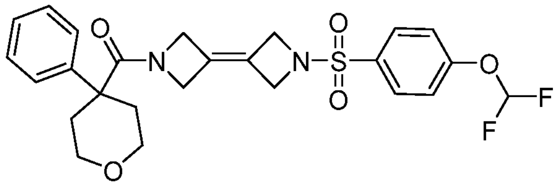 |
| 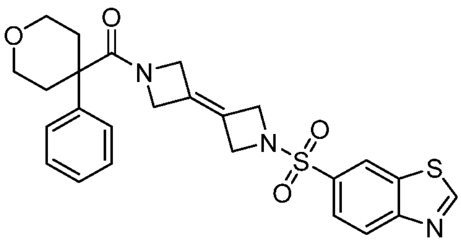 | 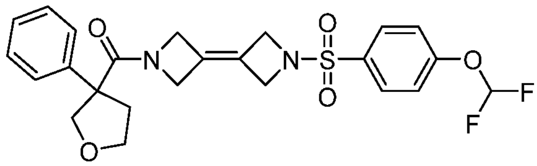 |
| 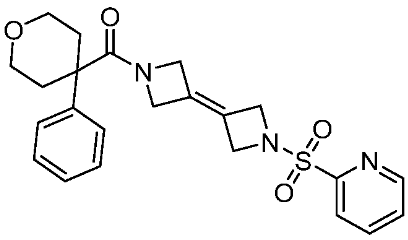 | 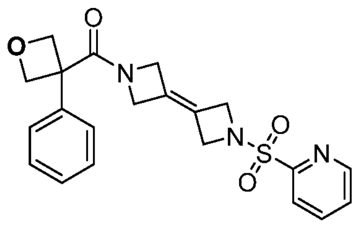 |

| | |
|---|---|
| 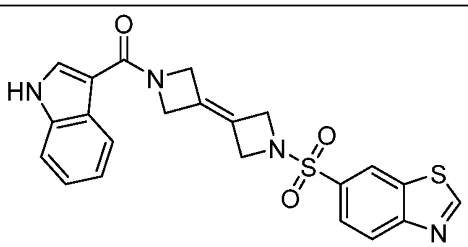 | 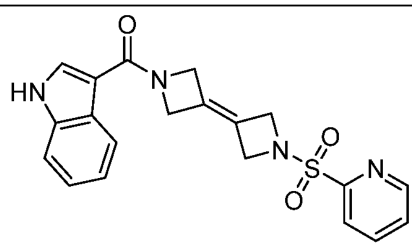 |
| 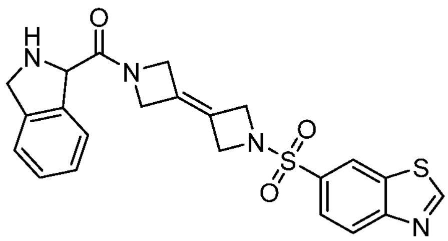 | |

**38.** A deuterated compound of formula I or II , or an isomer, pharmaceutically acceptable salt or solvate thereof:

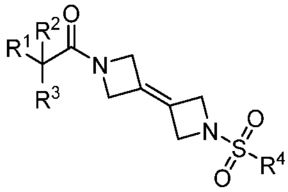

( I ),

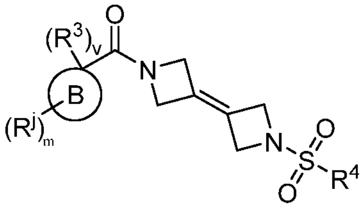

( II ),

wherein

$R^1$, $R^2$, $R^3$ and $R^4$ in the compound of formula I are as defined in any one of claims 1-28;
$R^3$, $R^j$, m, v, $R^4$ and ring B in the compound of formula II are as defined in any one of claims 32-36;
any one or more H in the compound of formula I or II are substituted by D.

**39. The** compound, or an isomer, pharmaceutically acceptable salt, or solvate thereof according to claim 38, wherein in the compound of formula I or II, any one or more H in the structure

“ 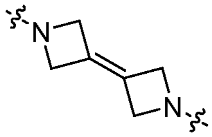 ”

are substituted by D, or any one or more H in $R^4$ are substituted by D.

**40.** The compound, or an isomer, pharmaceutically acceptable salt, or solvate thereof according to claim 39, wherein in the compound of formula I or II, all H in the structure

“ 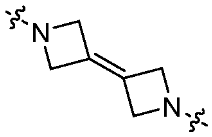 ”

are substituted by D.

**41.** The compound, or an isomer, pharmaceutically acceptable salt, or solvate thereof according to claim 38, wherein $R^1$, $R^2$ or $R^3$ is independently and optionally substituted by D when being selected as H.

**42.** The compound, or an isomer, pharmaceutically acceptable salt, or solvate thereof according to claim 38, wherein the compound is selected from:

| | |
|---|---|
| 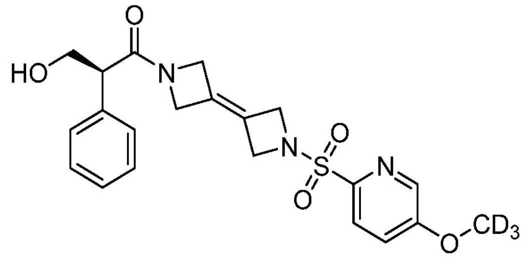 | 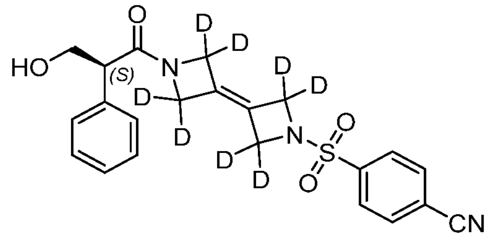 |
| 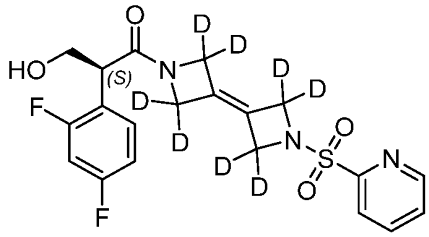 | 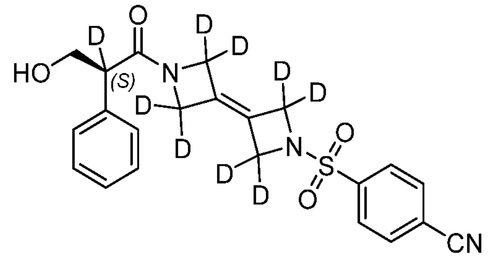 |

**43.** Use of the compound according to any one of claims 1-42 as a PKR agonist or USP9X inhibitor.

**44.** Use of the compound according to any one of claims 1-42 in the preparation of a medicament for treating a PKR or USP9X mediated disease.

**45.** The use according to claim 44, wherein the disease is pyruvate kinase deficiency, hemoglobinopathy, cancer, or tumor.

**46.** The use according to claim 44, wherein the disease comprises sickle cell anemia, β-thalassemia, hereditary non-spherocytic hemolytic anemia, hemolytic anemia, hereditary spherocytosis, hereditary elliptocytosis, abetalipoproteinemia, paroxysmal nocturnal hemoglobinuria, acquired hemolytic anemia, congenital anemia, anemia of chronic disease, colorectal cancer, renal cancer, pancreatic cancer, breast cancer, lung cancer, esophageal cancer, melanoma, lymphoma, glioblastoma, or multiple myeloma.

**47.** An intermediate compound represented by the following formula III:

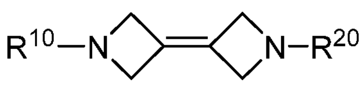 (III),

wherein, $R^{10}$ is -H or an amino-protecting group, $R^{20}$ is -H, an amino-protecting group or

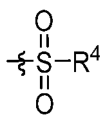 ,

and $R^4$ is as defined in any one of claims 1-28, 32-36 or 38-41 ; or, $R^{10}$ is -H, an amino-protecting group,

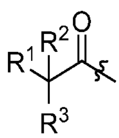 , 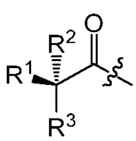 , 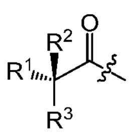 or 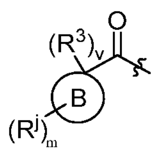 ,

wherein $R^1$, $R^2$ and $R^3$ are as defined in any one of claims 1-18 or 38-41 , m, v, $R^j$ and ring B are as defined in any one of claims 32-36, $R^{20}$ is -H or an amino-protecting group.

**48.** The compound according to claim 47, wherein each amino-protecting group is independently selected from -Cbz, -Boc, -Fmoc, -PMB, -Bn, -Trt, -Tos, -Pht, or -Alloc.

**49.** The compound according to claim 47, wherein any one or more H in the compound of formula III are substituted by D.

**50.** The compound according to claim 47, which is selected from:

| | |
|---|---|
| 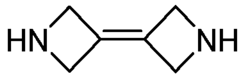 | 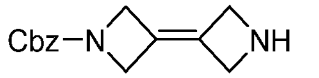 |
| 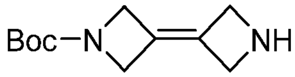 | 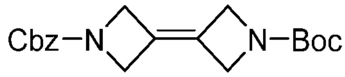 |
| 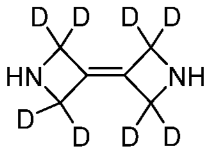 | 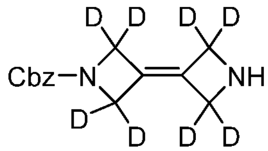 |
| 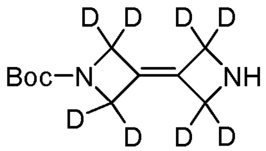 | 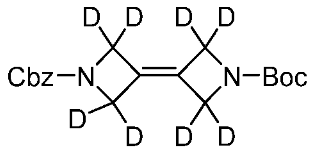 |

**51.** A pharmaceutical composition comprising the compound according to any one of claims 1-42 or an isomer, pharmaceutically acceptable salt or solvate thereof, and a pharmaceutically acceptable excipient.

**INTERNATIONAL SEARCH REPORT**

International application No.
**PCT/CN2022/140731**

**A. CLASSIFICATION OF SUBJECT MATTER**

C07D403/04(2006.01)i;C07D205/02(2006.01)i;A61K31/397(2006.01)i;A61P35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNPAT, WPI, CNKI, ISI Web of Knowledge, CAPLUS(STN), REGISTRY(STN): 赛诺哈勃药业(成都)有限公司, 成都倍特药业股份有限公司, 李建宗, 王早, 赵树春, 邵涛, 周瑞捷, 王静, 胡晓, 王叶叶, 张晓东, 唐军, 唐元清, 二联氮杂环丁亚基, 磺酰基, azetidinylidene, sulfonyl

**C. DOCUMENTS CONSIDERED TO BE RELEVANT**

| Category* | Citation of document, with indication, where appropriate, of the relevant passages | Relevant to claim No. |
|---|---|---|
| A | WO 2018175474 A1 (FORMA THERAPEUTICS, INC.) 27 September 2018 (2018-09-27) description, paragraphs [0008], [0013], [00137], [00139], [00141], [00155], [00160], [00162], [00172], [00174], [00176], [00178], [00207], [00219], [00235] | 1-51 |
| A | US 2020085798 A1 (FORMA THERAPEUTICS, INC.) 19 March 2020 (2020-03-19) description, paragraphs [0016]-[0020], [0024]-[0025] | 1-51 |
| A | WO 2020061261 A1 (FORMA THERAPEUTICS, INC.) 26 March 2020 (2020-03-26) description, paragraphs [0004], [0013], [0066], [0077], embodiments 1-94 | 1-51 |
| A | WO 2020061255 A1 (FORMA THERAPEUTICS, INC.) 26 March 2020 (2020-03-26) description, paragraphs [0009]-[0013], [00106], [00115]-[00116], [00132]-[00150], [00163], [00166] | 1-51 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 March 2023** | **14 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/140731**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **43**
because they relate to subject matter not required to be searched by this Authority, namely:

Claim 43 relates to a method for treating and/or preventing diseases. The present search report is provided on the basis of the use of a compound in the preparation of a drug for treating diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/140731**

| Patent document cited in search report | | | Publication date (day/month/year) | Patent family member(s) | | | Publication date (day/month/year) |
|---|---|---|---|---|---|---|---|
| WO | 2018175474 | A1 | 27 September 2018 | MA | 50589 | A | 15 September 2021 |
| | | | | HUE | 049979 | T2 | 30 November 2020 |
| | | | | US | 2021246143 | A1 | 12 August 2021 |
| | | | | US | 2021017184 | A1 | 21 January 2021 |
| | | | | US | 11396513 | B2 | 26 July 2022 |
| | | | | AU | 2019206013 | A1 | 01 August 2019 |
| | | | | AU | 2019206013 | B2 | 03 December 2020 |
| | | | | KR | 20190092597 | A | 07 August 2019 |
| | | | | KR | 102296703 | B1 | 01 September 2021 |
| | | | | RU | 2019137023 | A | 24 December 2019 |
| | | | | RU | 2019137023 | A3 | 30 April 2020 |
| | | | | RU | 2736217 | C2 | 12 November 2020 |
| | | | | AU | 2018240172 | A1 | 29 November 2018 |
| | | | | AU | 2018240172 | B2 | 16 May 2019 |
| | | | | AU | 2018240172 | C1 | 24 October 2019 |
| | | | | JP | 2019206587 | A | 05 December 2019 |
| | | | | JP | 7213775 | B2 | 27 January 2023 |
| | | | | AU | 2021201244 | A1 | 11 March 2021 |
| | | | | AU | 2021201244 | B2 | 20 October 2022 |
| | | | | LT | 3483164 | T | 11 May 2020 |
| | | | | RU | 2020136402 | A | 06 May 2022 |
| | | | | PT | 3483164 | T | 14 May 2020 |
| | | | | SI | 3483164 | T1 | 31 August 2020 |
| | | | | EP | 3448859 | A1 | 06 March 2019 |
| | | | | EP | 3448859 | B1 | 10 July 2019 |
| | | | | SG | 11201908670 | SA | 30 October 2019 |
| | | | | CY | 1122393 | T1 | 27 January 2021 |
| | | | | TW | 201837041 | A | 16 October 2018 |
| | | | | TWI | 772389 | B | 01 August 2022 |
| | | | | ES | 2747768 | T3 | 11 March 2020 |
| | | | | RS | 59306 | B1 | 31 October 2019 |
| | | | | MX | 2018014032 | A | 21 August 2019 |
| | | | | PL | 3483164 | T3 | 24 August 2020 |
| | | | | DK | 3483164 | T3 | 23 March 2020 |
| | | | | AR | 115993 | A2 | 17 March 2021 |
| | | | | JP | 2019521090 | A | 25 July 2019 |
| | | | | JP | 6594570 | B2 | 23 October 2019 |
| | | | | US | 2019218221 | A1 | 18 July 2019 |
| | | | | US | 10472371 | B2 | 12 November 2019 |
| | | | | AU | 2022241506 | A1 | 27 October 2022 |
| | | | | CA | 3024181 | A1 | 27 September 2018 |
| | | | | HUE | 045261 | T2 | 30 December 2019 |
| | | | | RS | 60209 | B1 | 30 June 2020 |
| | | | | SI | 3448859 | T1 | 28 February 2020 |
| | | | | IL | 289175 | A | 01 February 2022 |
| | | | | NZ | 748072 | A | 26 June 2020 |
| | | | | EA | 201992215 | A1 | 06 February 2020 |
| | | | | IL | 269447 | A | 28 November 2019 |
| | | | | IL | 269447 | B | 01 January 2022 |
| | | | | TN | 2020000045 | A1 | 04 October 2021 |

Form PCT/ISA/210 (patent family annex) (July 2022)

**INTERNATIONAL SEARCH REPORT**
**Information on patent family members**

International application No.
**PCT/CN2022/140731**

| Patent document cited in search report | Publication date (day/month/year) | Patent family member(s) | Publication date (day/month/year) |
|---|---|---|---|
| | | LT 3448859 T | 25 October 2019 |
| | | EP 3680241 A1 | 15 July 2020 |
| | | PT 3448859 T | 25 October 2019 |
| | | TN 2019000290 A1 | 07 May 2021 |
| | | MD 3483164 T2 | 31 July 2020 |
| | | SA 519410164 B1 | 10 March 2022 |
| | | BR 112018075312 A2 | 01 October 2019 |
| | | US 2020031839 A1 | 30 January 2020 |
| | | US 10836771 B2 | 17 November 2020 |
| | | KR 20180135494 A | 20 December 2018 |
| | | KR 102007135 B1 | 02 August 2019 |
| | | US 10208052 B1 | 19 February 2019 |
| | | RU 2707751 C1 | 29 November 2019 |
| | | HRP 20191665 T1 | 07 February 2020 |
| | | EP 3483164 A1 | 15 May 2019 |
| | | EP 3483164 B1 | 04 March 2020 |
| | | US 2020069643 A1 | 05 March 2020 |
| | | US 11014927 B2 | 25 May 2021 |
| | | ES 2788856 T3 | 23 October 2020 |
| | | MD 3448859 T2 | 31 March 2020 |
| | | AR 111295 A1 | 26 June 2019 |
| | | ZA 201906278 B | 27 January 2021 |
| | | MA 44795 A | 06 March 2019 |
| | | MA 44795 B1 | 30 September 2019 |
| | | PL 3448859 T3 | 28 February 2020 |
| | | MA 45614 B1 | 30 April 2020 |
| | | HRP 20200752 T1 | 24 July 2020 |
| | | DK 3448859 T3 | 23 September 2019 |
| US 2020085798 A1 | 19 March 2020 | US 2020129485 A1 | 30 April 2020 |
| | | US 2022031671 A1 | 03 February 2022 |
| | | EP 3853206 A1 | 28 July 2021 |
| | | EP 3853206 A4 | 20 July 2022 |
| | | WO 2020061378 A1 | 26 March 2020 |
| | | US 2020253939 A1 | 13 August 2020 |
| | | US 11071725 B2 | 27 July 2021 |
| | | MA 53668 A | 15 September 2021 |
| | | US 10675274 B2 | 09 June 2020 |
| | | US 2020405699 A1 | 31 December 2020 |
| | | BR 112021005188 A2 | 08 June 2021 |
| | | US 2021308108 A1 | 07 October 2021 |
| WO 2020061261 A1 | 26 March 2020 | IL 281483 A | 29 April 2021 |
| | | CA 3113423 A1 | 26 March 2020 |
| | | SG 11202102526 QA | 29 April 2021 |
| | | BR 112021004599 A2 | 25 May 2021 |
| | | JP 2022501362 A | 06 January 2022 |
| | | AU 2019345053 A1 | 06 May 2021 |
| | | EP 3852790 A1 | 28 July 2021 |
| | | EP 3852790 A4 | 10 August 2022 |
| | | KR 20210061400 A | 27 May 2021 |
| WO 2020061255 A1 | 26 March 2020 | US 2020087309 A1 | 19 March 2020 |

Form PCT/ISA/210 (patent family annex) (July 2022)

**INTERNATIONAL SEARCH REPORT**
**Information on patent family members**

International application No.
**PCT/CN2022/140731**

| Patent document cited in search report | Publication date (day/month/year) | Patent family member(s) | | | Publication date (day/month/year) |
|---|---|---|---|---|---|
| | | US | 11001588 | B2 | 11 May 2021 |
| | | JP | 2022501354 | A | 06 January 2022 |
| | | EP | 3852791 | A1 | 28 July 2021 |
| | | EP | 3852791 | A4 | 15 June 2022 |
| | | US | 2021221816 | A1 | 22 July 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018175474 A
- WO 2020061255 A
- WO 2021202796 A
- WO 2012083246 A
- WO 2011002817 A
- WO 2012092442 A
- WO 2014139325 A
- WO 2020061261 A


### Non-patent literature cited in the description


- *Nat Genet,* 2018, vol. 50, 478-480
- *Int J Mol Sci,* 2021, vol. 22, 7229
- *N Engl J Med,* 2014, vol. 371, 1908-1916
- *Sci Rep,* 2017, vol. 7, 920
- *Lancet,* 2010, vol. 376, 2018-2031
- *J Neonatal Screen,* 2018, vol. 4, 31
- *Engl J Med,* 2016, vol. 375, 435-442
- *Int J Mol Sci,* 2021, vol. 22, 7229
- *N Engl J Med,* 2018, vol. 378, 1479-1493
- *N Engl J Med,* 2021, vol. 384, 252-260
- *J Cell Mol Med,* 2020, vol. 24, 6162-6177
- *J Pain Res,* 2021, vol. 14, 849-856
- *Blood,* 2018, vol. 131, 263-265
- *Br J Haematol,* 2021, vol. 194, 474-477
- *J Clin Invest,* 2020, vol. 130, 491-506
- *Haematologica,* 2020, vol. 105, 623-631
- *N Engl J Med,* 2019, vol. 381, 933-944
- *Biomark Res,* 2021, vol. 9, 66
- *Cancer Med,* 2019, vol. 8, 6730-6740
- *Biochim Biophys Acta Rev Cancer,* 2019, vol. 1872, 188312
- *J Clin Invest,* 2018, vol. 128, 1326-1337
- *Nat Commun,* 2017, vol. 8, 14866
- *Blood,* 2015, vol. 125, 3588-3597
- *Cancer Lett,* 2018, vol. 436, 129-138
- *BiomarkRes,* 2021, vol. 9, 66
- *Cancers (Basel),* 2019, vol. 11, 344
- *Diagn Pathol,* 2013, vol. 8, 177
- *Nature,* 2012, vol. 486, 266-270
- *Am J Physiol Cell Physiol,* 2019, vol. 317, C534-C543
- *Nat Commun,* 2021, vol. 12, 2346
- *Cancer Med,* 2018, vol. 7, 4004-4011
- *nt J Mol Sci,* 2021, vol. 22, 10800